# EUROPÄISCHE PATENTSCHRIFT

(11) **EP 1 855 529 B1**
(45) Veröffentlichungstag und Bekanntmachung des Hinweises auf die Patenterteilung: **18.12.2013**
(21) Anmeldenummer: 06701829.1
(22) Anmeldetag: 08.02.2006
(51) Int. Cl.: A01N 43/08, A01N 43/36, C07D 487/10, C07D 491/10, C07D 493/10

(54) **SPIROKETAL-SUBSTITUIERTE CYCLISCHE KETOENOLE**
SPIROKETAL-SUBSTITUTED CYCLIC KETOENOLS
CETOENOLS CYCLIQUES SUBSTITUES PAR SPIROCETAL

(30) Priorität: 22.02.2005 DE 102005008021
(43) Veröffentlichungstag der Anmeldung: 21.11.2007
(73) Patentinhaber: Bayer CropScience AG, 40789 Monheim (DE)
(72) Erfinder: BRETSCHNEIDER, Thomas, 53797 Lohmar (DE); FISCHER, Reiner, 40789 Monheim (DE); GAERTZEN, Oliver, 50668 Köln (DE); LEHR, Stefan, 65835 Liederbach (DE); DREWES, Mark, Wilhelm, 40764 Langenfeld (DE); FEUCHT, Dieter, 65760 Eschborn (DE); MALSAM, Olga, 51503 Rösrath (DE); RECKMANN, Udo, 50823 Köln (DE); ARNOLD, Christian, 40764 Langenfeld (DE); AULER, Thomas, 42799 Leichlingen (DE); HEMPEL, Waltraud, 65835 Liederbach (DE); HILLS, Martin, Jeffrey, 65510 Idstein (DE); KEHNE, Heinz, 65719 Hofheim (DE); ROSINGER, Christopher, Hugh, 65719 Hofheim (DE); SANWALD, Erich, 24159 Kiel (DE)
(86) Internationale Anmeldenummer: PCT/EP2006/001089
(87) Internationale Veröffentlichungsnummer: WO 2006/089633

(56) Entgegenhaltungen:
- WO-A-98/06721
- WO-A-99/16748
- JP-A- 2002 205 984
- ITO, MITSURU ET AL: "Synthesis and insecticidal activity of novel N-oxydihydropyrrole derivatives with a substituted spirocyclohexyl group" BIOSCIENCE, BIOTECHNOLOGY, AND BIOCHEMISTRY , 67(6), 1230-1238 CODEN: BBBIEJ; ISSN: 0916-8451, 2003, XP002392344 in der Anmeldung erwähnt
- ITO, MITSURU ET AL: "Synthesis and insecticidal activity of N-oxydihydropyrroles: 4-hydroxy-3-mesityl-5,5-dimethyl derivatives with various substituents at the 1-position" BIOSCIENCE, BIOTECHNOLOGY, AND BIOCHEMISTRY , 67(6), 1230-1238 CODEN: BBBIEJ; ISSN: 0916-8451, 2002, Seiten 2406-2414, XP002392345
- FISCHER R ET AL: "Chemistry and stereochemistry of spirodiclofen (BAJ 2740)" PFLANZENSCHUTZ NACHRICHTEN BAYER, BAYER, LEVERKUSSEN, DE, Bd. 55, Nr. 2-3, 2002, Seiten 137-148, XP002298236 ISSN: 0340-1723
- DATABASE BEILSTEIN BEILSTEIN CROSSFIRE INSTITUT ZUR FOERDERUNG DER CHEMISCHEN WISSENSCHAFTEN, DE; Citation No. 156279 BRNs 9938211373325 1369330 1974, XP002392404 & BRITTEN A ET AL: "Photoluminescence studies of molecular interactions. I. Synthesis of some indolyl ketones and tryptophan analogues." JOURNAL OF THE CHEMICAL SOCIETY. PERKIN TRANSACTIONS 1. 1974, Bd. 0, Nr. 15, 1974, Seiten 1824-1827, ISSN: 0300-922X
- SATOH T ET AL: "1 CHLOROALKYL P-TOLYL SULFOXIDES AS USEFUL AGENTS FOR HOMOLOGATION OF CARBONYL COMPOUNDS CONVERSION OF CARBONYL COMPOUNDS TO ALPHA HYDROXY ACIDS ESTERS AND AMIDES AND ALPHA ALPHA' DIHYDROXY KETONES" JOURNAL OF ORGANIC CHEMISTRY, Bd. 56, Nr. 13, 1991, Seiten 4129-4134, XP002392346 ISSN: 0022-3263
- FELICE E ET AL: "Easy Access to alpha-Amino beta-Oxo Esters from beta-Enamino Esters" TETRAHEDRON LETTERS, ELSEVIER, AMSTERDAM, NL, Bd. 40, Nr. 23, 4. Juni 1999 (1999-06-04), Seiten 4413-4416, XP004164663 ISSN: 0040-4039
- OTA H ET AL: "A novel synthesis of cyclic alpha-amino aldehydes, amino alcohols, and alpha-amino acid methyl esters from cyclic ketones through sulfinylaziridines" TETRAHEDRON LETTERS, ELSEVIER, AMSTERDAM, NL, Bd. 45, Nr. 20, 10. Mai 2004 (2004-05-10), Seiten 3903-3907, XP004503827 ISSN: 0040-4039
- AVENOZA ALBERTO ET AL: "A new efficient synthesis of 2-phenyl-4-oxo-1-aminocyclohexanecarbox ylic acids" TETRAHEDRON, Bd. 50, Nr. 45, 1994, Seiten 12989-12998, XP002392347 ISSN: 0040-4020

## Beschreibung

Die vorliegende Erfindung betrifft neue spiroketalsubstituierte cyclische Ketoenole, mehrere Verfahren zu ihrer Herstellung und ihre Verwendung als Schädlingsbekämpfungsmittel und/oder Herbizide und/oder Mikrobizide. Auch Gegenstand der Erfindung sind selektiv herbizide Mittel, die Spiroketal-substituierte cyclische Ketoenole einerseits und eine die Kulturpflanzenverträglichkeit verbessernde Verbindung andererseits enthalten.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599, EP-A-415211 und JP-A-12-053 670) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 94/01 997, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/24437, WO 99/43649, WO 99/48869 und WO 99/55673, WO 01/17972, WO 01/23354, WO 01/74770, WO 03/013249, WO 03/062244, WO 2004/007448, WO 2004/024 688, WO 04/065366, WO 04/080962, WO 04/111042, WO 05/044791, WO 05/044796, WO 05/048710, WO 05/049596, WO 05/066125, WO 05/092897 und DE-A-04 030 753. Außerdem sind ketalsubstituierte 1-H-Arylpyrrolidin-2,4-dione aus WO 99/16748 und (spiro)-ketalsubstituierte N-Alkoxy-alkoxy-substituierte Aryl-pyrrolidindione aus JP-A-14 205 984 und Ito M. et. al Bioscience, Biotechnology and Biochemistry 67, 1230-1238, (2003) bekannt.

Es ist bekannt, dass bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156, EP-A-0 647 637, WO 95/26 345, WO 96/20 196, WO 96/25 395, WO 96/35 664, WO 97/01 535, WO 97/02 243, WO 97/36 868, WO 98/05638, WO 98/25928, WO 99/16748, WO 99/43649, WO 99/48869, WO 99/55673, WO 01/17972, WO 01/23354 und WO 01/74770, WO 03/013 249, WO 2004/024 688, WO 04/080962, WO 04/111042, WO 05/092897 und DE-A-04 030 753 bekannt.

Die herbizide und/oder akarizide und/oder insektizide Wirksamkeit und/oder Wirkungsbreite und/oder die Pflanzenverträglichkeit der bekannten Verbindungen, insbesondere gegenüber Kulturpflanzen, ist jedoch nicht immer ausreichend.

Es wurden nun neue Verbindungen der Formel (I) 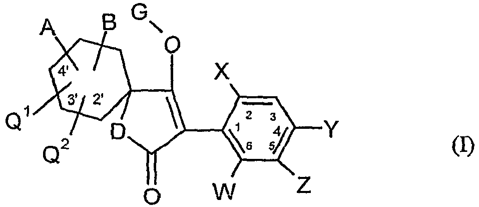 gefunden,
in welcher
- W: für Wasserstoff, Alkyl, Alkenyl, Alkinyl, Halogen, Alkoxy, Alkenyloxy, Halogenalkyl, Halogenalkoxy oder Cyano steht,
- X: für Halogen, Alkyl, Alkenyl, Alkinyl, Alkoxy, Alkenyloxy, Alkylthio, Alkylsulfinyl, Alkylsulfonyl, Halogenalkyl, Halogenalkoxy, Halogenalkenyloxy, Nitro oder Cyano steht,
- Y und Z: unabhängig voneinander für Wasserstoff, Alkyl, Alkenyl, Alkenyl, Alkoxy, Halogen, Halogenalkyl, Halogenalkoxy, Cyano, Nitro oder jeweils gegebenenfalls substituiertes Aryl oder Hetaryl steht,
mit der Maßgabe, dass mindestens einer der Reste W oder Z ungleich Wasserstoff sein muss, wenn X und Y für Methyl stehen,
- A und B: und das Kohlenstoffatom, an das sie gebunden sind, für ein jeweils gegebenenfalls durch Alkyl, Halogenalkyl, Alkoxy, Alkoxyalkyl oder gegebenenfalls substituiertes Phenyl substituiertes fünf- bis siebengliedriges Ketal, Thioketal oder Dithioketal steht, welches gegebenenfalls durch ein weiteres Heteroatom unterbrochen sein kann,
- D: für NH und Sauerstoff steht,
- Q¹, Q²: unabhängig voneinander für Wasserstoff, Alkyl, Halogenalkyl oder Alkoxy stehen,
- G: für Wasserstoff (a) oder für eine der Gruppen 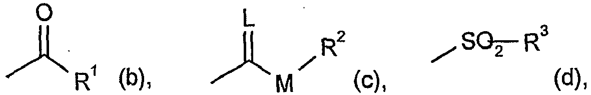 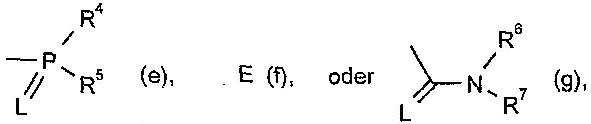 steht,
worin
E für ein Metallion oder ein Ammoniumion steht,
L für Sauerstoff oder Schwefel steht,
M für Sauerstoff oder Schwefel steht,
- R¹: für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxy-alkyl steht,
- R²: für jeweils gegebenenfalls durch Halogen oder Cyano substituiertes Alkyl, Alkenyl, Alkoxyalkyl oder Polyalkoxyalkyl oder für jeweils gegebenenfalls substituiertes Cycloalkyl, Phenyl oder Benzyl steht,
- R³, R⁴ und R⁵: unabhängig voneinander für jeweils gegebenenfalls durch Halogen substituiertes Alkyl, Alkoxy, Alkylamino, Dialkylamin, Alkylthio, Alkenylthio oder Cycloalkylthio oder für jeweils gegebenenfalls substituiertes Phenyl, Benzyl, Phenoxy oder Phenylthio stehen,
- R⁶ und R⁷: unabhängig voneinander für Wasserstoff, für jeweils gegebenenfalls durch Halogen oder Cyano substituiertes Alkyl, Cycloalkyl, Alkenyl, Alkoxy, Alkoxyalkyl, für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden und gegebenenfalls substituierten Cyclus bilden.

Die Verbindungen der Formel (I) können, auch in Abhängigkeit von der Art der Substituenten, als optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung von D für NH (1) und D für O (2) ergeben sich folgende hauptsächliche Strukturen (I-1) bis (I-2): 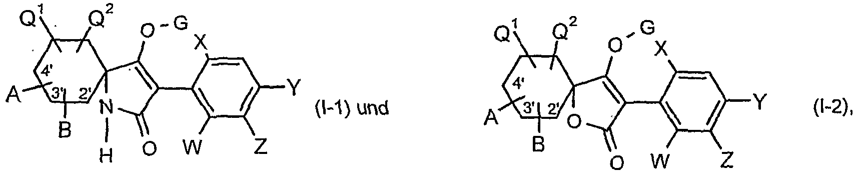 worin
A, B, G, Q¹, Q², W, X, Y und Z die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn D für NH (1) steht, 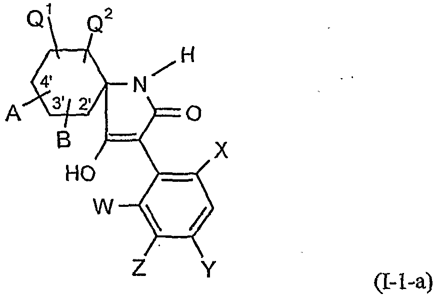 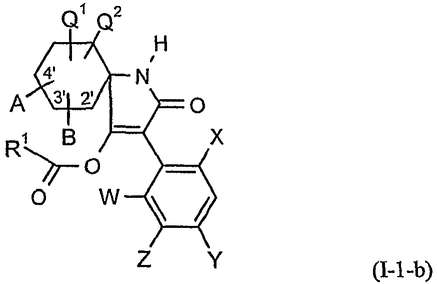 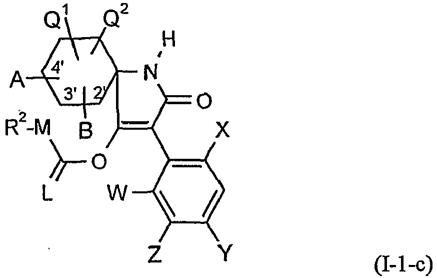 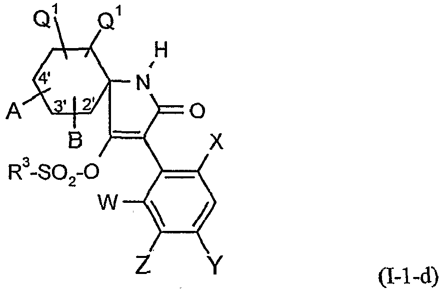 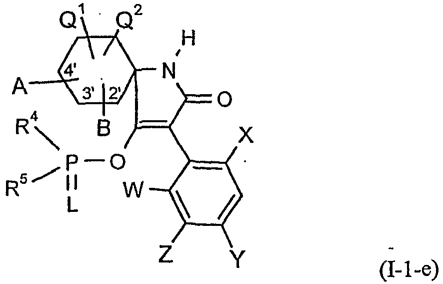 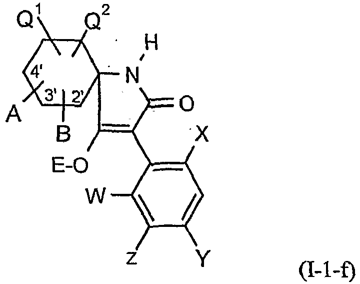 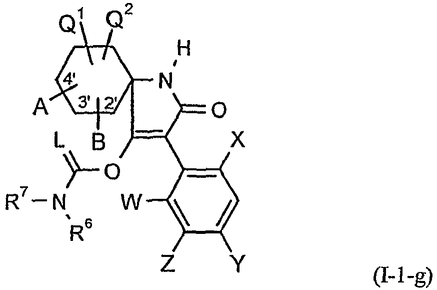 worin
A, B, E, L, M, Q¹, Q², W, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g), wenn D für O (2) steht, 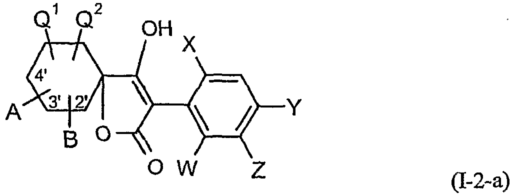 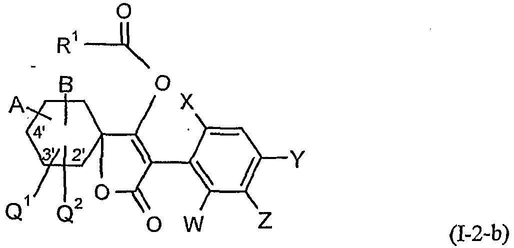 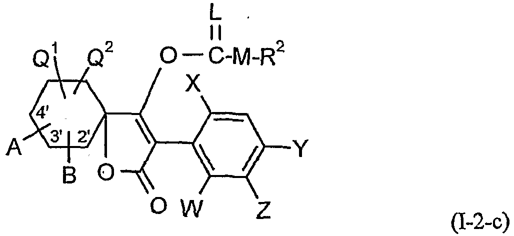 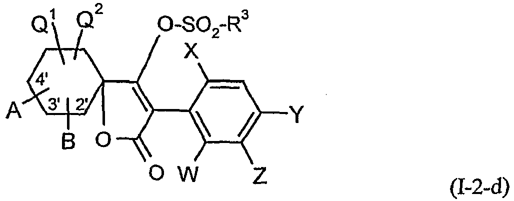 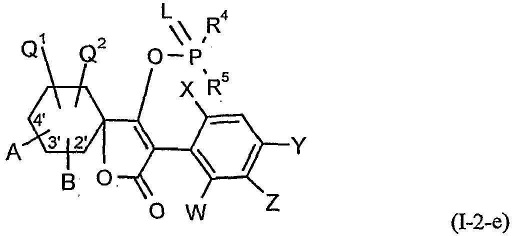 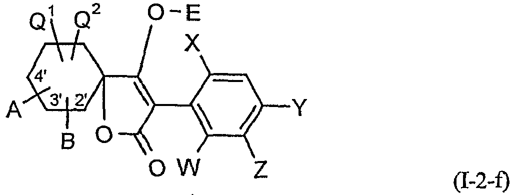 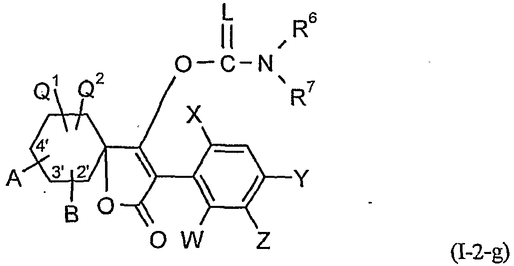 worin
A, B, E, L, M, Q¹, Q², W, X, Y, Z, R¹, R², R³, R⁴, R⁵, R⁶ und R⁷ die oben angegebene Bedeutung haben.

Weiterhin wurde gefunden, dass man die neuen Verbindungen der Formel (I) nach den im Folgenden beschriebenen Verfahren erhält:
(A) Man erhält Verbindungen der Formel (I-1-a) 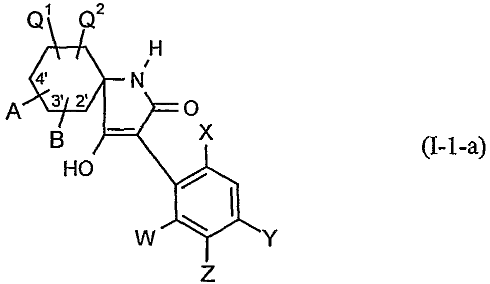 in welcher
   A, B, Q¹, Q², W, X, Y und Z die oben angegebenen Bedeutungen haben,
   wenn man
   Verbindungen der Formel (TI) 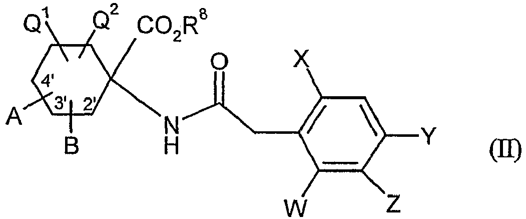 in welcher
   A, B, Q¹, Q², W, X, Y und Z die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, dass man Verbindungen der Formel (I-2-a) 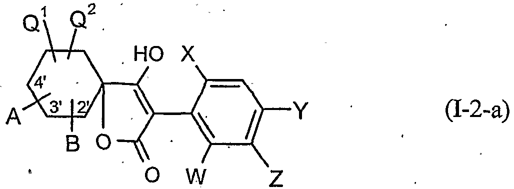 in welcher
   A, B, Q¹, Q², W, X, Y und Z die oben angegebenen Bedeutungen haben,
   erhält, wenn man
   Verbindungen der Formel (III) 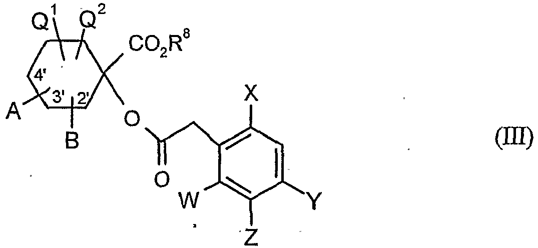 in welcher
   A, B, Q¹, Q², W, X, Y, Z und R⁸ die oben angegebenen Bedeutungen haben,
   in Gegenwart eines Verdünnungsmittels und in Gegenwart einer Base intramolekular kondensiert.
   Außerdem wurden gefunden
(C) dass man die Verbindungen der oben gezeigten Formeln (I-1-b) bis (I-2-b), in welchen R¹, A, B, Q¹, Q², W, X, Y und Z die oben angegebenen Bedeutungen haben, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   α) mit Verbindungen der Formel (IV) 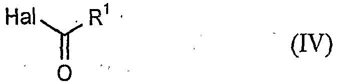 in welcher
      - R¹: die oben angegebene Bedeutung hat und'
      - Hal: für Halogen (insbesondere Chlor oder Brom) steht
      oder
   β) mit Carbonsäureanhydriden der Formel (V)

      R¹-CO-O-CO-R¹ (V)

      in welcher
      - R¹: die oben angegebene Bedeutung hat,
      gegebenenfalls in Gegenwart eines Verdünnungsmittels und gegebenenfalls in Gegenwart eines Säurebindemittels umsetzt;
(D) dass man die Verbindungen der oben gezeigten Formeln (I-1-c) bis (I-2-c), in welchen R², A, B, Q¹, Q², W, M, X, Y und Z die oben angegebenen Bedeutungen haben und L für Sauerstoff steht, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   mit Chlorameisensäureestern oder Chlorämeisensäurethioestern der Formel (VI)

   R²-M-CO-Cl (VI)

   in welcher
   - R² und M: die oben angegebenen Bedeutungen haben,
   gegebenenfalls in Gegenwart eines Verdünnungsmittels und gegebenenfalls in Gegenwert eines Säurebindemittels umsetzt;
(E) dass man Verbindungen der oben gezeigten Formeln (I-1-c) bis (I-2-c), in welchen R2, A, B, Q¹, Q², W, M, X, Y und Z die oben angegebenen Bedeutungen haben, und L für Schwefel steht, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   mit Chlormonothioameisensäureestern oder Chlordithioameisensäureestern der Formel (VII) 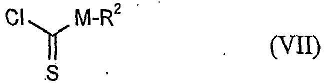 in welcher
   - M und R²: die oben angegebenen Bedeutungen haben,
   gegebenenfalls in Gegenwart eines Verdünnungsmittels und gegebenenfalls in Gegenwart eines Säurebindemittels umsetzt,
(F) dass man Verbindungen der oben gezeigten Formeln (I-1-d) bis (I-2-d), in welchen R³, A, B, W, Q¹, Q², X, Y und Z die oben angegebenen Bedeutungen haben, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   mit Sulfonsäurechloriden der Formel (VIII)

   R³-SO₂-Cl (VIII)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(G) dass man Verbindungen der oben gezeigten Formeln (I-1-e) bis (I-2-e), in welchen L, R⁴, R⁵, A, B, Q¹, Q², W, X, Y und Z die oben angegebenen Bedeutungen haben, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   mit Phosphorverbindungen der Formel (IX) 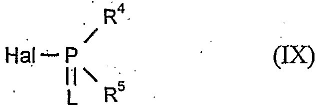 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(H) dass man Verbindungen der oben gezeigten Formeln (I-1-f) bis (I-2-f), in welchen E, A, B, Q¹, Q², W, X, Y und Z die oben angegebenen Bedeutungen haben, erhält, wenn man Verbindungen der Formeln (I-1-a) bis (I-2-a), in welchen A, B, Q¹, Q², W, X, Y und Z die oben angegebenen Bedeutungen haben, jeweils
   mit Metallverbindungen oder Aminen der Formeln (X) oder (XI) 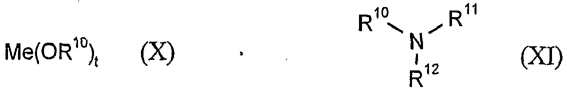 in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(I) dass man Verbindungen der oben gezeigten Formeln (I-1-g) bis (I-2-g), in welchen L, R⁶, R⁷, A, B, Q¹, Q², W, X, Y und Z die oben angegebenen Bedeutungen haben, erhält, wenn man Verbindungen der oben gezeigten Formeln (I-1-a) bis (I-2-a), in welchen A, B, Q¹, Q², W, X, Y und Z die oben angegebenen Bedeutungen haben, jeweils
   α) mit Isocyanaten oder Isothiocyanaten der Formel (XII)

      R⁶-N=C=L (XII)

      in welcher
      - R⁶ und L: die oben angegebenen Bedeutungen haben,
      gegebenenfalls in Gegenwart eines Verdünnungsmittels und gegebenenfalls in Gegenwart eines Katalysators umsetzt oder
   β) mit Carbamidsäurechloriden oder Thiocarbamidsäurechloriden der Formel (XIII) 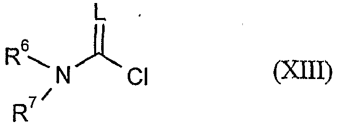 in welcher
      - L, R⁶ und R⁷: die oben angegebenen Bedeutungen haben,
      gegebenenfalls in Gegenwart eines Verdünnungsmittels und gegebenenfalls in Gegenwart eines Säurebindemittels, umsetzt,
(J) dass man Verbindungen der oben gezeigten Formeln (I-1-a) bis (I-1-g) und (I-2-a) bis (1-2-g), in welchen A, B, D, G, Q¹, Q², W, X, Y und Z die oben angegebenen Bedeutungen haben, erhält, wenn man Verbindungen der Formel (XIV) 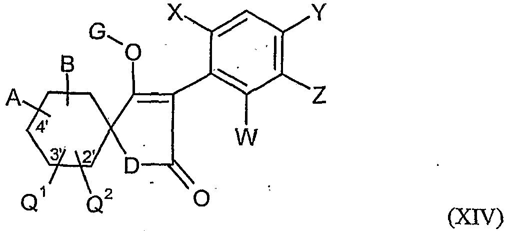 in welcher
   D, G, Q¹, Q², W, X, Y und Z die oben angegebenen Bedeutungen haben, und A, B und das Kohlenstoffatom, an das sie gebunden sind für eine 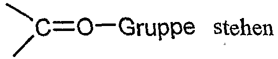 beispielsweise mit Diolen der Formel (XV)

   HO-A-B-OH (XV)

   in welcher
   A und B die oben genannte Bedeutung haben,
   gegebenenfalls in Gegenwart eines Verdünnungsmittels, in Gegenwart eines sauren Katalysators unter wasserentziehenden Bedingungen umsetzt.

Folgende Verbindung der Formel (I-a) ist 1994 im Rahmen des europäischen Patentprüfüngsverfahren zur EP 596 298 bekannt geworden.

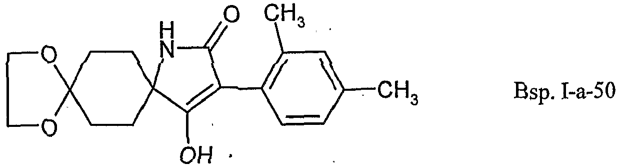

Weiterhin wurde gefunden, dass die neuen Verbindungen der Formel (I) eine gute Wirksamkeit als Schädlingsbekämpfungsmittel, vorzugsweise als Insektizide und/oder Akarizide und/oder Fungizide und/oder Herbizide aufweisen und darüber hinaus häufig sehr gut pflanzenverträglich, insbesondere gegenüber Kulturpflanzen, sind.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenem/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponente
(a') mindestens eine Verbindung der Formel (I), in welcher A, B, D, G, Q¹, Q², W, X, Y und Z die oben angegebene Bedeutung haben
   und
(b') zumindest eine die Kulturpflanzen-Verträglichkeit verbessernde Verbindung aus der folgenden Gruppe von Verbindungen:
   4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-' 3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenyl-ethyl)-hamstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2 yl-methyl)-N-(2-propenyl)-acetamid (PPG-1292), 3-Dichloracetyl-2,2-dimethyl-oxaiolidin (R-28725), 3-Dichloracetyl-2,2,5-trimethyl-oxazolidin (R-29148), 4-(4-Chlor-o-tolyl)-buttersäure, 4-(4-Chlor-phenoxy)-buttersäure, Diphenyl methoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-1H-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-buiylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-di-allylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlorphenoxy-essigsäure, 3,3'-Dimethyl-4-methoxy-benzophenon, 1-Brom-4-chlormethylsulfonyl-benzol, 1-[4-(N-2-Methoxybenzoylsulfamoyl)-phenyl]-3-methyl-harnstoff (alias N-(2-Methoxy-benzoyl)-4-[(methylamino-carbonyl)-amino]-benzolsulfonamid), 1-[4-(N-2-Methoxybenzoyl-sulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
   und/oder eine der folgenden durch allgemeine Formeln definierten Verbindungen
   der allgemeinen Formel (IIa) 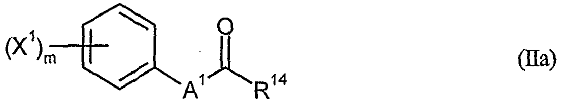 oder der allgemeinen Formel (IIb) 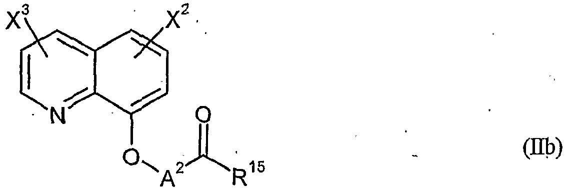 oder der Formel (IIc) 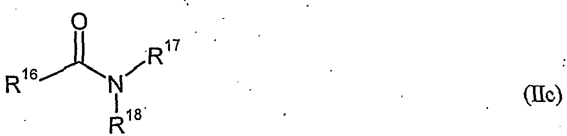 wobei
   - m: für eine Zahl 0, 1, 2, 3, 4 oder 5 steht;
   - A¹: für eine der nachstehend skizzierten divalenten heterocyclischen Gruppierungen steht, 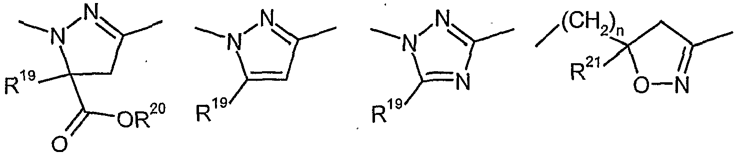
   - n: für eine Zahl 0, 1, 2, 3, 4 oder 5 steht,
   - A²: für gegebenenfalls durch C₁-C₄-Alkyl und/oder C₁-C₄-Alkoxy-carbonyl und/oder C₁-C₄-Alkenyloxy-carbonyl substituiertes Alkandiyl mit 1 oder 2 Kohlenstoffatomen steht,
   - R¹⁴: für Hydroxy, Mercapto, Amino, C₁-C₆-Alkoxy, C₁-C₆-Alkylthio, C₁-C₆-Alkylarnino oder Di-(C₁-C₄-alkyl)-amino steht,
   - R¹⁵: für Hydroxy, Mercapto, Amino, C₁-C₇-Alkoxy, C₁-C₆-Alkylthio, C₁-C₆-Alkenyloxy, C₁-C₆-Alkenyloxy-C₁-C₆-alkoxy, C₁-C₆-Alkylamino oder Di-(C₁-C₄-alkyl)-amino steht,
   - R¹⁶: für gegebenenfalls durch Fluor, Chlor und/oder Brom substituiertes C₁-C₄-Alkyl steht,
   - R¹⁷: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
   - R¹⁸: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl, R¹⁷ und R¹⁸ auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes C₃-C₆-Alkandiyl oder C₂-C₅-Oxaalkandiyl steht,
   - R¹⁹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
   - R²⁰: für Wasserstoff, jeweils gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
   - R²¹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
   - X¹: für Nitro, Cyano, Halogen, C₁-C₄-Alkyl, C₁-C₄-Halogenalkyl, C₁-C₄-Alkoxy oder C₁-C₄-Halogenalkoxy steht,
   - X²: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
   - X³: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
   und/oder die folgenden durch allgemeine Formeln definierten Verbindungen der allgemeinen Formel (IId) 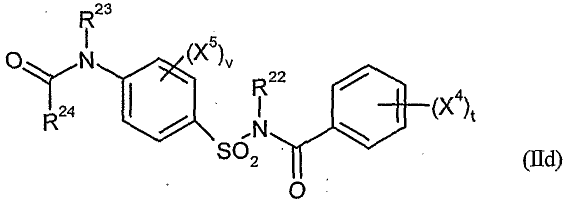 oder der allgemeinen Formel (IIe) 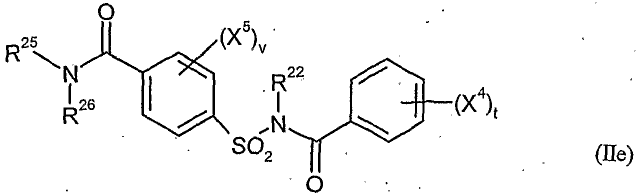 wobei
   - t: für eine Zahl 0, 1, 2, 3, 4 oder 5 steht,
   - v: für eine Zahl 0, 1, 2, 3, 4 oder 5 steht,
   - R²²: für Wasserstoff oder C₁-C₄-Alkyl steht,
   - R²³: für Wasserstoff oder C₁-C₄-Alkyl steht,
   - R²⁴: für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆-Cycloalkylthio oder C₃₋C₆-Cycloalkylamino steht,
   - R²⁵: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
   - R²⁶: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₅-Oxaalkandiyl steht,
   - X⁴: für Nitro, Cyano, Carboxy, Carbamoyl, Formyl, Sulfamoyl, Hydroxyl Amino, Halogen, C₁-C₄-Alkyl, C₁-C₄-Halogenalkyl, C₁-C₄-Alkoxy oder C₁-C₄-Halogenalkoxy steht, und
   - X⁵: für Nitro, Cyano, Carboxy, Carbamoyl, Formyl, Sulfamoyl, Hydroxy, Amino, Halogen, C₁-C₄-Alkyl, C₁-C₄-Halogenalkyl_{;} C₁-C₄-Alkoxy oder C₁-C₄-Halogenalkoxy steht.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:
- W: steht bevorzugt für Wasserstoff, C₁-C₆-Alkyl, C₂-C₆-Alkenyl, C₂-C₆-Alkinyl, Halogen, C₁-C₆-Alkoxy, C₁-C₄-Halogenalkyl, C₁-C₄-Halogenalkoxy oder Cyano,
- X: steht bevorzugt für Halogen, C₁-C₆-Alkyl, C₂-C₆-Alkenyl, C₂-C₆-Alkinyl, C₁-C₆-Halogenalkyl, C₁-C₆-Alkoxy, C₃-C₆-Alkenyloxy, C₁-C₆-Alkylthio, C₁-C₆-Alkylsulfinyl, C₁-C₆-Alkylsulfonyl, C₁-C₆-Halogenalkoxy, C₃-C₆-Halogenalkenyloxy, Nitro oder Cyano,
- Y und Z: stehen bevorzugt unabhängig voneinander für Wasserstoff, Halogen, C₁-C₆-Alkyl, C₂-C₆-Alkenyl, C₂-C₆-Alkinyl, C₁-C₆-Alkoxy, C₁-C₆-Halogenalkyl, C₁-C₆-Halogenalkoxyl, Cyano, C₂-C₆-Alkenyl, C₂-C₆-Alkinyl oder für einen der (Het)-arylreste 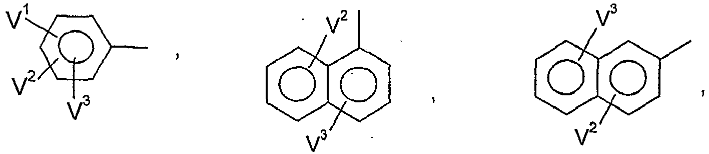 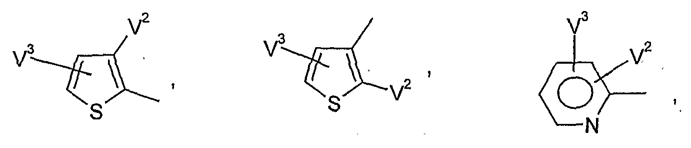 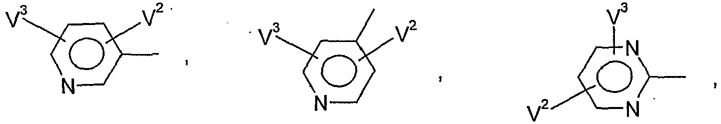 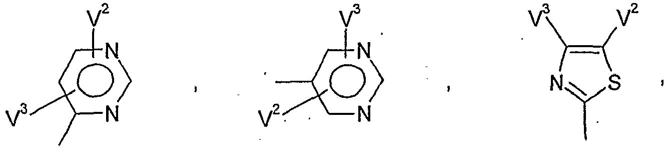 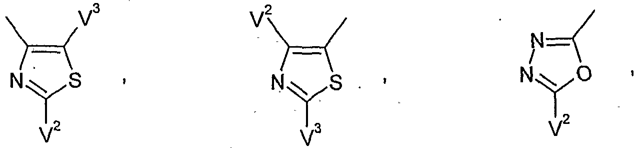 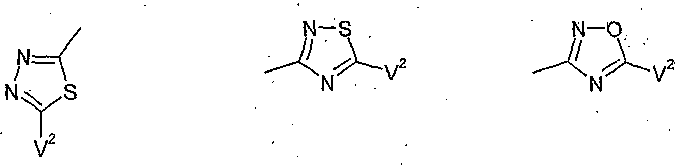 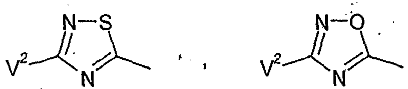 wobei im Falle von (Het)-aryl nur einer der Reste Y oder Z für (Het)-aryl stehen darf,
- V¹: steht bevorzugt für Wasserstoff, Halogen, C₁-C₁₂-Alkyl, C₁-C₆-Alkoxy, C₁-C₆-Alkylthio, C₁-C₆-Alkylsulfinyl, C₁-C₆-Alkylsulfonyl, C₁-C₄-Halogenalkyl, C₁-C₄-Halogenalkoxy, Nitro, Cyano oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-allcyl oder Phenyl-C₁-C₄-alkylthio,
- V² und V³: stehen bevorzugt unabhängig voneinander für Wasserstoff, Halogen, C₁-C₆-Alkyl, C₁-C₆-Alkoxy, C₁-C₄-Halogenalkyl oder C₁-C₄-Halogenalkoxy, mit der Maßgabe, dass mindestens einer der.Reste W oder Z ungleich Wasserstoff sein muss, wenn X und Y für Methyl stehen,
- A und B: und das Kohlenstoffatom, an dass sie gebunden sind, stehen bevorzugt für ein gegebenenfalls einfach bis vierfach durch C₁-C₆-Alkyl, C₁-C₄-Halogenalkyl, C₁-C₆-Alkoxy oder C₁-C₄-Alkoxy-C₁-C₄-alkyl substituiertes fünf- bis siebengliedriges Ketal, Thioketal oder Dithioketal, welches gegebenenfalls durch ein weiteres Sauerstoffatom, Schwefel-atom oder durch die Gruppe 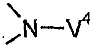 unterbrochen sein kann,
- V⁴: steht bevorzugt für Wasserstoff, C₁-C₆-Alkyl, C₃-C₆-Alkenyl, oder für die Gruppen -CO-V⁵, -CO₂V⁵, CO-NH-V⁵ oder CO-NH-O-V⁵,
- V⁵: steht bevorzugt für C₁-C₆-Alkyl,
- D: steht bevorzugt für NH (1) oder Sauerstoff (2),
- Q¹ und Q²: stehen bevorzugt unabhängig voneinander für Wasserstoff, C₁-C₆-Alkyl, C₁-C₂-Halogenalkyl oder C₁-C₄-Alkoxy,
- G: steht bevorzugt für Wasserstoff (a) oder für eine der Gruppen 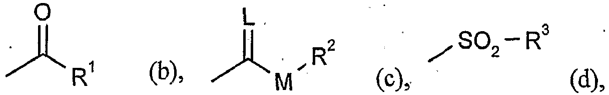 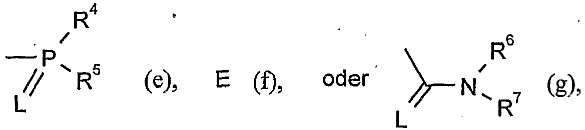 in welchen
E für ein Metallion oder ein Ammoniumion steht,
L für Sauerstoff oder Schwefel steht und
M für Sauerstoff oder Schwefel steht,
- R¹: steht bevorzugt für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl sub-stituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff,
- R²: steht bevorzugt für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl,
- R³: steht bevorzugt für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl,
- R⁴ und R⁵: stehen unabhängig voneinander bevorzugt für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Aklthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Akylthio, C₁-C₄-Halogen-alkylthio, C₁-C₄-Alkyl oder C₁-C₄-Halogenalkyl substituiertes Phenyl, Phenoxy oder Phenylthio,
- R⁶ und R⁷: stehen unabhängig voneinander bevorzugt für Wasserstoff, für jeweils gegebenenfalls durch Halogen oder Cyano substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Akyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest, in welchem gegebenenfalls eine Methylengruppe durch Sauerstoff oder Schwefel ersetzt ist.

In den als bevorzugt genannten Restedefnitionen steht Halogen für Fluor, Chlor, Brom und Iod, insbesondere für Fluor, Chlor und Brom.
- W: steht besonders bevorzugt für Wasserstoff, Chlor, Brom, C₁-C₄-Alkyl, C₂-C₄-Alkenyl, C₂-C₄-Alkinyl, C₁-C₄-Alkoxy, C₁-C₂-Halogenalkyl oder C₁-C₂-Halogenalkoxy,
- X: steht besonders bevorzugt für Chlor, Brom, C₁-C₄-Alkyl, C₂-C₄-Alkenyl; C₂-C₄-Alkinyl, C₁-C₄-Alkoxy, C₁-C₄-Halogenalkyl, C₁-C₄-Halogenalkoxy oder Cyano
- Y und Z: stehen besonders bevorzugt unabhängig voneinander für Wasserstoff, Fluor, Chlor, Brom, C₁-C₄-Alkyl, C₂-C₄-Alkenyl, C₂-C₄-Alkinyl, C₁-C₆-Alkoxy, C₁-C₄-Halogenalkyl, C₁-C₄-Halogenalkoxy, Cyano, C₂-C₄-Alkenyl, C₂-C₄-Alkinyl oder für einen der (Het)-arylreste, 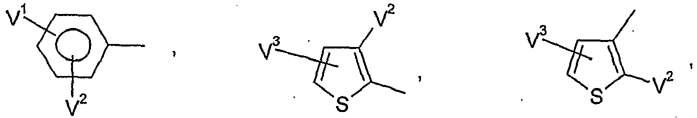 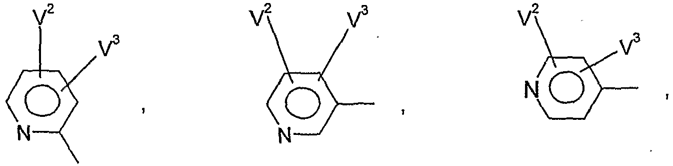 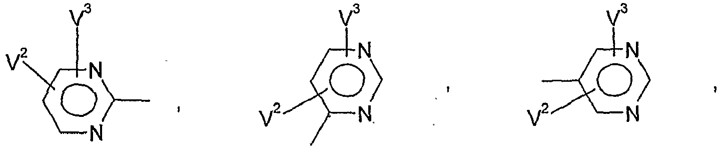 wobei im Falle von (Het)-aryl nur einer der Reste Y oder Z für (Het)-aryl stehen darf,
- V¹: steht besonders bevorzugt für Wasserstoff, Fluor, Chlor, Brom, C₁-C₆-Alkyl, C₁-C₄-Alkoxy, C₁-C₂- Halogenalkyl, C₁-C₂-Halogenalkoxy, Nitro, Cyano oder jeweils gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl,
- V² und V³: stehen besonders bevorzugt unabhängig voneinander für Wasserstoff, Fluor, Chlor, Brom, C₁-C₄-Alkyl, C₁-C₄-Alkoxy, C₁-C₂-Halogenalkyl oder C₁-C₂-Halogonalkoxy,
mit der Maßgabe, dass mindstens einer der Reste W oder Z ungleich Wasserstoff sein muss, wenn X und Y für Methyl stehen,
- A und B: und das Kohlenstoffatom, an dass sie gebunden sind, stehen besonders bevorzugt für ein gegebenenfalls einfach bis dreifach durch C₁-C₄-Alkyl, C₁-C₃-Halogenalkyl, C₁-C₄-Alkoxy oder C₁-C₄-Alkoxy-C₁-C₂-alkyl substituiertes fünf-, sechs- oder siebengliedriges Ketal, welches gegebenenfalls durch ein weiteres Sauerstoffatom unterbrochen sein kann,
- D: steht besonders bevorzugt für NH (1) oder Sauerstoff (2),
- Q¹ und Q²: stehen besonders bevorzugt unabhängig voneinander für Wasserstoff, Methyl, Ethyl, Trifluormethyl, Methoxy oder Ethoxy,
- G: steht besonders bevorzugt für Wasserstoff (a) oder für eine der Gruppen 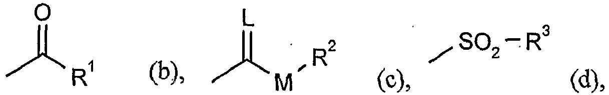 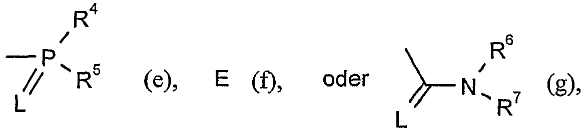 in welchen
E für ein Metallion oder ein Ammoniumion steht,
L für Sauerstoff oder Schwefel steht und
M für Sauerstoff oder Schwefel steht,
- R¹: steht besonders bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls einfach bis zweifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach bis zweifach durch Fluor, Chlor, Brom oder Ci-C₄-Alkyl substituiertes Pyrazolyl, Thiazolyl, Pyridyl, Pyrimidyl, Furanyl oder Thienyl,
für gegebenenfalls einfach bis zweifach durch Fluor, Chlor, Brom oder C₁-C₄-Alkyl substituiertes Phenoxy-C₁-C₅-alkyl oder
für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl,
- R²: steht besonders bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-allcoxy-C₂-C₆-alkyl,
für gegebenenfalls einfach bis zweifach durch Fluor, Chlor, C₁-C₄-Alkyl oder C₁-C₄-Alkoxy substituiertes C₃-C₇-Cycloalkyl oder
für jeweils gegebenenfalls einfach bis dreifach durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl,
- R³: steht besonders bevorzugt für gegebenenfalls einfach bis dreifach durch Fluor oder Chlor substituiertes C₁-C₆-Alkyl oder jeweils gegebenenfalls einfach bis zweifach durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl,
- R⁴ und R⁵: stehen unabhängig voneinander besonders bevorzugt für jeweils gegebenenfalls einfach bis dreifach durch Fluor oder Chlor substituiertes C₁-C₆-Alkyl, C₁-C₆-Alkoxy, C₁-C₆-Alkylamino, Di-(C₁-C₆-alkyl)amino, C₁-C₆-Alkylthio oder C₃-C₄-Alkenylthio oder für jeweils gegebenenfalls einfach bis zweifach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio,
- R⁶ und R⁷: stehen unabhängig voneinander besonders bevorzugt für Wasserstoff, für jeweils gegebenenfalls einfach bis dreifach durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls einfach bis dreifach durch Fluor, Chlor, Brom, C₁-C₅-Halo-genalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest, in welchem gegebenenfalls eine Methylengruppe durch Sauerstoff oder Schwefel ersetzt ist.

In den als besonders bevorzugt genannten Restedefinitionen steht Halogen für Fluor, Chlor und Brom, insbesondere für Fluor und Chlor.
- W: steht ganz besonders bevorzugt für Wasserstoff, Chlor, Brom, Methyl, Ethyl, Vinyl, Ethinyl, Propinyl, Methoxy, Ethoxy oder Trifluormethyl,
- X: steht ganz besonders bevorzugt für Chlor, Brom, Methyl, Ethyl, Propyl, iso-Propyl, Vinyl, Ethinyl, Propinyl, Methoxy, Ethoxy, Trifluormethyl, Difluormethoxy, Trifluormethoxy oder Cyano,
- Y und Z: stehen ganz besonders bevorzugt unabhängig voneinander für Wasserstoff, Fluor, Chlor, Brom, Methyl, Ethyl, Vinyl, Ethinyl, Propinyl, Methoxy, Trifluormethyl, Trifluormethoxy, Cyano oder einen Phenylrest, 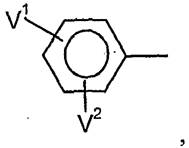 wobei im Falle von Phenyl nur einer der Reste Y oder Z für Phenyl stehen darf,
- V¹: steht ganz besonders bevorzugt für Wasserstoff, Fluor, Chlor, Brom, Methyl, Ethyl, n-Propyl, iso-Propyl, tert.-Butyl, Methoxy, Ethoxy, n-Propoxy, iso-Propoxy, Trifluormethyl oder Trifluormethoxy,
- V²: steht ganz besonders bevorzugt für Wasserstoff Fluor, Chlor, Methyl, Ethyl, n-Propyl, iso-Propyl, Methoxy, Ethoxy oder Trifluormethyl, mit der Maßgabe, dass mindestens einer der Reste W oder Z ungleich Wasserstoff sein muss, wenn X und Y für Methyl stehen,
- A und B: und das Kohlenstoffatom, an dass sie gebunden sind, stehen ganz besonders bevorzugt für ein gegebenenfalls einfach bis zweifach durch Methyl, Ethyl, Propyl, Trifluormethyl, Monochlormethyl, Methoxy, Ethoxy, Methoxymethyl oder Ethoxymethyl substituiertes fünf-, sechs- oder siebengliedriges Ketal, welches gegebenenfalls durch ein weiteres Sauerstoffatom unterbrochen sein kann,
- D: steht ganz besonders bevorzugt für NH (1) oder Sauerstoff (2),
- Q¹ und Q²: stehen ganz besonders bevorzugt für Wasserstoff,
- G: steht ganz besonders bevorzugt für Wasserstoff (a) oder für eine der Gruppen 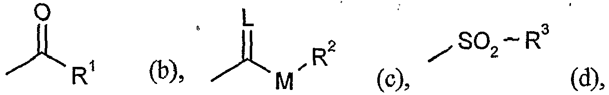 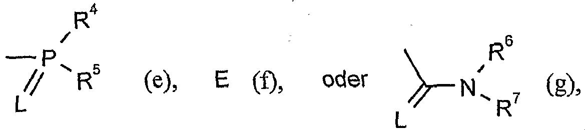 in welchen
E für ein Metallion oder ein Ammoniumion steht,
L für Sauerstoff oder Schwefel steht und
M für Sauerstoff oder Schwefel steht,
- R¹: steht ganz besonders bevorzugt für jeweils gegebenenfalls einfach bis dreifach durch Fluor oder Chlor substituiertes C₁-C₁₀-Alkyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloalkyl,
für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl,
für jeweils gegebenenfalls einfach durch Chlor, Brom oder Methyl substituiertes Furanyl, Thienyl oder Pyridyl,
- R²: steht ganz besonders bevorzugt für jeweils gegebenenfalls einfach bis dreifach durch Fluor oder Chlor substituiertes C₁-C₁₀-Alkyl, C₂-C₁₀-Alkenyl oder C₁-C₄-Alkoxy-C₂-C₄-alkyl,
für Cyclopentyl oder Cyclohexyl
oder für jeweils gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl,
- R³: steht ganz besonders bevorzugt für jeweils gegebenenfalls einfach bis dreifach durch Fluor oder Chlor substituiertes Methyl, Ethyl, Propyl oder iso-Propyl, oder jeweils gegebenenfalls einfach durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl,
- R⁴ und R⁵: stehen unabhängig voneinander ganz besonders bevorzugt für C₁-C₄-Alkoxy öder C₁-C₄-Alkylthio oder für jeweils gegebenenfalls einfach durch Fluor, Chlor, Brom, Nitro, Cyano, Methyl, Methoxy, Trifluormethyl oder Trifluormethoxy substituiertes Phenyl, Phenoxy oder Phenylthio,
- R⁶ und R⁷: stehen unabhängig voneinander ganz besonders bevorzugt für Wasserstoff, für C₁-C₄-Alkyl, C₃-C₆-Cycloalkyl, C₁-C₄-Alkoxy, C₃-C₄-Alkenyl oder C₁-C₄-Alkoxy-C₂-C₄-alkyl, für jeweils gegebenenfalls einfach bis zweifach durch Fluor, Chlor, Brom, Methyl, Methoxy oder Trifluormethyl substituiertes Phenyl, oder zusammen für einen C₅-C₆-Alkylenrest, in welchem gegebenenfalls eine Methylengruppe durch Sauerstoff oder Schwefel ersetzt ist.
- W: steht insbesondere bevorzugt für Wasserstoff, Chlor, Brom, Methyl, Ethyl oder Methoxy,
- X: steht insbesondere bevorzugt für Chlor, Brom, Methyl, Ethyl oder Methoxy,
- Y und Z: stehen insbesondere bevorzugt unabhängig voneinander für Wasserstoff, Chlor, Brom, Methyl oder für den Rest 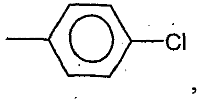 wobei in diesem Falle nur einer der Reste Y oder Z für 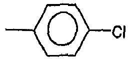 stehen darf,
mit der Maßgabe, dass mindestens einer der Reste W oder Z ungleich Wasserstoff sein muss, wenn X und Y für Methyl stehen,
- A und B: und das Kohlenstoffatom, an dass sie gebunden sind, stehen insbesondere bevorzugt für ein gegebenenfalls einfach bis zweifach durch Methyl, Ethyl, Propyl, Monochlormethyl oder Methoxymethyl substituiertes fünf- oder sechsgliedriges Ketal,
- D: steht insbesondere bevorzugt für NH (1) oder Sauerstoff (2),
- Q¹ und Q²: stehen insbesondere bevorzugt für Wasserstoff,
- G: steht insbesondere bevorzugt für Wasserstoff (a) oder für eine der Gruppen 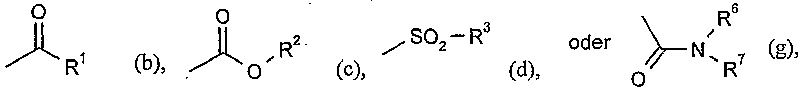
- R¹: steht insbesondere bevorzugt für C₁-C₁₀-Alkyl, C₁-C₄-Alkoxy-C₁-C₂-alkyl, C₃-C₆-Cycloalkyl,
für gegebenenfalls einfach durch Chlor substituiertes Phenyl, oder für Thienyl,
- R²: steht insbesondere bevorzugt für C₁-C₁₀-Alkyl, C₂-C₁₀-Alkenyl, oder für Benzyl,
- R³: steht insbesondere bevorzugt für Methyl,
- R⁶ und R⁷: stehen insbesondere bevorzugt zusammen für einen C₅-C₆-Alkylenrest, in welchem gegebenenfalls eine Methylengruppe durch Sauerstoff oder Schwefel ersetzt ist.

Die oben aufgeführten allgemeinen oder in Vorzugsbereichen aufgeführten Restedefmitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß insbesondere bevorzugt werden die Verbindungen der Formel (I), in welchen eine Kombination der vorstehend als insbesondere bevorzugt aufgeführten Bedeutungen vorliegt.

Hervorgehoben sind Verbindungen der Formel (I) in welchen G für Wasserstoff steht.

Im Fall der Verbindungen der Formel (I-1) ist der Phenylring hervorgehoben 3-fach substituiert, wobei sich folgende Substitutionsmuster ergeben: 2,4,6-; 2,4,5- oder 2,5,6-Substitution. Außerdem ist im der Fall der Verbindungen der Formel (I-1) der Phenylring hervorgehoben 4-fach substituiert, wobei sich folgendes Substitutionsmuster ergibt: 2,4,5,6-Substittution. Im Falle der Verbindungen der Formel (I-1) ist der Phenylring hervorgehoben auch 2-fach (2,5-Postition) substituiert. Im Falle der Verbindungen der Formel (I-I) ist der Phenylring hervorgehoben auch 1-fach (ortho Postition) substituiert. Die übrigen Substituenten W, X, Y, Z, G, A, B, Q¹ und Q² haben die im Text genannten Definitionen.

Außerdem steht im Fall der Verbindungen der Formel (I-1), in der der Phenylring in 2,4-Postion substituiert ist, der Substituent G hervorgehoben für die Gruppe (b), aber auch für die Gruppe (c) oder (d) oder (e) oder (f) oder (g). Insbesondere steht G in diesem Fall für die Gruppen (a), (b) oder (c). Die übrigen Substituenten W, X, Y, Z, A, B, Q¹ und Q² haben die im Text genannten Definitionen.

Weiterhin steht im Fall der Verbindungen der Formel (I-1), in der der Phenylring in 2,4-Position substituiert ist, die Substituenten A und B hervorgehoben in der 3'-Position am Spirocyclus. Weiterhin steht im Fall der Verbindungen der Formel (I-1), in der der Phenylring in 2,4-Position substituiert ist, die Substituenten A und B hervorgehoben in der 4'-Position am Spirocyclus.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nicht anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (1-1-a) genannt:

**Tabelle 1**

| | | | | |
|---|---|---|---|---|
| 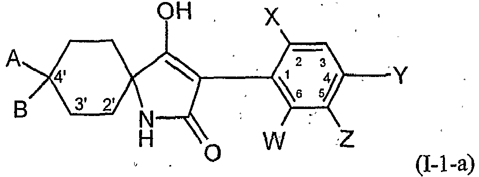 | | | | |

| **A-B** | **X** | **W** | **Y** | **Z** |
|---|---|---|---|---|
| -O-(CH₂)₂-O- | CH₃ | H | H | H |
| -O-(CH₂)₂-O- | Br | H | H | H |
| -O-(CH₂)₂-O- | Cl | H | H | H |
| -O-(CH₂)₂-O- | CF₃ | H | H | H |
| -O-(CH₂)₂-O- | OCH₃ | H | H | H |
| -O-(CH₂)₂-O- | Br | H | Cl | H |
| -O-(CH₂)₂-O- | Cl | H | Br | H |
| -O-(CH₂)₂-O- | Cl | H | Cl | H |
| -O-(CH₂)₂-O- | Cl | H | CH₃ | H |
| -O-(CH₂)₂-O- | CH₃ | H | Cl | H |
| -O-(CH₂)₂-O- | Cl | Cl | H | H |
| -O-(CH₂)₂-O- | Cl | OCH₃ | H | H |
| -O-(CH₂)₂-O- | Cl | CH₃ | H | H |
| -O-(CH₂)₂-O- | Cl | OC₂H₅ | H | H |
| -O-(CH₂)₂-O- | OCH₃ | OCH₃ | H | H |
| -O-(CH₂)₂-O- | CH₃ | CH₃ | H | H |
| -O-(CH₂)₂-O- | C₂H₅ | CH₃ | H | H |
| -O-(CH₂)₂-O- | C₂H₅ | C₂H₅ | H | H |
| -O-(CH₂)₂-O- | Br | CH₃ | Br | H |
| -O-(CH₂)₂-O- | Cl | CH₃ | Cl | H |
| -O-(CH₂)₂-O- | CH₃ | Br | CH₃ | H |
| -O-(CH₂)₂-O- | CH₃ | Cl | CH₃ | H |
| -O-(CH₂)₂-O- | OCH₃ | CH₃ | CH₃ | H |
| -O-(CH₂)₂-O- | OC₂H₅ | CH₃ | CH₃ | H |
| -O-(CH₂)₂-O- | OC₃H₇ | CH₃ | CH₃ | H |
| -O-(CH₂)₂-O- | CH₃ | CH₃ | CH₃ | H |
| -O-(CH₂)₂-O- | Br | Br | CH₃ | H |
| -O-(CH₂)₂-O- | Cl | Cl | CH₃ | H |
| -O-(CH₂)₂-O- | CH₃ | CH₃ | Br | H |
| -O-(CH₂)₂-O- | OCH₃ | C₂H₅ | CH₃ | H |
| -O-(CH₂)₂-O- | OC₂H₅ | C₂H₅ | CH₃ | H |
| -O-(CH₂)₂-O- | CH₃ | CH₃ | OCH₃ | H |
| -O-(CH₂)₂-O- | Br | Cl | CH₃ | H |
| -O-(CH₂)₂-O- | Br | CH₃ | Cl | H |
| -O-(CH₂)₂-O- | Cl | CH₃ | Br | H |
| -O-(CH₂)₂-O- | CH₃ | CH₃ | Cl | H |
| -O-(CH₂)₂-O- | C₂H₅ | CH₃ | CH₃ | H |
| -O-(CH₂)₂-O- | C₂H₅ | C₂H₅ | CH₃ | H |
| -O-(CH₂)₂-O- | C₂H₅ | CH₃ | C₂H₅ | H |
| -O-(CH₂)₂-O- | C₂H₅ | C₂H₅ | C₂H₅ | H |
| -O-(CH₂)₂-O- | C₂H₅ | CH₃ | Cl | H |
| -O-(CH₂)₂-O- | C₂H₅ | C₂H₅ | Cl | H |
| -O-(CH₂)₂-O- | C₂H₅ | CH₃ | Br | H |
| -O-(CH₂)₂-O- | C₂H₅ | C₂H₅ | Br | H |
| -O-(CH₂)₂-O- | C₂H₅ | Cl | CH₃ | H |
| -O-(CH₂)₂-O- | C₂H₅ | Br | CH₃ | H |
| -O-(CH₂)₂-O- | C₂H₅ | Cl | Cl | H |
| -O-(CH₂)₂-O- | C₂H₅ | Br | Br | H |
| -O-(CH₂)₂-O- | C₂H₅ | Cl | Br | H |
| -O-(CH₂)₂-O- | C₂H₅ | Br | Cl | H |
| -O-(CH₂)₂-O- | OCH₃ | CH₃ | Cl | H |
| -O-(CH₂)₂-O- | OCH₃ | C₂H₅ | Cl | H |
| -O-(CH₂)₂-O- | OC₂H₅ | CH₃ | Cl | H |
| -O-(CH₂)₂-O- | OC₂H₅ | C₂H₅ | Cl | H |
| -O-(CH₂)₂-O- | Cl | OCH₃ | CH₃ | H |
| -O-(CH₂)₂-O- | Cl | OC₂H₅ | CH₃ | H |
| -O-(CH₂)₂-O- | CH₃ | CH₃ | Cl | H |
| -O-(CH₂)₂-O- | Cl | H | Cl | Cl |
| -O-(CH₂)₂-O- | CH₃ | H | CH₃ | CH₃ |
| -O-(CH₂)₂-O- | CH₃ | H | Cl | CH₃ |
| -O-(CH₂)₂-O- | Br | H | Cl | CH₃ |
| -O-(CH₂)₂-O- | Br | H | CH₃ | CH₃ |
| -O-(CH₂)₂-O- | Cl | H | Br | CH₃ |
| -O-(CH₂)₂-O- | Cl | H | Cl | CH₃ |
| -O-(CH₂)₂-O- | CH₃ | H | Br | CH₃ |
| -O-(CH₂)₂-O- | Cl | H | CH₃ | Cl |
| -O-(CH₂)₂-O- | CH₃ | H | H | CH₃ |
| -O-(CH₂)₂-O- | Cl | H | H | CH₃ |
| -O-(CH₂)₂-O- | Br | H | H | CH₃ |
| -O-(CH₂)₂-O- | CH₃ | H | H | Cl |
| -O-(CH₂)₂-O- | CH₃ | H | H | Br |
| -O-(CH₂)₂-O- | CH₃ | CH₃ | CH₃ | CH₃ |
| -O-(CH₂)₂-O- | CH₃ | CH₃ | CH₃ | F |
| -O-(CH₂)₂-O- | CH₃ | CH₃ | CH₃ | Cl |
| -O-(CH₂)₂-O- | CH₃ | CH₃ | CH₃ | Br |
| -O-(CH₂)₂-O- | CH₃ | CH₃ | H | Cl |
| -O-(CH₂)₂-O- | CH₃ | CH₃ | H | Br |
| -O-(CH₂)₂-O- | Cl | Cl | H | Br |
| -O-(CH₂)₂-O- | CH₃ | CH₃ | 4-Cl-C₆H₄ | H |
| -O-(CH₂)₂-O- | C₂H₅ | CH₃ | 4-Cl-C₆H₄ | H |
| -O-(CH₂)₂-O- | C₂H₅ | C₂H₅ | 4-Cl-C₆H₄ | H |
| -O-(CH₂)₂-O- | Cl | CH₃ | 4-Cl-C₆H₄ | H |
| -O-(CH₂)₂-O- | Cl | C₂H₅ | 4-Cl-C₆H₄ | H |
| -O-(CH₂)₂-O- | CH₃ | H | H | 4-Cl-C₆H₄ |
| -O-(CH₂)₂-O- | CH₃ | CH₃ | H | 4-Cl-C₆H₄ |
| -O-(CH₂)₂-O- | CH₃ | H | CH₃ | 4-Cl-C₆H₄ |
| -O-(CH₂)₂-O- | CH₃ | CH₃ | CH₃ | 4-Cl-C₆H₄ |
| -O-(CH₂)₂-O- | Cl | H | H | 4-Cl-C₆H₄ |
| -O-(CH₂)₂-O- | J | H | H | H |
| -O-(CH₂)₂-O- | J | H | CH₃ | H |
| -O-(CH₂)₂-O- | J | CH₃ | H | H |
| -O-(CH₂)₂-O- | J | C₂H₅ | H | H |
| -O-(CH₂)₂-O- | CH₃ | H | H | J |
| -O-(CH₂)₂-O- | CH₃ | H | CH₃ | J |
| -O-(CH₂)₂-O- | J | CH₃ | CH₃ | H |
| -O-(CH₂)₂-O- | J | C₂H₅ | CH₃ | H |
| -O-(CH₂)₂-O- | J | CH₃ | Cl | H |
| -O-(CH₂)₂-O- | J | C₂H₅ | Cl | H |
| -O-(CH₂)₂-O- | J | Cl | CH₃ | H |
| -O-(CH₂)₂-O- | J | H | CH₃ | CH₃ |
| -O-(CH₂)₂-O- | CH₃ | H | J | H |
| -O-(CH₂)₂-O- | C₂H₅ | H | J | H |
| -O-(CH₂)₂-O- | CH₃ | CH₃ | J | H |
| -O-(CH₂)₂-O- | C₂H₅ | CH₃ | J | H |
| -O-(CH₂)₂-O- | C₂H₅ | C₂H₅ | J | H |
| -O-(CH₂)₂-O- | Cl | CH₃ | J | H |
| -O-(CH₂)₂-O- | Cl | C₂H₅ | J | H |
| -O-(CH₂)₂-O- | CH₃ | H | J | CH₃ |
| -O-(CH₂)₂-O- | CH₃ | CH₃ | H | J |
| -O-(CH₂)₂-O- | J | H | H | CH₃ |
| -O-(CH₂)₂-O- | C₂H₅ | H | H | H |

**Tabelle 2:** W, X, Y und Z wie in Tabelle 1 angegeben
A-B = 4'-O-CH₂-CHCH₃-O-

**Tabelle 3:** W, X, Y und Z wie in Tabelle 1 angegeben
A-B = 4'-O-CHCH₃-CHCH₃-O-

**Tabelle 4:** W, X, Y und Z wie in Tabelle 1 angegeben
A-B = 4'-O-(CH₂)₃-O-

**Tabelle 5:** W, X, Y und Z wie in Tabelle 1 angegeben
A-B = 4'-O-CHCH₃-(CH₂)₂-O-

**Tabelle 6:** W, X, Y und Z wie in Tabelle 1 angegeben
A-B = 4'-O-CHC-H₃-CH₂-CHCH₃-O-

**Tabelle 7:** W, X, Y und Z wie in Tabelle 1 angegeben
A-B = -4'O-CH₂-C(CH₃)₂-CH₂-O-

**Tabelle 8: W,** X, Y und Z wie in Tabelle 1 angegeben

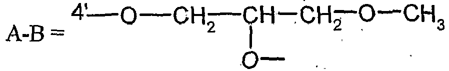

**Tabelle 9:** W, X, Y und Z wie in Tabelle 1 angegeben
A-B = 3'-O-(CH₂)₂-O-

**Tabelle 10:** W, X, Y und Z wie in Tabelle 1 angegeben
A-B = 3'-O-CH₂-CHCH₃-O-

**Tabelle 11:** W, X, Y und Z wie in Tabelle 1 angegeben
A-B = 3'-O-CHCH₃-CHCH₃-O-

**Tabelle 12:** W, X, Y und Z wie in Tabelle 1 angegeben
A-B = 3'-O-(CH₂)₃-O-

**Tabelle 13:** W, X, Y und Z wie in Tabelle 1 angegeben
A-B = 3'-O-CHCH₃-(CH₂)₂-O-

**Tabelle 14:** W, X, Y und Z wie in Tabelle 1 angegeben
A-B = 3'-O-CHCH₃-CH₂-CHCH₃-O-

**Tabelle 15:** W, X, Y und Z wie in Tabelle 1 angegeben
A-B = 3'-O-CH₂-C(CH₃)₂-CH₂-O-

**Tabelle 16:** W, X, Y und Z wie in Tabelle 1 angegeben

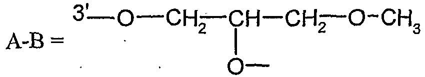

Im einzelnen seien außer den bei den Herstellungsbeispielen genannten Verbindungen die folgenden Verbindungen der Formel (I-2-a) genannt:

**Tabelle 17**

| | | | | |
|---|---|---|---|---|
| 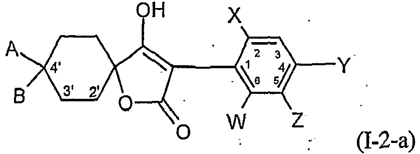 | | | | |

| **A-B** | **X** | **W** | **Y** | **Z** |
|---|---|---|---|---|
| -O-(CH₂)₂-O- | CH₃ | H | H | H |
| -O-(CH₂)₂-O- | Br | H | H | H |
| -O-(CH₂)₂-O- | Cl | H | H | H |
| -O-(CH₂)₂-O- | CF₃ | H | H | H |
| -O-(CH₂)₂-O- | OCH₃ | H | H | H |
| -O-(CH₂)₂-O- | Br | H | Cl | H |
| -O-(CH₂)₂-O- | Cl | H | Br | H |
| -O-(CH₂)₂-O- | Cl | H | Cl | H |
| -O-(CH₂)₂-O- | Cl | H | CH₃ | H |
| -O-(CH₂)₂-O- | CH₃ | H | Cl | H |
| -O-(CH₂)₂-O- | Cl | Cl | H | H |
| -O-(CH₂)₂-O- | Cl | OCH₃ | H | H |
| -O-(CH₂)₂-O- | Cl | CH₃ | H | H |
| -O-(CH₂)₂-O- | Cl | OC₂H₅ | H | H |
| -O-(CH₂)₂-O- | OCH₃ | OCH₃ | H | H |
| -O-(CH₂)₂-O- | CH₃ | CH₃ | H | H |
| -O-(CH₂)₂-O- | C₂H₅ | CH₃ | H | H |
| -O-(CH₂)₂-O- | C₂H₅ | C₂H₅ | H | H |
| -O-(CH₂)₂-O- | Br | CH₃ | Br | H |
| -O-(CH₂)₂-O- | Cl | CH₃ | Cl | H |
| -O-(CH₂)₂-O- | CH₃ | Br | CH₃ | H |
| -O-(CH₂)₂-O- | CH₃ | Cl | CH₃ | H |
| -O-(CH₂)₂-O- | OCH₃ | CH₃ | CH₃ | H |
| -O-(CH₂)₂-O- | OC₂H₅ | CH₃ | CH₃ | H |
| -O-(CH₂)₂-O- | OC₃H₇ | CH₃ | CH₃ | H |
| -O-(CH₂)₂-O- | CH₃ | CH₃ | CH₃ | H |
| -O-(CH₂)₂-O- | Br | Br | CH₃ | H |
| -O-(CH₂)₂-O- | Cl | Cl | CH₃ | H |
| -O-(CH₂)₂-O- | CH₃ | CH₃ | Br | H |
| -O-(CH₂)₂-O- | OCH₃ | C₂H₅ | CH₃ | H |
| -O-(CH₂)₂-O- | OC₂H₅ | C₂H₅ | CH₃ | H |
| -O-(CH₂)₂-O- | CH₃ | CH₃ | OCH₃ | H |
| -O-(CH₂)₂-O- | Br | Cl | CH₃ | H |
| -O-(CH₂)₂-O- | Br | CH₃ | Cl | H |
| -O-(CH₂)₂-O- | Cl | CH₃ | Br | H |
| -O-(CH₂)₂-O- | CH₃ | CH₃ | Cl | H |
| -O-(CH₂)₂-O- | C₂H₅ | CH₃ | CH₃ | H |
| -O-(CH₂)₂-O- | C₂H₅ | C₂H₅ | CH₃ | H |
| -O-(CH₂)₂-O- | C₂H₅ | CH₃ | C₂H₅ | H |
| -O-(CH₂)₂-O- | C₂H₅ | C₂H₅ | C₂H₅ | H |
| -O-(CH₂)₂-O- | C₂H₅ | CH₃ | Cl | H |
| -O-(CH₂)₂-O- | C₂H₅ | C₂H₅ | Cl | H |
| -O-(CH₂)₂-O- | C₂H₅ | CH₃ | Br | H |
| -O-(CH₂)₂-O- | C₂H₅ | C₂H₅ | Br | H |
| -O-(CH₂)₂-O- | C₂H₅ | Cl | CH₃ | H |
| -O-(CH₂)₂-O- | C₂H₅ | Br | CH₃ | H |
| -O-(CH₂)₂-O- | C₂H₅ | Cl | Cl | H |
| -O-(CH₂)₂-O- | C₂H₅ | Br | Br | H |
| -O-(CH₂)₂-O- | C₂H₅ | Cl | Br | H |
| -O-(CH₂)₂-O- | C₂H₅ | Br | Cl | H |
| -O-(CH₂)₂-O- | OCH₃ | CH₃ | Cl | H |
| -O-(CH₂)₂-O- | OCH₃ | C₂H₅ | Cl | H |
| -O-(CH₂)₂-O- | OC₂H₅ | CH₃ | Cl | H |
| -O-(CH₂)₂-O- | OC₂H₅ | C₂H₅ | Cl | H |
| -O-(CH₂)₂-O- | Cl | OCH₃ | CH₃ | H |
| -O-(CH₂)₂-O- | Cl | OC₂H₅ | CH₃ | H |
| -O-(CH₂)₂-O- | CH₃ | CH₃ | Cl | H |
| -O-(CH₂)₂-O- | Cl | H | Cl | Cl |
| -O-(CH₂)₂-O- | CH₃ | H | CH₃ | CH₃ |
| -O-(CH₂)₂-O- | CH₃ | H | Cl | CH₃ |
| -O-(CH₂)₂-O- | Br | H | Cl | CH₃ |
| -O-(CH₂)₂-O- | Br | H | CH₃ | CH₃ |
| -O-(CH₂)₂-O- | Cl | H | Br | CH₃ |
| -O-(CH₂)₂-O- | Cl | H | Cl | CH₃ |
| -O-(CH₂)₂-O- | CH₃ | H | Br | CH₃ |
| -O-(CH₂)₂-O- | Cl | H | CH₃ | Cl |
| -O-(CH₂)₂-O- | CH₃ | H | H | CH₃ |
| -O-(CH₂)₂-O- | Cl | H | H | CH₃ |
| -O-(CH₂)₂-O- | Br | H | H | CH₃ |
| -O-(CH₂)₂-O- | CH₃ | H | H | Cl |
| -O-(CH₂)₂-O- | CH₃ | H | H | Br |
| -O-(CH₂)₂-O- | CH₃ | CH₃ | CH₃ | CH₃ |
| -O-(CH₂)₂-O- | CH₃ | CH₃ | CH₃ | F |
| -O-(CH₂)₂-O- | CH₃ | CH₃ | CH₃ | Cl |
| -O-(CH₂)₂-O- | CH₃ | CH₃ | CH₃ | Br |
| -O-(CH₂)₂-O- | CH₃ | CH₃ | H | Cl |
| -O-(CH₂)₂-O- | CH₃ | CH₃ | H | Br |
| -O-(CH₂)₂-O- | Cl | Cl | H | Br |
| -O-(CH₂)₂-O- | CH₃ | CH₃ | 4-Cl-C₆H₄ | H |
| -O-(CH₂)₂-O- | C₂H₅ | CH₃ | 4-Cl-C₆H₄ | H |
| -O-(CH₂)₂-O- | C₂H₅ | C₂H₅ | 4-Cl-C₆H₄ | H |
| -O-(CH₂)₂-O- | Cl | CH₃ | 4-Cl-C₆H₄ | H |
| -O-(CH₂)₂-O- | Cl | C₂H₅ | 4-Cl-C₆H₄ | H |
| -O-(CH₂)₂-O- | CH₃ | H | H | 4-Cl-C₆H₄ |
| -O-(CH₂)₂-O- | CH₃ | CH₃ | H | 4-Cl-C₆H₄ |
| -O-(CH₂)₂-O- | CH₃ | H | CH₃ | 4-Cl-C₆H₄ |
| -O-(CH₂)₂-O- | CH₃ | CH₃ | CH₃ | 4-Cl-C₆H₄ |
| -O-(CH₂)₂-O- | Cl | H | H | 4-Cl-C₆H₄ |
| -O-(CH₂)₂-O- | J | H | H | H |
| -O-(CH₂)₂-O- | J | H | CH₃ | H |
| -O-(CH₂)₂-O- | J | CH₃ | H | H |
| -O-(CH₂)₂-O- | J | C₂H₅ | H | H |
| -O-(CH₂)₂-O- | CH₃ | H | H | J |
| -O-(CH₂)₂-O- | CH₃ | H | CH₃ | J |
| -O-(CH₂)₂-O- | J | CH₃ | CH₃ | H |
| -O-(CH₂)₂-O- | J | C₂H₅ | CH₃ | H |
| -O-(CH₂)₂-O- | J | CH₃ | Cl | H |
| -O-(CH₂)₂-O- | J | C₂H₅ | Cl | H |
| -O-(CH₂)₂-O- | J | Cl | CH₃ | H |
| -O-(CH₂)₂-O- | J | H | CH₃ | CH₃ |
| -O-(CH₂)₂-O- | CH₃ | H | J | H |
| -O-(CH₂)₂-O- | C₂H₅ | H | J | H |
| -O-(CH₂)₂-O- | CH₃ | CH₃ | J | H |
| -O-(CH₂)₂-O- | C₂H₅ | CH₃ | J | H |
| -O-(CH₂)₂-O- | C₂H₅ | C₂H₅ | J | H |
| -O-(CH₂)₂-O- | Cl | CH₃ | J | H |
| -O-(CH₂)₂-O- | Cl | C₂H₅ | J | H |
| -O-(CH₂)₂-O- | CH₃ | H | J | CH₃ |
| -O-(CH₂)₂-O- | CH₃ | CH₃ | H | J |
| -O-(CH₂)₂-O- | J | H | H | CH₃ |

**Tabelle 18:** W, X, Y und Z wie in Tabelle 17 angegeben
A-B = 4'-O-CH₂-CHCH₃-O-

**Tabelle 19:** W, X, Y und Z wie in Tabelle 17 angegeben
A-B = 4'-O-CHCH₃-CHCH₃-O-

**Tabelle 20:** W, X, Y und Z wie in Tabelle 17 angegeben
A-B = 4'-O-(CH₂)₃-O-

**Tabelle 21:** W, X, Y und Z wie in Tabelle 17 angegeben
A-B = 4'-O-CHCH₃-(CH₂)₂-O-

**Tabelle 22:** W, X, Y und Z wie in Tabelle 17 angegeben
A-B = 4'-O-CHCH₃-CH₂-CHCH₃-O-

**Tabelle 23:** W, X, Y und Z wie in Tabelle 17 angegeben
A-B = 4'-O-CH₂-C(CH₃)₂-CH₂-O-

**Tabelle 24:** W, X, Y und Z wie in Tabelle 17 angegeben

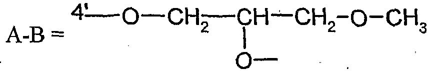

**Tabelle 25:** W, X, Y und Z wie in Tabelle 17 angegeben
A-B = 3'-O-(CH₂)₂-O-

**Tabelle 26:** W, X, Y und Z wie in Tabelle 17 angegeben
A-B = 3'-O-CH₂-CHCH₃-O-

**Tabelle 27:** W, X, Y und Z wie in Tabelle 17 angegeben
A-B = 3'-O-CHCH₃-CHCH₃-O-

**Tabelle 28:** W, X, Y und Z wie in Tabelle 17 angegeben
A-B = 3'-O-(CH₂)₃-O-

**Tabelle 29:** W, X, Y und Z wie in Tabelle 17 angegeben
A-B = 3'-O-CHCH₃-(CH₂)₂-O-

**Tabelle 30:** W, X, Y und Z wie in Tabelle 17 angegeben
A-B = 3'-O-CHCH₃-CH₂-CHCH₃-O-

**Tabelle 31:** W, X, Y und Z wie in Tabelle 17 angegeben
A-B = 3'-O-CH₂-C(CH₃)₂-CH₂-O-

**Tabelle 32:** W, X, Y und Z wie in Tabelle 17 angegeben

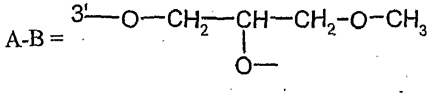

Bevorzugte Bedeutungen der oben in Zusammenhang mit den die Kulturpflanzen-Verträglichkeit verbessernden Verbindungen ("Herbizid-Safenern") der Formeln (IIa), (IIb), (IIc), (IId) und (IIe) aufgeführten Gruppen werden im Folgenden definiert.
- m: steht bevorzugt für die Zahlen 0, 1, 2, 3 oder 4.
- A¹: steht bevorzugt für eine der nachstehend skizzierten divalenten heterocyclischen Gruppierungen 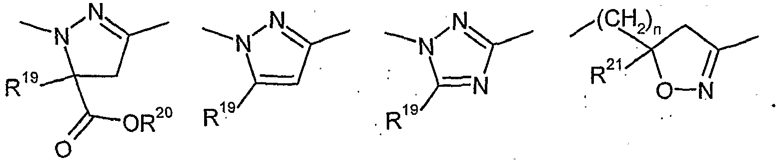
- n: steht bevorzugt für die Zahlen 0, 1, 2, 3 oder 4.
- A²: steht bevorzugt für jeweils gegebenenfalls durch Methyl, Ethyl, Methoxycarbonyl oder Ethoxycarbonyl oder Allyloxycarbonyl substituiertes Methylen oder Ethylen.
- R¹⁴: steht bevorzugt für Hydroxy, Mercapto, Amino, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁵: steht bevorzugt für Hydroxy, Mercapto, Amino, Methoxy, Ethoxy, n- oder i-Propoxy, n-, i-, s- oder t-Butoxy, 1-Methylhexyloxy, Allyloxy, 1-Allyloxymethyl-ethoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁶: steht bevorzugt für jeweils gegebenenfalls durch Fluor, Chlor und/oder Brom substituiertes Methyl, Ethyl, n- oder i-Propyl.
- R¹⁷: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl.
- R¹⁸: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl; Thiazolyl, Piperidinyl, oder gegebenenfalls durch Fluor, Chlor, Methyl, Ethyl, n- oder i-Propyl, n-, i-, s- oder t-Butyl substituiertes Phenyl, oder zusammen mit R¹⁷ für einen der Reste -CH₂-O-CH₂-CH₂- und -CH₂-CH₂-O-CH₂-CH₂-, die gegebenenfalls substituiert sind durch Methyl, Ethyl, Furyl, Phenyl, einen annellierten Benzolring oder durch zwei Substituenten, die gemeinsam mit dem C-Atom, an das sie gebunden sind, einen 5- oder 6-gliedrigen Carbocyclus bilden.
- R¹⁹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, Cyclopropyl, Cyclobutyl, Cyclopentyl, Cyclohexyl oder Phenyl.
- R²⁰: steht bevorzugt für Wasserstoff, gegebenenfalls durch Hydroxy, Cyano, Fluor, Chlor, Methoxy, Ethoxy, n- oder i-Propoxy substituiertes Methyl, Ethyl, n- oder i-Propyl, n-, i-, s- oder t-Butyl.
- R²¹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, n-, i-, s- oder t-Butyl, Cyclopropyl, Cyclobutyl, Cyclopentyl, Cyclohexyl oder Phenyl.
- X¹: steht bevorzugt für Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X²: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X³: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- t: steht bevorzugt für die Zahlen 0, 1, 2, 3 oder 4.
- v: steht bevorzugt für die Zahlen 0, 1, 2 oder 3.
- R²²: steht bevorzugt für Wasserstoff, Methyl, Ethyl, n- oder i-Propyl.
- R²³: steht bevorzugt für Wasserstoff, Methyl, Ethyl, n- oder i-Propyl.
- R²⁴: steht bevorzugt für Wasserstoff, jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, , Methylamino, Ethylamino, n- oder i-Propylamino, n-, i-, s- oder t-Butylamino, Dimethylamino oder Diethylamino, oder jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino oder Cyclohexylamino.
- R²⁵: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, oder jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl.
- R²⁶: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl, oder gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, oder zusammen mit R²⁵ für jeweils gegebenenfalls durch Methyl oder Ethyl substituiertes Butan-1,4-diyl (Trimethylen), Pentan-1,5-diyl, 1-Oxa-butan-1,4-diyl oder 3-Oxa-pentan-1,5-diyl.
- X⁴: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.
- X⁵: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

Beispiele für die als erfindungsgemäße Herbizid-Safener ganz besonders bevorzugten Verbindungen der Formel (IIa) sind in der nachstehenden Tabelle aufgeführt.

**Tabelle Beispiele für die Verbindungen der Formel (IIa)**

| | | | |
|---|---|---|---|
| 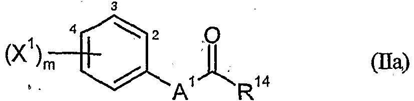 | | | |

| **Beispiel-Nr.** | **(Positionen) (X¹)ₘ** | **A¹** | **R¹⁴** |
|---|---|---|---|
| IIa-1 | (2) Cl, (4) Cl | 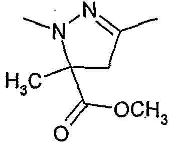 | OCH₃ |
| IIa-2 | (2) Cl, (4) Cl | 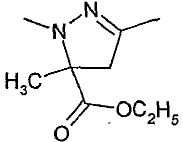 | OCH₃ |
| IIa-3 | (2) Cl, (4) Cl | 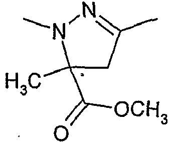 | OC₂H₅ |
| IIa-4 | (2) Cl, (4) Cl | 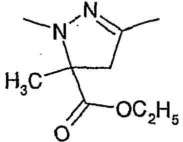 | OC₂H₅ |
| IIa-5 | (2) Cl | 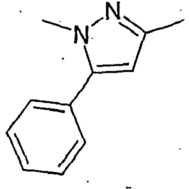 | OCH₃ |
| IIa-6 | (2) Cl, (4) Cl | 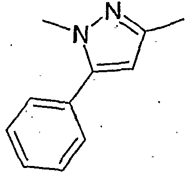 | OCH₃ |
| IIa-7 | (2) F | 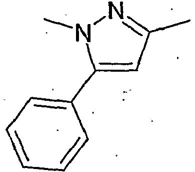 | OCH₃ |
| IIa-8 | (2) F | 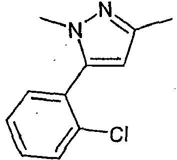 | OCH₃ |
| IIa-9 | (2) Cl, (4) Cl | 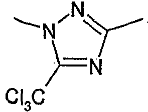 | OC₂H₅ |
| IIa-10 | (2) Cl, (4) CF₃ | 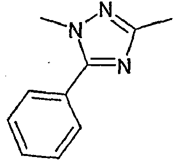 | OCH₃ |
| IIa-11 | (2) Cl | 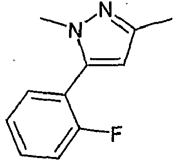 | OCH₃ |
| IIa-12 | - | 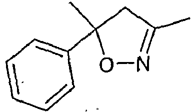 | OC₂H₅ |
| IIa-13 | (2) Cl, (4) Cl | 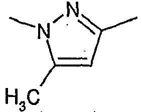 | OC₂H₅ |
| IIa-14 | (2) Cl, (4) Cl | 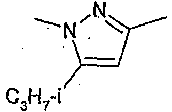 | OC₂H₅ |
| IIa-15 | (2) Cl, (4) Cl | 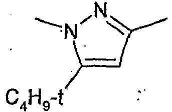 | OC₂H₅ |
| IIa-16 | (2) Cl, (4) Cl | 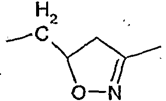 | OC₂H₅ |
| IIa-17 | (2) Cl, (4) Cl | 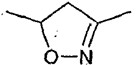 | OC₂H₅ |
| IIa-18 | - | 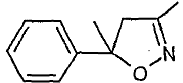 | OH |

Beispiele für die als erfindungsgemäße Herbizid-Safener ganz besonders bevorzugten Verbindungen der Formel (IIb) sind in der nachstehenden Tabelle aufgeführt.

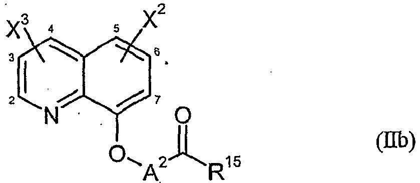

**Tabelle Beispiele für die Verbindungen der Formel (IIb)**

| **Beispiel-Nr.** | **(Position) X²** | **(Position) X³** | **A²** | **R¹⁵** |
|---|---|---|---|---|
| IIb-1 | (5) | - | CH₂ | OH |
| | Cl | | | |
| IIb-2 | (5) | - | CH₂ | OCH₃ |
| | Cl | | | |
| IIb-3 | (5) | - | CH₂ | OC₂H₅ |
| | Cl | | | |
| IIb-4 | (5) | - | CH₂ | OC₃H₇-n |
| | Cl | | | |
| IIb-5 | (5) | - | CH₂ | OC₃H₇-i |
| | Cl | | | |
| IIb-6 | (5) | - | CH₂ | OC₄H₉-n |
| | Cl | | | |
| IIb-7 | (5) | - | CH₂ | OCH(CH₃)C₅H₁₁-n |
| | Cl | | | |
| IIb-8 | (5) | (2) | CH₂ | OH |
| | Cl | F | | |
| IIb-9 | (5) | (2) | CH₂ | OH |
| | Cl | Cl | | |
| IIb-10 | (5) | - | CH₂ | OCH₂CH=CH₂ |
| | Cl | | | |
| IIb-11 | (5) | - | CH₂ | OC₄H₉-i |
| | Cl | | | |
| IIb-12 | (5) | - | CH₂ | 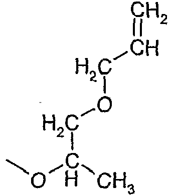 |
| | Cl | | | |
| IIb-13 | (5) | - | 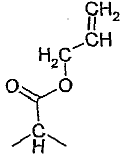 | OCH₂CH=CH₂ |
| | Cl | | | |
| IIb-14 | (5) | - | 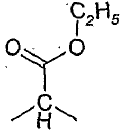 | OC₂H₅ |
| | Cl | | | |
| IIb-15 | (5) | - | 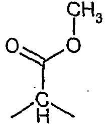 | OCH₃ |
| | Cl | | | |

Beispiele für die als erfindungsgemäße Herbizid-Safener ganz besonders bevorzugten Verbindungen der Formel (IIc) sind in der nachstehenden Tabelle aufgeführt.

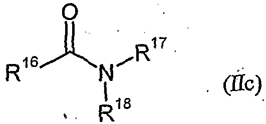

**Tabelle Beispiele für die Verbindungen der Formel (IIc)**

| **Beispiel-Nr.** | **R¹⁶** | **N(R¹⁷,R¹⁸)** |
|---|---|---|
| IIc-1 | CHCl₂ | N(CH₂CH=CH₂)₂ |
| IIc-2 | CHCl₂ | 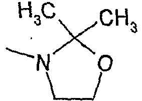 |
| IIc-3 | CHCl₂ | 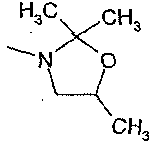 |
| IIc-4 | CHCl₂ | 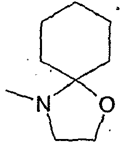 |
| IIc-5 | CHCl₂ | 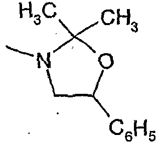 |
| IIc-6 | CHCl₂ | 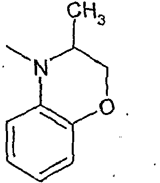 |
| IIc-7 | CHCl₂ | 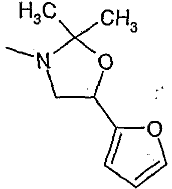 |

Beispiele für die als erfindungsgemäße Herbizid-Safener ganz besonders bevorzugten Verbindungen der Formel (IId) sind in der nachstehenden Tabelle aufgeführt.

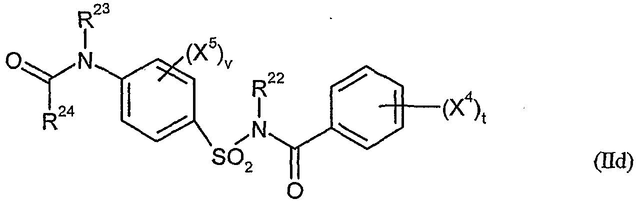

**Tabelle Beispiele für die Verbindungen der Formel (IId)**

| **Beispiel-Nr.** | **R²²** | **R²³** | **R²⁴** | **(Positionen) (X⁴)ₜ** | **(Positionen) (X⁵)ᵥ** |
|---|---|---|---|---|---|
| IId-1 | H | H | CH₃ | (2) OCH₃ | - |
| IId-2 | H | H | C₂H₅ | (2) OCH₃ | - |
| IId-3 | H | H | C₃H₇-n | (2) OCH₃ | - |
| IId-4 | H | H | C₃H₇-i | (2) OCH₃ | - |
| IId-5 | H | H | 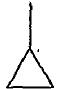 | (2) OCH₃ | - |
| IId-6 | H | H | CH₃ | (2) OCH₃ | - |
| | | | | (5) CH₃ | |
| IId-7 | H | H | C₂H₅ | (2) OCH₃ | - |
| | | | | (5) CH₃ | |
| IId-8 | H | H | C₃H₇-n | (2) OCH₃ | - |
| | | | | (5) CH₃ | |
| IId-9 | H | H | C₃H₇-i | (2) OCH₃ | - |
| | | | | (5) CH₃ | |
| IId-10 | H | H | 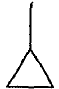 | (2) OCH₃ | - |
| | | | | (5) CH₃ | |
| IId-11 | H | H | OCH₃ | (2) OCH₃ | - |
| | | | | (5) CH₃ | |
| IId-12 | H | H | OC₂H₅ | (2) OCH₃ | - |
| | | | | (5) CH₃ | |
| IId-13 | H | H | OC₃H₇-i | (2) OCH₃ | - |
| | | | | (5) CH₃ | |
| IId-14 | H | H | SCH₃ | (2) OCH₃ | - |
| | | | | (5) CH₃ | |
| IId-15 | H | H | SC₂H₅ | (2) OCH₃ | - |
| | | | | (5) CH₃ | |
| IId-16 | H | H | SC₃H₇-i | (2) OCH₃ | - |
| | | | | (5) CH₃ | |
| IId-17 | H | H | NHCH₃ | (2) OCH₃ | - |
| | | | | (5) CH₃ | |
| IId-18 | H | H | NHC₂H₅ | (2) OCH₃ | - |
| | | | | (5) CH₃ | |
| IId-19 | H | H | NHC₃H₇-i | (2) OCH₃ | - |
| | | | | (5) CH₃ | |
| IId-20 | H | H | 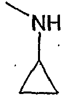 | (2) OCH₃ | - |
| | | | | (5) CH₃ | |
| IId-21 | H | H | NHCH₃ | (2) OCH₃ | - |
| IId-22 | H | H | NHC₃H₇-i | (2) OCH₃ | - |
| IId-23 | H | H | N(CH₃)₂ | (2) OCH₃ | - |
| IId-24 | H | H | N(CH₃)₂ | (3) CH₃ | - |
| | | | | (4) CH₃ | |
| IId-25 | H | H | CH₂-O-CH₃ | (2) OCH₃ | - |

Beispiele für die als erfindungsgemäße Herbizid-Safener ganz besonders bevorzugten Verbindungen der Formel (IIe) sind in der nachstehenden Tabelle aufgeführt.

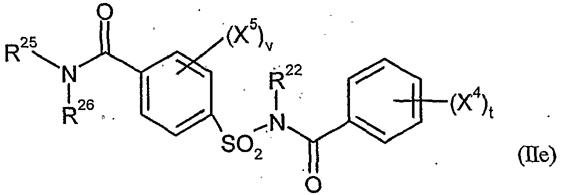

**Tabelle Beispiele für die Verbindungen der Formel (IIe)**

| **Beispiel-Nr.** | **R²²** | **R²⁵** | **R²⁶** | **(Positionen) (X⁴)ₜ** | **(Positionen) (X⁵)ᵥ** |
|---|---|---|---|---|---|
| IIe-1 | H | H | CH₃ | (2) OCH₃ | - |
| IIe-2 | H | H | C₂H₅ | (2) OCH₃ | - |
| IIe-3 | H | H | C₃H₇-n | (2) OCH₃ | - |
| IIe-4 | H | H | C₃H₇-i | (2) OCH₃ | - |
| IIe-5 | H | H | 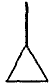 | (2) OCH₃ | - |
| IIe-6 | H | CH₃ | CH₃ | (2) OCH₃ | - |
| IIe-7 | H | H | CH₃ | (2) OCH₃ | - |
| | | | | (5) CH₃ | |
| IIe-8 | H | H | C₂H₅ | (2) OCH₃ | - |
| | | | | (5) CH₃ | |
| IIe-9 | H | H | C₃H₇-n | (2) OCH₃ | - |
| | | | | (5) CH₃ | |
| IIe-10 | H | H | C₃H₇-i | (2) OCH₃ | - |
| | | | | (5) CH₃ | |
| IIe-11 | H | H | 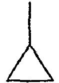 | (2) OCH₃ | - |
| | | | | (5) CH₃ | |
| IIe-12 | H | CH₃ | CH₃ | (2) OCH₃ | - |
| | | | | (5) CH₃ | |

Als die die Kulturpflanzen-Verträglichkeit verbessernde Verbindung [Komponente (b')] sind Cloquintocet-mexyl, Fenchlorazol-ethyl, Isoxadifen-ethyl, Mefenpyr-diethyl, Furilazole, Fenclorim, Cumyluron, Dymron, Dimepiperate und die Verbindungen IIe-5 und IIe-11 am meisten bevorzugt, wobei Cloquintocet-mexyl und Mefenpyr-diethyl besonders hervorgehoben seien.

Die als Safener erfindungsgemäß zu verwendenden Verbindungen der allgemeinen Formel (IIa) sind bekannt und/oder können nach an sich bekannten Verfahren hergestellt werden (vgl. WO-A-91/07874, WO-A-95/07897).

Die als Safener erfindungsgemäß zu verwendenden Verbindungen der allgemeinen Formel (IIb) sind bekannt und/oder können nach an sich bekannten Verfahren hergestellt werden (vgl. EP-A-191736).

Die als Safener erfindungsgemäß zu verwendenden Verbindungen der allgemeinen Formel (IIc) sind bekannt und/oder können nach an sich bekannten Verfahren hergestellt werden (vgl. DE-A-2218097, DE-A-2350547).

Die als Safener erfindungsgemäß zu verwendenden Verbindungen der allgemeinen Formel (IId) sind bekannt und/oder können nach an sich bekannten Verfahren hergestellt werden (vgl. DE-A-19621522/US-A-6235680).

Die als Safener erfindungsgemäß zu verwendenden Verbindungen der allgemeinen Formel (IIe) sind bekannt und können nach an sich bekannten Verfahren hergestellt werden (vgl. WO-A-99/66795/US-A-6251827).

Beispiele für die erfindungsgemäßen selektiv herbiziden Kombinationen aus jeweils einem Wirkstoff der Formel (I) und jeweils einem der oben definierten Safener sind in der nachstehenden Tabelle aufgeführt.

**Tabelle Beispiele für die erfindungsgemäßen Kombinationen**

| **Wirkstoffe der Formel (I)** | **Safener** |
|---|---|
| I-1-a | Cloquintocet-mexyl |
| I-1-a | Fenchlorazole-ethyl |
| I-1-a | Isoxadifen-ethyl |
| I-1-a | Mefenpyr-diethyl |
| I-1-a | Furilazole |
| I-1-a | Fenclorim |
| I-1-a | Cumyluron |
| I-1-a | Daimuron /Dymron |
| I-1-a | Dimepiperate |
| I-1-a | IIe-11 |
| I-1-a | IIe-5 |
| I-1-b | Cloquintocet-mexyl |
| I-1-b | Fenchlorazole-ethyl |
| I-1-b | Isoxadifen-ethyl |
| I-1-b | Mefenpyr-diethyl |
| I-1-b | Furilazole |
| I-1-b | Fenclorim |
| I-1-b | Cumyluron |
| 1-1-b | Daimuron /Dymron |
| I-1-b | Dimepiperate |
| I-1-b | IIe-11 |
| I-1-b | IIe-5 |
| I-1-c | Cloquintocet-mexyl |
| I-1-c | Fenchlorazole-ethyl |
| I-1-c | Isoxadifen-ethyl |
| I-1-c | Mefenpyr-diethyl |
| I-1-c | Furilazole |
| I-1-c | Fenclorim |
| I-1-c | Cumyluron |
| I-1-c | Daimuron /Dymron |
| I-1-c | Dimepiperate |
| I-1-c | IIe-5 |
| I-1-c | IIe-11 |
| I-1-d | Cloquintocet-mexyl |
| I-1-d | Fenchlorazole-ethyl |
| 1-1-d | Isoxadifen-ethyl |
| I-1-d | Mefenpyr-diethyl |
| I-1-d | Furilazole |
| I-1-d | Fenclorim |
| I-1-d | Cumyluron |
| I-1-d | Daimuron/Dymron |
| I-1-d | Dimepiperate |
| I-1-d | IIe-11 |
| I-1-d | IIe-5 |
| I-1-e | Cloquintocet-mexyl |
| I-1-e | Fenchlorazole-ethyl |
| I-1-e | Isoxadifen-ethyl |
| I-1-e | Mefenpyr-diethyl |
| I-1-e | Furilazole |
| I-1-e | Fenclorim |
| I-1-e | Cumyluron |
| I-1-e | Daimuron /Dymron |
| I-1-e | Dimepiperate |
| I-1-e | IIe-5 |
| I-1-e | IIe-11 |
| I-1-f | Cloquintocet-mexyl |
| I-1-f | Fenchlorazole-ethyl |
| I-1-f | Isoxadifen-ethyl |
| I-1-f | Mefenpyr-diethyl |
| I-1-f | Furilazole |
| I-1-f | Fenclorim |
| I-1-f | Cumyluron |
| I-1-f | Daimuron /Dymron |
| I-1-f | Dimepiperate |
| I-1-f | IIe-5 |
| I-1-f | IIe-11 |
| I-1-g | Cloquintocet-mexyl |
| I-1-g | Fenchlorazole-ethyl |
| I-1-g | Isoxadifen-ethyl |
| I-1-g | Mefenpyr-diethyl |
| I-1-g | Furilazole |
| I-1-g | Fenclorim |
| I-1-g | Cumyluron |
| I-1-g | Daimuron/Dymron |
| I-1-g | Dimepiperate |
| I-1-g | IIe-5 |
| I-1-g | IIe-11 |
| I-2-a | Cloquintocet-mexyl |
| I-2-a | Fenchlorazole-ethyl |
| I-2-a | Isoxadifen-ethyl |
| I-2-a | Mefenpyr-diethyl |
| I-2-a | Furilazole |
| I-2-a | Fenclorim |
| I-2-a | Cumyluron |
| I-2-a | Daimuron /Dymron |
| I-2-a | Dimepiperate |
| I-2-a | IIe-5 |
| I-2-a | IIe-11 |
| I-2-b | Cloquintocet-mexyl |
| I-2-b | Fenchlorazole-ethyl |
| I-2-b | Isoxadifen-ethyl |
| I-2-b | Mefenpyr-diethyl |
| I-2-b | Furilazole |
| I-2-b | Fenclorim |
| I-2-b | Cumyluron |
| I-2-b | Daimuron /Dymron |
| I-2-b | Dimepiperate |
| I-2-b | IIe-5 |
| I-2-b | IIe-11 |
| I-2-c | Cloquintocet-mexyl |
| I-2-c | Fenchlorazole-ethyl |
| I-2-c | Isoxadifen-ethyl |
| I-2-c | Mefenpyr-diethyl |
| I-2-c | Furilazole |
| I-2-c | Fenclorim |
| I-2-c | Cumyluron |
| I-2-c | Daimuron /Dymron |
| I-2-c | Dimepiperate |
| I-2-c | IIe-5 |
| I-2-c | IIe-11 |
| I-2-d | Cloquintocet-mexyl |
| I-2-d | Fenchlorazole-ethyl |
| I-2-c | Isoxadifen-ethyl |
| I-2-d | Mefenpyr-diethyl |
| I-2-d | Furilazole |
| I-2-d | Fenclorim |
| I-2-d | Cumyluron |
| I-2-d | Daimuron /Dymron |
| I-2-d | Dimepiperate |
| I-2-d | IIe-5 |
| I-2-d | IIe-11 |
| I-2-e | Cloquintocet-mexyl |
| I-2-e | Fenchlorazole-ethyl |
| I-2-e | Isoxadifen-ethyl |
| I-2-e | Mefenpyr-diethyl |
| I-2-e | Furilazole |
| I-2-e | Fenclorim |
| I-2-e | Cumyluron |
| I-2-e | Daimuron /Dymron |
| I-2-e | Dimepiperate |
| I-2-e | IIe-5 |
| I-2-e | IIe-11 |
| I-2-f | Cloquintocet-mexyl |
| I-2-f | Fenchlorazole-ethyl |
| I-2-f | Isoxadifen-ethyl |
| I-2-f | Mefenpyr-diethyl |
| I-2-f | Furilazole |
| I-2-f | Fenclorim |
| I-2-f | Cumyluron |
| I-2-f | Daimuron /Dymron |
| I-2-f | Dimepiperate |
| I-2-f | IIe-5 |
| I-2-f | IIe-11 |
| I-2-g | Cloquintocet-mexyl |
| I-2-g | Fenchlorazole-ethyl |
| I-2-g | Isoxadifen-ethyl |
| I-2-g | Mefenpyr-diethyl |
| I-2-g | Furilazole |
| I-2-g | Fenclorim |
| I-2-g | Cumyluron |
| I-2-g | Daimuron/Dymron |
| I-2-g | Dimepiperate |
| I-2-g | IIe-5 |
| I-2-g | IIe-11 |

Es wurde nun überraschend gefunden, dass die oben definierten Wirkstoffkombinationen aus Verbindungen, der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von substituierten cyclischen Ketoenolen auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Verwendet man beispielsweise gemäß Verfahren (A) N-[(4-Chlor-2,6-dimethyl)-phenylacetyl]-1-amino-4,4'-propylendioxy-cyclohexancarbonsäureethylester als Ausgangsstoff, so kann der Verlauf des erfindungsgemäßen Verfahrens durch folgendes Reaktionsschema wiedergegeben werden:

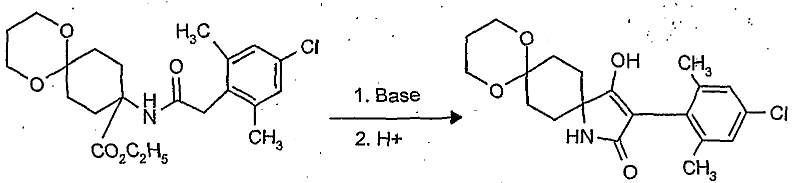

Verwendet man beispielsweise gemäß Verfahren (B) O-[(2-Chlor-6-methyl)-phenylacetyl]-1-hy-droxy-4,4'-ethylendioxy-cyclohexancarbonsäureethylester, so kann der Verlauf des erfindungsgemäßen Verfahrens durch folgendes Reaktionsschema wiedergegeben werden:

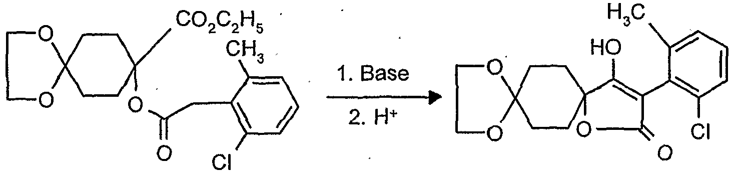

Verwendet man beispielsweise gemäß Verfahren (Cα) 8,8'-Ethylendioxy-3-[(4-chlor-2,6-dimethyl)-phenyl]-1-azaspiro[4,5]decan-2,4-dion und Pivaloylchlorid als Ausgangsstoffe, so kann der Verlauf des erfindungsgemäßen Verfahrens durch folgendes Reaktionsschema wiedergegeben werden:

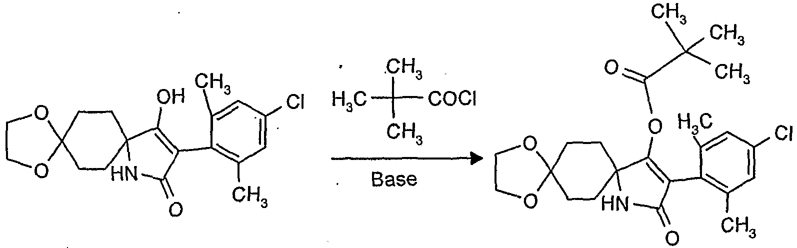

Verwendet man beispielsweise gemäß Verfahren (C) (Variante β) 8,8'-Propylendioxy-3-[(2,4-dichlor)-phenyl]-1-oxaspiro-[4,5]-decan-2,4-dion und Acetanhydrid als Ausgangsverbindungen, so kann der Verlauf des erfindungsgemäßen Verfahrens durch folgendes Reaktionsschema wiedergegeben werden:

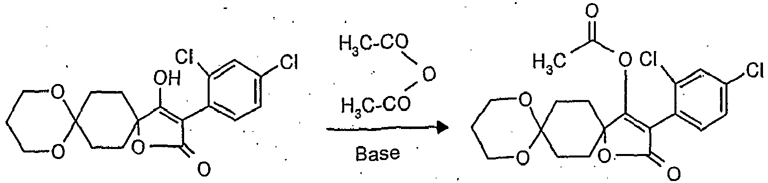

Verwendet man beispielsweise gemäß Verfahren (D) 8,8'-Propylendioxy-3-[(2,4-dichlor-6-methyl)-phenyl]-1-azaspiro[4,5]decan-2,4-dion und Chlorameisensäureethylester als Ausgangsverbindungen, so kann der Verlauf des erfindungsgemäßen Verfahrens durch folgendes Reaktionsschema wiedergegeben werden:

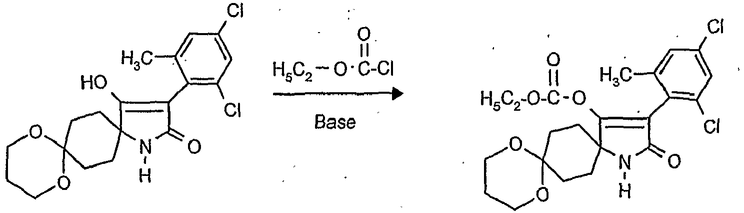

Verwendet man beispielsweise gemäß Verfahren (E) 8,8'-Ethylendioxy-3-[(2,4,6-trimethyl)-phe-nyl]-1-oxaspiro[4,5]decan-2,4-dion und Chlormonothioameisensäuremethylester als Ausgangsprodukte, so kann der Reaktionsverlauf folgendermaßen wiedergegeben werden:

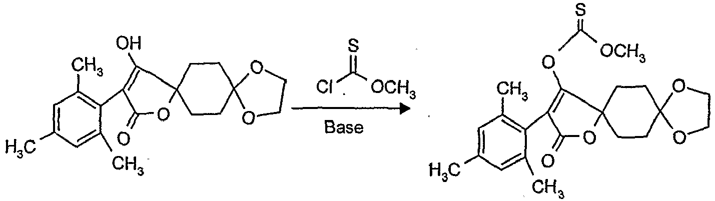

Verwendet man beispielsweise gemäß Verfahren (F) 8,8'-Propylendioxy-3-[(2,4,6-trimethyl)-phenyl]-1-azaspiro[4,5]decan-2,4-dion und Methansulfonsäurechlorid als Ausgangsprodukt, so kann der Reaktionsverlauf durch folgendes Reaktionsschema wiedergegeben werden:

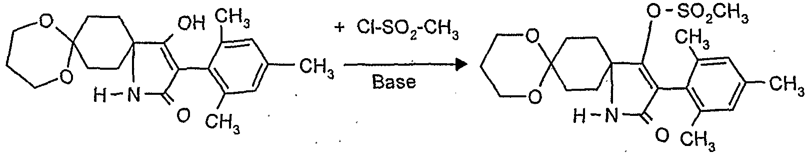

Verwendet man beispielsweise gemäß Verfahren (G) 8,8'-Propylendioxy-3-[(2,4-dichlor-6-methyl)-phenyl]-1-oxaspiro[4,5]decan-2,4-dion und Methanthio-phosphonsäurechlorid-(2,2,2-tri-fluorethylester) als Ausgangsprodukte, so kann der Reaktionsverlauf durch folgendes Reaktionsschema wiedergegeben werden:

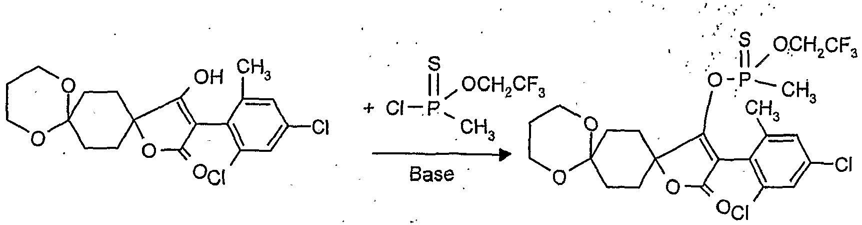

Verwendet man beispielsweise gemäß Verfahren (H) 8,8'-Ethylendioxy-3-[(2,3,4,6-tetramethyl-phenyl]-1-azapiro[4,5]decan-2,4-dion und NaOH als Komponenten, so kann der Verlauf des erfindungsgemäßen Verfahrens durch folgendes Reaktionsschema wiedergegeben werden:

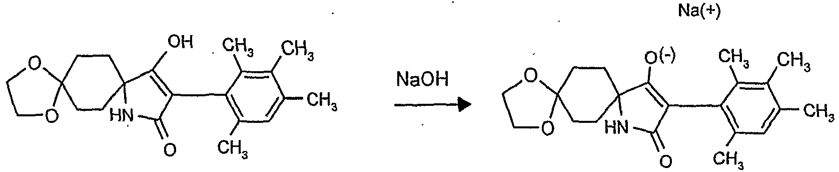

Verwendet man beispielsweise gemäß Verfahren (I) (Variante α) 8,8'-Ethylendioxy-3-[(2,4,5-trimethyl)-phenyl]-1-oxaspiro[4,5]decan-2,4-dion und Ethylisocyanat als Ausgangsprodukte, so kann der Reaktionsverlauf durch folgendes Reaktionsschema wiedergegeben werden:

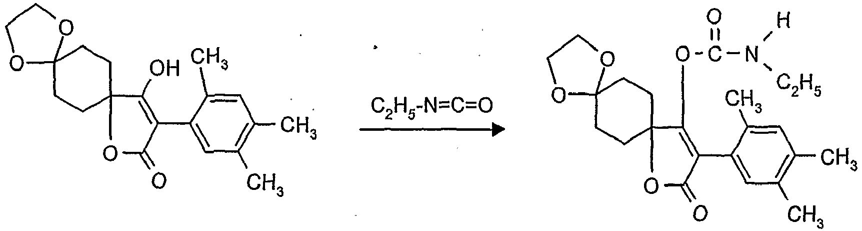

Verwendet man beispielsweise gemäß Verfahren (I) (Variante β) 8,8'-Ethylendioxy-3-[(2-4,6-trimethyl)-phenyl]-1-azaspiro[4,5]decan-2,4-dion und Dimethylcarbamidsäurechlorid als Ausgangsprodukte, so kann der Reaktionsverlauf durch folgendes Schema wiedergegeben werden:

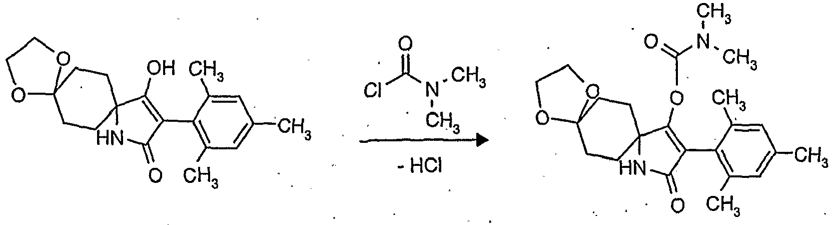

Die beim erfindungsgemäßen Verfahren (A) als Ausgangsstoffe benötigten Verbindungen der Formel (II) 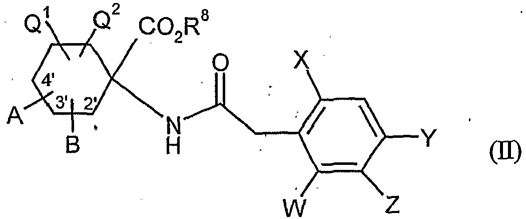 in welcher
- A, B, Q¹, Q², W, X, Y, Z und R⁸: die oben angegebenen Bedeutungen haben,
sind neu.

Man erhält die Acylaminosäureester der Formel (II) beispielsweise, wenn man Aminosäurederivate der Formel (XVI) 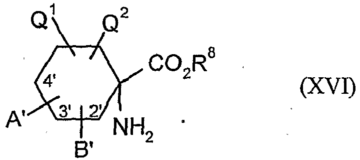 in welcher
- A, B, Q¹ und Q² und R⁸: die oben angegebene Bedeutung haben,
mit substituierten Phenylessigsäurederivaten der Formel (XVD) 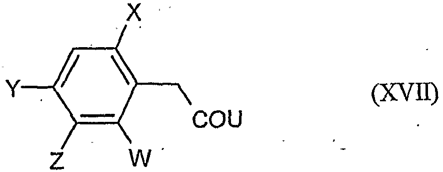 in welcher
- W, X, Y und Z: die oben angegebenen Bedeutungen haben und
- U: für eine durch Carbonsäureaktivierungsreagenzien wie Carbonyldiimidazol, Carbonyl-diimide (wie z.B. Dicyclohexylcarbondiimid), Phosphorylierungsreagonzien (wie z.B. POCl₃, BOP-Cl), Halogenierungsmittel wie z.B. Thionylchlorid, Oxalylchlorid, Phosgen oder Chlorameisensäureester eingeführte Abgangsgruppe steht,
acyliert (Chem. Reviews 52, 237-416 (1953); Bhattacharya, Indian J. Chem. 6, 341-5, 1968)
oder wenn man Acylaminosäuren der Formel (XVIII) 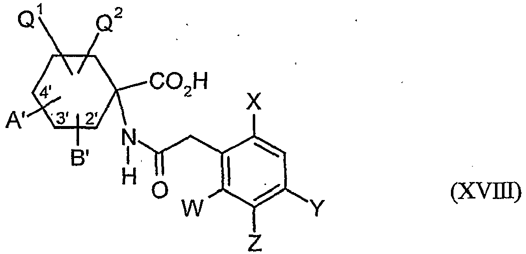 in welcher
- A, B, Q¹, Q², W, X, Y und Z: die oben angegebenen Bedeutungen haben,
verestert (Chem. Ind. (London) 1568 (1968)).

Die Verbindungen der Formel (XVIII) 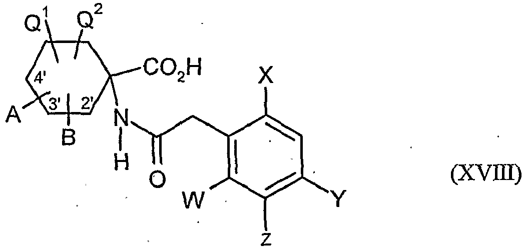 in welcher
- A, B, Q¹, Q², W, X, Y und Z: die oben angegebenen Bedeutungen haben,
sind neu.

Man erhält die Verbindungen der Formel (XVIII) beispielsweise, wenn man 1-Amino-cyclohexan-carbonsäuren der Formel (XIX) 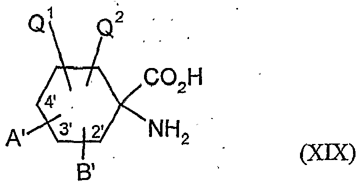 in welcher
- A, B, Q¹ und Q²: die oben angegebenen Bedeutungen haben
mit substituierten Phenylessigsäurederivaten der Formel (XVII) 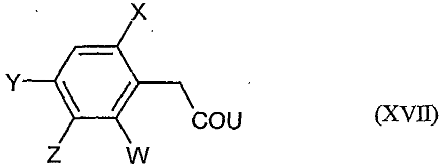 in welcher
- U, W, X, Y und Z: die oben angegebenen Bedeutungen haben und
z.B. nach Schotten-Baumann acyliert (Organikum, VEB Deutscher Verlag der Wissenschaften, Berlin 1977, S. 505).

Die Verbindungen der Formel (XVII) sind teilweise bekannt und/oder lassen sich nach den bekannten Verfahren in den eingangs zitierten Offenlegungsschriften herstellen.

Die Verbindungen der Formel (XVI) und (XIX) sind teilweise neu und lassen sich nach bekannten Verfahren darstellen (siehe z.B. Compagnon, Ann. Chim. (Paris) [14] 5, S. 11-22, 23-27 (1970), L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 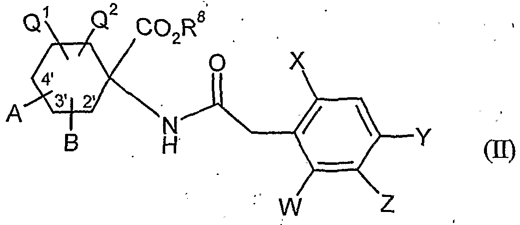 in welcher
- A, B, Q¹, Q², W, X, Y, Z und R⁸: die oben angegebenen Bedeutungen haben,
herstellen, wenn man 1-Amino-cyclohexan-carbonsäurenitrile der Formel (XX) 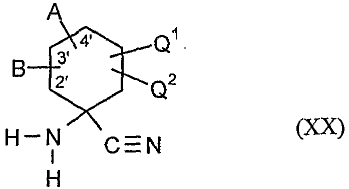 in welcher
- A, B, Q¹ und Q²: die oben angegebenen Bedeutungen haben,
mit substituierten Phenylessigsäurederivaten der Formel (XVII) 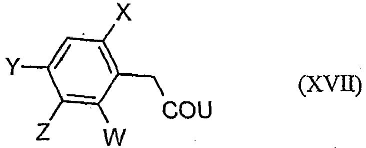 in welcher
- U, W, X, Y und Z: die oben angegebenen Bedeutungen haben,
zu Verbindungen, der Formel (XXI) 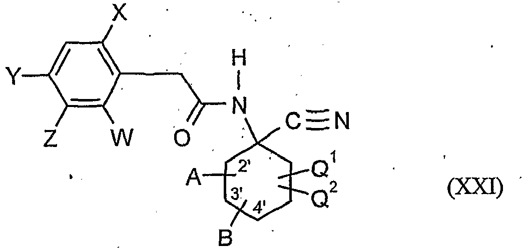 in welcher
- A, B, Q¹, Q², W, X, Y und Z: die oben angegebenen Bedeutungen haben,
umsetzt,
und diese anschließend einer sauren Alkoholyse unterwirft.

Die Verbindungen der Formel (XXI) sind ebenfalls neu. Die Verbindungen der Formel (XX) sind teilweise neu und lassen sich z.B. wie in EP-A-595 130 beschrieben herstellen.

Die bei dem erfindungsgemäßen Verfahren (B) als Ausgangstoffe benötigten Verbindungen der Formel (III) 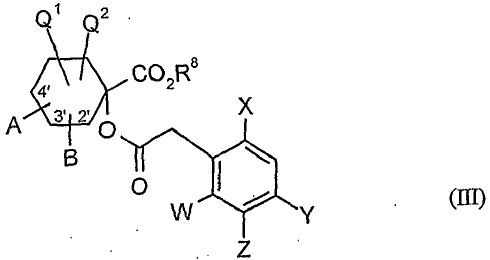 in welcher
- A, B, Q¹, Q², W, X, Y, Z und R⁸: die oben angegebenen Bedeutungen haben,
sind neu.

Sie lassen sich nach im Prinzip bekannten Methoden in einfacher Weise herstellen.

Man erhält die Verbindungen der Formel (III) beispielsweise, wenn man 1-Hydroxy-cyclohexan-carbonsäureester der Formel (XXII) 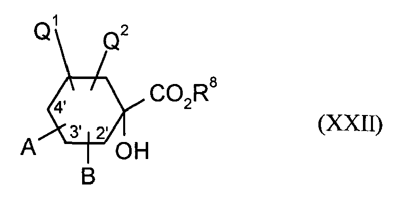 in welcher
- A, B, Q¹, Q² und R⁸: die oben angegebenen Bedeutungen haben,
substituierten Phenylessigsäurederivaten der Formel (XVII) 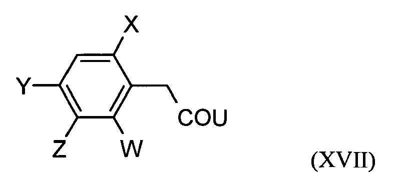 in welcher
- U, W, X, Y und Z: die oben angegebenen Bedeutungen haben,
acyliert (Chem. Reviews 52, 237-416 (1953)).

Die 1-Hydroxy-3-alkoxy-cyclohexyl-carbonsäureester der Formel (XXII) sind neu, ausgenommen die Verbindung 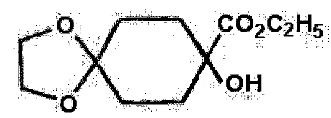 Man erhält sie beispielsweise, indem man substituierte 1-Hydroxy-4,4'-alkylidenyldioxy-cyclohexan-carbonsäurenitrile in Gegenwart von Säuren, z.B. nach Pinner mit Alkoholen umsetzt und die entstandenen 1-Hydroxy-4-oxo-cylohexancarbonsäureester bekannt aus WO 99/16748 (Bsp. XXI-1) mit Diolen erneut ketalisiert. Das Cyanhydrin erhält man beispielsweise durch Umsetzung von substituierten 4,4'-Alkylidenyldioxy-cyclohexan-1-onen mit Blausäure (s. WO 99/16748).

Die zur Durchführung der erfindungsgemäßen Verfahren (C), (D), (E), (F), (G), (H), (I) und (J) außerdem als Ausgangsstoffe benötigten Säurehalogenide der Formel (IV), Carbonsäureanhydride der Formel (V), Chlorameisensäureester oder Chlorameisensäurethioester der Formel (VI), Chlormonothioameisensäureester oder Chlordithioameisensäureester der Formel (VII), Sulfonsäurechloride der Formel (VIII), Phosphorverbindungen der Formel (IX) und Metallhydroxide, Metallalkoxide oder Amine der Formel (X) und (XI) und Isocyanate der Formel (XII) und Carbamidsäurechloride der Formel (XIII) und Diole der Formel (XV) sind allgemein bekannte Verbindungen der organischen bzw. anorganischen Chemie. Die Verbindungen der Formel (XIV) sind teilweise bekannt aus WO 99/16748.

Die Verbindungen der Formeln (XVII) sind darüber hinaus aus den eingangs zitierten Patentanmeldungen bekannt und/oder lassen sich nach den dort angegebenen Methoden herstellen.

Das Verfahren (A) ist dadurch gekennzeichnet, dass man Verbindungen der Formel (II), in welcher A, B, Q¹, Q², W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkätalysatoren wie z.B. Triethylbenzylammoniumchlorid, Tetrabutylammoniumbromid, Adogen 464 (=Methyltrialkyl(C₈-C₁₀)ammoniumchlorid) oder TDA 1 (=Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen -75°C und 200°C, vorzugsweise zwischen -50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppeltäquimolaren Mengen ein. Es ist jedoch auch möglich, die eine oder andere Komponente in einem größeren Überschuss (bis zu 3 Mol) zu verwenden.

Das Verfahren (B) ist dadurch gekennzeichnet, dass man Verbindungen der Formel (III), in welcher A, B, Q¹, Q², W, X, Y, Z und R⁸ die oben angegebenen Bedeutungen haben, in Gegenwart eines Verdünnungsmittels und in Gegenwart einer Base intramolekular kondensiert.

Als Verdünnungsmittel können bei dem erfindungsgemäßen Verfahren (B) alle gegenüber den Reaktionsteilnehmern inerten organischen Solventien eingesetzt werden. Vorzugsweise verwendbar sind Kohlenwasserstoffe, wie Toluol und Xylol, ferner Ether, wie dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (B) innerhalb eines größeren Bereiches variiert werden. Im allgemeinen arbeitet man bei Temperaturen zwischen -75°C und 200°C, vorzugsweise zwischen -50°C und 150°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuss (bis zu 3 Mol) zu verwenden.

Das Verfahren (C_{α}) ist dadurch gekennzeichnet, dass man Verbindungen der Formeln (I-1-a) bis (I-2-a) jeweils mit Carbonsäurehalogeniden der Formel (IV) gegebenenfalls in Gegenwart einer Verdünnungsmittels und gegebenenfalls in Gegenwart eines Säurebindemittels umsetzt.

Als Verdünnungsmittel können bei dem erfindungsgemäßen Verfahren (C_{α}) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (C_{α}) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (C_{α}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (C_{α}) werden die Ausgangsstoffe der Formeln (I-1-a) bis (I-2-a) und das Carbonsäurehalogenid der Formel (IV) im allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (C_{ß}) ist dadurch gekenntzeichnet, dass man Verbindungen der Formeln (I-1-a) bis (I-2-a) jeweils mit Carbonsäureanhydriden der Formel (V) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (C_{ß}) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (C_{ß}) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (C_{ß}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (C_{ß}) werden die Ausgangsstoffe der Formeln (I-1-a) bis (I-2-a) und das Carbonsäureanhydrid der Formel (V) im allgemeinen in jeweils angenähert äquivalenten Mengen verwender. Es ist jedoch auch möglich, das Carbonsäureanhydrid in einem größeren Überschuss (bis zu 5 Mol) einzusetzen. Die Aufarbeitung erfolgt nach üblichen Methoden.

Im allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (D) ist dadurch gekennzeichnet, dass man Verbindungen der Formeln (I-1-a) bis (I-2-a) jeweils mit Chlorameisensäureestern oder Chlorameisensäurethiolestern der Formel (VI) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (D) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (D) alle gegenüber den Chlorameisensäureestern bzw. Chlorameisensäurethiolestern inerten Solventien eingesetzt werden. Vorzugsweise- verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (D) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (D) wird im allgemeinen unter Normaldruck durchgeführt.

Bei der Durchführung des erfindungsgemäßen Verfahrens (D) werden die Ausgangsstoffe der Formeln (I-1-a) bis (I-2-a) und der entsprechende Chlorameisensäureester bzw. Chlorameisensäurethiolester der Formel (VI) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im allgemeinen geht man so vor, dass man ausgefallene Salze entfernt und das verbleibende Reaktionsgemisch durch Abziehen des Verdünnungsmittels einengt.

Das erfindungsgemäße Verfahren (E) ist dadurch gekennzeichnet, dass man Verbindungen der Formeln (I-1-a) bis (I-2-a) jeweils mit Verbindungen der Formel (VII) in Gegenwart eines Verdünnungsmittels und gegebenenfalls in Gegenwart eines Säurebindemittels umsetzt.

Beim Herstellungsverfahren (E) setzt man pro Mol Ausgangsverbindung der Formeln (I-1-a) bis (I-2-a) ca. 1 Mol Chlormonothioameisensäureester bzw. Chlordithioameisensäureester der Formel (VII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid, Essigsäureethylester oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-2-a) dar, kann auf den weiteren Zusatz von Säurebindemitteln verzichtet werden.

Als Basen können beim Verfahren (E) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (F) ist dadurch gekennzeichnet, dass man Verbindungen der Formeln (I-1-a) bis (I-2-a) jeweils mit Sulfonsäurechloriden der Formel (VIII) gegebenenfalls in Gegenwart eines Verdünnungsmittels und gegebenenfalls in Gegenwart eines Säurebindemittels umsetzt.

Beim Herstellungsverfahren (F) setzt man pro Mol Ausgangsverbindung der Formel (I-1-a) bis (I-2-a) ca. 1 Mol Sulfonsäurechlorid der Formel (VIII) bei -20 bis 150°C, vorzugsweise bei 0 bis 70°C um.

Das Verfahren (F) wird vorzugsweise in Gegenwart eines Verdünnungsmittels durchgeführt.

Als Verdünnungsmittel kommen alle inerten polaren organischen Lösungsmittel in Frage wie Ether, Amide, Ketone, Carbonsäureester, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Essigsäureethylester, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-2-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (G) ist dadurch gekennzeichnet, dass man Verbindungen der Formeln (I-1-a) bis (I-2-a) jeweils mit Phosphorverbindungen der Formel (IX) gegebenenfalls in Gegenwart eines Verdünnungsmittels und gegebenenfalls in Gegenwart eines Säurebindemittels umsetzt.

Beim Herstellungsverfahren (G) setzt man zum Erhält von Verbindungen der Formeln (I-1-e) bis (I-2-e) auf 1 Mol der Verbindungen, (I-1-a) bis (I-2-a), 1 bis 2, vorzugsweise 1 bis 1,3 Mol der Phosphorverbindung der Formel (IX) bei Temperaturen zwischen -40°C und 150°C, vorzugsweise zwischen -10 und 110°C um.

Das Verfahren (G) wird vorzugsweise in Gegenwart eines Verdünnungsmittels durchgeführt.

Als Verdünnungsmittel kommen alle inerten, polaren organischen Lösungsmittel in Frage wie Ether, Carbonsäureester, halogenierte Kohlenwasserstoffe, Ketone, Amide, Nitrile, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chromatographische Reinigung oder durch sogenanntes "Andestillieren", d,h, Entfernung der flüchtigen Bestandteile im Vakuum gereinigt.

Das Verfahren (H) ist dadurch gekennzeichnet, dass man Verbindungen der Formeln (I-1-a) bis (I-2-a) jeweils mit Metallhydroxiden bzw. Metallalkoxiden der Formel (X) oder Aminen der Formel (XI), gegebenenfalls in Gegenwart eines Verdünnungsmittels, umsetzt.

Als Verdünnungsmittel können bei dem erfindungsgemäßen Verfahren (H) vorzugsweise Ether wie Tetrahydrofuran, Dioxan, Diethylether oder aber Alkohole wie Methanol, Ethanol, Iso-propanol, aber auch Wasser eingesetzt werden. Das erfindungsgemäße Verfahren (H) wird im allgemeinen unter Normaldruck durchgeführt. Die Reaktionstemperatur liegt im allgemeinen zwischen -20°C und 100°C, vorzugsweise zwischen 0°C und 50°C.

Das erfindungsgemäße Verfahren (I) ist dadurch gekennzeichnet, dass man Verbindungen der Formeln (I-1-a) bis (I-2-a) jeweils mit (Iα) Verbindungen der Formel (XII) gegebenenfalls in Gegenwart eines Verdünnungsmittels und gegebenenfalls in Gegenwart eines Katalysators oder (Iß) mit Verbindungen der Formel (XIII) gegebenenfalls in Gegenwart eines Verdünnungsmittels und gegebenenfalls in Gegenwart eines Säurebindemittels umsetzt.

Bei Herstellungsverfahren (Iα) setzt man pro Mol Ausgangsverbindung der Formeln (I-1-a) bis (I-2-a) ca. 1 Mol Isocyanat der Formel (XII) bei 0 bis 100°C, vorzugsweise bei 20 bis 50°C um.

Das Verfahren (Iα) wird vorzugsweise in Gegenwart eines Verdünnungsmittels durchgeführt.

Als Verdünnungsmittel kommen alle inerten organischen Lösungsmittel in Frage, wie aromatische Kohlenwasserstoffe, halogenierte Kohlenwasserstoffe, Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet

Beim Herstellungsverfahren (Iß) setzt man pro Mol Ausgangsverbindung der Formeln (I-1-a) bis (I-2-a) ca. 1 Mol Carbamidsäurechlorid der Formel (XIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Carbonsäureester, Nitrile, Ketone, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) bis (I-2-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (J) setzt man pro Mol der Ausgangsverbindungen (I-1-a) bis (I-1-g) oder (I-2-a) bis (I-2-g) bis zu 50 Mol Diol der Formel (XVI), bevorzugt 1 bis 10 Mol bei -50 bis 250°C, bevorzugt 0 bis 150°C, um.

Als Verdünnungsmittel kommen alle inerten, organischen Lösungsmittel in Frage wie aromatische Kohlenwasserstoffe, halogenierte Kohlenwasserstoffe Ether, Ester, Amide, Nitrile, Sulfone oder Sulfoxide.

Gegebenenfalls können Katalysatoren zur Beschleunigung der Reaktion zugesetzt werden. Als Katalysatoren können sehr vorteilhaft Säuren wie z.B. p-Toluolsulfonsäure, konz. Schwefelsäure aber auch Lewis-Säuren wie z.B. Bortrifluoretherat eingesetzt werden.

Es wird vorzugsweise bei Normaldruck gearbeitet.

Die wasserentziehenden Bedingungen lassen sich sowohl durch azeotrope Entfernung des Wassers als auch durch Zugabe geeigneter wasserentziehender Reagenzien wie z.B. Ameisensäureorthoester, Dimethoxypropan aber auch Molekularsieb erzielen.

Die Aufarbeitung geschieht nach den üblichen Methoden.

Die erfindungsgemäßen Wirkstoffe/Wirkstoffkombinationen eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp.

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp.

Aus der Ordnung der Chilopoda z.B. Geophilus spp., Scutigera spp.

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus, spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Promnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa.

Aus der Klasse der Gastropoda z.B. Arion spp., Biomphalaria spp., Bulinus spp., Deroceras spp., Galba spp., Lymnaea spp., Oncomelania spp., Succinea spp.

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenotepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B: Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotmophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp.

Aus der Ordnung der Isopoda z.B. Armadillidium vulgare, Oniscus asellus, Porcellio scaber.

Aus der Ordnung der Isoptera z.B. Reticulitermes spp.

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Rhipiphorothrips cruentatus, Scirtothrips spp., Taeniothrips cardamoni, Thrips spp.

Aus der Ordnung der Thysanura z.B. Lepisma saccharina.

Zu den pflanzenparasitären Nematoden gehören z.B. Aphelenchoides spp., Bursaphelenchus spp., Ditylenchus dipsaci, Globodera spp., Heterodera spp., Longidorus spp., Meloidogyne spp., Pratylenchus spp., Radopholus similis, Trichodorus spp., Tylenchulus semipenetrans, Xiphinema spp.

Die erfindungsgemäßen Verbindungen/Wirkstoffkombinationen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen/Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Samen, weiterhin durch ein- oder mehrschichtiges Umhüllen.

Die Wirkstoffe/Wirkstoffkombinationen können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-. Konzentrate, Wirkstoff-imprägnierte Naturstoffe, Wirkstoffi-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe/Wirkstoffkombinationen mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyolohexanon, stark polare Lösungsmittel, wie Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gew.% Wirkstoff, vorzugsweise zwischen 0,5 und 90 %.

Der erfindungsgemäße Wirkstoff/Wirkstoffkombinationen kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Besonders günstige Mischpartner sind z.B. die folgenden:

### Fungizide:

2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesilate); Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothalisopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-chlorophenyl)-2-propynyl]oxy]-3-methoxyphenyl]-ethyl]-3-methyl-2-[(methylsulfonyl)amino]-butanamide; 1-(1-naphthalenyl)-1H-pyrrole-2,5-dione; 2,3,5,6-tetrachloro-4-(methylsulfonyl)-pyridine; 2-amino-4-methyl-N-phenyl-5-thiazole-carboxamide; 2-chloro-N-(2,3-dihydro-1,1,3-trimethyl-1H-inden-4-yl)-3-pyridincarboxamide; 3,4,5-trichloro-2,6-pyridinedicarbonitrile; Actinovate; cis-1-(4-chlorophenyl)-2-(1H-1,2,4-triazole-1-yl)-cycloheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine; Sodium tetrathiocarbonate; sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxine-copper.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

### Acetylcholinesterase (AChE) Inhibitoren

1.1 Carbamate,
   zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
1.2 Organophosphate,
   zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion

### Natrium-Kanal-Modulatoren / Spannungsabhängige Natrium-Kanal-Blocker

2.1 Pyrethroide,
   zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum)
   DDT
2.2 Oxadiazine,
   zum Beispiel Indoxacarb
2.3 Semicarbazone
   zum Beispiel Metaflumizone (BAS 3201)

### Acetylcholin-Rezeptor-Agonisten/-Antagonisten

3.1 Chloronicotinyle,
   zum Beispiel Acetamiprid, Clothianidin, Dinotefuran, Imidacloprid, Nitenpyram, Nithiazine, Thiacloprid, Thiamethoxam
   3.2 Nicotine, Bensultap, Cartap

### Acetylcholin-Rezeptor-Modulatoren

4.1 Spinosyne,
   zum Beispiel Spinosad

### GABA-gesteuerte Chlorid-Kanal-Antagonisten

5.1 Organochlorine,
   zum Beispiel Camphechlor, Chlordane, Endosulfan, Gamma-HCH, HCH, Heptachlor, Lindane, Methoxychlor
5.2 Fiprole,
   zum Beispiel Acetoprole, Ethiprole, Fipronil, Pyrafluprole, Pyriprole, Vaniliprole

### Chlorid-Kanal-Aktivatoren

6.1 Mectine,
   zum Beispiel Avermectin, Emamectin, Emamectin-benzoate, Ivermectin, Milbemycin Juvenilhormon-Mimetika,
   zum Beispiel Diofenolan, Epofenonane, Fenoxycarb, Hydroprene, Kinoprene, Methoprene, Pyriproxifen, Triprene

### Ecdysonagonisten/disruptoren

8.1 Diacylhydrazine,
   zum Beispiel Chromafenozide, Halofenozide, Methoxyfenozide, Tebufenozide

### Inhibitoren der Chitinbiosynthese

9.1 Benzoylharnstoffe,
   zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
9.2 Buprofezin
9.3 Cyromazine

### Inhibitoren der oxidativen Phosphorylierung, ATP-Disruptoren

10.1 Diafenthiuron
10.2 Organozinnverbindungen,
   zum Beispiel Azocyclotin, Cyhexatin, Fenbutatin-oxide

### Entkoppler der oxidativen Phoshorylierung durch Unterbrechung des H-Protongradienten

11.1 Pyrrole,
   zum Beispiel Chlorfenapyr
11.2 Dinitrophenole,
   zum Beispiel Binapacyrl, Dinobuton, Dinocap, DNOC

### Elektronentransportinhibitoren

12.1 Seite-I-Elektronentransportinhibitoren
   aus der Gruppe der
   METIs,
   zum Beispiel Fenazaquin, Fenpyroximate, Pyrimidifen, Pyridaben, Tebufenpyrad, Tolfenpyrad sowie
   Hydramethylnon
   Dicofol
12.2 Seite-II-Elektronentransportinhibitoren
   Rotenone
12.3 Seite-III-Elektronentransportinhibitoren
   Acequinocyl, Fluacrypyrim

### Mikrobielle Disruptoren der Insektendarmmembran

Bacillus thuringiensis-Stämme

### Inhibitoren der Fettsäurebiosynthese

14.1 Tetronsäuren,
   zum Beispiel Spirodiclofen, Spiromesifen
14.2 Tetramsäuren,
   zum Beispiel Spirotetramat

### Carboxamide,

zum Beispiel Flonicamid

### Oktopaminerge Agonisten,

zum Beispiel Amitraz

### Inhibitoren der Magnesium-stimulierten ATPase,

Propargite

### Ryanodinrezeptor-Effektoren

18.1 Benzoesäuredicarboxamide,
   zum Beispiel Flubendiamide
18.2 Anthranilamide, zum Beispiel DPX E2 Y45

### Nereistoxin-Analoge,

zum Beispiel Thiocyclam hydrogen oxalate, Thiosultap-sodium

### Biologika, Hormone oder Pheromone

Azadirachtin, Bacillus spec., Beauveria spec., Codlemone, Metarrhizium spec., Paecilomyces spec., Thuringiensin, Verticillium spec.

### Wirkstoffe mit unbekannten oder nicht spezifischen Wirkmechanismen

21.1 Begasungsmittel,
   zum Beispiel Aluminium phosphite, Methyl bromide, Sulfuryl fluoride
21.2 Fraßhemmer,
   zum Beispiel Cryolite, Flonicamid, Pymetrozine
21.3 Milbenwachstumsinhibitoren,
   zum Beispiel Clofentezine, Etoxazole, Hexythiazox
21.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe/Wirkstoffkombinationen können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muss.

Die erfindungsgemäßen Wirkstoffe/Wirkstoffkombinationen können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepassten üblichen Weise.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch, die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe/Wirkstoffkombinationen wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocorina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe/Wirkstoffkombinationen der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe/Wirkstoffkombinationen geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feedthrough-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markieningsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen/Wirkstoffkombinationen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium perlinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hautflügler wie Sirex juvencus, Urocerus gigas, Urocerus gigas taignus, Urocerus augur;

Termiten wie Kalotermes flavicollis, Crypfotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:
Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im Allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion; Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid, Triflumuron, Chlothianidin, Spinosad, Tefluthrin, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis(triallrylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-tert.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[b]thiophencarbonsäurecyclohexylamid-S,S-dioxid, Dichlofluanid, Fluorfolpet, 3-Iod-2-propinyl-butylcarbamat, Tolylfluanid und Azole wie
   Azaconazole, Cyproconazole, Epoxyconazole, Hexaconazole, Metconazole, Propiconazole und Tebuconazole;
Molluskizide wie
   Fentinacetat, Metaldehyd, Methiocarb, Niclosamid, Thiodicarb und Trimethacarb, Fe-chelate;
oder herkömmliche Antifouling-Wirkstoffe wie
4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenyiboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichtorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp.
Aus der Ordnung der Chilopoda z.B. Geophilus spp.
Aus der Ordnung der Zygentoma z.B. Ctenolepisma spp., Lepisma saccharina, Lepismodes inquilinus.
Aus der Ordnung der Blattaria z.B. Blätta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coloptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe/Wirkstoffkombinationen können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe/Wirkstoffkombinationen können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe/Wirkstoffkombinationen ist jedoch keineswegs auf diese Gattungen beschränkt, sondern erstreckt sich in gleicher Weise auch auf andere Pflanzen.

Die erfindungsgemäßen Wirkstoffe/Wirkstoffkombinationen eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I)/Wirkstoffkombinationen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe/Wirkstoffkombinationen können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe/Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschaten, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe/Wirkstoffkombinationen können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Ferfigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise
Acetochlor, Acifluorfen (-sodium), Aclonifen, Alachlor, Alloxydim (-sodium), Ametryne, Amicarbazone, Amidochlor, Amidosulfuron, Aminopyralid, Anilofos, Asulam, Atrazine, Azafenidin, Azimsulfuron, Beflubutamid, Benazolin (-ethyl), Benfuresate, Bensulfuron (-methyl), Bentazon, Benzcarbazone, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropyl-ammonium), Halosafen, Haloxyfop (-ethoxyethyl, -P-methyl), Hexazinone, HOK-201, Imazamethabenz -methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, KIH 485, Lactofen, Lenacil, Linuron, MCPA, Mecoprop, Mefenacet, Mesosulfurone, Mesotrione, Metamifop, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Orthosulfamuron, Oryzalin, Oxadiargyl, Oxadiazon, Oxasulfuron, Oxaziclomefone, Oxyfluorfen, Paraquat, Pelargonsäure, Pendimethalin, Pendralin, Penoxsulam, Pentoxazone, Phenmedipham, Picolinafen, Pinoxaden, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrasulfotole, Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrimisulfan, Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tembotrione, Tepraloxydim, Terbuthylazine, Terbutryn, , Thenylchlor, Thiafluamide, Thiazöpyr, Thidiazimin, Thifensulfuron (-methyl), Thiobencarb, Tiocarbazil, Topramezone, Tralkoxydim, Triallate, Triasulfuron, Tribenuron (-methyl), Triclopyr, Tridiphane, Trifluralin, Trifloxysulfuron, Triflusulfuron (-methyl), Tritosulfuron und

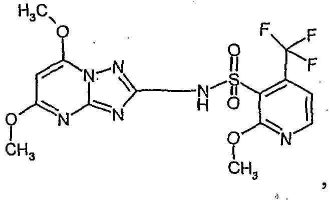

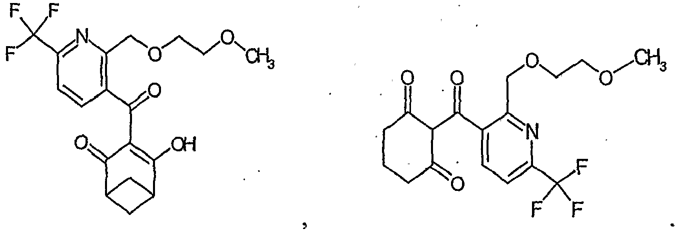

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe/Wirkstoffkombinationen können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe/Wirkstoffkombinationen können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Die erfindungsgemäßen Stoffe/Wirkstoffkombinationen weisen eine starke mikrobizide Wirkung auf und können zur Bekämpfung von unerwünschten Mikroorganismen, wie Fungi und Bakterien, im Pflanzenschutz und im Materialschutz.eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen. Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Altemaria brassicae;
Pseudocercosporella-Arten, wie beispielsweise Pseudocercosporella herpotrichoides.

Die erfindungsgemäßen Wirkstoffe/Wirkstoffkombinationen weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokolation mit unerwünschten Mikroorgansimen weitgehende Resistenz gegen diese Mirkroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe/Wirkstoffkombinationen in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe/Wirkstoffkombinationen eignen sich auch zur Steigerung des Ernteertrages. Sie sind außerdem mindertoxisch und weisen eine gute Pflanzenverträglichkeit auf.

Die erfindungsgemäßen Wirkstoffe/Wirkstoftkombinationen können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe/Wirkstoffkombinationen können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe/Wirkstoffkombinationen mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycotether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe/Wirkstoffkombinationen können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos Binanacryl Biphenyl Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothalisopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-chlorophenyl)-2-propynyl]oxy]-3-methoxyphenyl]ethyl]-3-methyl- 2-[(methylsulfonyl)amino]-butanamide; 1-(1-naphthalenyl)-1H-pyrrole-2,5-dione; 2,3,5,6-tetrachloro-4-(methylsulfonyl)-pyridine; 2-amino-4-methyl-N-phenyl-5-thiazolecarboxamide; 2-chloro-N-(2,3-dihydro-1,1,3-trimethyl-1H-inden-4-yl)-3-pyridincarboxam ide; 3,4,5-trichloro-2,6-pyridinedicarbonitrile; Actinovate; cis-1-(4-chlorophenyl)-2-(1H-1,2,4-triazole-1-yl)-cycloheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine;Sodium tetrathiocarbonate; sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxinecopper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,

Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,

Cadusafos, Camphechlor, Carbaryl, Carbofuran, Caxbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,

DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinotefuran, Diofenolan, Disulfoton, Docusat-sodium, Dofenapyn, DOWCO-439,

Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos,

Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,

Gamma-HCH, Gossyplure, Grandlure, Granuloseviren,
Halfenprox, Halofenozide, HCH, HCN-801, Heptenophos, Hexaflumuron, Hexythiazox, Hydramethylnone, Hydroprene,
IKA-2002, Imidacloprid, Imiprothrin, Indoxacarb, Iodofenphos, Iprobenfos, Isazofos, Isofenphos, Isoprocarb, Isoxathion, Ivermectin,
Japonilure,
Kadethrin, Kernpolyederviren, Kinoprene,
Lambda-Cyhalothrin, Lindane, Lufenuron,
Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800,
Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
OK-5101, OK-5201, OK-9601, OK-9602, OK-9701, OK-9802, Omethoate, Oxamyl, Oxydemetonmethyl,
Paecilomyces fumosoroseus, Parathion-methyl Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen,
Quinalphos,
Resmethrin, RH-5849, Ribavirin, RU-12457, RU-15525,
S-421, S-1833, Salithion, Sebufos, SI-0009, Silafluofen, Spinosad, Spirodiclofen, Spiromesifen, Sulfluramid, Sulfotep, Sulprofos, SZI-121,
Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb,
Vamidothion, Vaniliprole, Verbutin, Verticillium lecanii,
WL-108477, WL-40027,
YI-5201, YI-5301, YI-5302,
XMC, Xylylcarb,
ZA-3274, Zeta-Cypermethrin, Zolaprofos, ZXI-8901,
die Verbindung 3-Methyl-phenyl-propylcarbamat (Tsumacide Z),
die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I)/Wirkstoffkombinationen auch sehr gute antimykotische Wirkungen aüf. Sie besitzen ein sehr breites anti-mykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes,

Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe/Wirkstoffkombinationen können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe/Wirkstoffkombinationen als Fungizide können die Aufwandmengen je nach Applikationsart innerhalb eines größeren Bereiches variiert werden. Bei der Behandlung von Pflanzenteilen liegen die Aufwandmengen an Wirkstoff im Allgemeinen zwischen 0,1 und 10.000 g/ha, vorzugsweise zwischen 10 und 1.000 g/ha. Bei der Saatgutbehandlung liegen die Aufwandmengen an Wirkstoff im Allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im Allgemeinen zwischen 0,1 und 10.000 g/ha, vorzugsweise zwischen 1 und 5.000 g/ha.

Die Herstellung und die Verwendung der erfindungsgemäßen Wirkstoffe/Wirkstoffkombinationen geht aus den nachfolgenden Beispielen hervor.

### Herstellungsbeispiele

### Beispiel I-1-a-1

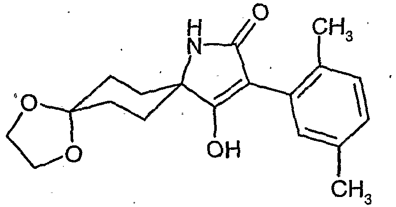

4.88 g (0.042 Mol) Kalium-tert.-butylat werden in 13 ml wasserfreiem Dimethylformamid (DMF) vorgelegt und bei 60°C tropft man 6.82 g gemäß Beispiel II-1 in 14 ml wasserfreiem DMF zu und rührt bei 80°C unter dünnschichtchromatographischer Kontrolle.

Nach Reaktionsende gibt man die Reaktionslösung auf 150 ml Eiswasser und säuert bei 0 bis 10°C mit konzentrierter Salzsäure auf pH=2 an, saugt den Niederschlag ab, wäscht mit Eiswasser nach und trocknet anschließend. Es erfolgt säulenchromatographische Reinigung an Kieselgel (Dichlormethan/Aceton = 5:1).

Ausbeute: 2,5 g (40 % d. Theorie), Fp. 233°C.

### Beispiel I-1-a-8

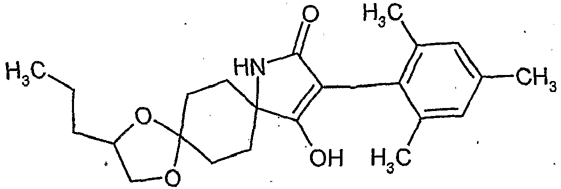

0,644 g (2 mmol) der Verbindung gemäß Beispiel XIV-1-1 werden unter Argon-Schutzgas in 30 ml Xylol vorgelegt, bei Raumtemperatur mit 2.083 g (20 mmol) 1,2-Pentandiol und 0,059 g (0,29 mmol) p-Toluolsulfonsäure versetzt und ca. 0,5 g Mol-Sieb 4 A° (Pulver) zugegeben und drei Tage unter Rückfluss und dünnschichtchromatographischer Kontrolle gerührt.

Nach Reaktionsende wird das Lösungsmittel abdestilliert. Es erfolgt säulenchromatographische Reinigung an Kieselgel (Dichlomethan/Aceton = 5:1).

Ausbeute: 0,27 g(≙ 35 % d. Theorie), Fp. 273°C.

In Analogie zu Beispiel (I-1-a-1) und gemäß den allgemeinen Angaben zur Herstellung erhält man folgende Verbindungen der Formel (I-1-a)

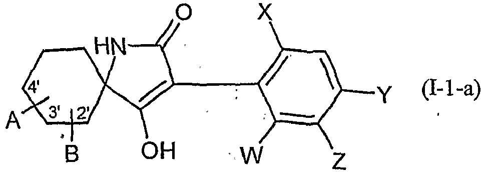

| **Bsp.-Nr.** | **W** | **X** | **Y** | **Z** | **A** | **B** | **Fp °C** | **Isomer** |
|---|---|---|---|---|---|---|---|---|
| I-1-a-2 | CH₃ | CH₃ | CH₃ | H | 4'-O-(CH₂)₂-O- | | >220 | - |
| I-1-a-3 | CH₃ | CH₃ | CH₃ | CH₃ | 4'-O-(CH₂)₂-O- | | >240 | - |
| I-1-a-4 | CH₃ | CH₃ | Cl | H | 4'-O-(CH₂)₂-O | | >240 | - |
| I-1-a-5 | H | CH₃ | CH₃ | CH₃ | 4'-O-(CH₂)₂-O- | | 240 | - |
| I-1-a-6 | CH₃ | Cl | Cl | H | 4'-O-(CH₂)₂-O- | | >245 | - |
| I-1-a-7 | CH₃ | CH₃ | CH₃ | H | 4'-O-CHCH₃-CHCH₃-O- | | * | Gemisch |
| I-1-a-8 | CH₃ | CH₃ | CH₃ | H | 4'-O-CHC₃H₇-CH₂-O- | | 273 | A logP 2.7 |
| I-1-a-9 | CH₃ | CH₃ | CH₃ | H | 4'-O-CHC₃H₇-CH₂-O- | | Z | B logP 2.7 |
| I-1-a-10 | CH₃ | CH₃ | CH₃ | H | 4'-O-CHCH₃-CHCH₃-O- | | 275 | Gemisch |
| I-1-a-11 | CH₃ | Cl | Cl | H | 4'-O-(CH₂)₃-O- | | 262 | - |
| I-1-a-12 | H | CH₃ | H | CH₃ | 4'-O-(CH₂)₃-O- | | 166 | - |
| I-1-a-13 | CH₃ | CH₃ | CH₃ | CH₃ | 4'-O-(CH₂)₃-O- | | 263 | - |
| I-1-a-14 | CH₃ | CH₃ | Cl | H | 4'-O-(CH₂)₃-O- | | 312 | - |
| I-1-a-15 | H | Cl | H | 4-Cl-Ph | 4'-O-(CH₂)₃-O- | | 133 | - |
| I-1-a-16 | H | CH₃ | H | 4-Cl-Ph | 4'-O-(CH₂)₃-O- | | 210 | - |
| I-1-a-17 | H | CH₃ | CH₃ | CH₃ | 4'-O-(CH₂)₃-O- | | 189 | - |
| I-1-a-18 | CH₃ | CH₃ | CH₃ | H | 4'-O-(CH₂)₃-O- | | 229 | - |
| I-1-a-19 | CH₃ | CH₃ | Br | H | 4'-O-(CH₂)₃-O- | | 255 | - |
| I-1-a-20 | CH₃ | CH₃ | Cl | H | 4'-O-CH₂-C(CH₃)₂-CH₂-O- | | 273 | - |
| I-1-a-21 | CH₃ | CH₃ | CH₃ | H | 4'-O-CHCH₃-CH₂-CHCH₃-O- | | 265 | Gemisch |
| I-1-a-22 | CH₃ | CH₃ | CH₃ | H | 4'-O-CHCH₃-C(CH₂)₂-O- | | 230 | Gemisch |
| I-1-a-23 | CH₃ | CH₃ | CH₃ | H | 4'-O-CH₂-C(CH₃)₂-CH₂-O- | | 230 | - |
| I-1-a-24 | CH₃ | OCH₃ | CH₃ | H | 4'-O-(CH₂)₃-O- | | 202 | - |
| I-1-a-25 | CH₃ | C₂H₅ | CH₃ | H | 4'-O-(CH₂)₂-O- | | Z | - |
| I-1-a-26 | CH₃ | CH₃ | Br | H | 4'-O-(CH₂)₂-O- | | 306 | - |
| I-1-a-27 | H | CH₃ | H | 5-(4-Cl-Ph) | 4'-O-(CH₂)₂-O- | | 154 | - |
| I-1-a-28 | H | CH₃ | H | CH₃ | 4'-O-CHCH₃-CH₂-O- | | Wachs | Gemisch |
| I-1-a-29 | H | CH₃ | H | CH₃ | 4'-O-CHCH₃-CHCH₃-O- | | 84 | Gemisch |
| I-1-a-30 | CH₃ | CH₃ | Cl | H | 4'-O-CHCH₃-CHCH₃-O- | | 268 | Gemisch |
| I-1-a-31 | CH₃ | CH₃ | Cl | H | -4'O-CHCH₃-CH₂-O- | | 301 | Gemisch |
| I-1-a-32 | CH₃ | CH₃ | Br | H | 4'-O-CH₂-CHCH₃-CH₂-O- | | 171 | Gemisch |
| I-1-a-33 | CH₃ | CH₃ | Br | H | 4'-O-CH₂-C(CH₃)₂ -CH₂-O- | | 266 | - |
| I-1-a-34 | CH₃ | CH₃ | Cl | H | 4'-O-CH₂-CHCH₃-CH₂-O- | | 265 | Gemisch |
| I-1-a-35 | CH₃ | CH₃ | Cl | H | 4'-O-CHCH₃-(CH₂)₂-O- | | 243 | Gemisch |
| I-1-a-36 | H | CH₃ | H | CH₃ | 4'-O-CHCH₃-CH₂-CHCH₃-O- | | 161 | Gemisch |
| I-1-a-37 | H | CH₃ | H | CH₃ | 4'-O-CHCH₃-(CH₂)₂-O- | | Öl | Gemisch |
| I-1-a-38 | CH₃ | CH₃ | Cl | H | 4'-O-CHCH₃-CH₂-CHCH₃-O- | | 276 | Gemisch |
| I-1-a-39 | CH₃ | C₂H₅ | Br | H | 4'-O-(CH₂)₂-O- | | 293 | - |
| I-1-a-40 | C₂H₅ | C₂H₅ | CH₃ | H | 4'-O-(CH₂)₂-O- | | 269 | - |
| I-1-a-41 | H | CH₃ | Cl | CH₃ | 4'-O-(CH₂)₂-O- | | 236 | - |
| I-1-a-42 | C₂H₅ | OCH₃ | Cl | H | 4'-O-(CH₂)₂-O- | | 234 | - |
| I-1-a-43 | CH₃ | OCH | CH₃ | H | 4'-O-(CH₂)₂-O- | | 237 | - |
| I-1-a-44 | C₂H₅ | Cl | Cl | H | 4'-O-(CH₂)₂-O- | | 305 | - |
| I-1-a-45 | C₂H₅ | CH₃ | Br | H | 4'-O-CHCH₃-CHCH₃-O- | | 148 | Gemisch |
| I-1-a-46 | CH₃ | CH₃ | Br | H | 4'-O-CHCH₃-CHCH₃-O- | | 275 | Gemisch |
| I-1-a-47 | CH₃ | CH₃ | Br | H | 4'-O-CHCH₃-CH₂-O- | | 292 | Gemisch |
| I-1-a-48 | C₂H₅ | Br | CH₃ | H | 4'-O-(CH₂)₂-O- | | 296 | - |
| I-1-a-49 | H | Cl | H | 4-Cl-Ph | 4'-O-(CH₂)₂-O- | | 257 | - |
| I-1-a-50 | CH₃ | CH₃ | Br | H | 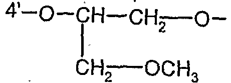 | | 116 | Gemisch |
| I-1-a-51 | CH₃ | CH₃ | H | 4-Cl-Ph | 4'-O-CHCH₃-CH₂-O- | | 288 | Gemisch |
| I-1-a-52 | H | CH₃ | H | 4-Cl-Ph | 4'-O-CHCH₃-CH₂-O- | | 198 | Gemisch |
| I-1-a-53 | CH₃ | C₂H₅ | Br | H | 4'-O-CHCH₃-CH₂-O- | | 255 | Gemisch |
| I-1-a-54 | H | CH₃ | H | 4-Cl-Ph | 4'-O-CHCH₃-CHCH₃-O- | | 213 | Gemisch |
| I-1-a-55 | H | CH₃ | Cl | CH₃ | 4'-O-CHCH₃-CH₂-O- | | 272 | Gemisch |
| I-1-a-56 | CH₃ | CH₃ | Br | H | 4'-O-CHCH₃-CH₂-CHCH₃-O- | | 262 | Gemisch |
| I-1-a-57 | H | Cl | Cl | H | 4'-O-(CH₂)₃-O- | | 237 | - |
| I-1-a-58 | CH₃ | CH₃ | Br | H | 4'-O-CHCH₃-(CH₂)₂-O- | | 80 | Gemisch |
| I-1-a-59 | CH₃ | Cl | Cl | H | 4'-O-CH₂-C(CH₃)₂-CH₂-O- | | 264 | - |
| I-1-a-60 | CH₃ | C₂H₅ | Br | H | 4'-O-CHCH₃-(CH₂)₂-O- | | 190 | Gemisch |
| I-1-a-61 | H | CH₃ | H | CH₃ | 4'-O-CH₂-C(CH₃)₂-CH₂-O- | | 240 | - |
| I-1-a-62 | H | CH₃ | H | CH₃ | 4'-O-CH₂-CHCH₃-CH₂-O- | | 276 | Gemisch |
| I-1-a-63 | CH₃ | C₂H₅ | Br | H | 4'-O-CHCH₃-CH₂-CHCH₃-O- | | >300 | Gemisch |
| I-1-a-64 | H | Cl | H | 4-Cl-Ph | 4'-O-CH₂-C(CH₃)₂-CH₂-O- | | 276 | - |
| I-1-a-65 | H | Cl | H | 4-Cl-Ph | 4'-O-CH₂-CHCH₃-CH₂-O- | | 79 | Gemisch |
| I-1-a-66 | H | CH₃ | H | 4-Cl-Ph | 4'-O-CHCH₃-(CH₂)₂-O- | | 67 | Gemisch |
| I-1-a-67 | H | CH₃ | H | 4-Cl-Ph | 4'-O-CH₂-C(CH₃)₂-CH₂-O- | | 234 | - |
| I-1-a-68 | H | CH₃ | H | CH₃ | 3'-O-(CH₂)₂-O- | | 211 | - |
| I-1-a-69 | C₂H₅ | Br | CH₃ | H | 3'-O-(CH₂)₂-O- | | Z | - |
| I-1-a-70 | CH₃ | CH₃ | Cl | H | 3'-O-(CH₂)₂-O- | | 260 | - |
| I-1-a-71 | CH₃ | CH₃ | CH₃ | H | 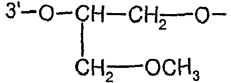 | | Öl | Gemisch |
| I-1-a-72 | C₂H₅ | Br | CH₃ | H | 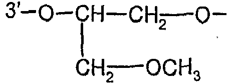 | | Öl | Gemisch |
| I-1-a-73 | H | CH₃ | H | CH₃ | 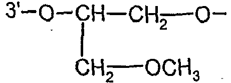 | | Öl | Gemisch |
| I-1-a-74 | CH₃ | CH₃ | Cl | H | 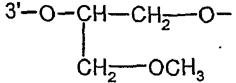 | | 82 | Gemisch |
| I-1-a-75 | CH₃ | CH₃ | Cl | H | 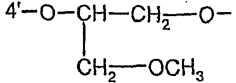 | | 67 | Gemisch |
| I-1-a-76 | H | Cl | H | 4-Cl-Ph | 4'-O-CHCH₃-CH₂-O- | | 68 | Gemisch |
| I-1-a-77 | C₂H₅ | Br | CH₃ | H | 4'-O-CHCH₃-CH₂-O- | | 281 | Gemisch |
| I-1-a-78 | H | Cl | H | 4-Cl-Ph | 4'-O-CHCH₃-(CH₂)₂-O- | | 232 | Gemisch |
| I-1-a-79 | H | Cl | H | 4-Cl-Ph | 4'-O-CH₂-CHCH₃-CH₂-O- | | 160 | Gemisch |
| I-1-a-80 | H | Cl | H | 4-Cl-Ph | 4'-O-CHCH₃-CH₂-CHCH₃-O- | | 268 | Gemisch |
| I-1-a-81 | H | Cl | H | 4-Cl-Ph | 4'-O-CH₂-C(CH₃)₂-CH₂-O- | | 151 | - |
| I-1-a-82 | CH₃ | | Br | H | 4'-O-(CH₂)₃-O- | | 244 | - |
| I-1-a-83 | CH₃ | CH₃ | Cl | H | 3'-O-CHCH₃-CH₂-O- | | 167 | Gemisch |
| I-1-a-84 | CH₃ | CH₃ | Cl | H | 3'-O-CH₂-C(CH₃)₂-CH₂-O- | | 266 | Gemisch |
| I-1-a-85 | CH₃ | CH₃ | Cl | H | 3'-O-(CH₂)₃-O- | | 261 | Gemisch |
| I-1-a-86 | CH₃ | C₂H₅ | 4-Cl-Ph | H | 4'-O-CHCH₃-CH₂-O- | | 219-222 | Gemisch |

| | | | | | | | | |
|---|---|---|---|---|---|---|---|---|
| * ¹H-NMR ( 400 MHz, d₆-DMSO): δ = 1.05-1.09 (2d, 6H, 2xCHCH₃); 4.18-4.23 (m, 2H, 2xCHCH₃) ppm Z = Zersetzung Ph = 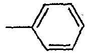 | | | | | | | | |

### Beispiel I-1-b-1

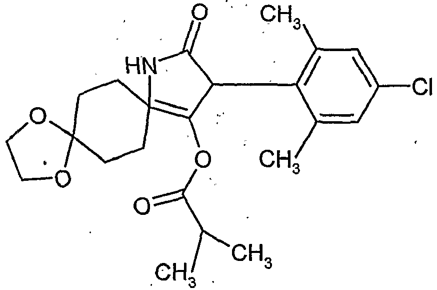

1.82 g der Verbindung gemäß Beispiel I-1-a-4 werden in 50 ml wasserfreiem Essigsäureethylester und 0,77 ml (5.5 mmol) Triethylamin unter Rückfluss vorgelegt. Man gibt 0,55 ml (0.0055 Mol) Isobuttersäurechlorid in 5 ml wasserfreiem Essigsäureethylester zu und rührt unter Rückfluss.

Nach Abschluss der Reaktion (dünnschichtchromatographische Kontrolle) wird das Lösungsmittel abdestilliert und der Rückstand in Dichlormethan aufgenommen. Man wäscht 2 mal mit 30 ml 0.5 N NaOH, trocknet und destilliert das Lösungsmittel ab.

Es erfolgt säulenchromatographische Reinigung an Kieselgel (Dichlormethan/Essigsäureethylester = 3:1).

Ausbeute: 0,3 g (14 % d. Theorie), Fp. 215°C

In Analogie zu Beispiel (I-1-b-1) und gemäß den allgemeinen Angaben zur Herstellung erhält man folgende Verbindungen der Formel (I-1-b)

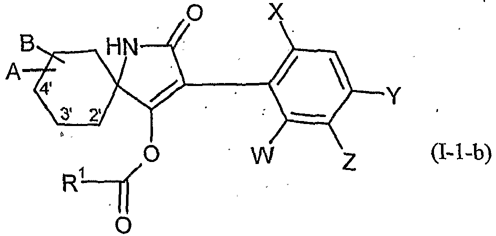

| **Bsp.-Nr.** | **W** | **X** | **Y** | **Z** | **A** | **B** | **R¹** | **Fp °C** | **Isomer** |
|---|---|---|---|---|---|---|---|---|---|
| I-1-b-2 | CH₃ | C₂H₅ | Br | H | 4'-O-(CH₂)₂-O- | | H₃CO-CH₂- | *3.23 (s,3H, OCH₃) | - |
| | | | | | | | | 7.21 (s,2H, -Ar-H) | |
| I-1-b-3 | CH₃ | C₂H₅ | Br | H | 4'-O-(CH₂)₂-O- | | i-C₃H₇ | erstarrter Schaum | - |
| I-1-b-4 | C₂H₅ | Cl | Cl | H | 4'-O-(CH₂)₂-O- | | i-C₃H₇ | 106-112 | - |
| I-1-b-5 | C₂H₅ | Br | CH₃ | H | 4'-O-CHCH₃-CHCH₃-O- | | i-C₃H₇ | 198-200 | Gemisch |
| I-1-b-6 | C₂H₅ | Cl | Cl | H | 4'-O-(CH₂)₂-O- | | H₃CO-CH₂- | *2.63(m,2H,Ar-CH₂) | - |
| | | | | | | | | 3.29(s,3H,OCH₃) | |
| I-1-b-7 | C₂H₅ | OCH₃ | Cl | H | 4'-O-(CH₂)₂-O- | | H₃CO-CH₂- | 170-175 | - |
| I-1-b-8 | C₂H₅ | Br | CH₃ | H | 3'-O-(CH₂)₂-O- | | i-C₃H₇ | erstarrter Schaum | - |
| | | | | | | | | **1.07(m,6H, CHCH₂)₂) | |
| | | | | | | | | 2.28(s,3H,ArCH₃) | |
| I-1-b-9 | C₂H₅ | Br | CH₃ | H | 3'-O-(CH₂)₂-O- | | H₃CO-CH₂- | *2.29(s,3H,Ar-CH₃) | - |
| | | | | | | | | 3.26(s,3H,OCH₃) | |
| I-1-b-10 | C₂H₅ | Br | CH₃ | H | 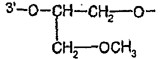 | | i-C₃H₇ | Öl **1,05(m,6H, CHCH₃)₂) | Gemisch |
| | | | | | | | | 2.29(s,3H,ArCH₃) | |
| I-1-b-11 | C₂H₅ | Br | CH₃ | H | 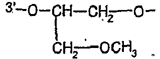 | | H₃CO-CH₂- | Öl **2.29(s,3H, ArCH₃) | Gemische |
| | | | | | | | | 3.26(s,3H,OCH₃) | |
| I-1-b-12 | CH₃ | C₂H₅ | Br | H | 4'-O-CHCH₃₋CH₂-CHCH₃-O- | | 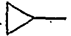 | 225 | Gemisch |
| I-1-b-13 | CH₃ | C₂H₅ | Br | H | 4'-O-CHCH₃-(CH₂)₂-O- | | 2-Cl-Ph | 225 | Gemisch |
| I-1-b-14 | CH₃ | C₂H₅ | Br | H | 4'-O-CHCH₃-CHCH₃-O- | | 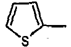 | 246 | Gemisch |
| I-1-b-15 | CH₃ | C₂H₅ | Br | H | 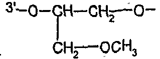 | | t-C₄H₉ | erstarrter Schaum | Gemisch |
| I-1-b-16 | CH₃ | C₂H₅ | Br | H | 4'-O-CHCH₃-CH₂-O- | | i-C₃H₇ | 194 | Gemisch |
| I-1-b-17 | CH₃ | C₂H₅ | Br | H | 4'-O-CHCH₃-(CH₂)₂-O- | | i-C₃H₇ | *1.06(m,6H, CH(CH₃)₂) | Gemisch |
| | | | | | | | | 7.21(s,2H,ArH) | |
| I-1-b-18 | C₂H₅ | CH₃ | Br | H | 4'-O-CHCH₃-CHCH₃-O- | | H₃CO-CH₂ | *3.24(s,3H, OCH₃) | Gemisch |
| | | | | | | | | 7.21(s,2H,Ar-H) | |
| I-1-b-19 | C₂H₅ | CH₃ | CH₃ | H | 4'-O-(CH₂)₂-O- | | i-C₃H₇ | *0.98 (dd, 6H, CH(CH₃)₂) | - |
| | | | | | | | | 3.98 (m, 4H, CH₂O) | |
| | | | | | | | | 6.86 (s, 2H, Ar-H) | |
| I-1-b-20 | C₂H₅ | CH₃ | Br | H | 4'-O-CHCH₃-CH₂-CHCH₃-O- | | i-C₃H₇ | 120 | Gemisch |

| | | | | | | | | | |
|---|---|---|---|---|---|---|---|---|---|
| * ¹H-NMR (400 MHz, CDCl₃): Verschiebungen δ in ppm ** ¹H-NMR (300 MHz, CDCl₃): Verschiebungen δ in ppm | | | | | | | | | |

### Beispiel I-1-c-1

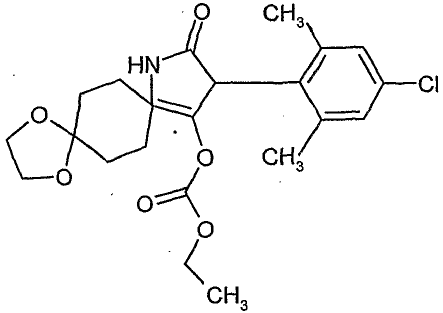

1.82 g der Verbindung gemäß Beispiel I-1-a-4 werden in 30 ml wasserfreiem Dichlormethan und 0.7 ml Triethylamin bei 10 bis 20°C gerührt. Man gibt 0.5 ml Chlorameisensäure-ethylester in 2 ml wasserfreiem Dichlormethan hinzu und rührt bei Raumtemperatur.

Nach Reaktionsende (dünnschichtchromatographische Kontrolle) wird 2 mal mit 10 ml 0.5 N NaOH gewaschen und über Magnesiumsulfat getrocknet. Das Lösungsmittel wird anschließend abdestilliert.

Es erfolgt säulenchromatographische Reinigung an Kieselgel (Dichlormethan/Ethanol = 30:1).

Ausbeute: 1,15 g (52 % der Theorie), Fp 198°C.

In Analogie zu Beispiel (I-1-c-1) und gemäß den allgemeinen Angaben zur Herstellung erhält man folgende Verbindungen der Formel (I-1-c):

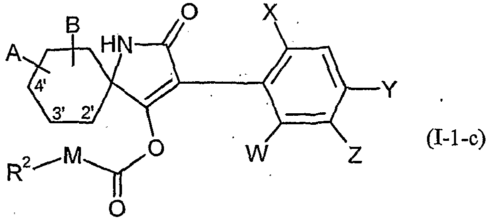

| Bsp.-Nr. | W | X | Y | Z | A | B | M | R² | Fp °C | Isomer |
|---|---|---|---|---|---|---|---|---|---|---|
| I-1-c-2 | CH₃ | CH₃ | CH₃ | H | 4'-O-(CH₂)₂-O- | | O | C₂H₅ | 181 | - |
| I-1-c-3 | H | CH₃ | CH₃ | CH₃ | 4'-O-(CH₂)₂-O- | | O | C₂H₅ | 221 | - |
| I-1-c-4 | CH₃ | Cl | Cl | H | 4'-O-(CH₂)₂-O- | | O | C₂H₅ | 233 | - |
| I-1-c-5 | CH₃ | CH₃ | CH₃ | CH₃ | 4'-O-(CH₂)₂-O- | | O | C₂H₅ | 209 | - |
| I-1-c-6 | CH₃ | CH₃ | CH₃ | H | 4'-O-CHCH₃-CH₂-O- | | O | C₂H₅ | 218 | Gemischs |
| I-1-c-7 | CH₃ | Cl | Cl | H | 4'-O-(CH₂)₃-O- | | O | C₂H₅ | 235 | - |
| I-1-c-8 | CH₃ | CH₃ | Cl | H | 4'-O-(CH₂)₃-O- | | O | C₂H₅ | 195 | - |
| I-1-c-9 | H | CH₃ | CH₃ | CH₃ | 4'-O-(CH₂)₃-O- | | O | C₂H₅ | 235 | - |
| I-1-c-10 | CH₃ | CH₃ | Br | H | 4'-O-(CH₂)₃-O- | | O | C₂H₅ | 234 | - |
| I-1-c-11 | CH₃ | CH₃ | CH₃ | H | 4'-O-(CH₂)₃-O- | | O | C₂H₅ | 229 | - |
| I-1-c-12 | CH₃ | CH₃ | Cl | H | 4'-O-CH₂-CHCH₃-CH₂-O- | | O | C₂H₅ | 241 | Gemisch |
| I-1-c-13 | CH₃ | CH₃ | Cl | H | 4'-O-CH₂-C(CH₃)₂-CH₂-O- | | O | C₂H₅ | 247 | - |
| I-1-c-14 | CH₃ | C₂H₅ | Br | H | 4'-O-(CH₂)₂-O- | | O | C₂H₅ | 200-201 . | - |
| I-1-c-15 | CH₃ | C₂H₅ | CH₃ | H | 4'-O-(CH₂)₂-O- | | O | C₂H₅ | Öl | - |
| I-1-c-16 | H | CH₃ | H | 4-Cl-Ph | 4'-O-(CH₂)₂-O- | | O | C₂H₅ | 197 | - |
| I-1-c-17 | CH₃ | CH₃ | Cl | H | 4'-O-CHCH₃-CH₂-O- | | O | C₂H₅ | 223 | Gemisch |
| I-1-c-18 | CH₃ | CH₃ | Cl | H | 4'-O-CHCH₃-CHCH₃-O- | | O | C₂H₅ | 232 | Gemisch . |
| I-1-c-19 | H | CH₃ | H | CH₃ | 4'-O-CHCH₃-CH₂-O- | | O | C₂H₅ | 141 | Gemisch |
| I-1-c-20 | CH₃ | CH₃ | Br | H | 4'-O-(CH₂)₂-O- | | O | C₂H₅ | 222 | - |
| I-1-c-21 | H | CH₃ | Cl | CH₃ | 4'-O-(CH₂)₂-O- | | O | C₂H₅ | 187 | - |
| I-1-c-22 | H | CH₃ | H | CH₃ | 4'-O-CHCH₃-CHCH₃-O- | | O | C₂H₅ | 180 | Gemisch |
| I-1-c-23 | C₂H₅ | C₂H₅ | CH₃ | H | 4'-O-(CH₂)₂-O- | | O | C₂H₅ | 179 | - |
| I-1-c-24 | CH₃ | O-CH₃ | CH₃ | H | 4'-O-(CH₂)₂-O- | | O | C₂H₅ | 176 - | - |
| I-1-c-25 | C₂H₅ | O-CH₃ | Cl | H | 4'-O-(CH₂)₂-O- | | O | C₂H₅ | **1.16 ("q",6H, - -Ar-CH₂-CH₃ u. O-CH₂-CH₃) 3.79 (s,3H,Ar-OCH₃) | - |
| I-1-c-26 | C₂H₅ | Cl | Cl | H | 4'-O-(CH₂)₂-O- | | O | C₂H₅ | 175-183 | - |
| I-1-c-27 | CH₃ | C₂H₅ | Br | H | 4'-O-CHCH₃-CH₂-O- | | O | C₂H₅ | 181 | Gemisch |
| I-1-c-28 | C₂H₅ | Cl | Cl | H | 4'-O-(CH₂)₂-O- | | O | C₆H₅ -CH₂- | 173 | - |
| I-1-c-29 | CH₃ | C₂H₅ | Br | H | 4'-O-CHCH₃-CHCH₃-O- | | O | CH₂= CH-CH₂- | 187 | Gemisch |
| I-1-c-30 | CH₃ | CH₃ | Cl | H | 4'-O-CHCH₃-(CH₂)₂-O- | | O | C₂H₅ | 229 | Gemisch |
| I-1-c-31 | H | CH₃ | H | 4-Cl-Ph | 4'-O-(CH₂)₃-O- | | O | C₂H₅ | 207 | - |
| I-1-c-32 | CH₃ | CH₃ | Br | H | 4'-O-CH₂-CHCH₃-CH₂-O- | | O | C₂H₅ | 242 | Gemisch |
| I-1-c-33 | CH₃ | CH₃ | Br | H | 4'-O-CH₂-C(CH₃)₂-CH₂-O- | | O | C₂H₅ | 250 | - |
| I-1-c-34 | CH₃ | O-CH₃ | CH₃ | H | 4'-O-(CH₂)₃-O- | | O | C₂H₅ | 222 | - |
| I-1-c-35 | H | CH₃ | H | CH₃ | 4'-O-CH₂-C(CH₃)₂-CH₂-O- | | O | C₂H₅ | 194 | - |
| I-1-c-36 | H | CH₃ | H | CH₃ | 4'-O-CH₂-CHCH₃-CH₂-O- | | O | C₂H₅ | 169 | Gemisch |
| I-1-c-37 | CH₃ | C₂H₅ | Br | H | 4'-O-CHCH₃-CH₂-CHCH₃-O- | | O | C₂H₅ | 175-178 | Gemisch |
| I-1-c-38 | CH₃ | C₂H₅ | Br | H | 4'-O-CHCH₃-(CH₂)₂-O- | | O | C₂H₅ | 97 | Gemisch |
| I-1-c-39 | H | Cl | H | 4-Cl-Ph | 4'-O-(CH₂)₃-O- | | O | C₂H₅ | 237 | - |
| I-1-c-40 | H | CH₃ | H | CH₃ | 3'-O-(CH₂)₂-O- | | O | C₂H₅ | 156 | - |
| I-1-c-41 | CH₃ | CH₃ | Cl | H | 3'-O-(CH₂)₂-O- | | O | C₂H₅ | 193 | - |
| I-1-c-42 | C₂H₅ | Br | CH₃ | H | 3'-O-(CH₂)₂-O- | | O | C₂H₅ | **2.58 (m,2H,Ar-CH₂-), 4.08 (q, 2H, OCH₂-CH₃) | - |
| I-1-c-43 | C₂H₅ | Br | CH₃ | H | 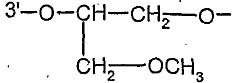 | | O | C₂H₅ | Öl**1.18 ("q",6H, Ar-CH₂-CH₃ u. O-CH₂-CH₃) 2.32 (s,3H,Ar-CH₃) | Gemisch |
| I-1-c-44 | C₂H₅ | Br | CH₃ | H | 3'-O-(CH₂)₂-O- | | O | (H₃C)₃ C-CH₂- | Schaum **0.8 (s,9H, C(CH₃)₃) 2.28 (s,3H,Ar-CH₃) | - |
| I-1-c-45 | H | Cl | H | 4-Cl-Ph | 4'-O-CHCH₃-CH₂-O- | | O | C₂H₅ | 219 | Gemisch |
| I-1-c-46 | H | Cl | H | 4-Cl-Ph | 4'-O-(CH₂)₂-O- | | O | C₂H₅ | 239 | - |

| | | | | | | | | | | |
|---|---|---|---|---|---|---|---|---|---|---|
| * ¹H-NMR (400 MHz, CDCl₃): Verschiebungen δ in ppm ** ¹H-NMR (300 MHz, CDCl₃): Verschiebungen δ in ppm | | | | | | | | | | |

### Beispiel I-1-d-1

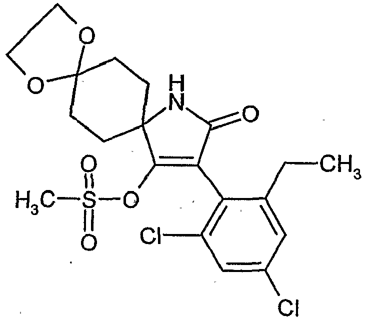

0,159 g der Verbindung gemäß Beispiel I-1-a-44 werden in 10 ml Trichlormethan vorgelegt, bei Raumtemperatur mit 0,098 ml Diisopropylethylamin und 1,5 mg DMAP versetzt, 0,037 ml Methansulfonsäurechlorid zugeben und bei 20 Stunden nachgerührt, mit 5 ml NaCl-Lösung versetzt, ca. 0,5 Stunden bei Raumtemperatur nachgerührt, die organische Phase abgetrennt, mit Natriumsulfat getrocknet und einrotiert. Das erhaltene Rohprodukt wird säulenchromatographisch an Kieselgel mit einem Gradienten n-Heptan/Essigsäureethylester 4:1 nach 0:100 gereinigt.

Ausbeute 0,07 g (37 % d. Theorie), Fp.: 217°C.

### Beispiel I-1-a-1

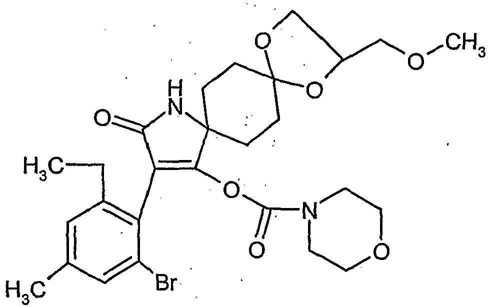

0,233 g der Verbindung gemäß Beispiel I-1-a-72 werden in 8 ml Chloroform vorgelegt, 0,098 ml Diisopropylethylamin und 1,5 mg DMAP zugegeben, anschließend noch 0,056 ml Morpholin-carbonsäurechlorid hinzugefügt, 20 Stunden bei Raumtemperatur gerührt, mit 5 ml 5 %iger Natriumbicarbonat-Lösung versetzt, 0,5 Stunden nachgerührt, die organische Phase abgetrennt, mit Natriumsulfat getrocknet und einrotiert. Das Rohprodukt wird säulenchromatographisch an Kieselgel mit einem Gradientn n-Heptan/Essigsäureethylester 4:1 nach 0:100 gereinigt.

Ausbeute: 0,148 g (64 % d. Theorie), Öl

¹H-NMR (400 MHz, CDCl₃): δ = 2.31 (2, 3H, Ar-CH₃), 3.1, 3.2 (je m, 2H, CH₂O) ppm.

### Beispiel II-1

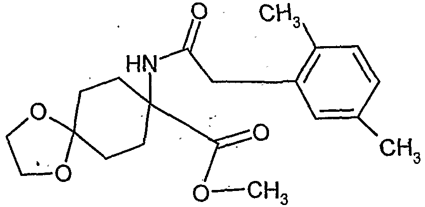

9.56 g 1,4-Dioxaspiro[4.5]decan-8-carbonsäure-8-amino-methylester-hydrochlorid werden in 38 ml wasserfreiem Acetonitril vorgelegt und 17.5 g (0.127 Mol) gemahlenes Kaliumcarbonat bei 5 bis 10°C zugegeben. Dann tropft man 6.94 g 2,5-Dimethyl-phenylessigsäurechlorid in 10 ml wasserfreiem Acetonitril in 12 Min. zu.

Man führt 3 h bei Raumtemperatur.

Die Reaktionslösung gießt man in 250 ml Eiswasser und saugt den Niederschlag ab. Man wäscht mit Wasser nach und nimmt den Niederschlag in Dichlormethan auf, trocknet und destilliert das Lösungsmittel ab. Es erfolgt säulenchromatographische Reinigung an Kieselgel (Dichlormethan/- Essigsäureethylester = 3:1).

Ausbeute: 6,82 g (49 % der Theorie), Fp. 115°C.

### Beispiel (II-2)

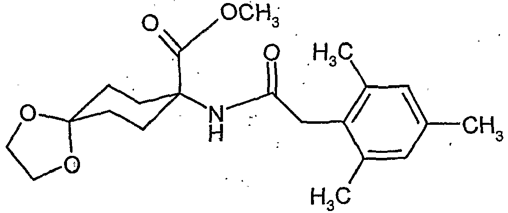

Es werden 154.6 g (1.579 Mol) konz. Schwefelsäure vorgelegt und 107.9 g der Verbindung gemäß Beispiel (XXI-1) in 630 ml Methylenchlorid als Suspension bei einer Innentemperatur von 30 bis 40°C zugetropft. Man rührt 2 h bei 30 bis 40°C und tropft dann 220 ml wasserfreies Methanol zu, dass sich eine Innentemperatur von 40°C einstellt.

Man rührt weitere 6 h bei einer Außentemperatur von 40 bis 70°C.

Die Reaktionslösung wird auf 1,5 kg Eis gegeben, mit Dichlormethan extrahiert und mit NaHCO₃-Lösung gewaschen. Man trocknet und destilliert das Lösungsmittel ab.

Es erfolgt säulenchromatographische Reinigung an Kieselgel (Toluol/Essigsäureethylester = 1:1).

Ausbeute: 11,4 g (10 % der Theorie).

In Analogie zu den Beispielen (II-1) und (II-2) und gemäß den allgemeinen Angaben zur Herstellung erhält man folgende Verbindungen der Formel (II)

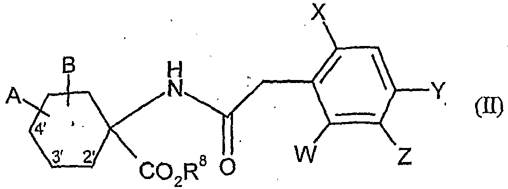

| Bsp.-Nr. | W | X | Y | Z | A | B | R⁸ | Fp °C |
|---|---|---|---|---|---|---|---|---|
| II-2 | CH₃ | CH₃ | CH₃ | H | 4'-O-(CH₂)₂-O- | | CH₃ | Log P 2.68 |
| II-3 | CH₃ | CH₃ | CH₃ | CH₃ | 4'-O-(CH₂)₂-O- | | CH₃ | 141 |
| II-4 | CH₃ | CH₃ | Cl | H | 4'-O-(CH₂)₂-O- | | CH₃ | 181 |
| II-5 | H | CH₃ | CH₃ | CH₃ | 4'-O-(CH₂)₂-O- | | CH₃ | 129 |
| II-6 | CH₃ | Cl | Cl | H | 4'-O-(CH₂)₂-O- | | CH₃ | 159 |
| II-7 | CH₃ | CH₃ | Br | H | 4'-O-(CH₂)₂-O- | | CH₃ | Wachs log P 2.81* |
| II-8 | CH₃ | C₂H₅ | CH₃ | H | 4'-O-(CH₂)₂-O- | | CH₃ | Wachs log P 2.95* |
| II-9 | CH₃ | C₂H₅ | Br | H | 4'-O-(CH₂)₂-O- | | CH₃ | Wachs log P 3.19* |
| II-11 | CH₃ | Cl | Cl | H | 4'-O-(CH₂)₃-O- | | CH₃ | 175 |
| II-12 | H | CH₃ | H | CH₃ | 4'-O-(CH₂)₃-O- | | CH₃ | 144 |
| II-13 | CH₃ | CH₃ | CH₃ | CH₃ | 4'-O-(CH₂)₃-O- | | CH₃ | 148 |
| II-14 | CH₃ | CH₃ | Cl | H | 4'-O-(CH₂)₃-O- | | CH₃ | 184 |
| II-15 | H | Cl | H | 4-Cl-Ph | 4'-O-(CH₂)₃-O- | | CH₃ | 177 |
| II-16 | H | CH₃ | H | 4-Cl-Ph | 4'-O-(CH₂)₃-O- | | CH₃ | 181 |
| II-17 | H | CH₃ | CH₃ | CH₃ | 4'-O-(CH₂)₃-O- | | CH₃ | log P 2.59* |
| II-18 | CH₃ | CH₃ | CH₃ | H | 4'-O-(CH₂)₃-O- | | CH₃ | 143 |
| II-19 | CH₃ | CH₃ | Br | H | 4'-O-(CH₂)₃-O- | | CH₃ | 189 |
| II-20 | CH₃ | CH₃ | Cl | H | 4'-O-CH₂-C(CH₃)₂-CH₂-O- | | CH₃ | 178 |
| II-21 | CH₃ | CH₃ | Cl | H | 4'-O-CH₂-CHCH₃-CH₂-O- | | CH₃ | 170 |
| II-22 | CH₃ | CH₃ | Br | H | 4'-O-CH₂-C(CH₃)₂-CH₂-O- | | CH₃ | 181 |
| II-23 | CH₃ | CH₃ | Br | H | 4'-O-CH₂-CHCH₃-CH₂-O- | | CH₃ | 177 |
| II-24 | CH₃ | OCH₃ | CH₃ | H | 4'-O-(CH₂)₃-O- | | CH₃ | log P 2.49* |
| II-25 | CH₃ | CH₃ | Br | H | 4'-O-CHCH₃-CHCH₃-O- | | CH₃ | 189 |
| II-26 | CH₃ | CH₃ | Br | H | 4'-O-CHCH₃-CH₂-O- | | CH₃ | 161 |
| II-27 | H | CH₃ | H | CH₃ | 4'-O-CHCH₃-CHCH₃-O- | | CH₃ | 166 |
| II-28 | CH₃ | CH₃ | Cl | H | 4'-O-CHCH₃-CHCH₃-O- | | CH₃ | 183 |
| II-29 | CH₃ | CH₃ | Cl | H | 4'-O-CHCH₃-CH₂-O- | | CH₃ | 170 |
| II-30 | H | CH₃ | H | CH₃ | 4'-O-CHCH₃-CH₂-O- | | CH₃ | 141 |
| II-31 | H | CH₃ | H | 5-(4-Cl-Ph) | 4'-O-(CH₂)₂-O- | | CH₃ | log P 3.47* |
| II-32 | C₂H₅ | C₂H₅ | CH₃ | H | 4'-O-(CH₂)₂-O- | | CH₃ | log P 3.28* |
| II-33 | CH₃ | OCH₃ | CH₃ | H | 4'-O-(CH₂)₂-O- | | CH₃ | 139 |
| II-34 | H | CH₃ | Cl | CH₃ | 4'-O-(CH₂)₂-O- | | CH₃ | 182 |
| II-35 | C₂H₅ | OCH₃ | Cl | H | 4'-O-(CH₂)₂-O- | | CH₃ | 153 |
| II-36 | C₂H₅ | Br | CH₃ | H | 4'-O-(CH₂)₂-O- | | CH₃ | log P 3.04* |
| II-37 | H | CH₃ | H | CH₃ | 4'-O-CHCH₃-CH₂-CHCH₃-O- | | CH₃ | 122 |
| II-38 | CH₃ | CH₃ | Cl | H | 4'-O-CHCH₃-CH₂-CHCH₃-O- | | CH₃ | 208 |
| II-39 | H | CH₃ | H | CH₃ | 4'-O-CHCH₃-(CH₂)₂-O- | | CH₃ | 144 |
| II-40 | CH₃ | CH₃ | Cl | H | 4'-O-CHCH₃-(CH₂)₂-O- | | CH₃ | 174 |
| II-41 | CH₃ | CH₃ | Br | H | 4'-O-CHCH₃-(CH₂)₂-O- | | CH₃ | 175 |
| II-42 | CH₃ | CH₃ | Br | H | 4'-O-CHCH₃-CH₂-CHCH₃-O- | | CH₃ | 202 |
| II-43 | CH₃ | C₂H₅ | Br | H | 4'-O-CHCH₃-CH₂-CHCH₃-O- | | CH₃ | 200 |
| II-44 | H | CH₃ | H | CH₃ | 4'-O-CH₂-C(CH₃)₂-CH₂-O- | | CH₃ | 157 |
| II-45 | H | CH₃ | H | CH₃ | 4'-O-CH₂-CHCH₃-CH₂-O- | | CH₃ | 154 |
| II-46 | CH₃ | C₂H₅ | Br | H | 4'-O-CHCH₃-(CH₂)₂-O- | | CH₃ | 158 |
| II-47 | CH₃ | Cl | Cl | H | 4'-O-CH₂-C(CH₃)₂-CH₂-O- | | CH₃ | 197 |
| II-48 | CH₃ | Cl | Cl | H | 4'-O-CH₂-CHCH₃-CH₂-O- | | CH₃ | 174 |
| II-49 | H | Cl | H | 4-Cl-Ph | 4'-O-CH₂-C(CH₃)₂-CH₂-O- | | CH₃ | 205 |
| II-50 | H | Cl | H | 4-Cl-Ph | 4'-O-CH₂-CHCH₃-CH₂-O- | | CH₃ | 166 |
| II-51 | H | CH₃ | H | 4-Cl-Ph | 4'-O-CH₂-C(CH₃)₂-CH₂-O- | | CH₃ | 184 |
| II-52 | H | CH₃ | H | 4-Cl-Ph | 4'-O-CH₂-CHCH₃-CH₂-O- | | CH₃ | 163 |
| II-53 | H | CH₃ | H | 4-Cl-Ph | 4'-O-CHCH₃-CH₂-CHCH₃-O- | | CH₃ | 149 |
| II-54 | CH₃ | C₂H₅ | Br | H | 4'-O-CH₂-C(CH₃)₂-CH₂-O- | | CH₃ | 241 |
| II-55 | CH₃ | C₂H₅ | Br | H | 4'-O-CH₂-CHCH₃-CH₂-O- | | CH₃ | 147 |
| II-56 | CH₃ | C₂H₅ | Br | H | 4'-O-CHCH₃-CH₂-O- | | CH₃ | 157 |
| II-57 | CH₃ | C₂H₅ | Br | H | 4'-O-CHCH₃-CHCH₃-O- | | CH₃ | 167 |
| II-58 | C₂H₅ | Cl | Cl | H | 4'-O-(CH₂)₂-O- | | CH₃ | 281 |
| II-59 | H | Cl | H | 4-Cl-Ph | 4'-O-(CH₂)₂-O- | | CH₃ | |
| II-60 | CH₃ | CH₃ | H | 4-Cl-Ph | 4'-O-CHCH₃-CH₂-O- | | CH₃ | 173 |
| II-61 | H | CH₃ | Cl | CH₃ | 4'-O-CHCH₃-CH₂-O- | | CH₃ | 165 |
| II-62 | H | CH₃ | H | 4-Cl-Ph | 4'-O-CHCH₃-CH₂-O- | | CH₃ | 174 |
| II-63 | H | CH₃ | H | 4-Cl-Ph | 4'-O-CHCH₃-CHCH₃-O- | | CH₃ | 151 |
| II-64 | H | Cl | H | 4-Cl-Ph | 4'-O-CHCH₃-CH₂-O- | | CH₃ | 181 |
| II-65 | H | Cl | H | 4-Cl-Ph | 4'-O-CHCH₃-CHCH₃-O- | | CH₃ | 182 |
| II-66 | H | CH₃ | H | CH₃ | 3'-O-(CH₂)₂-O- | | CH₃ | 111 |
| II-67 | C₂H₅ | Br | CH₃ | H | 3'-O-(CH₂)₂-O- | | CH₃ | 174 |
| II-68 | CH₃ | CH₃ | Cl | H | 3'-O-(CH₂)₂-O- | | CH₃ | 154 |
| II-69 | CH₃ | CH₃ | Cl | H | 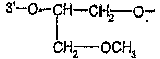 | | CH₃ | Öl |
| II-70 | CH₃ | CH₃ | CH₃ | H | 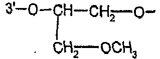 | | CH₃ | Öl |
| II-71 | H | CH₃ | H | CH₃ | 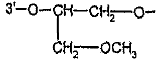 | | CH₃ | Öl |
| II-72 | C₂H₅ | Br | CH₃ | H | 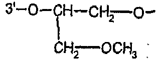 | | CH₃ | Öl |
| II-73 | C₂H₅ | Br | CH₃ | H | 4'-O-CHCH₃-CH₂-O- | | CH₃ | 143 |
| II-74 | H | Cl | H | 4-Cl-Ph | 4'-O-CH₂-CHCH₃-CH₂-O- | | CH₃ | 169 |
| II-75 | CH₃ | C₂H₅ | Br | H | 4'-O-(CH₂)₃-O- | | CH₃ | 165 |
| II-76 | H | Cl | H | 4-Cl-Ph | 4'-O-CHCH₃-(CH₂)₂-O- | | CH₃ | 161 |
| II-77 | H | Cl | H | 4-Cl-Ph | 4'-O-CHCH₃-CH₂-CHCH₃-O- | | CH₃ | 74 |
| II-18 | C₂H₅ | Br | CH₃ | H | 4'-O-CHCH₃-CH₂-CHCH₃-O- | | CH₃ | 84 |

| | | | | | | | | |
|---|---|---|---|---|---|---|---|---|
| * Die Bestimmung der in den voran stehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C | | | | | | | | |

Die Bestimmung erfolgt im sauren Bereich bei pH 2.3 mit 0,1 % wässriger Phosphorsäure und Acetonitril als Eluenten; linearer Gradient von 10 % Acetonitril bis 95 % Acetonitril.

Die Bestimmung mit der LC-MS im sauren Bereich erfolgt bei pH 2,7 mit 0,1 % wässriger Ameisensäure und Acetonitril (enthält 0,1 % Ameisensäure) als Eluenten; linearer Gradient von 10 % Acetonitril bis 95 % Acetonitril.

Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigtem Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanolen).

Die lambda-max-Werte wurden an Hand der UV-Spektren von 200 nm bis 400 nm in den Maxima der chromatographischen Signale ermittelt.

### Beispiel (XXI-1)

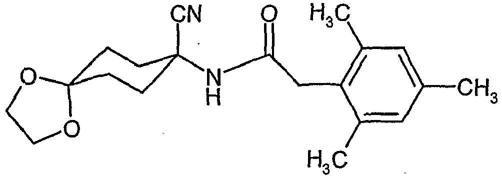

Zu 65 g 1,4-Dioxaspiro[4.5]decan-8-amino-8-carbonsäurenitril in 800 ml wasserfreiem Tetrahydrofuran (THF) gibt man 49.4 ml Triethylamin. Bei 0 bis 10°C tropft man 70.2 g Mesitylenessigsäurechlorid in 80 ml wasserfreiem THF zu.

Man rührt bei Raumtemperatur.

Nach Reaktionsende (dünnschichtchromatographische Kontrolle) rührt man die Reaktionslösung in 1,2 1 Eiswasser ein, gibt 200 ml IN HCl zu und saugt den Niederschlag ab. Dieser wird in Dichlormethan aufgenommen und die Wasserphase abgetrennt Die organische Phase wird getrocknet, das Lösungsmittel abdestilliert und der Rückstand aus 320 ml Toluol/640 ml n-Hexan umkristallisiert.

Ausbeute: 107,9 g (88 % der Theorie), Fp. >220°C.

### Beispiel XIV-1-1

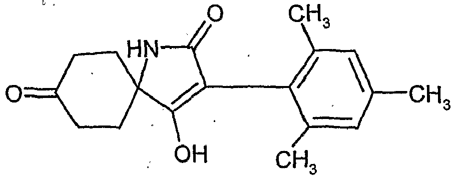

20.6 g der Verbindung gemäß Beispiel I-1-c-2 werden in 24 g halbkonzentrierter Natronlauge und ca. 100 ml Ethanol bei Raumtemperatur eine Stunde unter Argon-Schutzgas gerührt und anschließend das Ethanol abgedampft. Dann wird die Lösung mit Salzsäure angesäuert, alles in Methylenchlorid gelöst, die organische Phase abgetrennt und eingedampft. Der Rückstand wird zwei Stunden bei 60°C in 4 N Salzsäure gerührt.

Der Niederschlag wird abgetrennt und in Essigsäureethylester gerührt.

Ausbeute: 6,67 g; Fp: 293,9°C

### Beispiel XVI-1

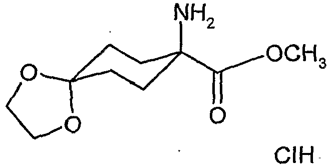

46 g 1,4-Dioxa[4.5]decan-8-amino-8-carbonsäure werden in 350 ml wasserfreiem Methanol vorgelegt und bei 0 bis 5°C 32.6 g (0.275 Mol) Thionylchlorid zugetropft. Es wird 30 Min. bei 0°C (Suspension) und dann über Nach bei 40°C gerührt.

Man kühlt ab, saugt das Kaliumchlorid ab und destilliert das Lösungsmittel ab. Der Rückstand wird mit MTB-Ether verrieben und abgesaugt.

Ausbeute: 48,63 g (83 % der Theorie), Fp: Zersetzung

In Analogie zu Beispiel XVI-1 erhält man folgende neuen Verbindungen der Formel (XVI)

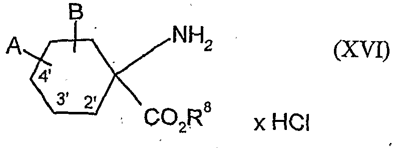

| Bsp.-Nr. | A | B | R⁸ | Fp °C |
|---|---|---|---|---|
| XVI-2 | 4'-O-CHCH₃-CH₂-O- | | CH₃ | * 1.18-1.19 (2d, 3H, CHCH₃) |
| | | | | 3.76 (2s, 3H, OCH₃) |
| XVI-3 | 4'-O-CHCH₃-CHCH₃-O- | | CH₃ | * 3.76 (s, 3H, OCH₃) |
| | | | | 4.18-4.23 (m, 2H, 2xCH-O) |
| XVI | 4'-O-(CH₂)₃-O- | | CH₃ | Harz |
| XVI-5 | 4'-O-CHCH₃-(CH₂)₂-O- | | CH₃ | * 1.07-1.08 (d, 3H, CHCH₃) |
| | | | | 3.77 (s, 3H, OCH₃) |
| XVI-6 | -O-CH₂-CHCH₃-CH₂-O- | | CH₃ | * 0.77 (d, 3H, CHCH₃) |
| | | | | 8.71 (3H, br, NH₃+) |
| XVI-7 | 4'-O-CHCH₃-CH₂-CHCH₃-O- | | CH₃ | * 1.07-1.15 (m, 6H, 2xCHCH₃) |
| | | | | 3.93-4.0 (m, 2H, O-CH) |
| XVI-8 | 4'-O-CH₂-C(CH₃)₂-CH₂-O- | | CH₃ | * 0.9 (s, 6H, C(CH₃)₂) |
| | | | | 3.76 (s, 3H, OCH₃) |
| XVI-9 | 3'-O-(CH)₂-O- | | CH₃ | * 3.74,3.75 (2s, 3H, CO₂CH₃) |
| | | | | 3.79-3.99 (m, 4H, -O-(CH₂)₂-O-) |
| XVI-10 | 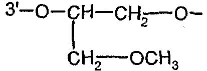 | | CH₃ | * 3.28 (2s, 3H, OCH₃) |
| | | | | 3.73-3.75 (4s, 3H, CO₂CH₃) |

| | | | | |
|---|---|---|---|---|
| *¹H-NMR (400 MHz, d₆-DMSO): Verschiebung δ in ppm | | | | |

Die substituierten 1,4-Dioxa[4,5]decan-8-amino-8-carbonsäureester und deren Salze der Formel (XVI'), die gegebenenfalls substituierten 1,4-Dioxa[4,5]decan-7-amino-7-carbonsäureester und deren Salze der Formel (XVI") und die gegebenenfalls substituierten 1,5-Dioxa[5,5]undecan-9-amino-9-carbonsäureester und deren Salze der Formel (XVI"'), in welchen als Substituenten die eingangs in der Definition von A, B genannten Substituenten in Frage kommen, sind neu und Gegenstand der Erfindung: 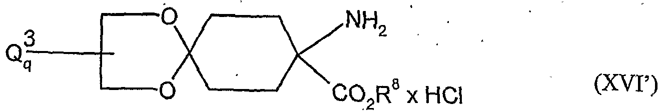 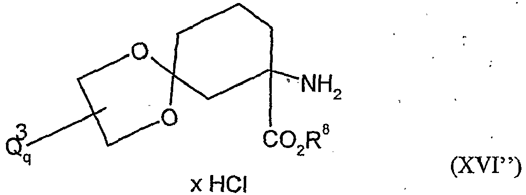 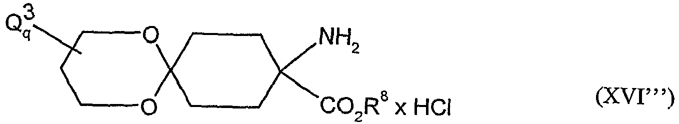 in welcher
- Q³: für C₁-C₄-Alkyl, C₁-C₃-Halogenalkyl, C₁-C₄-Alkoxy oder C₁-C₄-Alkoxy-C₁-C₂-alkyl steht,
- q: für 1, 2 oder 3 steht und im Fall der Verbindungen der Formeln (XVI") und (XVI"') auch für 0 stehen kann,
- R⁸: die oben angegebene Bedeutung hat.

### Beispiel XIX-1

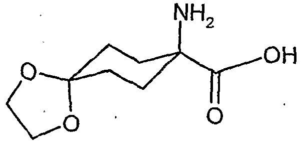

112 g der Verbindung gemäß Beispiel XXIII-8 werden in 710 ml 30 %iger KOH suspendiert und unter Rückfluss unter Stickstoffatmosphäre gerührt.

Bei 0 bis 20°C mit konz. HCl aus pH 5.2 bis 5.3 ansäuern, absaugen, das Filtrat auf ca. 0,5 l azeotrop mit Methanol einengen, Kaliumchlorid absaugen, einrotierten, mit Methanol azeotrop entwässern.

Ausbeute: 46 g (46 % der Theorie).

In Analogie zu Beispiel XIX-1, erhält man folgende neuen Verbindungen der Formel (XIX)

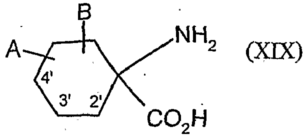

| Bsp.-Nr. | A | B |
|---|---|---|
| XIX-2 | 4'-O-CHCH₃-CH₂-O- | |
| XIX-3 | 4'-O-CHCH₃-CHCH₃-O- | |
| XIX-4 | 4'-O-(CH₂)₃-O- | |
| XIX-5 | 4'-O-CHCH₃-(CH₂)₂-O- | |
| XIX-6 | 4'-O-CH₂-CHCH₃-CH₂-O- | |
| XIX-7 | 4'-O-CHCH₃-CH₂-CHCH₃-O- | |
| XIX-8 | 4'-O-CH₂-C(CH₃)₂-CH₂-O- | |
| XIX-9 | 3'-O-(CH₂)₂-O- | |
| XIX-10 | 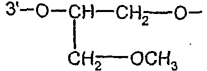 | |

Die Aminosäuren wurden ohne weitere Reinigung direkt in die Veresterung zu den Verbindungen (XVI) umgesetzt. Dort erfolgte die Charakterisierung. Die substituierten 1,4-Dioxa[4,5]decan-8-amino-8-carbonsäuren (XIX'), die gegebenenfalls substituierten 1,4-Dioxa[4,5]-decan-7-amino-7-carbonsäuren der Formel (XIX") und die gegebenenfalls substituierten 1,5-Dioxa[5,5]undecan-9-amino-carbonsäuren der Formel (XIX"'), in welchen als Substituenten die eingangs in der Definition der Reste A, B genannten Substituenten in Frage kommen, sind neu und Gegenstand der Erfindung: 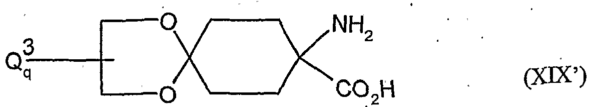 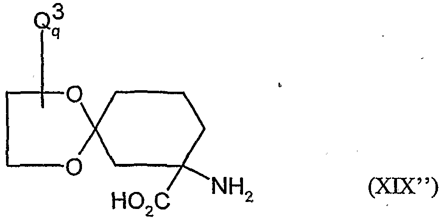 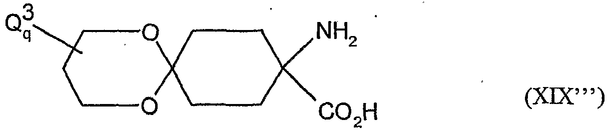 in welcher
- Q³: die oben genannten Bedeutungen hat und
- q: für 1, 2 oder 3 steht und im Fall der Verbindungen der Formeln (XIX") und (XIX"') auch für 0 stehen kann.

### Beispiel XXIII-1

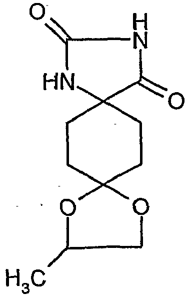

16,4 g (335 mmol) Natriumcyanid und 128,7 g (1339 mmol) Ammoniumcarbonat werden in 600 ml Wasser vorgelegt und bei Raumtemperatur 57 g (335 mmol) 1,4-Dioxa[4,5]decan-2-methyl-8-on (XXIV-1) in 30 ml Ethanol gelöst langsam zugetropft.

Man rührt ca. 12 bis 15 Stunden bei 55 bis 60°C.

Auf 0°C kühlen, Feststoff absaugen, mit Wasser erneut anschlämmen, absaugen, mit Hexan waschen, trocknen.

Ausbeute: 73,6 g (92 % der Theorie). 2 Isomere 1:1.

In Analogie zu Beispiel (XXIII-1) erhält man folgende neuen Verbindungen der Formel (XXIII)

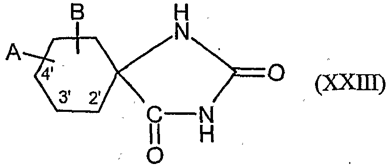

| Bsp.-Nr. | A | B | ¹H-NMR (400 MHz) | Verschiebungen δ in ppm |
|---|---|---|---|---|
| XXIII-1 | 4'-O-CHCH₃-CH₂-O- | | d₆-DMSO | 10.53 (s); 8.35 (s); 4.15 (m, 1H); 4.02 (m, 1H); 3.37 (m, 1H); 1.93-1.50 (6m, 8H); 1.18 (dd, J=6.2 Hz, 6.5 Hz. 3H) |
| XXIII-2 | 4'-O-CHCH₃-CHCH₃-O- | | d₆-DMSO 2 Diastereomeren 2:1 | 10.52 (s); 8.35 (s); 4.18 (m) + 3.59 (m): Σ2H; 1.90-1.50 (bm, Σ8H); 1.18 (dd, J=6.3Hz, 5.8 Hz) + 1.06 (dd,J=6.1 Hz, 7.7 Hz): Σ6H |
| XXIII-3 | 4'-O-(CH₂)₃-O- | | d₆-DMSO | 10.50 (s); 8.35 (s); 3.81 (m, 4H); 2.12 (m, 2H); 1.78 (m, 2H); 1.60 (m, 4H); 1.48 (m, 2H) |
| XXIII-4 | 4'-O-CHCH₃-(CH₂)₂-O- | | d₆-DMSO 2 Diastereomeren | 10.50 (s); 8.31/8.30 (je s.); 4.09-3.67 (6m, 3H); 2.54 (m, 1H); 1.88 (m, 1H); 1.76-1.39 (6m, 8H); 1.08 (dd,J=6Hz) |
| XXIII-5 | 4'-O-CH₂-CHCH₃-CH₂-O- | | d₆-DMSO | 10.51 (s); 8.34 (s); 3.73 (m,2H); 3.51 (dd,J=9.7 Hz, 11.5 Hz, 1H); 3.43 (dd,J=9.7 Hz, 11.5 Hz, 1H); 2.41 (m, 1H); 1.88-1.41 (bm, 8H); 0,76 (d,J=6.8 Hz, 3H) |
| XXIII-6 | 4'-O-CHCH₃-CH₂-CHCH₃-O- | | d₆-DMSO 2 Diastereomeren 2:1 | 10.53 (s); 8.28 (s); 4.02 + 3.92 (je m, Σ2H); 1.95 (6m).+ 1.75-1.40 (bm): Σ10H; 1.12 (dd,J=6.3 Hz, 10,8 Hz) + 1,08 (dd,J=6.1 Hz, 11.3 Hz): Σ6H |
| XXIII-7 | 4'-O-CH₂-C(CH₃)₂-CH₂-O- | | d₆-DMSO | 10.50 (s); 8.34 (s); 3.45 (s, 2H); 3.43 (s, 2H); 2.11 (m, 2H); 1.78 (m, 2H); 1.62 (m, 2H); 1.50 (m, 2H); 0.89 (s, 6H) |
| XXIII-8 | 3'-O-(CH₂)₂-O- | | d₆-DMSO | 10.6 (bm, 1H), 7.32 (bm, 1H), 3.9 (m, 4H), 1.71 (m, 4H), 1.61 (m, 1H), 1.52 (m, 3H) |
| XXIII-9 | 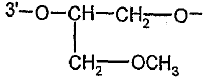 | | d₆-DMSO Isomerengemisch | 10.5 (bm), 7.16 (bm), 6.98 (bm), 6.87 (bm), 6.60 (bm), 4.22 (m), 4.01 (m), 3.64 (m), 3.34 (m), 3.27 (m), 3.17 (s), 2.40 (m), 2.22 (m), 2.01 (m), 1.90-1.60 (bm) |

Die Verbindungen der Formeln (XXIII'), (XXIII") und (XXIII"') 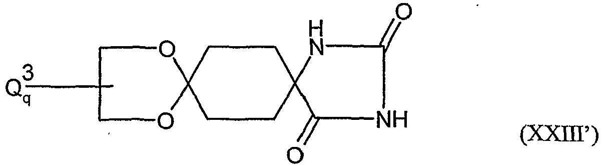 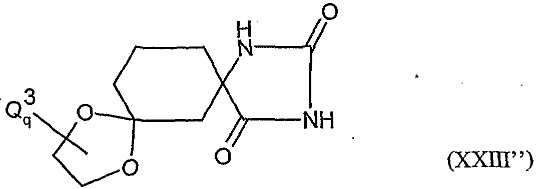 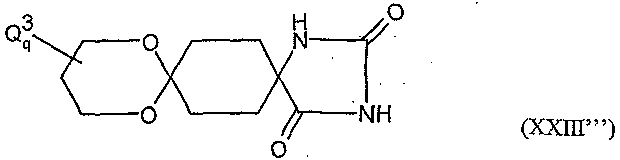 in welcher
- Q³: die oben genannten Bedeutungen hat und
- q: für 1, 2 oder 3 steht und im Fall der Verbindungen der Formel (XXIII") und (XXIII"') auch für 0 stehen kann, sind neu und Gegenstand der Erfindung.

### Beispiel XXIV-1

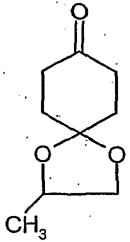

53,7 g (423 mmol) Oxalsäuredichlorid werden in 1000 ml Dichlormethan vorgelegt. Bei -70°C wird eine Lösung von 60 ml Dimethylsulfoxid in 100 ml Dichlormethan hinzugetropft und 20 min. nachgerührt. 66 g (385 mmol) 1,4-Dioxa[4,5]decan-2-methyl-8-ol werden in 300 ml Dichlormethan gelöst und bei gleicher Temperatur zur Reaktionslösung über 1 h hinzugetropft. 1 Stunde nachrühren, dann das Eisbad herunterfahren und innerhalb von 30 min. auf -40°C kommen lassen. 30 min. bei dieser Temperatur rühren, dann Triethylamin (140 ml) hinzugeben und auf Raumtemperatur kommen lassen (1 h); und über Nacht rühren lassen.

Es wird mit 500 ml Wasser gequencht und 15 Min. nachgerührt,

Die organische Phase wird abgetrennt und die wässrige Lösung 3 x mit 200 ml Dichlormethan extrahiert. Die vereinigten organischen Phasen werden mit Natriumsulfat getrocknet und das Lösungsmittel abdestilliert. Chromatographische Reinigung an Kieselgel, Hexan-Essigester 2:1.

Ausbeute: 58 g (88 % d. Theorie)

¹H-NMR (400 MHz, d₆-DMSO): δ = 4.24 (m, 1H), 3.46 (t, 1H), 4.10 (dd, 1H), 2.36 (m, 4H), 1.94 (m, 4H), 1.23 (d, 3H) ppm.

### Beispiel XXIV-7

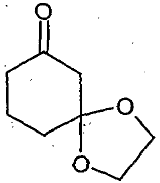

50 g Cyclohexan-1,3-dion, 27,67 g 1,2-Ethandiol, 500 ml Toluol und 0,849 g 4-Toluolsulfon-säuremonohydrat werden zusammengeben und am Wasserabscheider 3 bis 4 h lang unter Rückfluss gekocht. Das Gemisch wird mit 100 ml ges. NaHCO₃-Lösung und 2 x mit ges. NaCl-Lösung gewaschen, getrocknet und einrotiert. Anschließend wird eine frakt. Destillation im HV (Dest. des Produkts bei 85°C/lmbar bzw. 62-75°C/0,15 mm Hg) durchgeführt.

Ausbeute: 21,9 g (27 % d. Theorie)

¹H-NMR (400 MHz, CD₃CN): δ = 3.9 (m, 4H), 2.52 (s, 2H), 2.27 (m, 2H), 1.87 (m, 2H), 1.79 (m, 2H) ppm.

In Analogie zu den Beispielen (XXIV-1) und (XXIV-7) erhält man folgende neuen Verbindungen der Formel (XXIV)

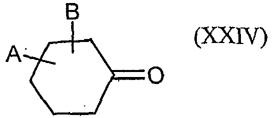

| Bsp.-Nr. | A | B | 1H-NMR (400 MHz) | Verschiebung δ in ppm |
|---|---|---|---|---|
| XXIV-1 | 4'-O-CHCH₃-CH₂-O- | | d₆-DMSO | 4.24 (m, 1H); 3.46 (t, 1H, J=7.7 Hz); 4.10 (dd, 1H, J=5.8 HZ, 7.9 Hz); 2.36 (m, 4H); 1.94 (m, 4H); 1.23 (d, J==6Hz/3H) |
| XXIV-2 | 4'-O-CHCH₃-CHCH₃-O- | | d₆-DMSO 2 Isomeren 2:1 | 4.28/3.68 (je m, ∑2H); 2.35/1.94 (je m, ∑8H); 1.22 (d.J=5.7 Hz) + 1.10 (d, J=6.1Hz); ∑6H |
| XXIV-3 | 4'-O-(CH₂)₃-O- | | d₆-DMSO | 3.88 (dd,J=5.6Hz, 4H); 2.25 (m, 4H); 2.08 (m, 4H); 1.64 (m, 2H) |
| XXIV-4 | 4'-O-CHCH₃-(CH₂)₂-O- | | in d₆-DMSO | 4.03 (m, 1H); 3.93 (m, 1H); 3.80 (m, 1H); 2.30 (m, 2H); 2.22 (m, 4H); 1.87 (m, 2H); 1.51 (m, 2H); 1.10 (d, 3H, J=6.1 Hz) |
| XXIV-5 | 4'-O-CH₂-CHCH₃-CH₂-O- | | d₆-DMSO 1 Isomer | 3.81 (dd, J=4.7 Hz, 12.0 Hz, 2H); 3.52 (dd, 2H, J=9.7 Hz); 2.28 (m, 2H); 2.21 (m, 4H); 1.95 (m, 2H); 1.91 (m, 1H); 0.80 (d, J=6.8 Hz, 1H) |
| XXIV-6 | 4'-O-CHCH₃-CH₂-CHCH₃-O- | | d₆-DMSO 2 Isomere=45:55 | 4.02 (m, 2H); 2.31/2.21/1.97/ 1.87/1.61 (jeweils m, ∑10H); 1.17 (d, 3H, J=6.3 Hz); 1.12 (d, 3H, J=6.1 Hz) |
| XXIV-8 | 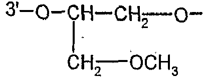 | | CD₃CN Isomerengemisch | 4.23 (m), 4.01 (m), 3.66 (m), 3.38 (m), 3.31 (s), 2.54 (s), 2.26 (m), 1.87 (m), 1.78 (m), 1.56 (m) |

Die Ketone der Formel (XXIV) sind teilweise käuflich, teilweise bekannt (JACS 108, 2691-2699; 1986; JACS 109, 1363-1370, 1987) und teilweise neu.

### Beispiel XXV-1

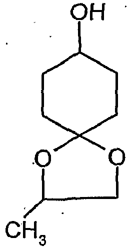

114 g 4-Hydroxycyclohexanon und 80 g 1,2 Propandiol (77 ml) und 2 g 4-Toluolsulfonsäuredihydrat werden in 500 ml Toluol zusammengegeben. Man kocht am Wasserabscheider bis kein

Wasser mehr abgeschieden wird. Zur Aufarbeitung gibt man 1 ml Triethylamin, wäscht mit 200 ml Wasser, trocknet und rotiert die Reaktionslösung ein. Der Rückstand (104 g) wird im Vakuum destilliert.

Ausbeute: 76,3 g ≙ 44 % d. Theorie

In Analogie zu Beispiel (XXIV-1) erhält man folgende neuen Verbindungen der Formel (XXIV)

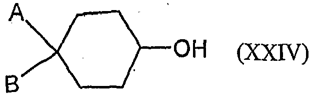

| Bsp.-Nr. | A | B | 1H-NMR (400 MHz, d₆-DMSO) Verschiebung δ in ppm |
|---|---|---|---|
| XXV-1 | -O-CHCH₃-CH₂-O- | | 4.38 (dd, 1 H J=4.2 Hz, 5.3 Hz); 4.12 (m, 1H); 3.97 (m, 1H); 3.54 (6s, 1H); 3.33 (m, 1H); 1.67 (m, 4H); 1.44 (m, 4H); 1.16 (dd,J=6.1HZ, 3H); |
| XXV-2 | -O-CHCH₃-CHCH₃-O- | | 4.35 (m); 4.16 (m); 3.54 (m); 1.67 (m); 1.44 (m); 1.15 (dd,J= 5.7 Hz); 1.03 (dd,J=6.1 Hz) |
| XXV-3 | -O-(CH₂)₃-O- | | 4.35 (OH); 3.77 (m, 4H); 3.52 (m, 1H); 1.96 (m, 2H); 1.57 (m, 4H); 1.50-128 (m,4H) |
| XXV-4 | -O-CHCH₃-(CH₂)₂-O- | | 4.34 (m, 1H); 3.95 (m, 1H); 3.87 (m, 1H); 3.67 (m, 1H); 3.53 (m, 1H); 2.25 (m, 1H); 2.66-2.20 (m, 10H); 1.06 (dd,J=6.1 Hz, 3H) |
| XXV-5 | -O-CH₂-CHCH₃-CH₂-O- | | 4.41 (bs, OH); 3.69 (m) + 3.53 (m) + 3.43 (m) + 3.35 (m) + 3.25 (m): ∑5 H; 2.12 (m, 1H); 1.74 (m, 2H); 1.62-1.23 (m, 5H); 0.80 (d.J=6.8 Hz) + 0.75 (d,J = 6.8 Hz): ∑3 H |
| XXV-6 | -O-CHCH₃-CH₂-CHCH₃-O- | | 4.34 (s,OH); 3.94 (m); 3,53 (m) 2.20 (m); 1.78 (m); 2.65-2.20 (bm); 1.10 (dd,J= 6.3 Hz); 1.05 (dd,J = 6 Hz) |

Die Verbindungen der Formel (XXV) sind teilweise kommerziell erhältlich, teilweise bekannt (J. Med. Chem. 24, 341-346, 1981) und teilweise neu.

### Beispiel I-2-a-1

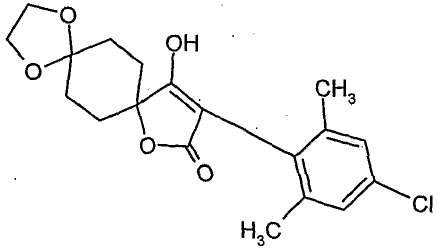

0,32 g (1,00 mmol) der Verbindung gemäß Beispiel XIV-2-1, 0,62 g (10 mmol) Ethylenglykol und 50 mg 4-Toluolsulfonsäure werden in 15 ml Toluol über Nacht refluxiert.

Zur Aufarbeitung wird abgekühlt, der Feststoff abgesaugt und getrocknet.

Ausbeute: 0,35 g (96 % der Theorie), Fp. 267°C.

In Analogie zu Beispiel (I-2-a-1) und gemäß den allgemeinen Angaben zur Herstellung erhält man folgende Verbindungen der Formel (I-2-a):

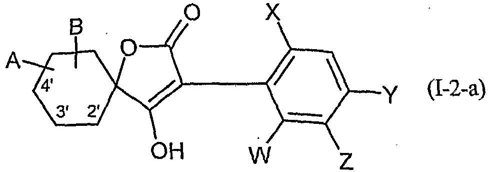

| Bsp.-Nr. | W | X | Y | Z | A | B | Fp °C |
|---|---|---|---|---|---|---|---|
| I-2-a-2 | CH₃ | CH₃ | CH₃ | H | 4'-O-(CH₂)₂-O- | | >250 |
| I-2-a-3 | CH₃ | CH₃ | Cl | H | 4'-O-CHCH₃-CH₂-O- | | 198 |
| I-2-a-4 | CH₃ | CH₃ | Cl | H | 4'-O-CHCH₂Cl-CH₂-O- | | 248 |
| I-2-a-5 | CH₃ | CH₃ | Br | H | 4'-O-(CH₂)₂-O- | | 266 |
| I-2-a-6 | CH₃ | CH₃ | Cl | H | 4'-O-(CH₂)₃-O- | | 227 |
| I-2-a-7 | CH₃ | CH₃ | Cl | H | 4'-O-CH₂-C(CH₃)₂-CH₂-O- | | 270 |
| I-2-a-8 | H | CH₃ | H | 4-Cl-Ph | 4'-O-(CH₂)₂-O- | | * |

| | | | | | | | |
|---|---|---|---|---|---|---|---|
| 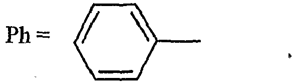 *¹H-NMR (400 MHz, d₆-DMSO): δ = 2.19 (s, 3H, Ar-CH₃), 3.92 (s, 4H,(O-CH₂-)₂), 7.3 (m, 1H, Ar-H), 7.4 (m, 1H, Ar-H), 7.5 (m, 3H, Ar-H), 7.65 (m, 2H-Ar-H) ppm. | | | | | | | |

### Beispiel I-2-b-1

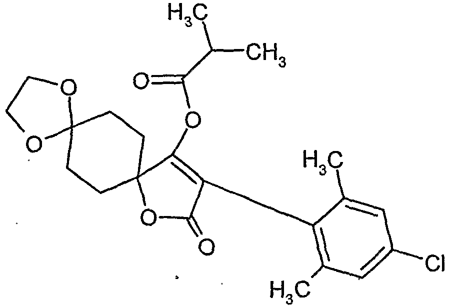

0,22 g (0,60 mmol) der Verbindung gemäß Beispiel (I-2-a-1) werden in 10 ml Dichlormethan vorgelegt, 0,067 g (0,66 mmol) Triethylamin zugesetzt, unter Eiskühlung 0,071 g (0,66 mol) Isobuttersäurechlorid zugetropft und über Nacht bei Raumtemperatur gerührt.

Zur Aufarbeitung wird mit 10 %iger Zitronensäurelösung und gesättigter Natriumhydrogencarbonatlösung extrahiert, die organische Phase mit Natriumsulfat getrocknet und einrotiert.

Ausbeute: 0,22 g (77 % der Theorie), Fp. 142°C

In Analogie zu Beispiel (I-2-b-1) und gemäß den allgemeinen Angaben zur Herslellung erhält man folgende Verbindungen der Formel (I-2-b):

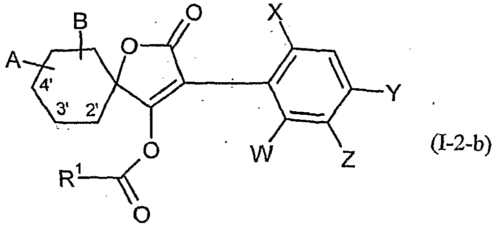

| Bsp.-Nr. | W | X | Y | Z | A | B | R¹ | Fp °C |
|---|---|---|---|---|---|---|---|---|
| I-2-b-2 | CH₃ | CH₃ | CH₃ | H | 4'-O-(CH₂)₂-O- | | t-C₄H₉ | 165-169 |
| I-2-b-3 | CH₃ | CH₃ | Cl | H | 4'-O-CHCH₂Cl-CH₂-O- | | i-C₃H₇ | 240-243 |
| I-2-b-4 | CH₃ | CH₃ | Cl | H | 4'-O-CHCH₃-CH₂-O- | | i-C₃H₇ | 232 |

### Beispiel XIV-2-1

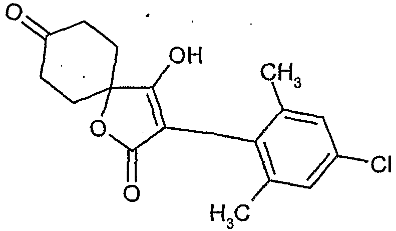

4,59 g (40,89 mmol) Kalium-tert.-butylat werden in 35 ml Dimethylformamid vorgelegt, eine Lösung von 10,00 g (27,26 mmol) der Verbindung gemäß Beispiel (III-A-1) in 15 ml Dimethyl-formamid unter Eiskühlung bei 0 bis 10°C zugetropft und über Nacht bei Raumtemperatur gerührt.

Zur Aufarbeitung rotiert man das DMF am Rotationsverdampfer weitgehend ab und verteilt den Rückstand zwischen Wasser und Essigester. Die wässrige Phase wird mit Salzsäure angesäuert, das ausgefallene Produkt abgesaugt, mit Wasser gewaschen und getrocknet.

Die weitere Reinigung erfolgt durch präparative HPLC (Kieselgel RP-18, Eluent Acetonitril/-Wasser).

Ausbeute: 2,20 g (25 % der Theorie), Fp. 210°C.

In Analogie zu Beispiel (XIV-2-1) und gemäß den allgemeinen Angaben zur Herstellung von Verbindungen der Formel (XIV-2) in der WO 99/16 748 erhält man folgende Verbindungen der Formel (XIV-2):

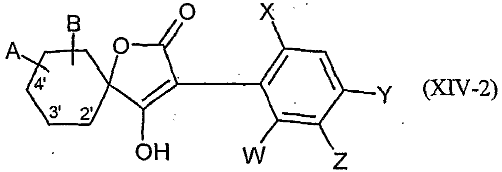

| Bsp.-Nr. | W | X | Y | Z | A | B | Fp °C |
|---|---|---|---|---|---|---|---|
| XIV-2-2 | CH₃ | CH₃ | CH₃ | H | 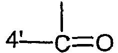 | | 187-190 |
| XIV-2-3 | CH₃ | CH₃ | Br | H | 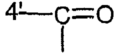 | | 222 |

### Beispiel III-A-1

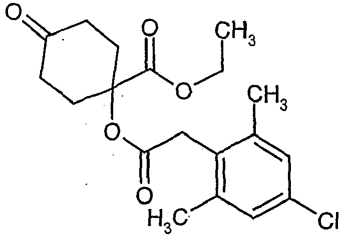

5,00 g (26,85 mmol) 4-Oxo-1-hydroxy-cyclohexancarbonsäure-ethylester und 3,26 g (32,22 mmol) Triethylamin werden in 35 ml Dichlormethan vorgelegt, eine Lösung von 6,41 g (29,53 mmol) 2,6-Dimethyl-4-chlor-phenylessigsäurechlorid in 15 ml Dichlormethan unter Eisbadkühlung bei 0 bis 10°C zugetropft und über Nacht bei Raumtemperatur gerührt.

Zur Aufarbeitung wird mit 10 %iger Zitronensäurelösung und gesättigter Natriumhydrogencarbonatlösung extrahiert, die organische Phase mit Natriumsulfat getrocknet und einrotiert.

Ausbeute: 10,00 g Öl (67 % der Theorie).

In Analogie zu Beispiel (III-A-1) und gemäß den allgemeinen Angaben zur Herstellung erhält man folgende Verbindungen der Formel (III-A):

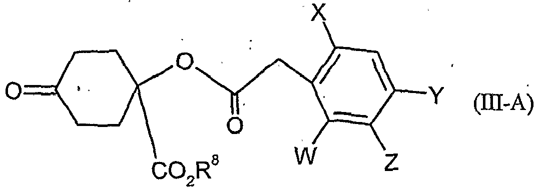

| Bsp.-Nr. | W | X | Y | Z | R⁸ | Fp°C |
|---|---|---|---|---|---|---|
| III-A-2 | CH₃ | CH₃ | CH₃ | H | C₂H₅ | Öl |
| III-A-3 | CH₃ | CH₃ | Br | H | C₂H₅ | Öl |

Die Öle wurden ohne weitere Charakterisierung in die Synthese von Verbindungen der Formel (XIV-2) eingesetzt.

### Beispiel A

### Myzus persicae -Test (7:1)

| | | |
|---|---|---|
| Lösungsmittel: | 7 | Gewichtsteile Dimethylformamid |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea),* die stark von der Grünen Pfirsichblattlaus *(Myzus persicae)* befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele eine Wirkung von ≥ 80 % bei einer Konzentration von 1000 ppm: Bsp. I-1-a-2, Bsp. I-1-a-1, Bsp. I-1-a-3, Bsp. I-1-a-4, Bsp. I-1-b-1, Bsp. I-1-c-3, Bsp. I-1-c-4, Bsp. I-1-c-1.

### Beispiel B

### Aphis gossypii -Test (7:1)

| | | |
|---|---|---|
| Lösungsmittel: | 7 | Gewichtsteile Dimethylformamid |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblätter *(Gossypium hirsutum),* die stark von der Baumwollblattlaus *(Aphis gossypii)* befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Konzentration von 200 ppm: Bsp. I-1-a-2, Bsp. I-1-a-3, Bsp. I-1-a-4, Bsp. I-1-c-1.

### Beispiel C

### Phaedon cochleariae - Larven -Test (7:1)

| | | |
|---|---|---|
| Lösungsmittel: | 7 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea)* werden durch Tauchen in die Wirkstoffzubereitung der gewünschten Konzentration behandelt und mit Larven des Meerrettichblattkäfers *(Phaedon cochleariae)* besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele eine Wirkung von ≥ 90 % bei einer Konzentration von 1000 ppm: Bsp. I-2-a-2, Bsp. I-2-b-2, Bsp. I-1-a-2, Bsp. I-1-a-1, Bsp. I-1-a-3, Bsp. I-1-a-4, Bsp. I-1-c-3, Bsp. I-1-c-4, Bsp. I-1-c-5, Bsp. I-1-b-1, Bsp. I-1-c-1.

### Beispiel D

### Plutella-Test (7:1)

| | | |
|---|---|---|
| Lösungsmittel: | 7 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea)* werden durch Tauchen in die Wirkstoffzubereitung der gewünschten Konzentration behandelt und mit Raupen der Kohlschabe *(Plutella xylostella)* besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele eine Wirkung von 100 % bei einer Konzentration von 1000 ppm: Bsp. I-2-a-2, Bsp. I-2-b-2, Bsp. I-1-a-2.

### Beispiel E

### Spodoptera frugiperda-Test (7:1)

| | | |
|---|---|---|
| Lösungsmittel: | 7 | Gewichtsteile Dimethylformamid |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea)* werden durch Tauchen in die Wirkstoffzubereitung der gewünschten Konzentration behandelt und mit Raupen des Heerwurms *(Spodoptera frugiperda)* besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z.B. die folgenden Verbindungen der Herstellungsbeispiele eine Wirkung von ≥ 85 % bei einer Konzentration von 1000 ppm: Bsp. I-2-a-2, Bsp. I-2-b-2, Bsp. I-1-a-2, Bsp. I-1-a-3, Bsp. I-1-c-3.

### Beispiel F

### Nephotettix-Test (7:1)

| | | |
|---|---|---|
| Lösungsmittel: | 7 | Gewichtsteile Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Reiskeimlinge *(Oryza sativa)* werden durch Tauchen in die Wirkstoffzubereitung der gewünschten Konzentration behandelt und mit der Grünen Reiszikade *(Nephotettix cincticeps)* besetzt, solange die Blätter noch feucht sind.

Nach der gewünschten Zeit wird die Abtötung in % bestimmt. Dabei bedeutet 100%, dass alle Zikaden abgetötet wurden; 0% bedeutet, dass keine Zikaden abgetötet wurden.

Bei diesem Test zeigen z.B. die folgenden Verbindungen der Herstellungsbeispiele eine Wirkung von 100 % bei einer Konzentration von 1000 ppm: Bsp. I-2-a-2, Bsp. I-2-b-2, Bsp. I-1-a-2.

### Beispiel G

### Tetranychus-Test (7:1) (OP-resistent/Tauchbehandlung)

| | | |
|---|---|---|
| Lösungsmittel: | 7 | Gewichtsteile Dimethylformamid |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen *(Phaseolus vulgaris),* die stark von allen Stadien der Gemeinen Spinnmilbe *(Tetranychus urticae)* befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von ≥ 98 % bei einer Konzentration von 100 ppm: Bsp. I-1-a-1, Bsp. I-1-a-3, Bsp. I-1-a-4, Bsp. I-1-c-4.

### Beispiel H

### Meloidogyne-Test (7:1)

| | | |
|---|---|---|
| Lösungsmittel: | 7 | Gewichtsteile Dimethylformamid |
| Emulgator: | 1 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, *Meloidogyne incognita*-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigt z. B. die folgende Verbindung der Herstellungsbeispiele eine Wirkung von 95 % bei einer Konzentration von 20 ppm: Bsp. I-1-a-4.

### Beispiel I

### Myzus persicae -Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea),* die stark von der Grünen Pfirsichblattlaus *(Myzus persicae)* befallen sind, werden durch Tauchen in die Wirkstoffzubereitung der gewünschten Konzentration behandelt

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Konzentration von 100 ppm: Bsp. I-2-a-5, Bsp. I-1-a-11, Bsp. I-1-a-14, Bsp. I-1-a-12, Bsp. I-1-a-13.

### Beispiel J

### Tetranychus-Test (OP-resistent/Tauchbehandlung)

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen *(Phaseolus vulgaris),* die stark von allen Stadien der Gemeinen Spinnmilbe *(Tetranychus urticae)* befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgende Verbindungen der Herstellungsbeispiele eine Wirkung von ≥ 90 % bei einer Konzentration von 100 ppm: Bsp. I-1-a-11, Bsp. I-1-a-14, Bsp, I-1-a-12, Bsp. I-1-a-21, Bsp. I-1-a-22, Bsp. I-1-a-9, Bsp. I-1-a-43, Bsp. I-1-a-46, Bsp. I-1-a-53, Bsp. I-1-a-70, Bsp. I-1-c-16, Bsp. I-1-c-20.

### Beispiel K

### Meloidogyne-Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung *Meloidogyne incognita*-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigt z. B. die folgende Verbindung der Herstellungsbeispiele eine Wirkung von 80 % bei einer Konzentration von 20 ppm: Bsp. I-1-a-12.

### Beispiel L

### Aphis gossypii -Test

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblätter *(Gossypium hirsutum),* die stark von der Baumwollblattlaus *(Aphis gossypii)* befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen.der Herstellungsbeispiele eine Wirkung von ≥ 80 % bei einer Konzentration von 100 ppm: Bsp. I-1-a-19, Bsp. I-1-a-7, Bsp. I-1-a-14, Bsp. I-1-a-30, Bsp. I-1-a-31, Bsp. I-1-a-38, Bsp. I-1-a-48, Bsp. I-1-a-50, Bsp. I-1-a-53, Bsp. I-1-a-56, Bsp. I-1-a-58, Bsp. I-1-a-63, Bsp. I-1-b-4, Bsp. I-1-c-15, Bsp.I-1-c-19, Bsp.I-1-c-20, Bsp.I-1-c-32, Bsp.I-2-a-5.

### Beispiel M

### Phaedon cochleariae - Larven -Test

| | | |
|---|---|---|
| Lösungsmittel: | 7 | Gewichtsteile Dimethylformamid |
| Emulgator: | 2 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea)* werden durch Tauchen in die Wirkstoffzubereitung der gewünschten Konzentration behandelt und mit Larven des Meerrettichblattkäfers *(Phaedon cochleariae)* besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden.

Bei diesem Test zeigt z. B. die folgende Verbindung der Herstellungsbeispiele eine Wirkung von 100 % bei einer Konzentration von 100 ppm: Bsp. I-1-a-23.

### Beispiel N

### Phaedon-Test (Spritzbehandlung)

| | | |
|---|---|---|
| Lösungsmittel: | 78 | Gewichtsteile Aceton |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirk-stoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettich-blattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z.B. die folgenden Verbindungen der Herstellungsbeispiele eine Wirkung von ≥ 80 % bei einer Aufwandmenge von 100 g/ha: Bsp. I-1-a-15, Bsp. I-1-c-9, Bsp. I-1-a-21 und eine Wirkung von 100 % bei einer Aufwandmenge von 500 g/ha: Bsp. I-1-a-18, Bsp. I-1-a-20, Bsp.I-1-a-22, Bsp.I-1-a-8, Bsp.I-1-a-10, Bsp.I-1-a-7, Bsp. I-1-a-9, Bsp. I-1-c-6, Bsp.I-1-a-16, Bsp. I-1-a-24, Bsp. I-1-a-27, Bsp. I-1-a-30, Bsp. I-1-a-31, Bsp. I-1-a-32, Bsp. I-1-a-33, Bsp. I-1-a-34, Bsp. I-1-a-38, Bsp. I-1-a-39, Bsp. I-1-a-43, Bsp. I-1-a-45, Bsp. I-1-a-46, Bsp. I-1-a-47, Bsp. I-1-a-49, Bsp. I-1-a-51, Bsp. I-1-a-52, Bsp. I-1-a-53, Bsp. I-1-a-54, Bsp. I-1-a-56, Bsp. I-1-a-61, Bsp. I-1-a-63, Bsp. I-1-a-64, Bsp. I-1-a-65, Bsp. I-1-a-66, Bsp. I-1-a-67, Bsp. I-1-a-68, Bsp. I-1-a-71, Bsp. I-1-c-19, Bsp. I-1-c-31, Bsp. I-1-c-34.

### Beispiel O

### Tetranychus-Test (OP-resistent/Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von ≥ 80 % bei einer Aufwandmenge von 100 g/ha: Bsp. I-2-b-1, Bsp. I-1-a-16, Bsp.I-1-a-15, Bsp.I-1-c-9, Bsp.I-1-c-11, Bsp.I-1-a-23, Bsp. I-1-c-6, Bsp. I-1-a-24, Bsp. I-1-a-27 und Bsp. I-1-a-38, Bsp. I-1-a-50, Bsp. I-1-a-58, Bsp. I-1-c-24, Bsp. I-1-c-43.

### Beispiel P

### Meloidogyne-Test (Spritzbehandlung)

| | | |
|---|---|---|
| Lösungsmittel: | 80 | Gewichtsteile Aceton |

Zur Herstellung einer zweckmäßigen Wirkstoffzubereitung vermischt man 1 Gewichtsteil Wirk-stoff mit der angegebenen Menge Lösungsmittel und verdünnt das Konzentrat mit Wasser auf die gewünschte Konzentration.

Gefäße werden mit Sand, Wirkstofflösung, Meloidogyne incognita-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigt z. B. die folgende Verbindung der Herstellungsbeispiele eine Wirkung von 100 % bei einer Konzentration von 20 ppm: Bsp. I-1-a-15.

### Beispiel Q

### Myzus-Test (Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (Brassica pekinensis), die von allen Stadien der Grünen Pfirsichblattlaus (Myzus persicae) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele eine Wirkung von ≥ 90 % bei einer Aufwandmenge von 500 g/ha: Bsp. I-1-a-18, Bsp. I-1-a-19, Bsp.I-1-c-10, Bsp.I-1-a-20, Bsp.I-1-c-11, Bsp.I-1-a-23, Bsp. I-1-a-21, Bsp. I-1-a-22, Bsp. I-1-a-8, Bsp. I-1-a-10, Bsp. I-1-a-7, Bsp.I-1-a-9, Bsp.I-1-c-6, Bsp.I-2-a-1, Bsp.I-2-a-3, Bsp. I-2-a-4, Bsp. I-2-a-7, Bsp. I-2-b-1, Bsp.I-2-b-3, Bsp.I-2-b-4, Bsp. I-2-a-5, Bsp. I-1-a-16, Bsp. I-1-a-24, Bsp. I-1-a-26, Bsp. 1-1-a-28, Bsp. I-1-a-29, Bsp. I-1-a-30, Bsp. I-1-a-31, Bsp. I-1-a-32, Bsp. I-1-a-33, Bsp. I-1-a-34, Bsp. I-1-a-35, Bsp. I-1-a-36, Bsp. I-1-a-37, Bsp. I-1-a-38, Bsp. I-1-a-39, Bsp. I-1-a-40, Bsp. I-1-a-41, Bsp. I-1-a-42, Bsp. I-1-a-43, Bsp. I-1-a-44, Bsp. I-1-a-45, Bsp. I-1-a-46, Bsp. I-1-a-47, Bsp.I-1-a-48, Bsp. I-1-a-49, Bsp. I-1-a-50, Bsp. I-1-a-51, Bsp. I-1-a-52, Bsp. I-1-a-53, Bsp. I-1-a-54, Bsp. I-1-a-56, Bsp. I-1-a-57, Bsp. I-1-a-58, Bsp. I-1-a-59, Bsp. I-1-a-60, Bsp. I-1-a-61, Bsp. I-1-a-62, Bsp. I-1-a-63, Bsp. I-1-a-64, Bsp. I-1-a-65, Bsp. I-1-a-66, Bsp. I-1-a-67, Bsp. I-1-a-69, Bsp. I-1-a-70, Bsp. I-1-a-71, Bsp. I-1-b-4, Bsp. I-1-b-6, Bsp. I-1-b-7, Bsp. I-1-b-8, Bsp. I-1-b-10, Bsp. I-1-b-11, Bsp. I-1-c-14, Bsp. I-1-c-15, Bsp. I-1-c-16, Bsp. I-1-c-17, Bsp. I-1-c-18, Bsp. I-1-c-19, Bsp. I-1-c-20, Bsp. I-1-c-21, Bsp. I-1-c-22, Bsp. I-1-c-23, Bsp. I-1-c-24, Bsp. I-1-c-25, Bsp. I-1-c-26, Bsp. I-1-c-30, Bsp. I-1-c-31, Bsp.I-1-c-32, Bsp. I-1-c-33, Bsp. I-1-c-34, Bsp. I-1-c-35, Bsp. I-1-c-36, Bsp. I-1-c-40, Bsp. I-1-c-41, Bsp. I-1-c-42, Bsp. I-1-c-43.

### Beispiel R

### Spodoptera frugiperda-Test (Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (Zea mays) werden mit einer Wirkstoffzubereitung der gewünschten Konzentration gespritzt und nach dem Abtrocknen mit Raupen des Heerwurms (Spodoptera frugiperda) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z.B. die folgenden Verbindungen der Herstellungsbeispiele eine Wirkung von ≥ 80 % bei einer Aufwandmenge von 500 g/ha: Bsp. I-2-a-7, Bsp. I-1-a-16, Bsp.I-1-a-21, Bsp.I-1-a-22, Bsp.I-1-a-8, Bsp.I-1-a-10, Bsp. I-1-a-27, Bsp. I-1-a-31, Bsp.I-1-a-49, Bsp.I-1-a-51, Bsp.I-1-a-52, Bsp.I-1-a-54, Bsp.I-1-a-64, Bsp.I-1-a-65, Bsp.I-1-a-66, Bsp.I-1-a-67, Bsp.I-1-c-16, Bsp.I-1-c-19, Bsp.I-1-c-31, Bsp. Bsp.I-1-c-36, Bsp.I-1-c-40.

### Beispiel S

### Myzus persicae -Test (hydroponischeBehandlung)

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Die Wirkstoffzubereitung wird mit Wasser gemischt. Die angegebene Konzentration bezieht sich auf die Wirkstoffmenge pro Volumeneinheit Wasser (mg/l = ppm). Man füllt das behandelte Wasser in Gefäße mit einer Erbsenpflanze *(Pisum sativum).* Nach der gewünschten Zeit wird mit der Grünen Pfirsichblattlaus *(Myzus persicae)* infiziert.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgende Verbindungen der Herstellungsbeispiele eine Wirkung von ≥ 95 % bei einer Konzentration von 20 ppm: Bsp. I-1-a-23, Bsp. I-1-a-21, Bsp.I-1-a-7, Bsp.I-1-a-24, Bsp.I-1-a-26, Bsp.I-1-a-30, Bsp. I-1-a-32, Bsp. I-1-a-34, Bsp. I-1-a-35, Bsp. I-1-a-36, Bsp. I-1-a-38, Bsp. I-1-a-39, Bsp. I-1-a-40, Bsp.I-1-a-42, Bsp.I-1-a-43, Bsp.I-1-a-44, Bsp.I-1-a-45, Bsp.I-1-a-46, Bsp.I-1-a-47, Bsp.I-1-a-48, Bsp.I-1-a-50, Bsp.I-1-a-53, Bsp.I-1-a-56, Bsp.I-1-a-57, Bsp.I-1-a-58, Bsp.I-1-a-59, Bsp.I-1-a-69, Bsp.I-1-a-70, Bsp.I-1-a-71, Bsp.I-1-b-4, Bsp.I-1-b-8, Bsp.I-1-b-10, Bsp.I-1-b-11, Bsp.I-1-c-15, Bsp.I-1-c-17, Bsp.I-1-c-19, Bsp.I-1-c-20, Bsp.I-1-c-23, Bsp.I-1-c-24, Bsp.I-1-c-25, Bsp.I-1-c-32, Bsp.I-1-c-43.

### Beispiel T

### Post-emergence-Test

| | | |
|---|---|---|
| Lösungsmittel: | 5 | Gewichtsteile Aceton |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5- 15 cm haben so, dass die jeweils gewünschten Wirkstoffmengen pro Flächeneinheit ausgebracht werden. Die Konzentration der Spritzbrühe wird so gewählt, dass in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten
0 % = keine Wirkung (wie unbehandelte Kontrolle)
100 % = totale Vernichtung

### Beispiel U

### Pre-emergence-Test (Prüfung von Wirkstoffen Herbizide Monheim)

| | | |
|---|---|---|
| Lösungsmittel: | 5 | Gewichtsteile Aceton |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung bespritzt so, dass die jeweils gewünschten Wirkstoffmengen pro Flächeneinheit ausgebracht werden. Die Konzentration der Spritzbrühe wird so gewählt, dass in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten
0% = keine Wirkung (wie unbehandelte Kontrolle)
100 % = totale Vernichtung

| Gewächshaus | Wirkstoffkonzentration g a.i./ha | Echinochloa | Setaria | Amaranthus | Galium | Sinapis |
|---|---|---|---|---|---|---|
| Bsp. I-1-a-4 | 250 | 100 | 100 | 95 | 70 | 70 |

| Gewächshaus | Wirkstoffkonzentration g a.i./ha | Alopecurus | A vena fatua | Echinochloa | Setaria | Sinapis |
|---|---|---|---|---|---|---|
| Bsp. I-1-c-4 | 250 | 80 | - | 90 | 99 | 80 |
| Bsp. I-1-b-1 | 250 | 70 | 70 | 80 | 95 | 70 |
| Bsp. I-1-a-11 | 250 | 90 | 70 | 80 | 90 | - |

| Gewächs-haus | Wirkstoffkonzentration g a.i./ha | Zuckerrüben | Alopecurus | Avena fatua | Echinochloa | Setaria |
|---|---|---|---|---|---|---|
| Bsp. I-1-a-14 | 250 | 0 | 95 | 80 | 80 | 90 |
| Bsp. I-1-a-15 | 250 | 0 | 95 | 80 | 99 | 70 |

| Gewächshaus | Wirkstoffkonzentration g a.i./ha | Alopecurus | Echinochloa | Setaria |
|---|---|---|---|---|
| Bsp. I-1-a-11 | 250 | 80 | 80 | 100 |

### Beispiel V

### 1. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten Testverbindungen werden dann als wässrige Suspension mit einer Wasseraufwandmenge von umgerechnet 600 l/ha unter Zusatz von 0,2% Netzmittel in unterschiedlichen Dosierungen auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Auflaufschäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen).

Folgende Verbindungen zeigen neben den zuvor genannten Verbindungen im Vorauflauf mit 320 g/ha a.i. gegen Solium multiflorum und Setaria virichs eine Wirkung von ≥ 80 %: Bsp. I-1-a-24, Bsp. I-1-a-39, Bsp. I-1-a-40, Bsp. I-1-a-42, Bsp. I-1-a-43, Bsp. I-1-a-44, Bsp. I-1-a-45, Bsp. I-1-a-48, Bsp. I-1-a-69, Bsp. I-1-a-71, Bsp. I-1-b-4, Bsp. I-1-b-5, Bsp. I-1-b-8, Bsp. I-1-b-9, Bsp. I-1-b-10, Bsp. I-1-c-14, Bsp. I-1-c-15, Bsp. I-1-c-23, Bsp. I-1-c-25, Bsp. I-1-c-42 und Bsp. I-1-c-43.

### 2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2-3 Wochen nach der Aussaat werden die Versuchspflanzen im Einblattstadium behandelt. Die als Spritzpulver (WP) formulierten Testverbindungen werden in verschiedenen Dosierungen mit einer Wasseraufwandmenge von umgerechnet 600 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

| Gewächshaus | Wirkstoffkonzentration g a.i./ha | Lolium | Setaria | Sinapis | Echinochloa |
|---|---|---|---|---|---|
| Bsp. I-1-a-18 | 320 | 80 | 100 | - | 90 |
| Bsp. I-1-a-19 | 320 | 90 | 90 | 70 | 90 |
| Bsp. I-2-a-6 | 320 | 80 | 80 | 60 | 60 |
| Bsp. I-2-b-3 | 320 | 100 | 90 | - | 80 |
| Bsp. T-1-a-2 | 320 | 70 | 90 | 60 | 80 |
| Bsp. I-1-a-7 | 320 | 80 | 80 | 60 | 60 |
| Bsp. I-1-a-21 | 320 | 80 | 90 | 70 | 80 |
| Bsp. I-1-a-22 | 320 | 70 | 80 | 70 | 100 |
| Bsp. I-1-c-6 | 320 | 90 | 90 | 60 | 80 |

| Gewächshaus | Wirkstoffkonzentration g a.i./ha | Lolium | Setaria | Amaranthus | Sinapis | Echinochloa |
|---|---|---|---|---|---|---|
| Bsp. I-2-a-2 | 320 | 90 | 100 | 60 | 70 | 80 |

| Gewächshaus | Wirkstoffkonzentration g a.i./ha | Lolium | Setaria | Amaranthus | Stellaria |
|---|---|---|---|---|---|
| Bsp. I-2-a-5 | 320 | 100 | 70 | 70 | 60 |

Neben den zuvor genannten Verbindungen zeigen folgende Verbindungen im Nachauflauf mit 320 g/ha a.i. gegen Echinocloa crus-galli, Lolium multiflorum und Setaria viridis eine Wirkung von ≥ 80 %: Bsp. I-1-a-10, Bsp. I-1-a-18, Bsp. I-1-a-19, Bsp. I-1-a-21, Bsp. I-1-a-24, Bsp. I-1-a-25, Bsp. I-1-a-27, Bsp. I-1-a-39, Bsp. I-1-a-40, Bsp. I-1-a-42, Bsp. I-1-a-45, Bsp. I-1-a-48, Bsp. I-1-a-71, Bsp. I-1-b-8, Bsp. I-1-b-9, Bsp. I-1-b-10, Bsp. I-1-b-11, Bsp. I-1-c-6, Bsp. I-1-c-15, Bsp. I-1-c-23, Bsp. I-1-c-25, Bsp. I-1-c-26, Bsp. I-1-c-42, Bsp. I-1-c-43, Bsp. I-2-a-2, und Bsp. I-2-bs3.

### Versuchsbeschreibung für Profiling - Versuche

### 2. Herbizide Wirkung im Nachauflauf

Samen von mono- bzw. dikotylen Unkraut- bzw. Kulturpflanzen werden in Holzfasertöpfen oder in Plastiktöpfen in sandigem Lehmboden ausgelegt, mit Erde abgedeckt und im Gewächshaus, während der Vegetationsperiode auch im Freien außerhalb des Gewächshauses, unter guten Wachstumsbedingungen angezogen. 2 bis 3 Wochen nach der Aussaat werden die Versuchspflanzen im Ein- bis Drei-Blattstadium behandelt Die als Spritzpulver (WP) oder Flüssigkeit (EC) formulierten Testverbindungen werden in verschiedenen Dosierungen mit einer Wasseraufwandmenge von umgerechnet 300 l/ha unter Zusatz von Netzmittel (0,2 bis 0,3 %) auf die Pflanzen und die Bodenoberfläche gespritzt. 3 bis 4 Wochen nach Behandlung der Versuchspflanzen wird die Wirkung der Präparate visuell im Vergleich zu unbehandelten Kontrollen bonitiert (herbizide Wirkung in Prozent (%): 100 % Wirkung = Pflanzen sind abgestorben, 0 % Wirkung = wie Kontrollpflanzen).

### Verwendung von Safenern:

Soll zusätzlich getestet werden, ob Safener die Pflanzenverträglichkeit von Testsubstanzen bei den Kulturpflanzen verbessern können, werden folgende Möglichkeiten für die Anwendung des Sageners verwendet:
- Samen der Kulturpflanzen werden vor der Aussaat mit der Safenersubstanz gebeizt (Angabe der Safenermenge in Prozent bezogen auf das Samengewicht)
- Kulturpflanzen werden vor Anwendung der Testsubstanzen mit dem Safener mit einer bestimmten Hektaraufwandmenge gespritzt (üblicherweise 1 Tag vor Anwendung der Prüfsubstanzen)
- der Safener wird zusammen mit der Testsubstanz als Tankmischung appliziert (Angabe der Safenermenge in g/ha oder als Verhältnis zum Herbizid).

Durch Vergleich der Wirkung von Testsubstanzen auf Kulturpflanzen, welche ohne und mit Safener behandelt wurden, kann die Wirkung der Safenersubstanz beurteilt werden.

### Gefäßversuche mit Getreide im Gewächshaus

### Mefenpyr 1 Tag vor Herbizidapplikation

**Tabelle 1**

| | | 10 Tage nach Applikation | |
|---|---|---|---|
| | Aufwandmenge g a.i./ha | Sommergerste beobachtet (%) | Sommerweizen beobachtet (%) |
| | 25 | 50 | 40 |
| I-1-a-25 | 12,5 | 30 | 20 |
| I-1-a-25 | 25 + 100 | 15 | 10 |
| + Mefenpyr | 12,5 + 100 | 10 | 5 |

**Tabelle 2**

| | | 28 Tage nach Applikation |
|---|---|---|
| | Aufwandmenge g a.i./ha | Sommerweizen beobachtet (%) |
| | 50 | 60 |
| | 25 | 20 |
| T-1-a-25 | 12,5 | 10 |
| | 50 + 100 | 10 |
| I-1-a-25 | 25 + 100 | 5 |
| + Mefenpyr | 12,5 + 100 | 0 |

**Tabelle 3**

| | 28 Tage nach Applikation | | |
|---|---|---|---|
| | Aufwandmenge g a.i./ha | Sommergerste beobachtet (%) | Sommerweizen beobachtet (%) |
| | 23,8 | 50 | 70 |
| I-1-c-25 | 11,9 | | 20 |
| I-1-c-25 | 23,8 | 20 | 20 |
| + Mefenpyr | 11,9 | | 10 |

### Beispiel W

### Grenzkonzentrations-Test / Bodeninsekten- Behandlung transgener Pflanzen

| | |
|---|---|
| Testinsekt: | Diabrotica balteata - Larven im Boden |
| Lösungsmittel: | 7 Gewichtsteile Aceton |
| Emulgator: | 1 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 Töpfe und lässt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel X

### Heliothis virescens - Test - Behandlung transgener Pflanzen

| | | |
|---|---|---|
| Lösungsmittel: | 7 | Gewichtsteile Aceton |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

## Patentansprüche

1. Verbindungen der Formel (I) 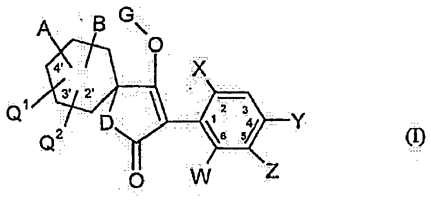 in welcher
W für Wasserstoff, Alkyl, Alkenyl, Alkinyl, Alkoxy, Halogen, Alkenyloxy, Halogenalkyl, Halogenalkoxy oder Cyano steht,
X film Halogen, Alkyl Alkenyl, Alkinyl, Alkoxy, Alkenyloxy, Alkylthio, Alkylsulfinyl, Alkylsulfonyl, Halogenalkyl, Halogenalkoxy, Halogenalkenyloxy, Nitro oder Cyano steht,
Y und Z unabhängig voneinander für Wasserstoff, Alkyl, Alkenyl, Alkinyl, Alkoxy, Halogen, Halogenalkyl, Halogenalkoxy, Cyano, Nitro oder jeweils gegebenenfalls substituiertes Aryl oder Hetaryl steht,
mit der Maßgabe, dass mindestens einer der Reste W odar Z ungleich Wasserstoff sein muss, wenn X und Y für Methyl stehen,
A und B und das Kohlenstoffiatorm an das sie gebunden sind, für jeweils gegebenenfalls durch Alkyl, Halogenalkyl, Alkoxy, Alkoxyalkyl oder gegebenenfalls substituiertes Phenyl substituiertes fünf- bis siebengliedriges Ketal, Thioketal oder Dithioketal steht, welches gegebenenfalls durch ein weiteres Heteroatom unterbrochen sein kann,
D für NH und Sauerstoff steht,
Q¹, Q² unabhängig voneinander für Wasserstoff, Alkyl, Halogenalkyl oder Alkoxy stehen,
G für Wasserstoff (a) oder für eine der Gruppen 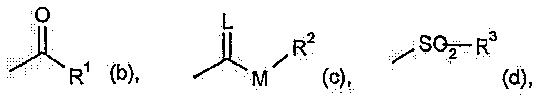 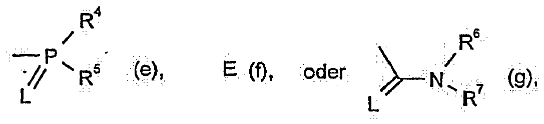 steht, worin
E für ein Metallion oder ein Ammoniumion steht,
L für Sauerstoff oder Schwefel steht,
M für Sauerstoff oder Schwefel steht,
R¹ für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht,
R² für jeweils gegebenenfalls durch Halogen oder Cyano substituiertes Alkyl, Alkenyl, Alkoxyalkyl oder Polyalkoxyalkyl oder für jeweils gegebenenfalls substituiertes Cycloakyl, Phenyl oder Benzyl steht,
R³, R⁴ und R⁵ unabhängig voneinander für jeweils gegebenenfalls durch Halogen substituiertes Alkyl, Alkoxy, Alkylamino, Dialkylamino, Alkylthio, Alkenylthio oder Cycloalkylthio oder für jeweils gegebenenfalls substituiertes Phenyl, Benzyl, Phenoxy oder Phenylthio stehen,
R⁶ und R⁷ unabhängig voneinander für Wasserstoff, für jeweils gegebenenfalls durch Halogen oder Cyano substituiertes Alkyl, Cycloalkyl, Alkenyl, Alkoxy, Alkoxy-alkyl, für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden und gegebenenfalls substituierten Cyclus bilden.

2. Verbindungen, der Formel (I) gemäß Anspruch 1, in welcher
W für Wasserstoff C₁-C₆-Alkyl, C₂-C₆-Alkenyl, C₂-C₆-Alkinyl, Halogen, C₁-C₆-Alkoxy, C₁-C₄-Halogenalkyl, C₁-C₄-Halogenalkoxy oder Cyano steht,
X für Halogen, C₁-C₆-Alkyl, C₂-C₆-Alkenyl; C₂-C₆-Alkinyl, C₁-C₆-Halogenalkyl; C₁-C₆-Alkoxy, C₃-C₆-Alkenyloxy, C₁-C₆-Alkylthio, C₁-C₆-Alkylsulfinyl, C₁-C₆-Alkylsulfonyl, C₁-C₆-Halogenalkoxy, C₃-C₆-Halogenakenyloxy, Nitro oder Cyano steht,
Y und Z unabhängig voneinander für Wasserstoff, Halogen, C₁-C₆-Alkyl, C₂-C₆-Alkenyl, C₂-C₆-Alkinyl, C₁-C₆-Alkoxy, C₁-C₆-Halogenalkyl, C₁-C₆-Halogenalkoxyl, Cyano, C₂-C₆-Alkenyl, C₂-C₆-Alkinyl oder für einen der (Het)-arylreste 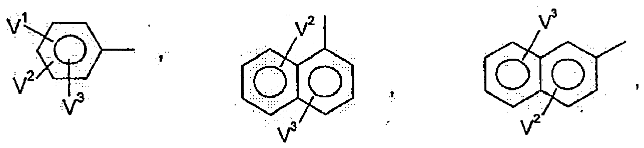 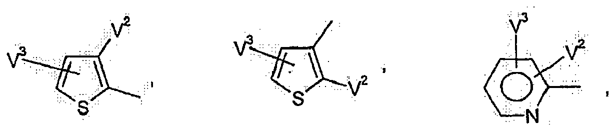 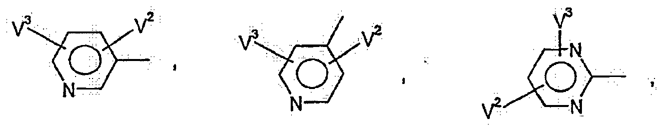 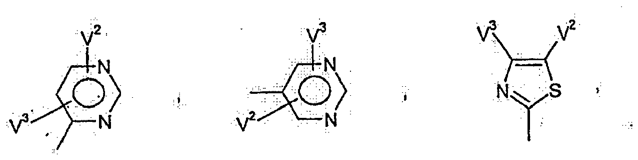 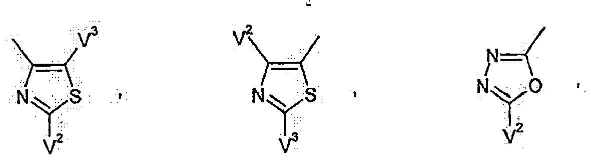 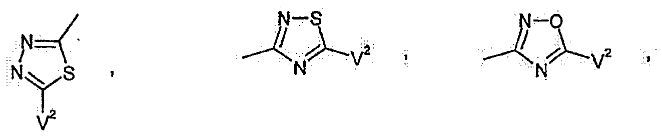 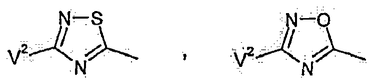 stehen,
wobei im Falle von (Het)-aryl nur einer der Reste Y oder Z für (Het)-aryl stehen darf,
V¹ für Wasserstoff, Halogen, C₁-C₁₂-Alkyl, C₁-C₆-Alkoxy, C₁-C₆-Alkylthio, C₁-C₆-Alkylsulfinyl, C₁-C₆-Alkylsulfonyl, C₁-C₄-Halogenalkyl, C₁-C₄-Halogen-alkoxy, Nitro, Cyano oder jeweils gegebenenfalls einfach oder mehrfach durch Halogen, C₁-C₆-Aikyl, C₁-C₆-Alkoxy, C₁-C₄-Halogenalkyl, C₁-C₄-Halogen-alkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio steht,
V² und V³ unabhängig voneinander für Wasserstoff, Halogen, C₁-C₆-Alkyl, C₁-C₆-Alkoxy, C₁-C₄-Halogenalkyl oder C₁-C₄-Halogenalkoxy stehen,
mit der Maßgabe, dass mindestens einer der Reste W oder Z ungleich Wasserstoff sein muss, wenn X und Y für Methyl stehen,
A und B und das Kohlenstoffatom, an dass sie gebunden sind, für ein gegebenenfalls einfach bis vierfach durch C₁-C₆-Alkyl, C₁-C₄-Halogenalkyl, C₁-C₆-Alkoxy oder C₁-C₄-Alkoxy-C₁-C₄-alkyl substituiertes fünf- bis siebengliedriges Ketal, Thioketal oder Dithioketal stehen, welches gegebenenfalls durch ein weiteres Sauerstoffatom, Schwefelatom oder durch die Gruppe 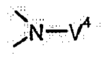 unterbrochen sein kann,
V⁴ für Wasserstoff, C₁-C₆-Alkyl, C₃-C₆-Alkenyl, oder für die Gruppen -CO-V⁵, -CO₂V⁵, CO-NH-V⁵ oder CO-NH-O-V⁵ steht,
V⁵ für C₁-C₆-Alkyl steht,
D für NH (1) oder Sauerstoff (2) steht,
Q¹ und Q² unabhängig voneinander für Wasserstoff, C₁-C₆-Allryl, C₁-C₂-Halogenalkyl oder C₁-C₄-Alkoxy stehen,
G für Wasserstoff (a) oder für eine der Gruppen 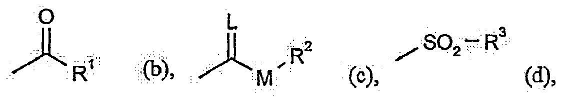 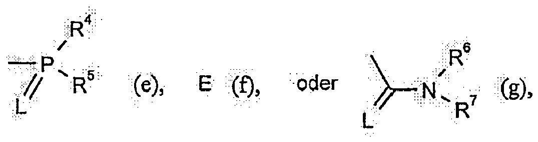 steht,
in welchen
E für ein Metallion oder ein Ammoniumion steht,
L für Sauerstoff oder Schwefel steht und
M für Sauerstoff oder Schwefel steht,
R¹ für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆₋Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkyl-sulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff steht,
R² für jeweils gegebenenfalls durch Halogen oder Cyano substituiertes C₁-C₂₀-Alkyl C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenakyl oder C₁-C₆-Halogenalkoxy substituiertes Phenyl oder Benzyl steht,
R³ für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio; C₁-C₄-Alkyl oder C₁-C₄-Halogenalkyl substituiertes Phenyl, Phenoxy oder Phenylthio stehen,
R⁶ und R⁷ unabhängig voneinander für Wasserstoff, für jeweils gegebenenfalls durch Halogen oder Cyano substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest stehen, in welchem gegebenenfalls eine Methylengruppe durch Sauerstoff oder Schwefel ersetzt ist.

3. Verbindungen der Formel (I) gemäß Anspruch 1, in welcher
W für Wasserstoff, Chlor, Brom, C₁-C₄-Alkyl, C₂-C₄-Alkenyl, C₂-C₄-Alkinyl, C₁-C₄-Alkoxy, C₁-C₂-Halogenalkyl oder C₁-C₂-Halogenalkoxy steht,
X für Chlor, Brom, C₁-C₄-Alkyl, C₂-C₄-Alkenyl, C₂-C₄-Alkinyl, C₁-C₄-Alkoxy, C₁-C₄-Halogenalkyl, C₁-C₄-Halogenalkoxy oder Cyano steht,
Y und Z unabhängig voneinander für Wasserstoff, Fluor, Chlor, Brom, C₁-C₄-Alkyl, C₂-C₄-Alkenyl, C₂-C₄-Alkinyl, C₁-C₆-Alkoxy, C₁-C₄-Halogenalkyl C₁-C₄-Halogenalkoxy, Cyano, C₂-C₄-Alkenyl, C₂-C₄-Alkinyl oder für einen der (Het)-arylreste, 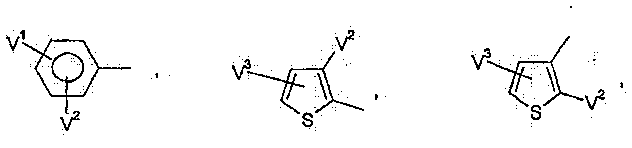 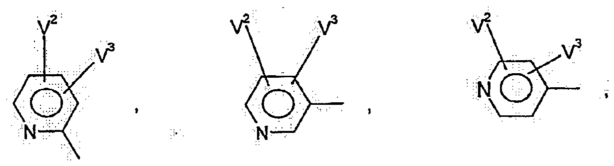 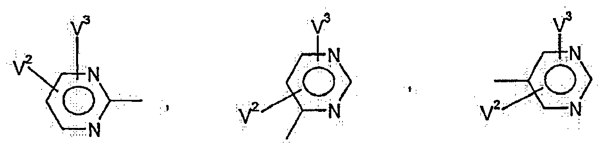 stehen.
wobei im Falle von (Het)-aryl nur einer der Reste Y oder Z für (Het)-aryl stehen darf,
V¹ für Wasserstoff, Fluor, Chlor, Brorn, C₁-C₆-Alkyl, C₁-C₄-Alkoxy, C₁-C₂-Halogenalkyl, C₁-C₂-Halogenalkoxy, Nitro, Cyano oder jeweils gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl steht,
V² und V³ unabhängig voneinander für Wasserstoff, Fluor, Chlor, Brom, C₁-C₄₋Alkyl, C₁-C₄-Alkoxy, C₁-C₂-Halogenalkyl oder C₁-C₂-Halogenalkoxy stehen, mit der Maßgabe, dass mindstens einer der Reste W oder Z ungleich Wasserstoff sein muss, wenn X und Y für Methyl stehen,
A und B und das Kohlenstoffatom, an dass sie gebunden sind, für ein gegebenenfalls einfach bis dreifach durch C₁-C₄-Alkyl, C₁-C₃-Halogenalkyl, C₁-C₄-Alkoxy oder C₁-C₄-Alkoxy-C₁-C₂-alkyl substituiertes fünf-, sechs- oder siebengliedriges Ketal stehen, welches gegebenenfalls durch ein weiteres Sauerstoffatom unterbrochen sein kann,
D für NH (1) oder Sauerstoff (2) steht,
Q¹ und Q² unabhängig voneinander für, Wasserstoff, Methyl, Ethyl, Trifluormethyl, Methoxy oder Ethoxy stehen,
G für Wasserstoff (a) oder für eine der Gruppen 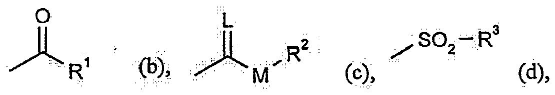 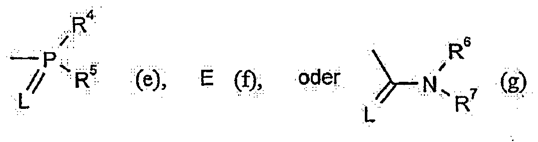 steht, in welchen
E für ein Metallion oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Cyano, Nitro, C₁-C₄-Alkyl, C₁-C₄ Alkoxy, C₁-C₃-Halogenalkyl, C₁-C₃-Halogenalkoxy, C₁-C₄-Alkylthio oder C₁-C₄-4lkylsulfonyl substituiertes Phenyl,
für gegebenenfalls einfach, bis zweifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach bis zweifach durch Fluor, Chlor, Brom oder C₁-C₄-Alkyl substituiertes Pyrazolyl, Thiazolyl, Pyridyl, Pyrimidyl, Furanyl oder Thienyl;
für gegebenenfalls einfach bis zweifach durch Fluor, Chlor, Brom oder C₁-C₄-Alkyl substituiertes Phenoxy-C₁-C₅-alkyl oder
für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiamlyloxy-C₁-C₅-alkyl steht,
R² für jeweils gegebenenfalls einfach bis dreifach durch Fluor oder Chlor substituiertes C₁-C₁₆-Alkyl, C₂-C₁₆-Alkenyl, C₁-C₆-Allcoxy-C₂-C₆-alkyl oder Poly-C₁-C₆-alkoxy-C₂-C₆-alkyl,
für gegebenenfalls einfach bis zweifach durch Fluor, Chlor, C₁-C₄-Alkyl oder C₁-C₄-Alkoxy substituiertes C₃-C₇-Cycloalkyl oder
für jeweils gegebenenfalls einfach bis dreifach durch Fluor, Chlor, Brom, Cyano, Nitro, C₁-C₄-Alkyl, C₁-C₃₋Alkoxy, C₁-C₃-Halogenalkyl oder C₁-C₃-Halogenalkoxy substituiertes Phenyl oder Benzyl steht,
R³ für gegebenenfalls einfach bis dreifach durch Fluor oder Chlor substituiertes C₁-C₆-Alkyl oder jeweils gegebenenfalls einfach bis zweifach durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl steht,
R⁴ und R⁵ unabhängig voneinander für jeweils Gegebenenfalls einfach bis dreifach durch Fluor oder Chlor substituiertes C₁-C₆-Alkyl; C₁-C₆₋Alkoxy, C₁-C₆-Alkylamino, Di-(C₁-C₆-alkyl)amno, C₁-C₆-Alkylthio oder C₃-C₄-Alkenylthio oder für jeweils gegebenenfalls einfach bis zweifach durch Fluor, Chlor, Brom, Nitro, Cyano, C₁-C₃-Alkoxy, C₁-C₃-Haldgenalkoxy, C₁-C₃-Alkylthio, C₁-C₃-Halogenalkylthio, C₁-C₃-Alkyl oder C₁-C₃-Halogenalkyl substituierte Phenyl, Phenoxy oder Phenylthio, stehen;
R⁶ und R⁷ unabhängig voneinander für Wasserstoff, für jeweils gegebenenfalls einfach bis dreifach durch Fluor oder Chlor substituiertes C₁-C₆-Alkyl C₃-C₆-Cycloalkyl, C₁-C₆-Alkoxy, C₃-C₆-Alkenyl oder C₁-C₆-Alkoxy-C₂-C₆-alkyl, für jeweils gegebenenfalls einfach bis zweifach durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-alkyl substituierten C₃-C₆-Alkylenrest stehen, in welchem gegebenenfalls eine Methylengruppe durch Sauerstoff oder Schwefel ersetzt ist.

4. Verbindungen der Formel (1) gemäß Anspruch 1, in welcher
W für Wasserstoff, Chlor, Brom, Methyl, Ethyl, Vinyl, Ethinyl, Propinyl, Methoxy, Ethoxy oder Trifluormethyl steht,
X für Chlor, Brom, Methyl, Ethyl, Propyl, iso-Propyl, Vinyl, Ethinyl, Propinyl, Methoxy, Ethoxy, Trifluormethyl, Difluormethoxy, Trifluormethoxy oder Cyano steht,
Y und Z unabhängig voneinander für Wasserstoff, Fluor, Chlor, Brom, Methyl, Ethyl, Vinyl, Ethinyl, Propinyl, Methoxy, Trifluormethyl, Trifluormethoxy, Cyano oder einen Phenylrest 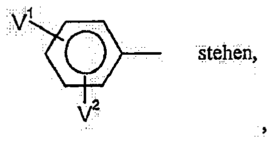 wobei im Falle von Phenyl nur einer der Reste Y oder Z für Phenyl stehen darf,
V¹ für Wasserstoff, Fluor, Chlor, Brom, Methyl, Ethyl, n-Propyl, iso-Propyl, tert.-Butyl, Methoxy, Ethoxy, n-Propoxy; , iso-Propoxy, Trifluormethyl oder Trifluormethoxy steht,
V² für Wasserstoff, Fluor, Chlor, Methyl, Ethyl n-Propyl, iso-Propyl, Methoxy, Ethoxy oder Trifluormethyl steht,
mit der Maßgabe, dass mindestens einer der Reste W oder Z ungleich Wasserstoff sein muss, wenn X und Y für Methyl stehen,
A und B und das Kohlenstoffatom, an dass sie gebunden sind, für ein gegebenenfalls einfach bis zweifach durch Methyl, Ethyl, Propyl, Trifluormethyl, Monochlormethyl, Methoxy, Ethoxy, Methoxymethyl oder Ethoxymethyl substituiertes fünf-, sechs- oder siebengliedriges Ketal stehen, welches gegebenenfalls durch ein weiteres Sauerstoffatom unterbrochen sein kann,
D für NH (1) oder Sauerstoff (2) steht,
Q¹ und Q² für Wasserstoff steht,
G für Wasserstoff (a) oder für eine der Gruppen 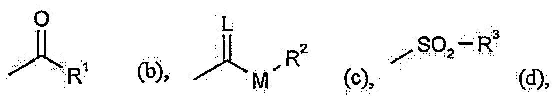 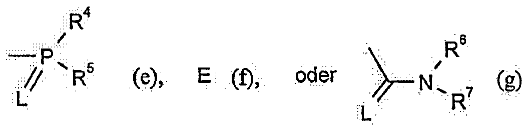 steht, in welchen
E für ein Metallion oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituiertes C₁-C₁₀-Alkyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloakl,
für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl,
für jeweils gegebenenfalls einfach durch Chlor, Brom oder Methyl substituiertes Furanyl, Thienyl oder Pyridyl steht,
R² für jeweils gegebenenfalls einfach bis dreifach durch Fluor oder Chlor substifuiertes C₁-C₁₀-Alkyl, C₂-C₁₀-Alkenyl oder C₁-C₄-Alkoxy-C₂-C₄-alkyl,
für Cyclopentyl oder Cyclohexyl
oder für jeweils gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl steht,
R³ für jeweils gegebenenfalls einfach bis dreifach durch Fluor oder Chlor substituiertes Methyl, Ethyl, Propyl oder iso-Propyl, oder jeweils gegebenenfalls einfach durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl steht,
R⁴ und R⁵ unabhängig voneinander für C₁-C₄-Alkoxy oder C₁-C₄-Alkylthio oder für jeweils gegebenenfalls einfach durch Fluor, Chlor, Brom, Nitro, Cyano, Methyl, Methxy, Trifluormethyl oder Trifluormethoxy substituiertes Phenyl, Phenoxy oder Phenylthio steht,
R⁶ und R⁷ unabhängig voneinander für Wasserstoff, für C₁-C₄-Alkyl, C₃-C₆-Cycloalkyl, C₁-C-₄-Alkoxy, C₃-C₄-Alkenyl oder C₁-C₄-Alkoxy-C₂-C₄-alkyl, für jeweils gegebenenfalls einfach bis zweifach durch Fluor, Chlor, Brom, Methyl, Methoxy oder Trifluormethyl substituiertes Phenyl, oder zusammen für einen C₅-C₆-Alkylenrest stehen, in welchem gegebenenfalls eine Methylengruppe durch Sauerstoff oder Schwefel ersetzt ist.

5. Verbindungen der Formel (I) gemäß Anspruch 1, in welcher
W für Wasserstoff Chlor, Brom, Methyl, Ethyl oder Methoxy steht,,
X für Chlor, Brom, Methyl, Ethyl oder Methoxy,
Y und Z unabhängig voneinander für Wasserstoff, Chlor, Brom, Methyl oder für den Rest 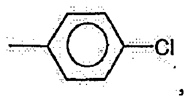 wobei in diesem Falle nur einer der Reste Y oder Z für 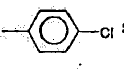 stehen darf, mit der Maßgabe, dass mindestens einer der Reste W oder Z ungleich Wasserstoff, sein muss, wenn X und Y für Methyl stehen,
A und B und das Kohlenstoffatom, an dass sie gebunden sind, für ein gegebenenfalls einfach bis zweifach durch Methyl, Ethyl, Propyl, Monochlormethyl substituiertes fünf- oder sechsgliedriges Ketal stehen,
D für NH (1) oder Sauerstoff (2) steht,
Q¹ und Q² für Wasserstoff stehen,
G für Wasserstoff (a) oder für eine der Gruppen 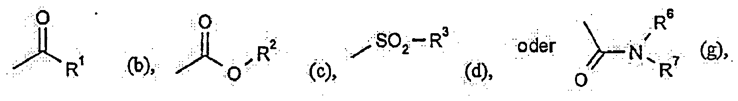
R¹ für C₁-C₁₀-Alkyl, C₁-C₄-Alkoxy-C₁-C₂-alkyl, C₃-C₆-Cycloalkyl,
für gegebenenfalls einfach durch Chlor, substituiertes Phenyl, oder für Thienyl Steht,
R² für C₁-C₁₀-Alkyl, C₂-C₁₀-Alkenyl, oder Benzyl steht,
R³ für Methyl steht,
R⁶ und R⁷ zusammen für einen C₅-C₆-Alkylenrest stehen, in Welchem gegebenenfalls eine Methylengruppe durch Sauerstoff oder Schwefel ersetzt ist

6. Verfahren zur Herstellung von Verbindungen der Formel (I) gemäß Anspruch 1, **dadurch gekennzeichnet; dass** man zum Gehalt von
(A) Verbindungen der Formel (I-1-a) 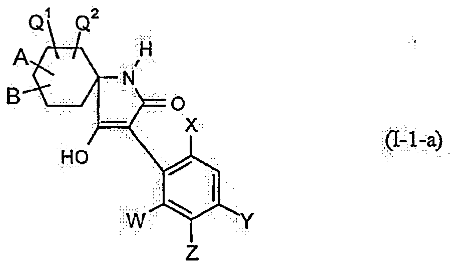 in welcher A, B, Q¹, Q², W, X, Y und Z die oben angegebenen Bedeutungen haben,
Verbindungen der Formel (II) 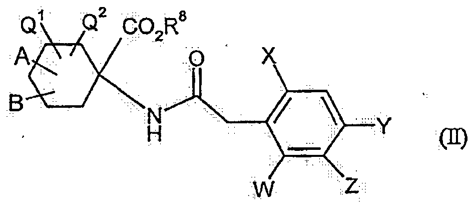 in welcher A, B, Q¹, Q², W, X, Y und Z die oben angegebenen Bedeutungen haben,
und
R⁸ für Alkyl steht,
in Gegenwart eines Verdünnungsmittels und in Gegenwart einer Base intramolekular kondensiert,
(B) Verbindungen der Formel (I-2-a) 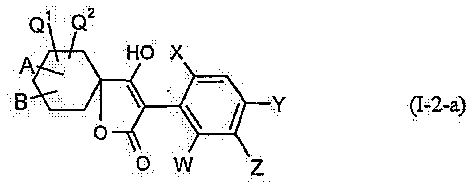 in welcher
A, B, Q¹, Q², W, X, Y und Z die oben angegebenen Bedeutungen haben,
Verbindungen der Formel (III) 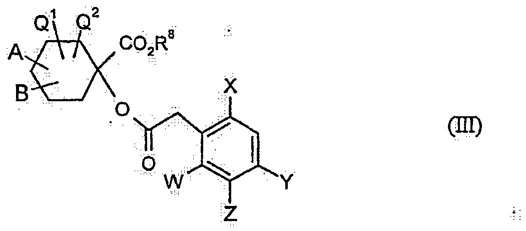 in welcher
A, B, Q¹, Q², W, Y, Y, Z und R⁸ die oben angegebenen Bedeutungen haben,
in Gegenwart eines Verdünnungsmittels und in Gegenwart einer Base intramolekular kondensiert,
(C) Verbindungen der Formel (I-1-b) bis (I-2-b), 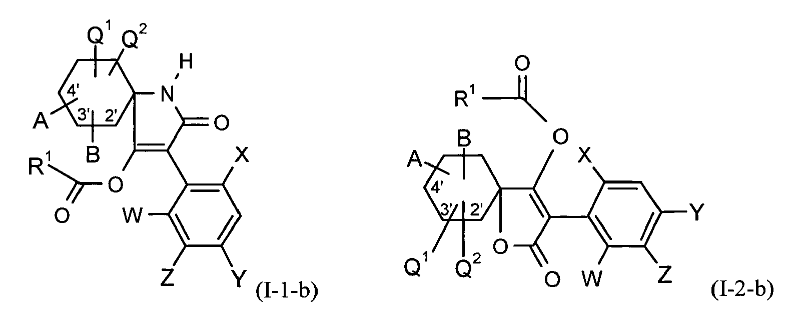 in welchen R¹; A, B, Q¹, Q², W, X, Y und Z die oben angegebenen Bedeutungen haben, Verbindungen der oben gezeigten Formeln (I-1-a) bis (I-2-a), in welchen A, B, Q¹, Q², W, X, Y und Z die oben angegebenen Bedeutungen haben, jeweils
α) mit Verbindungen der Formel (IV) 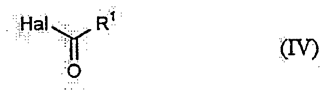 in welcher
R¹ die oben angegebene Bedeutung hat und
Hal für Halogen steht
oder
ß) mit Carbonsäureanhydriden der Formel (V)
R¹-CO-O-CO-R¹ (V)
in welcher
R¹ die oben angegebene Bedeutung hat,
gegebenenfalls in Gegenwart eines Verdünnungsmittels und gegebenenfalls in Gegenwart eines Säurebindemittels umsetzt;
(D) Verbindungen der Formeln (I-1-c) bis (I-2-c), 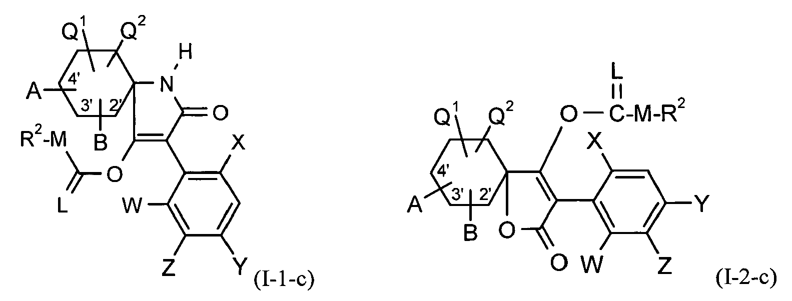 in welchen R², A, B, Q¹, Q², W, M, X, Y und Z die oben angegebenen Bedeutungen haben und L für Sauerstoff steht, Verbindungen der oben gezeigten Formeln (I-1-a) bis (I-2-a), in welchen, B, Q¹, Q², W, X, Y und Z die oben angegebenen Bedeutungen haben, jeweils
mit Chlorarneisensäureestern oder Chlorameisensäurethioestern der Formel (VI)
R²-M-CO-Cl (VI)
in welcher
R² und M die oben angegebenen Bedeutungen haben,
gegebenenfalls in Gegenwart eines Verdünnuhgsmittels und gegebenenfalls in Gegenwart eines Säurebindemittels umsetzt;
(E) Verbindungen der Formeln (I-1-c) bis (I-2-c), 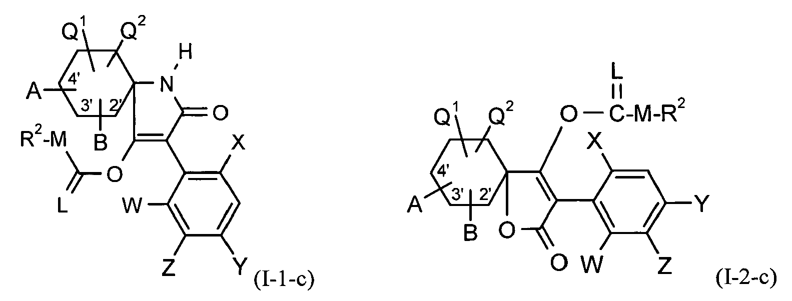 in welchen R², A, B, Q¹, Q², W, M, X, Y und Z die oben angegebenen Bedeutungen haben und L für Schwefel steht, Verbindungen der oben gezeigten Formeln (I-1-a) bis (I-2-a), in welchen A, B, Q¹ Q², W, X, Y und Z die oben angegebenen Bedeutungen haben, jeweils
mit Chlormonothioameisensäureestern oder Chlordithioameisensäureestern der Formel (VII) 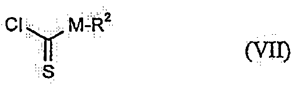 in welcher
M und R² die oben angegebenen Bedeutungen haben,
gegebenenfalls in Gegenwart eines Verdünnungsmittels und gegebenenfalls in Gegenwart eines Säurebindemittels umsetzt,
(F) Verbindungen der Formeln (I-1-d) bis (I-2-d), 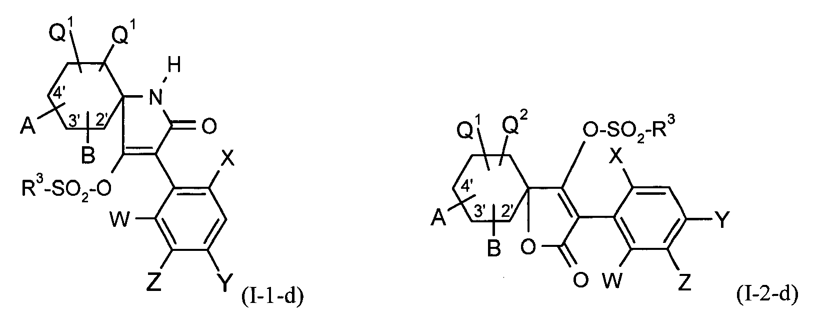 in welchen R³, A, B, W, Q¹, Q², X, Y und Z die oben angegebenen Bedeutungen haben, Verbindungen der oben gezeigten Formeln (I-1-a) bis (I-2-a), in welchen A, B, Q¹, Q², W, X, Y und Z die oben angegebenen Bedeutungen haben, jeweils
mit Sulfonsäurechloriden der Formel (VIII)
R³-SO₂-Cl (VIII)
in welcher
R³ die oben angegebene Bedeutung hat,
gegebenenfalls in Gegenwart eines Verdünnungsmittels und gegebenenfalls in Gegenwart eines Säurebindemittels umsetzt,
(G) Verbindungen der Formeln (I-1-e) bis (I-2-e), 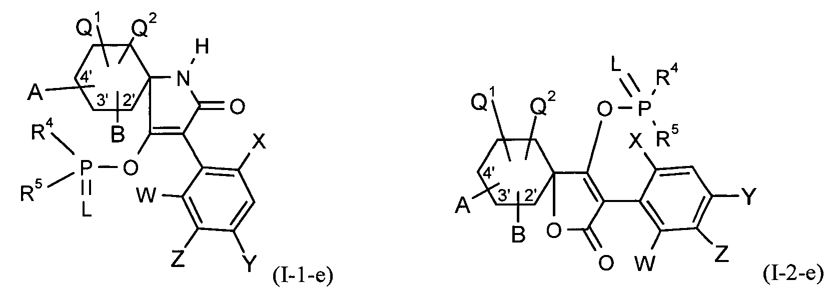 in welchen L, R⁴, R⁵, A, B, Q¹, Q², W, X, Y und Z die oben angegebenen Bedeutungen haben, Verbindungen der oben gezeigten Formeln (I-1-a) bis (I-2-a), in welchen A, B, Q¹, Q², W, X, Y und Z die oben angegebenen Bedeutungen haben, jeweils
mit Phosphorverbindungen der Formel (IX) 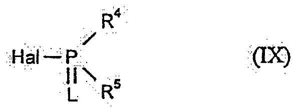 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(H) Verbindungen der Formeln (I-1-f) bis (I-2-f), 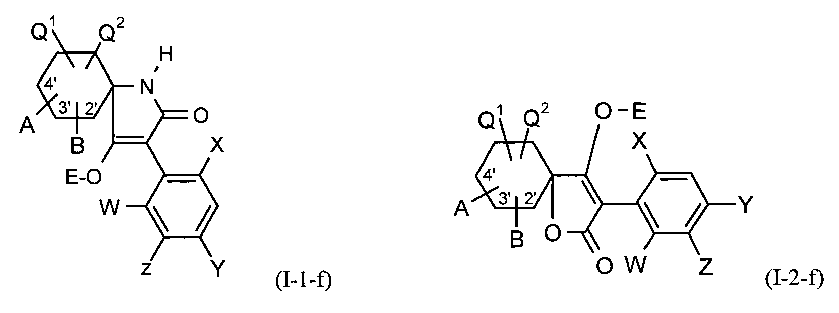 in welchen E, A, B, Q¹, Q², W, X, Y und Z die oben angegebenen Bedeutungen haben, Verbindungen der Formeln (I-1-a) bis (I-2-a), in welchen A, B, Q¹, Q², W, X, Y und Z die oben angegebenen Bedeutungen haben, jeweils
mit Metallverbindungen oder Aminen der Formeln (X) oder (XI) 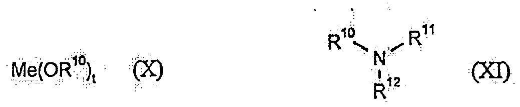 in welchen
Me für ein ein- oder zweiwertiges Metall
t für die Zahl 1 der 2 und
R¹⁰, R¹¹, R¹² unabhängig voneinander für Wasserstoff oder Alkyl stehen,
gegebenenfalls in Gegenwart eines Verdünnungsmittels umsetzt.
(I) Verbindungen der Formeln (I-1-g) bis (I-2-g), in welchen L, R⁶, R⁷, A, B, Q¹, Q², W, X, Y und Z die oben angegebenen Bedeutungen haben, Verbindungen der oben gezeigten Formeln (I-1-a) bis (I-2-a), 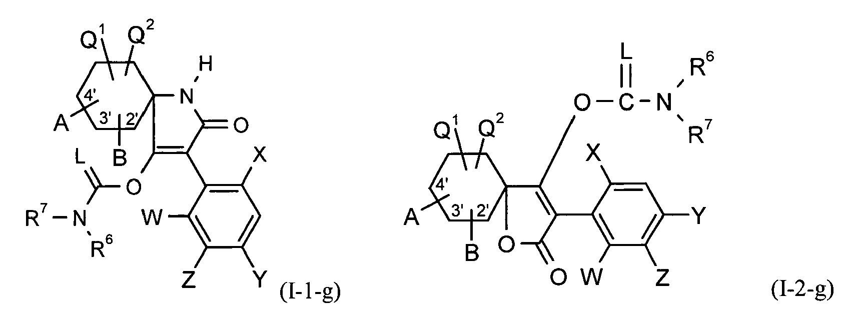 in welchen A, B, Q¹, Q², W, X, Y und Z die oben angegebenen Bedeutungen haben, jeweils
α) mit Isocyanaten oder Isothiocyanaten der Formel (XII)
R⁶-N=C=L (XII)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
ß) mit Carbamidsäurechloriden oder Thiocarbamidsäurechloriden oder Formel (XIII) 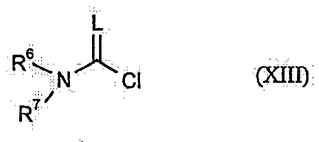 in welcher
L, R⁶ und R⁷ die oben angegebenen Bedeutungen haben,
gegebenenfalls in Gegenwart eines Verdünnungsmittels und Gegebenenfalls in Gegenwart eines Säurebindemittels, umsetzt,
(J) Verbindungen der Formeln (I-1-a) bis (I-1-g) und (I-2-a) bis (I-2-g), 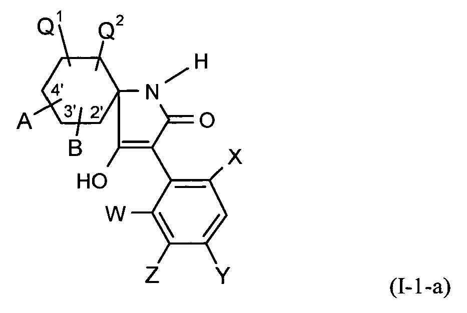 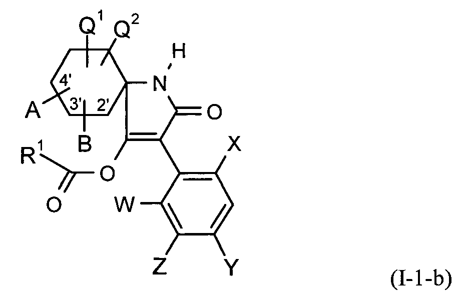 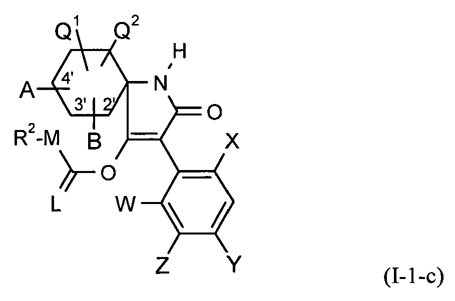 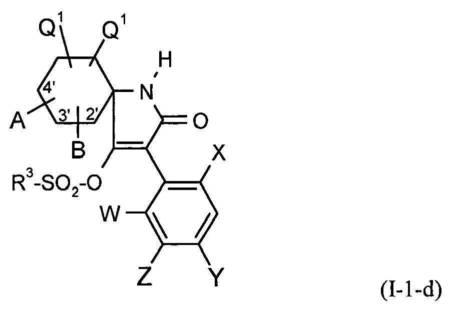 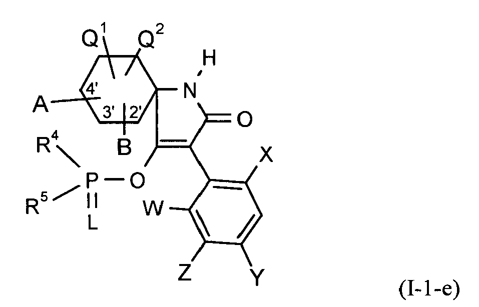 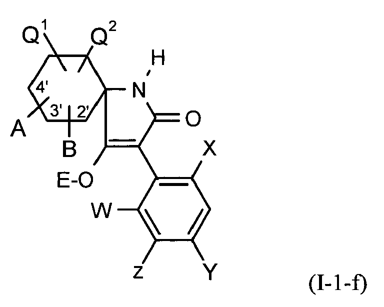 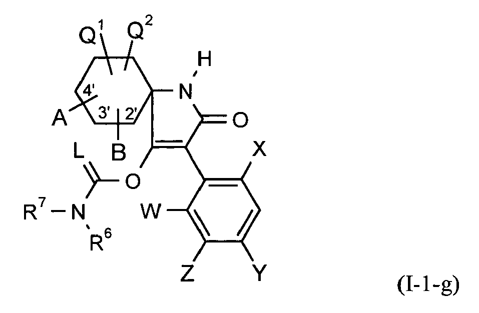 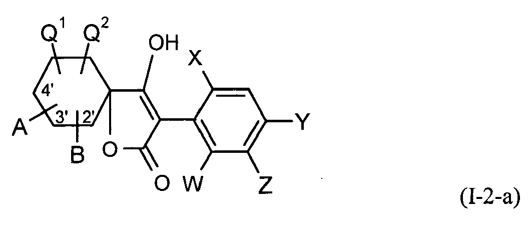 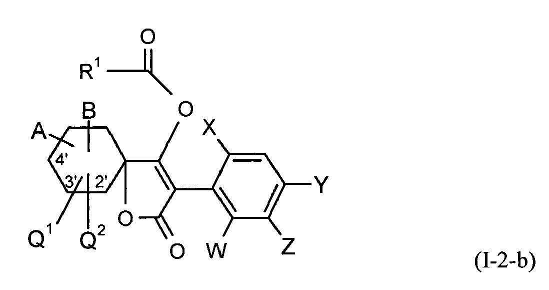 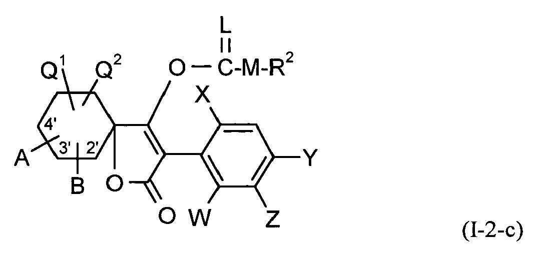 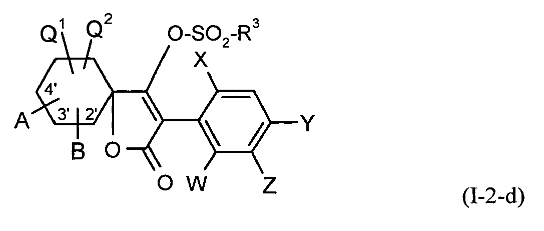 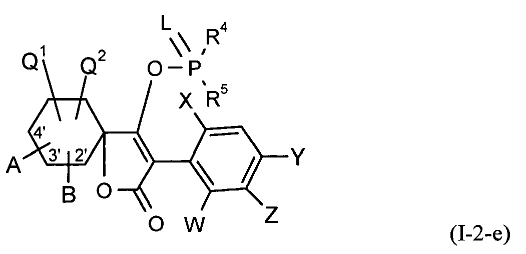 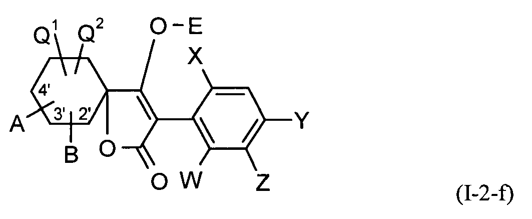 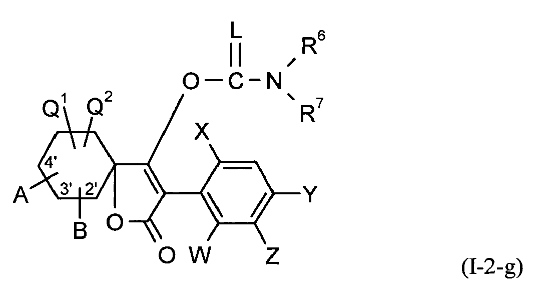 in welchen A, B, D, G, Q¹, Q², W, X, Y und Z die oben angegebenen Bedeutungen haben, Verbindungen der Formel (XIV) 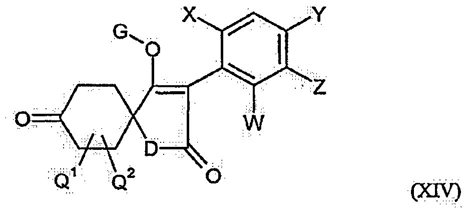 in welcher
D, G, Q¹, Q², W, X, Y und Z die oben angegebenen Bedeutungen haben,
mit Diolen der Formel (XV)
HO - A - B - OH (XV)
in welcher
A und B die oben genannte Bedeutung haben,
gegebenenfalls in Gegenwart eines Verdünnungsmittels, in Gegenwart eines sauren Katalysators unter wasserentziehenden Bedingungen umsetzt.

7. Mittel zur Bekämpfung von Schädlingen, unerwünschten Pflanzenwuchs und/oder unerwünschten Mikroorganismen, **gekennzeichnet durch** einen Gehalt an mindestens einer Verbindung der Formel (I) gemäß Anspruch 1.

8. Verfahren zur Bekämpfung von tierischen Schädlingen, unerwünschtem Pflanzenbewuchs und/oder unerwünschten Mikroorganismen, **dadurch gekennzeichnet, dass** man Verbindungen der Formel (I) gemäß Anspruch 1 suf Schädlinge, unerwünschtern Pflanzenwuchs, unerwünschte Mikroorganismen und/oder ihren Lebensraum einwirken lässt, ausgenommen sind Verfahren zur chirurgischen oder therapeutischen Behandlung des menschlichen oder tierischen Körpers.

9. Verwendung von Verbindungen der Formel (I) gemäß Anspruch 1 zur Bekämpfung von tierischen Schädlingen, unerwünschtem Pflanzenbewuchs und/oder unerwünschten Mikro-organismen, ausgenommen zur Verwendung in Verfahren zur chirurgischen oder therapeutischen Behandlung des menschlichen oder tierischen Körpers.

10. Verfahren zur Herstellung von Mittels zur Bekämpfung von Schädlingen, unerwünschtem Pflanzenbewuchs und/oder unerwünschten Mikroorganismen, **dadurch gekennzeichnet, dass** man Verbindungen der Formel (I) gemäß Anspruch 1 mit Streckmitteln und/oder oberflächenaktiven Stoffen vermischt.

11. Verwendung von Verbindungen der Formel (I) gemäß Anspruch 1 zur Herstellung von Mitteln zur Bekämpfung von Schädlingen, unerwünschtem Pflanzenwuchs und/oder unerwünschten Mikroorganismen.

12. Mittel enthaltend einen wirksamen Gehalt an einer Wirkstoffkombination umfassend als Komponenten
(a') mindestens eine Verbindung der Formel (I), gemäß Anspruch 1 in welcher A, B, D, G, Q¹, Q², W, X, Y und Z die oben angegebene Bedeutung haben
und
(b') zumindest eine die Kulturpflanzen-Verträglichkeit verbessernde Verbindung aus der folgenden Gruppe von Verbindungen:
4-Dichloracetyl-1-oxa-4-aza-spiro[4.5]-decan (AD-67, MON-4660), 1-Dichloracetyl-hexa-hydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl), 3-(2-Chlor-benzyl)-1-(1-methyl-1-phenyl-ethyl-harnstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlormethyl-1H-1,2,4-triazol-3-carbonsäure-ethylester (Fenchlorazole-ethyl), 2-Chlor-4-trifluormethyl-thiazol-5-carbonsäure-phenylmethylester (Flurazole), 4-Chlor-N-(1,3-di-oxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureohydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl-buttersäure, 4-(4-Chlor-phenoxy)-buttersaure, Diphenylmethoxyessigsäure, Diphenylmethoxyessigsäuremethylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester, 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester, 5-Chlor-chinolin-8-oxy-essigsänre-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester, 4-Carboxy-chroman-4-yl-essigsäure (AC-304415), 4-Chlor-phenoxy-essigsäure, 3,3'-Dimethyl-4-methoxy-benzophenon, 1-Brom-4-chlormethyl-sulfonyl-benzol, 1-[4-(N-2-Methoxybenzoylsulfamoyl)-phenyl]-3-methyl-harnstoff (alias N-(2-Methoxy-benzoyl)-4-[(methylamino-carbonyl)-amino]-benzolsulfonamid), 1-[4-(N-2-Methoxybenzoylsulfamoyl)-phenyl]-3,3-dimethyl-harnstoff, 1-[4-(N4,5-Dimethylbenzoyl-sulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfon-amid,
und/oder eine der folgenden durch allgemeine Formeln definierten Verbindungen der allgemeinen Formel (IIa) 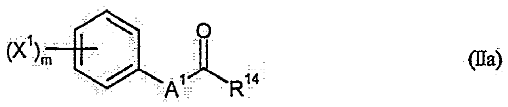 oder der allgemeinen Formel (IIb) 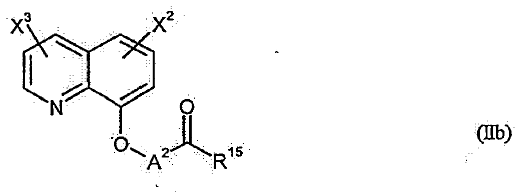 oder der Formel (IIc) 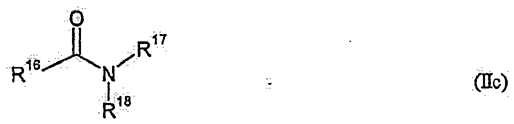 wobei
m für eine Zahl 0, 1, 2, 3, 4 oder 5 steht,
A¹ für eine der nachstehend skizzierten divalenten heterocyclischen Gruppierungen steht, 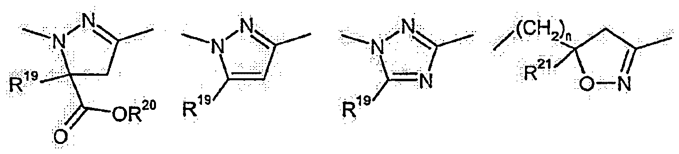
n für eine Zahl 0, 1, 2, 3, 4 oder 5 steht,
A² für gegebenenfalls durch C₁-C₄-Alkyl und/oder C₁-C-₄-Alkoxy-carbonyl und/oder C₁-C₄-Alkenyloxy-carbonyl substituiertes Alkandiyl mit 1 oder 2 Kohlenstoffatomen steht,
R¹⁴ für Hydroxy, Mercapto, Amino, C₁-C₆-Alkoxy, C₁-C₆-Alkylthio, C₁-C₆-Alkylamino oder Di-(C₁-C₄-alkyl)-amino steht,
R¹⁵ für Hydroxy, Mercapto, Amino, C₁-C₇-Alkoxy, C₁-C₆-Alkylthio, C₁-C₆-Alkenyloxy, C₁-C₆-Alkenyloxy-C₁-C₆-alkoxy, C₁-C₆-Alkylamino oder Di-(C₁-C₄-alkyl)-amino steht,
R¹⁶ für gegebenenfalls durch Fluor, Chlor und/oder Brom substituiertes C₁-C₄-Alkyl steht,
R⁷ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
R¹⁸ für Wasserstoff, jeweils gegebenenfalls durch Fluor, Chlor und/oder Brom substituiertes C₁-C₆-Alkyl, C₂-C₆-Alkenyl oder C₂-C₆-Akinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl, R¹⁷ und R¹⁸ auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes C₃-C₆-Alkandiyl oder C₂-C₅-Oxaalkandiyl steht,
R¹⁹ für Wasserstoff Cyano, Halogen, oder für jeweils gegebenenfalls durch Fluor, Chlor und/oder Brom substituiertes C₁-C₄-Akyl, C₃-C₆-Cycloalkyl oder Phenyl steht,
R²⁰ für Wasserstoff, jeweils gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
R²¹ für Wasserstoff, Cyano, Halogen, oder für jeweils gegebenenfalls durch Fluor, Chlor und/öder Brom substituiertes C₁-C₄-Alkyl, C₃-C₆-Cycloalkyl oder Phenyl steht,
X¹ für Nitro, Cyano, Halogen, C₁-C₄-Alkyl, C₁-C₄-Halogenalkyl, C₁-C₄-Alkoxy oder C₁-C₄-Halogenalkoxy steht,
X² für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
X³ für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen der allgemeinen Formel (IId) 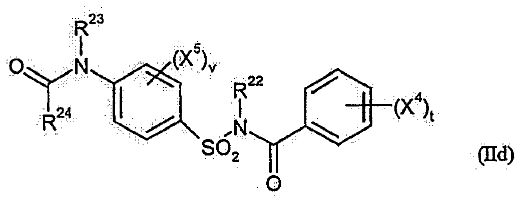 oder der allgemeinen Formeln (IIe) 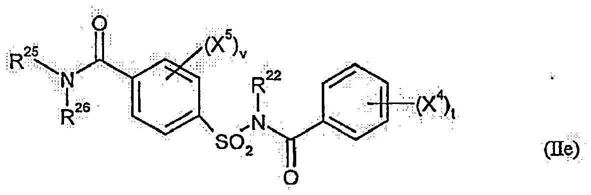 wobei
t für eine Zahl 0, 1, 2, 3, 4 oder 5 steht,
v für eine Zahl 0, 1, 2, 3, 4 oder 5 steht,
R²² für Wasserstoff oder C₁-C₄-Alkyl steht,
R²³ für Wasserstoff oder C₁-C₄-Alkyl steht,
R²⁴ für Wasserstoff, jeweils gegebenenfalls durch Cyano, Halogen oder C₁-C₄ Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cycloalkyl, C₃-C₆-Cycloakyloxy, C₃-C₆-Cycloalkylthio oder C₃-C₆-Cycloalkylamino steht,
R²⁵ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
R²⁶ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen sub-stituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₅-Oxa-alkandiyl steht,
X⁴ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
X⁵ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

13. Mittel nach Anspruch 12, bei dem die die Kulturpflanzen-Verträglichkeit verbessernde Verbildung aus der folgenden Gruppe von Verbindungen ausgewählt ist: Gloquintocet-mexyl, Fenchlorazole-ethyl, Isoxadifen-ethyl, Mefenpyr-diethyl, Furilazole, Fenclorim, Cumyluron, Dymron oder die Verbindungen 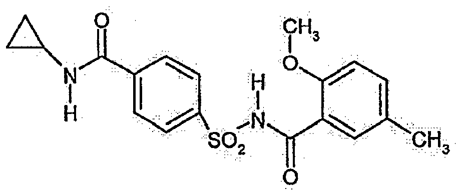 und

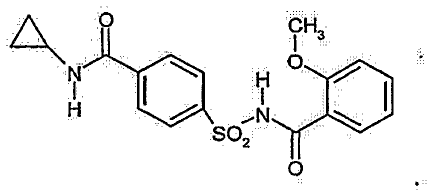

14. Mittel gemäß einem der Anspruche 12 der 13, bei denen die Kulturpflanzen-Verträglichkeit verbessernde Verbindung Cloquintocet-mexyl ist.

15. Mittel gemäß einem der Ansprüche 12 oder 13, bei denen die Kulturpflanzen-Verträglichkeit verbessernde Verbindung Mefenpyr-diethyl ist

16. Verfahren zum Bekämpfen von unerwünschtem Pflanzenwuchs, **dadurch gekennzeichnet, dass** man ein Mittel gemäß Anspruch 12 auf die Pflanzen oder ihre Umgebung einwirken lässt.

17. Verwendung eines Mittel gemäß Anspruch 12 zum Bekämpfen von unerwünschten Pflanzenwuchs.

18. Verfahren zum Bekämpfen von unerwünschtem Pflanzenwuchs, **dadurch gekennzeichnet, dass** man eine Verbindung der Formel (I) gemäß Anspruch 1 und die die Kulturpflanzenverträglichkeit verbessernde Verbindung gemäß Anspruch 12 in zeitlich naher Abfolge getrennt auf die Pflanzen oder ihre Umgebung einwirken lässt.

19. Verbindungen der Formel (II) 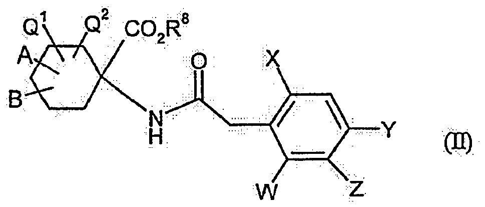 in welcher
A, B, Q¹, Q², R⁸, W, X, Y und Z die in Ansprüche 1 und 6 angegebenen Bedeutungen haben.

20. Verbindungen der Formel (III) 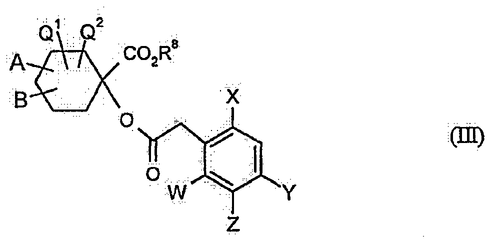 in welcher
A, B, Q¹, Q², W, X, Y, Z und R⁸ die in Ansprüche 1 und 6 angegebenen Bedeutungen haben

21. Verbindungen der Formel (XVIII) 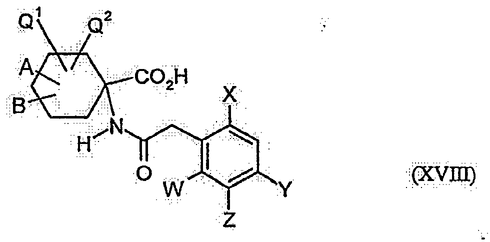 in welcher
A, B, Q¹, Q², W, X, Y und Z die in Anspruch 1 angegebenen Bedeutungen haben.

22. Verbindungen der Formel (XXI) 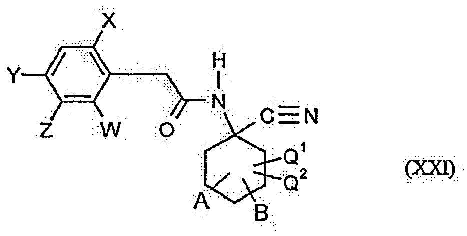 in welcher
A, B, Q¹, Q², W, X, Y und Z die in Anspruch 1 angegebenen Bedeutungen haben.

23. Verbindungen der Formel (XXII) 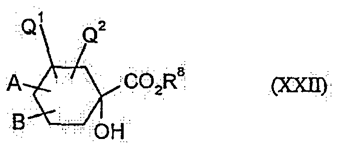 in welcher
A, B, Q¹ , Q² und R⁸ die in Ansprüche 1 und 6 angegebenen Bedeutungen haben, ausgenommen

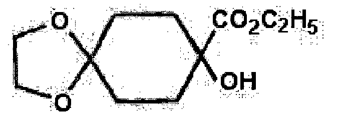

24. Verbindungen der Formel (XVI') und deren Salze 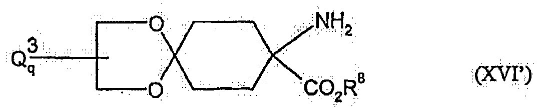 in welcher
Q³ für C₁-C₄-Alkyl, C₁-C₃-Halogenalkyl, C₁-C₄-Alkoxy oder C₁-C₄-Alkoxy-C₁-C₂-alkyl steht,
q für 1, 2 oder 3 steht,
R⁸ die in Anspruch 6 angegebene Bedeutung hat.

25. Verbindungen der Formel (XVI") und deren Salze 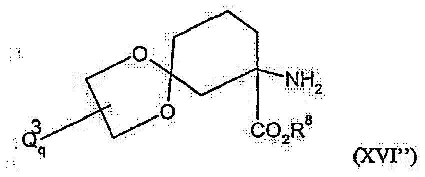 in welcher
Q³ und R⁸ die in Ansprüche 6 und 24 angegebenen Bedeutungen haben und
q für 0, 1, 2 oder 3 steht.

26. Verbindungen der Formel (XVI"') und deren Salze 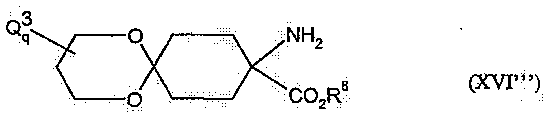 in welcher
Q³ und R⁸ die in Ansprüche 6 und 24 angegebenen Bedeutungen haben und
q für 0, 1, 2 oder 3 steht.

27. Verbindungen der Formel (XIX') 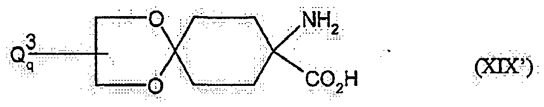 in welcher
Q³ die in Ansprüche 6 und 24 angegebenen Bedeutungen haben und
q für 1, 2 oder 3 steht.

28. Verbindungen der Formel (XIX") 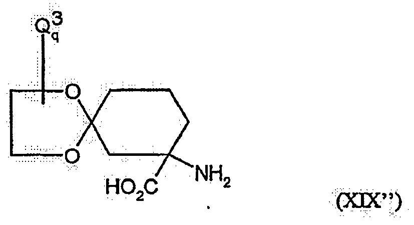 in welcher
Q³ die in Anspruch 24 angegebenen Bedeutungen haben und
q für 0, 1, 2 oder 3 steht.

29. Verbindungen der Formel (XIX''') 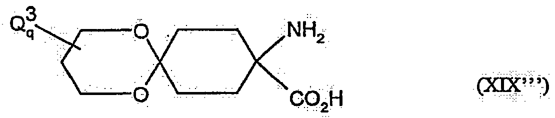 in welcher
Q³ die in Anspruch 24 angegebenen Bedeutungen haben und
q für 0,1, 2 oder 3 steht.

30. Verbindungen der Formel (XXIII') 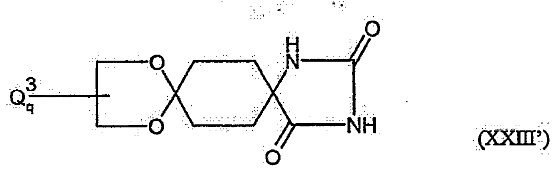 in welcher
Q³ die in Anspruch 24 angegebenen Bedeutungen haben und
q für 1, 2 oder 3 steht.

31. Verbindungen der Formeln (XXIII'') 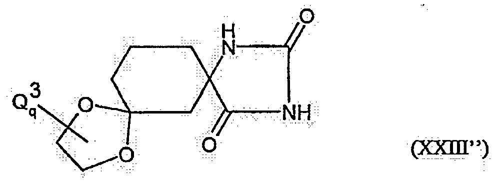 in welcher
Q³ die in Anspruch 24 genannten Bedeutungen haben und
q für 0, 1, 2, oder 3 steht.

32. Verbindungen der Formel (XXIII''') 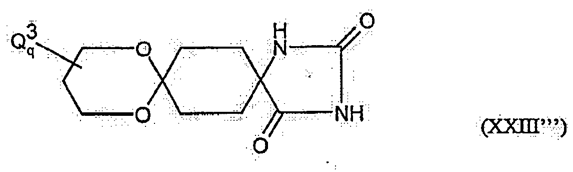 in welcher
Q³ die in Anspruch 24 genannten Bedeutungen hat und
q für 0, 1, 2 oder 3 steht.

33. Verbindung der Formel (I) gemäß Anspruch 1, in welcher die Substituenten die in der Tabelle angegebenen Bedeutungen haben:
| W | X | Y | Z | D | Q¹ | Q² | G | A | B |
|---|---|---|---|---|---|---|---|---|---|
| CH₃ | CH₃ | CH₃ | H | NH | H | H | H | 4'-O-(CH₂)₂-O- | |
| CH₃ | CH₃ | Cl | H | NH | H | H | H | 4'-O-(CH₂)₂-O- | |
| CH₃ | CH₃ | Br | H | NH | H | H | H | 4'-O-(CH₂)₂-O- | |
| CH₃ | CH₃ | CH₃ | H | NH | H | H | H | 4'-O-(CH₂)₃-O- | |
| CH₃ | CH₃ | Cl | H | NH | H | H | H | 4'-O-(CH₂)₃-O- | |
| CH₃ | CH₃ | Br | H | NH | H | H | H | 4'-O-(CH₂)₃-O- | |

## Claims

1. Compounds of the formula (I) 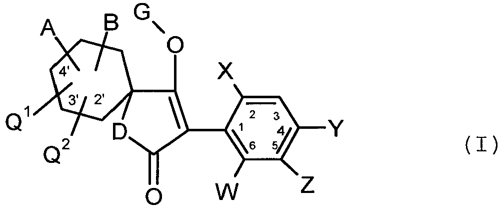 in which
W represents hydrogen, alkyl, alkenyl, alkynyl, alkoxy, halogen, alkenyloxy, haloalkyl, haloalkoxy or cyano,
X represents halogen, alkyl, alkenyl, alkynyl, alkoxy, alkenyloxy, alkylthio, alkylsulphinyl, alkylsulphonyl, haloalkyl, haloalkoxy, haloalkenyloxy, nitro or cyano,
Y and Z independently of one another represent hydrogen, alkyl, alkenyl, alkynyl, alkoxy, halogen, haloalkyl, haloalkoxy, cyano, nitro or in each case optionally substituted aryl or hetaryl,
with the proviso that at least one of the radicals W or Z has to be different from hydrogen if X and Y represent methyl,
A and B and the carbon atom to which they are attached represent five- to seven-membered ketal, thioketal or dithioketal which may optionally be interrupted by a further heteroatom, each of which radicals is optionally substituted by alkyl, haloalkyl, alkoxy, alkoxyalkyl or optionally substituted phenyl,
D represents NH and oxygen,
Q¹, Q² independently of one another represent hydrogen, alkyl, haloalkyl or alkoxy,
G represents hydrogen (a) or represents one of the groups 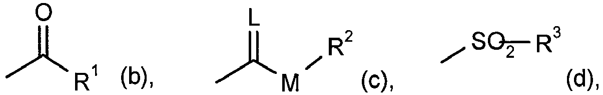 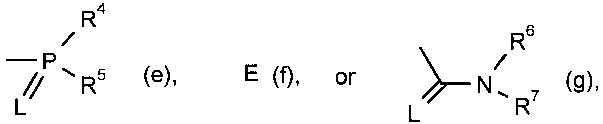 in which
E represents a metal ion or an ammonium ion,
L represents oxygen or sulphur,
M represents oxygen or sulphur,
R¹ represents in each case optionally halogen- or cyano-substituted alkyl, alkenyl, alkoxyalkyl, alkylthioalkyl or polyalkoxyalkyl or represents in each case optionally halogen-, alkyl- or alkoxy-substituted cycloalkyl or heterocyclyl or represents in each case optionally substituted phenyl, phenylalkyl, hetaryl, phenoxyalkyl or hetaryloxyalkyl,
R² represents in each case optionally halogen- or cyano-substituted alkyl, alkenyl, alkoxyalkyl or polyalkoxyalkyl or represents in each case optionally substituted cycloalkyl, phenyl or benzyl,
R³, R⁴ and R⁵ independently of one another represent in each case optionally halogensubstituted alkyl, alkoxy, alkylamino, dialkylamino, alkylthio, alkenylthio or cycloalkylthio or represent in each case optionally substituted phenyl, benzyl, phenoxy or phenylthio,
R⁶ and R⁷ independently of one another represent hydrogen, represent in each case optionally halogen- or cyano-substituted alkyl, cycloalkyl, alkenyl, alkoxy, alkoxyalkyl, represent in each case optionally substituted phenyl or benzyl, or together with the N atom to which they are attached form an optionally substituted cycle which optionally contains oxygen or sulphur.

2. Compounds of the formula (I) according to Claim 1 in which
W represents hydrogen, C₁-C₆-alkyl, C₂-C₆-alkenyl, C₂-C₆-alkynyl, halogen, C₁-C₆-alkoxy, C₁-C₄-haloalkyl, C₁-C₄-haloalkoxy or cyano,
X represents halogen, C₁-C₆-alkyl, C₂-C₆-alkenyl, C₂-C₆-alkynyl, C₁-C₆-haloalkyl, C₁-C₆-alkoxy, C₃-C₆-alkenyloxy, C₁-C₆-alkylthio, C₁-C₆-alkylsulphinyl, C₁-C₆-alkylsulphonyl, C₁-C₆-haloalkoxy, C₃-C₆-haloalkenyloxy, nitro or cyano,
Y and Z independently of one another represent hydrogen, halogen, C₁-C₆-alkyl, C₂-C₆-alkenyl, C₂-C₆-alkynyl, C₁-C₆-alkoxy, C₁-C₆-haloalkyl, C₁-C₆-haloalkoxy, cyano, C₂-C₆-alkenyl, C₂-C₆-alkynyl or one of the (het)aryl radicals 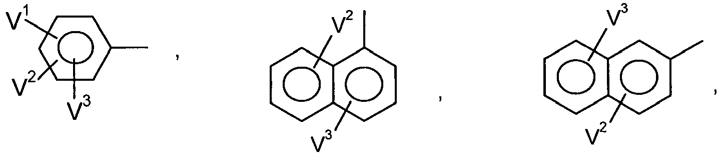 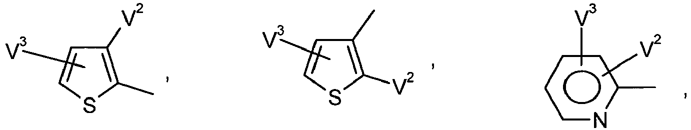 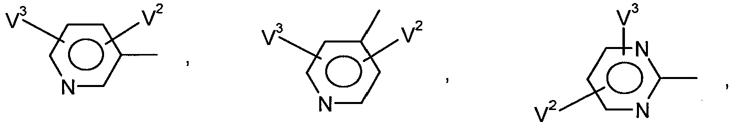 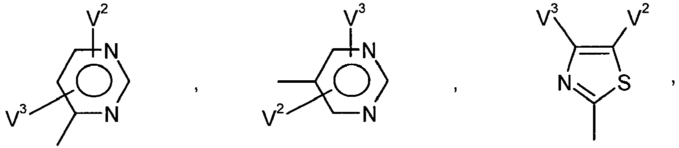 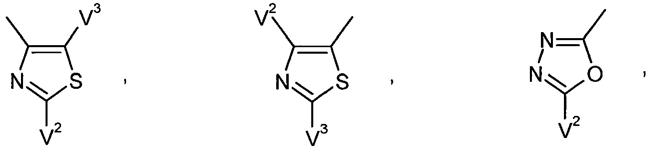 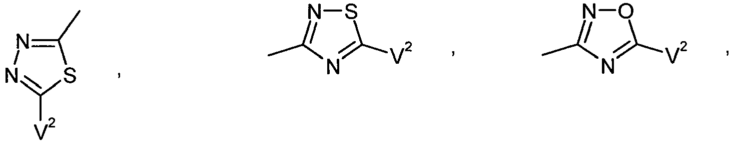 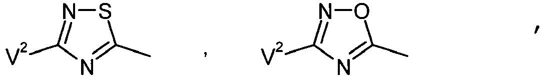 where in the case of (het)aryl only one of the radicals Y or Z may represent (het)aryl,
V¹ represents hydrogen, halogen, C₁-C₁₂-alkyl, C₁-C₆-alkoxy, C₁-C₆-alkylthio, C₁-C₆-alkylsulphinyl, C₁-C₆-alkylsulphonyl, C₁-C₄-haloalkyl, C₁-C₄-haloalkoxy, nitro, cyano or represents phenyl, phenoxy, phenoxy-C₁-C₄-alkyl, phenyl-C₁-C₄-alkoxy, phenylthio-C₁-C₄-alkyl or phenyl-C₁-C₄-alkylthio, each of which is optionally mono- or polysubstituted by halogen, C₁-C₆-alkyl, C₁-C₆-alkoxy, C₁-C₄-haloalkyl, C₁-C₄-haloalkoxy, nitro or cyano,
V² and V³ independently of one another represent hydrogen, halogen, C₁-C₆-alkyl, C₁-C₆-alkoxy, C₁-C₄-haloalkyl or C₁-C₄-haloalkoxy, with the proviso that at least one of the radicals W or Z has to be different from hydrogen if X and Y represent methyl,
A and B and the carbon atom to which they are attached represent five- to seven-membered ketal, thioketal, or dithioketal which may optionally be interrupted by a further oxygen atom, sulphur atom or by the group 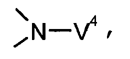 each of which radicals is optionally mono- to tetrasubstituted by C₁-C₆-alkyl, C₁-C₄-haloalkyl, C₁-C₆-alkoxy or C₁-C₄-alkoxy-C₁-C₄-alkyl,
V⁴ represents hydrogen, C₁-C₆-alkyl, C₃-C₆-alkenyl, or represents the groups -CO-V⁵, -CO₂V⁵, CO-NH-V⁵ or CO-NH-O-V⁵,
V⁵ represents C₁-C₆-alkyl,
D represents NH (1) or oxygen (2),
Q¹ and Q² independently of one another represent hydrogen, C₁-C₆-alkyl, C₁-C₂-haloalkyl or C₁-C₄-alkoxy,
G represents hydrogen (a) or represents one of the groups 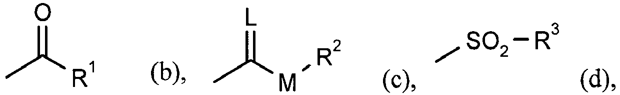 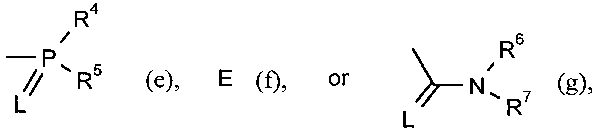 in which
E represents a metal ion or an ammonium ion,
L represents oxygen or sulphur and
M represents oxygen or sulphur,
R¹ represents in each case optionally halogen- or cyano-substituted C₁-C₂₀-alkyl, C₂-C₂₀-alkenyl, C₁-C₈-alkoxy-C₁-C₈-alkyl, C₁-C₈-alkylthio-C₁-C₈-alkyl or poly-C₁-C₈-alkoxy-C₁-C₈-alkyl or represents optionally halogen-, C₁-C₆-alkyl- or C₁-C₆-alkoxy-substituted C₃-C₈-cycloalkyl in which optionally one or two not directly adjacent methylene groups are replaced by oxygen and/or sulphur,
represents optionally halogen-, cyano-, nitro-, C₁-C₆-alkyl-, C₁-C₆-alkoxy-, C₁-C₆-haloalkyl-, C₁-C₆-haloalkoxy-, C₁-C₆-alkylthio- or C₁-C₆-alkylsulphonyl-substituted phenyl,
represents optionally halogen-, nitro-, cyano-, C₁-C₆-alkyl-, C₁-C₆-alkoxy-, C₁-C₆-haloalkyl- or C₁-C₆-haloalkoxy-substituted phenyl-C₁-C₆-alkyl,
represents optionally halogen- or C₁-C₆-alkyl-substituted 5- or 6-membered hetaryl having one or two heteroatoms from the group consisting of oxygen, sulphur and nitrogen, represents optionally halogen- or C₁-C₆-alkyl-substituted phenoxy-C₁-C₆-alkyl or represents optionally halogen-, amino- or C₁-C₆-alkyl-substituted 5- or 6-membered hetaryloxy-C₁-C₆-alkyl having one or two heteroatoms from the group consisting of oxygen, sulphur and nitrogen,
R² represents in each case optionally halogen- or cyano-substituted C₁-C₂₀-alkyl, C₂-C₂₀-alkenyl, C₁-C₈-alkoxy-C₂-C₈-alkyl or poly-C₁-C₈-alkoxy-C₂-C₈-alkyl,
represents optionally halogen-, C₁-C₆-alkyl- or C₁-C₆-alkoxy-substituted C₃-C₈-cycloalkyl or
represents in each case optionally halogen-, cyano-, nitro-, C₁-C₆-alkyl-, C₁-C₆-alkoxy-, C₁-C₆-haloalkyl- or C₁-C₆-haloalkoxy-substituted phenyl or benzyl,
R³ represents optionally halogen-substituted C₁-C₈-alkyl or in each case optionally halogen-, C₁-C₆-alkyl-, C₁-C₆-alkoxy-, C₁-C₄-haloalkyl-, C₁-C₄-haloalkoxy-, cyano- or nitro-substituted phenyl or benzyl,
R⁴ and R⁵ independently of one another represent in each case optionally halogen-substituted C₁-C₈-alkyl, C₁-C₈-alkoxy, C₁-C₈-alkylamino, di(C₁-C₈-alkyl)amino, C₁-C₈-alkylthio or C₃-C₈-alkenylthio or represent in each case optionally halogen-, nitro-, cyano-, C₁-C₄-alkoxy-, C₁-C₄-haloalkoxy-, C₁-C₄-alkylthio-, C₁-C₄-haloalkylthio-, C₁-C₄-alkyl- or C₁-C₄-haloalkyl-substituted phenyl, phenoxy or phenylthio,
R⁶ and R⁷ independently of one another represent hydrogen, represent in each case optionally halogen- or cyano-substituted C₁-C₈-alkyl, C₃-C₈-cycloalkyl, C₁-C₈-alkoxy, C₃-C₈-alkenyl or C₁-C₈-alkoxy-C₂-C₈-alkyl, represent in each case optionally halogen-, C₁-C₈-alkyl-, C₁-C₈-haloalkyl- or C₁-C₈-alkoxy-substituted phenyl or benzyl or together represent an optionally C₁-C₆-alkyl-substituted C₃-C₆-alkylene radical in which optionally one methylene group is replaced by oxygen or sulphur.

3. Compounds of the formula (I) according to Claim 1 in which
W represents hydrogen, chlorine, bromine, C₁-C₄-alkyl, C₂-C₄-alkenyl, C₂-C₄-alkynyl, C₁-C₄-alkoxy, C₁-C₂-haloalkyl or C₁-C₂-haloalkoxy,
X represents chlorine, bromine, C₁-C₄-alkyl, C₂-C₄-alkenyl, C₂-C₄-alkynyl, C₁-C₄-alkoxy, C₁-C₄-haloalkyl, C₁-C₄-haloalkoxy or cyano,
Y and Z independently of one another represent hydrogen, fluorine, chlorine, bromine, C₁-C₄-alkyl, C₂-C₄-alkenyl, C₂-C₄-alkynyl, C₁-C₆-alkoxy, C₁-C₄-haloalkyl, C₁-C₄-haloalkoxy, cyano, C₂-C₄-alkenyl, C₂-C₄-alkynyl or represent one of the (het)aryl radicals 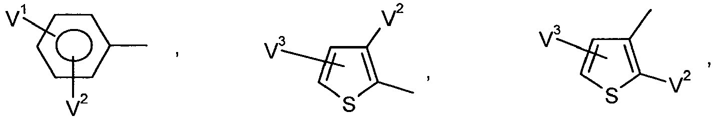 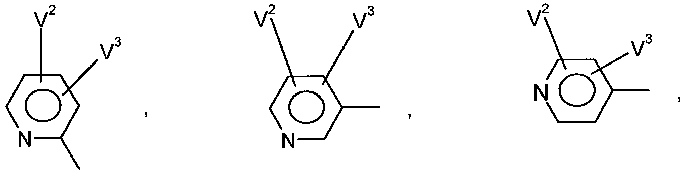 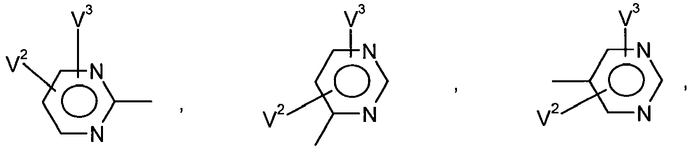
V¹ where in the case of (het)aryl only one of the radicals Y or Z may represent (het)aryl, represents hydrogen, fluorine, chlorine, bromine, C₁-C₆-alkyl, C₁-C₄-alkoxy, C₁-C₂-haloalkyl, C₁-C₂-haloalkoxy, nitro, cyano or represents phenyl which is optionally mono-or disubstituted by fluorine, chlorine, bromine, C₁-C₄-alkyl, C₁-C₄-alkoxy, C₁-C₂-haloalkyl, C₁-C₂-haloalkoxy, nitro or cyano,
V² and V³ independently of one another represent hydrogen, fluorine, chlorine, bromine, C₁-C₄-alkyl, C₁-C₄-alkoxy, C₁-C₂-haloalkyl or C₁-C₂-haloalkoxy,
with the proviso that at least one of the radicals W or Z has to be different from hydrogen if X and Y represent methyl,
A and B and the carbon atom to which they are attached represent a five-, six- or seven-membered ketal which may optionally be interrupted by a further oxygen atom and which is optionally mono- to trisubstituted by C₁-C₄-alkyl, C₁-C₃-haloalkyl, C₁-C₄-alkoxy or C₁-C₄-alkoxy-C₁-C₂-alkyl,
D represents NH (1) or oxygen (2),
Q¹ and Q² independently of one another represent hydrogen, methyl, ethyl, trifluoromethyl, methoxy or ethoxy,
G represents hydrogen (a) or represents one of the groups 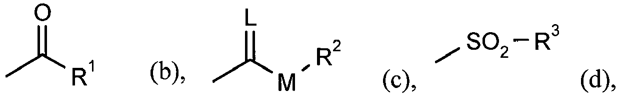 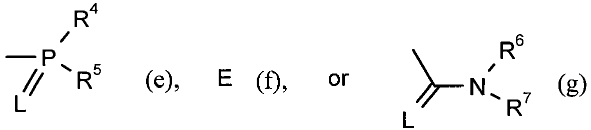 in which
E represents a metal ion or an ammonium ion,
L represents oxygen or sulphur and
M represents oxygen or sulphur,
R¹ represents C₁-C₁₆-alkyl, C₂-C₁₆-alkenyl, C₁-C₆-alkoxy-C₁-C₄-alkyl, C₁-C₆-alkylthio-C₁-C₄-alkyl or poly-C₁-C₆-alkoxy-C₁-C₄-alkyl, each of which is optionally mono- to trisubstituted by fluorine or chlorine, or represents C₃-C₇-cycloalkyl which is optionally mono- or disubstituted by fluorine, chlorine, C₁-C₅-alkyl or C₁-C₅-alkoxy and in which optionally one or two not directly adjacent methylene groups are replaced by oxygen and/or sulphur,
represents phenyl which is optionally mono-to trisubstituted by fluorine, chlorine, bromine, cyano, nitro, C₁-C₄-alkyl, C₁-C₄-alkoxy, C₁-C₃-haloalkyl, C₁-C₃-haloalkoxy, C₁-C₄-alkylthio or C₁-C₄-alkylsulphonyl,
represents phenyl-C₁-C₄-alkyl which is optionally mono- or disubstituted by fluorine, chlorine, bromine, C₁-C₄-alkyl, C₁-C₄-alkoxy, C₁-C₃-haloalkyl or C₁-C₃-haloalkoxy,
represents pyrazolyl, thiazolyl, pyridyl, pyrimidyl, furanyl or thienyl, each of which is optionally mono- or disubstituted by fluorine, chlorine, bromine or C₁-C₄-alkyl, represents phenoxy-C₁-C₅-alkyl which is optionally mono- or disubstituted by fluorine, chlorine, bromine or C₁-C₄-alkyl or
represents pyridyloxy-C₁-C₅-alkyl, pyrimidyloxy-C₁-C₅-alkyl or thiazolyloxy-C₁-C₅-alkyl, each of which is optionally mono-or disubstituted by fluorine, chlorine, bromine, amino or C₁-C₄-alkyl,
R² represents C₁-C₁₆-alkyl, C₂-C₁₆-alkenyl, C₁-C₆-alkoxy-C₂-C₆-alkyl or poly-C₁-C₆-alkoxy-C₂-C₆-alkyl, each of which is optionally mono- to trisubstituted by fluorine or chlorine,
represents C₃-C₇-cycloalkyl which is optionally mono- or disubstituted by fluorine, chlorine, C₁-C₄-alkyl or C₁-C₄-alkoxy or
represents phenyl or benzyl, each of which is optionally mono- to trisubstituted by fluorine, chlorine, bromine, cyano, nitro, C₁-C₄-alkyl, C₁-C₃-alkoxy, C₁-C₃-haloalkyl or C₁-C₃-haloalkoxy,
R³ represents C₁-C₆-alkyl which is optionally mono- to trisubstituted by fluorine or chlorine or represents phenyl or benzyl, each of which is optionally mono- or disubstituted by fluorine, chlorine, bromine, C₁-C₄-alkyl, C₁-C₄-alkoxy, C₁-C₂-haloalkoxy, C₁-C₂-haloalkyl, cyano or nitro,
R⁴ and R⁵ independently of one another represent C₁-C₆-alkyl, C₁-C₆-alkoxy, C₁-C₆-alkylamino, di(C₁-C₆-alkyl)amino, C₁-C₆-alkylthio or C₃-C₄-alkenylthio, each of which is optionally mono- to trisubstituted by fluorine or chlorine, or represent phenyl, phenoxy or phenylthio, each of which is optionally mono-or disubstituted by fluorine, chlorine, bromine, nitro, cyano, C₁-C₃-alkoxy, C₁-C₃-haloalkoxy, C₁-C₃-alkylthio, C₁-C₃-haloalkylthio, C₁-C₃-alkyl or C₁-C₃-haloalkyl,
R⁶ and R⁷ independently of one another represent hydrogen, represent C₁-C₆-alkyl, C₃-C₆-cycloalkyl, C₁-C₆-alkoxy, C₃-C₆-alkenyl or C₁-C₆-alkoxy-C₂-C₆-alkyl, each of which is optionally mono- to trisubstituted by fluorine or chlorine, represent phenyl or benzyl, each of which is optionally mono- to trisubstituted by fluorine, chlorine, bromine, C₁-C₅-haloalkyl, C₁-C₅-alkyl or C₁-C₅-alkoxy, or together represent an optionally C₁-C₄-alkyl-substituted C₃-C₆-alkylene radical in which optionally one methylene group is replaced by oxygen or sulphur.

4. Compounds of the formula (I) according to Claim 1 in which
W represents hydrogen, chlorine, bromine, methyl, ethyl, vinyl, ethynyl, propynyl, methoxy, ethoxy or trifluoromethyl,
X represents chlorine, bromine, methyl, ethyl, propyl, isopropyl, vinyl, ethynyl, propynyl, methoxy, ethoxy, trifluoromethyl, difluoromethoxy, trifluoromethoxy or cyano,
Y and Z independently of one another represent hydrogen, fluorine, chlorine, bromine, methyl, ethyl, vinyl, ethynyl, propynyl, methoxy, trifluoromethyl, trifluoromethoxy, cyano or a phenyl radical 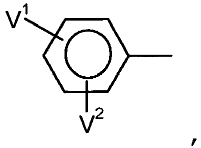 where in the case of phenyl only one of the radicals Y or Z may represent phenyl,
V¹ represents hydrogen, fluorine, chlorine, bromine, methyl, ethyl, n-propyl, isopropyl, tert-butyl, methoxy, ethoxy, n-propoxy, isopropoxy, trifluoromethyl or trifluoromethoxy,
V² represents hydrogen, fluorine, chlorine, methyl, ethyl, n-propyl, isopropyl, methoxy, ethoxy or trifluoromethyl, with the proviso that at least one of the radicals W or Z has to be different from hydrogen if X and Y represent methyl,
A and B and the carbon atom to which they are attached represent a five-, six- or seven-membered ketal which may optionally be interrupted by a further oxygen atom and which is optionally mono- or disubstituted by methyl, ethyl, propyl, trifluoromethyl, monochloromethyl, methoxy, ethoxy, methoxymethyl or ethoxymethyl,
D represents NH (1) or oxygen (2),
Q¹ and Q² represent hydrogen,
G represents hydrogen (a) or represents one of the groups 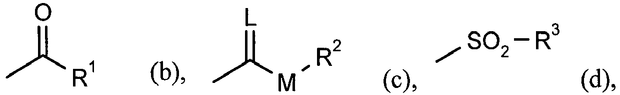 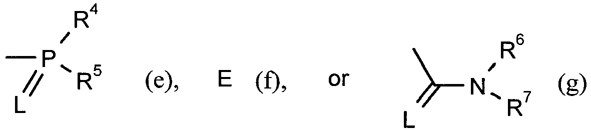 in which
E represents a metal ion or an ammonium ion,
L represents oxygen or sulphur and
M represents oxygen or sulphur,
R¹ represents C₁-C₁₀-alkyl, C₂-C₁₀-alkenyl, C₁-C₄-alkoxy-C₁-C₂-alkyl, C₁-C₄-alkylthio-C₁-C₂-alkyl, each of which is optionally mono- to trisubstituted by fluorine or chlorine, or
represents C₃-C₆-cycloalkyl which is optionally monosubstituted by fluorine, chlorine, methyl, ethyl or methoxy,
represents phenyl which is optionally mono-or disubstituted by fluorine, chlorine, bromine, cyano, nitro, methyl, ethyl, n-propyl, isopropyl, methoxy, ethoxy, trifluoromethyl or trifluoromethoxy, represents furanyl, thienyl or pyridyl, each of which is optionally monosubstituted by chlorine, bromine or methyl,
R² represents C₁-C₁₀-alkyl, C₂-C₁₀-alkenyl or C₁-C₄-alkoxy-C₂-C₄-alkyl, each of which is optionally mono- to trisubstituted by fluorine or chlorine,
represents cyclopentyl or cyclohexyl, or represents phenyl or benzyl, each of which is optionally mono- or disubstituted by fluorine, chlorine, cyano, nitro, methyl, ethyl, methoxy, trifluoromethyl or trifluoromethoxy,
R³ represents methyl, ethyl, propyl or isopropyl, each of which is optionally mono-to trisubstituted by fluorine or chlorine, or represents phenyl which is optionally monosubstituted by fluorine, chlorine, bromine, methyl, ethyl, isopropyl, tert-butyl, methoxy, ethoxy, isopropoxy, trifluoromethyl, trifluoromethoxy, cyano or nitro,
R⁴ and R⁵ independently of one another represent C₁-C₄-alkoxy or C₁-C₄-alkylthio or represent phenyl, phenoxy or phenylthio, each of which is optionally monosubstituted by fluorine, chlorine, bromine, nitro, cyano, methyl, methoxy, trifluoromethyl or trifluoromethoxy,
R⁶ and R⁷ independently of one another represent hydrogen, represent C₁-C₄-alkyl, C₃-C₆-cycloalkyl, C₁-C₄-alkoxy, C₃-C₄-alkenyl or C₁-C₄-alkoxy-C₂-C₄-alkyl, represent phenyl which is optionally mono- or disubstituted by fluorine, chlorine, bromine, methyl, methoxy or trifluoromethyl, or together represent a C₅-C₆-alkylene radical in which optionally one methylene group is replaced by oxygen or sulphur.

5. Compounds of the formula (I) according to Claim 1 in which
W represents hydrogen, chlorine, bromine, methyl, ethyl or methoxy,
X represents chlorine, bromine, methyl, ethyl or methoxy,
Y and Z independently of one another represent hydrogen, chlorine, bromine, methyl or represent the radical 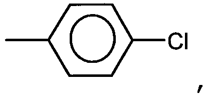 where in this case only one of the radicals Y or Z may represent 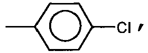 with the proviso that at least one of the radicals W or Z has to be different from hydrogen if X and Y represent methyl,
A and B and the carbon atom to which they are attached represent a five- or six-membered ketal which is optionally mono- or disubstituted by methyl, ethyl, propyl, monochloromethyl,
D represents NH (1) or oxygen (2),
Q¹ and Q² represent hydrogen,
G represents hydrogen (a) or represents one of the groups 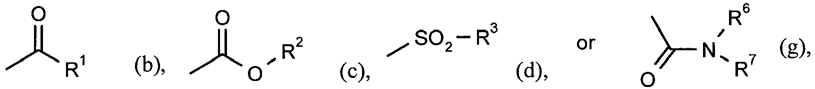
R¹ represents C₁-C₁₀-alkyl, C₁-C₄-alkoxy-C₁-C₂-alkyl, C₃-C₆-cycloalkyl, represents phenyl which is optionally monosubstituted by chlorine, or represents thienyl,
R² represents C₁-C₁₀-alkyl, C₂-C₁₀-alkenyl or benzyl,
R³ represents methyl,
R⁶ and R⁷ together represent a C₅-C₆-alkylene radical in which optionally one methylene group is replaced by oxygen or sulphur.

6. Process for preparing compounds of the formula (I) according to Claim 1, **characterized in that**, to obtain
(A) compounds of the formula (I-1-a) 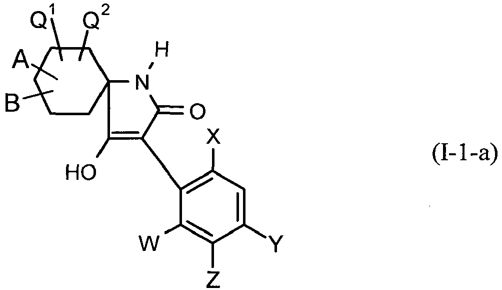 in which
A, B, Q¹, Q², W, X, Y and Z are as defined above,
compounds of the formula (II) 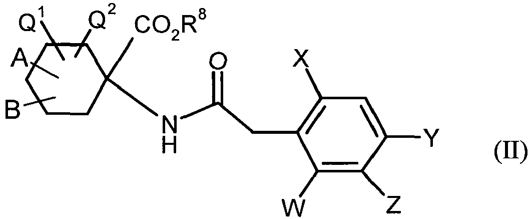 in which
A, B, Q¹, Q², W, X, Y and Z are as defined above,
and
R⁸ represents alkyl
are condensed intramolecularly in the presence of a diluent and in the presence of a base,
(B) compounds of the formula (I-2-a) 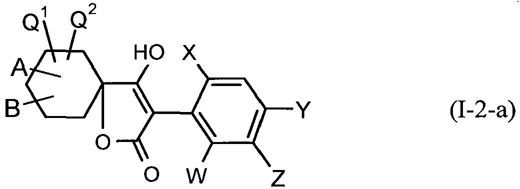 in which
A, B, Q¹, Q², W, X, Y and Z are as defined above,
compounds of the formula (III) 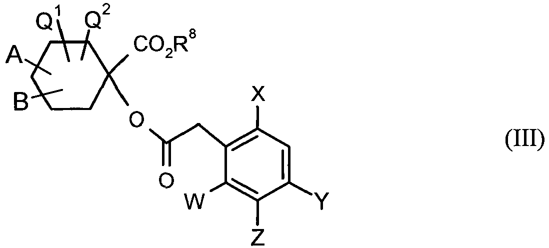 in which
A, B, Q¹, Q², W, X, Y, Z and R⁸ are as defined above
are condensed intramolecularly in the presence of a diluent and in the presence of a base,
(C) compounds of the formulae (I-1-b) to (I-2-b) 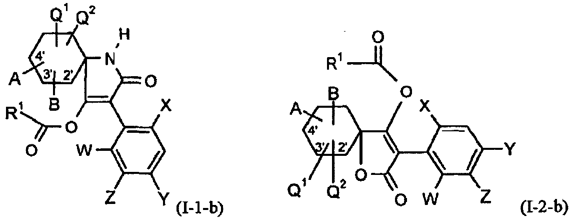 in which R¹, A, B, Q¹, Q², W, X, Y and Z are as defined above, compounds of the formulae (I-1-a) to (I-2-a) shown above in which A, B, Q¹, Q², W, X, Y and Z are as defined above are in each case reacted
α) with compounds of the formula (IV) 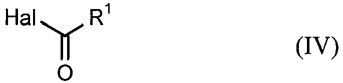 in which
R¹ is as defined above and
Hal represents halogen
or
ß) with carboxylic anhydrides of the formula (V)
R¹-CO-O-CO-R¹ (V)
in which
R¹ is as defined above,
if appropriate in the presence of a diluent and if appropriate in the presence of an acid binder;
(D) compounds of the formulae (I-1-c) to (I-2-c) 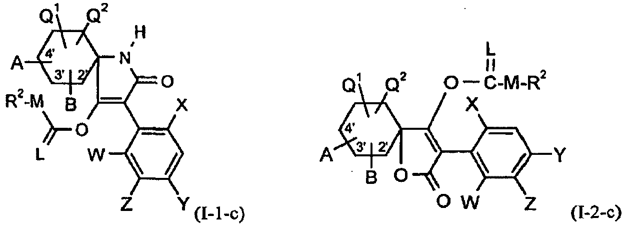 in which R², A, B, Q¹, Q², W, M, X, Y and Z are as defined above and L represents oxygen, compounds of the formulae (I-1-a) to (I-2-a) shown above in which A, B, Q¹, Q², W, X, Y and Z are as defined above are in each case reacted
with chloroformic esters or chloroformic thioesters of the formula (VI)
R²-M-CO-Cl (VI)
in which
R² and M are as defined above,
if appropriate in the presence of a diluent and if appropriate in the presence of an acid binder;
(E) compounds of the formulae (I-1-c) to (I-2-c) 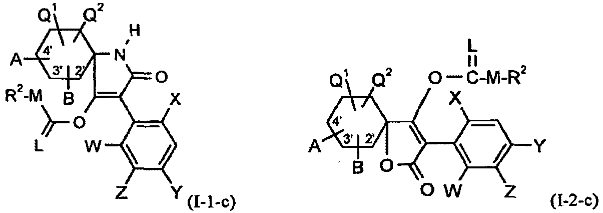 in which R², A, B, Q¹, Q², W, M, X, Y and Z are as defined above and L represents sulphur, compounds of the formulae (I-1-a) to (I-2-a) shown above in which A, B, Q¹, Q², W, X, Y and Z are as defined above are in each case reacted
with chloromonothioformic esters or chlorodithioformic esters of the formula (VII) 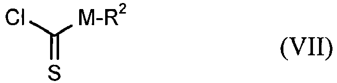 in which
M and R² are as defined above,
if appropriate in the presence of a diluent and if appropriate in the presence of an acid binder,
(F) compounds of the formulae (I-1-d) to (I-2-d) 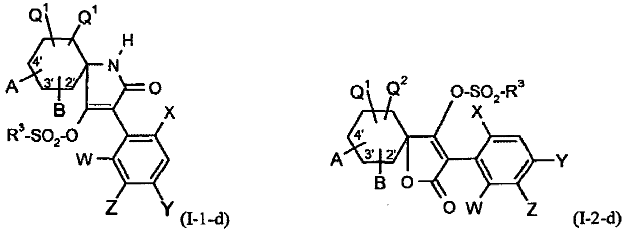 in which R³, A, B, W, Q¹, Q², X, Y and Z are as defined above, compounds of the formulae (I-1-a) to (I-2-a) shown above in which A, B, Q¹, Q², W, X, Y and Z are as defined above are in each case reacted
with sulphonyl chlorides of the formula (VIII)
R³-SO₂-Cl (VIII)
in which
R³ is as defined above,
if appropriate in the presence of a diluent and if appropriate in the presence of an acid binder,
(G) compounds of the formulae (I-1-e) to (I-2-e) 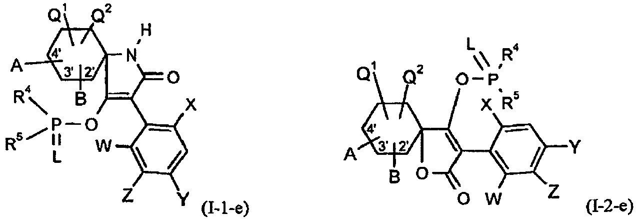 in which L, R⁴, R⁵, A, B, Q¹, Q², W, X, Y and Z are as defined above, compounds of the formulae (I-1-a) to (I-2-a) shown above in which A, B, Q¹, Q², W, X, Y and Z are as defined above are in each case reacted
with phosphorus compounds of the formula (IX) 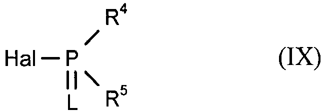 in which
L, R⁴ and R⁵ are as defined above and
Hal represents halogen,
if appropriate in the presence of a diluent and if appropriate in the presence of an acid binder,
(H) compounds of the formulae (I-1-f) to (I-2-f) 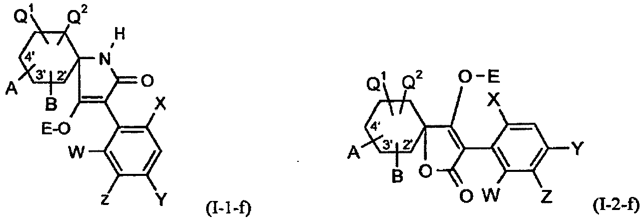 in which E, A, B, Q¹, Q², W, X, Y and Z are as defined above, compounds of the formulae (I-1-a) to (I-2-a) in which A, B, Q¹, Q², W, X, Y and Z are as defined above are in each case reacted
with metal compounds or amines of the formula (X) or (XI) 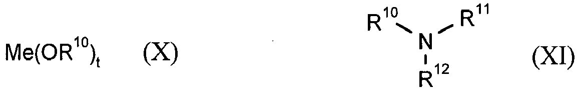 in which
Me represents a mono- or divalent metal,
t represents the number 1 or 2 and
R¹⁰, R¹¹, R¹² independently of one another represent hydrogen or alkyl,
if appropriate in the presence of a diluent,
(I) compounds of the formulae (I-1-g) to (I-2-g) 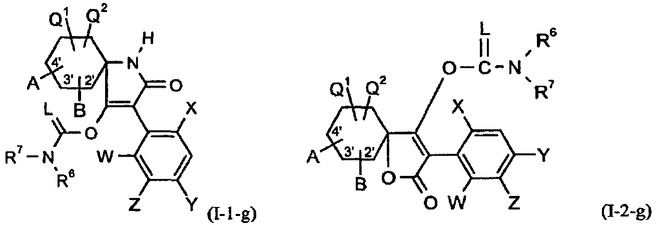 in which L, R⁶, R⁷, A, B, Q¹, Q², W, X, Y and Z are as defined above, compounds of the formulae (I-1-a) to (I-2-a) shown above in which A, B, Q¹, Q², W, X, Y and Z are as defined above are in each case
α) reacted with isocyanates or isothiocyanates of the formula (XII)
R⁶-N=C=L (XII)
in which
R⁶ and L are as defined above,
if appropriate in the presence of a diluent and if appropriate in the presence of a catalyst, or
ß) reacted with carbamoyl chlorides or thiocarbamoyl chlorides of the formula (XIII) 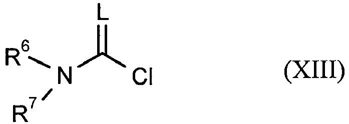 in which
L, R⁶ and R⁷ are as defined above,
if appropriate in the presence of a diluent and if appropriate in the presence of an acid binder,
(J) compounds of the formulae (I-I-a) to (I-1-g) and (I-2-a) to (I-2-g) 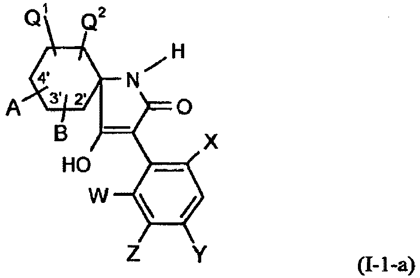 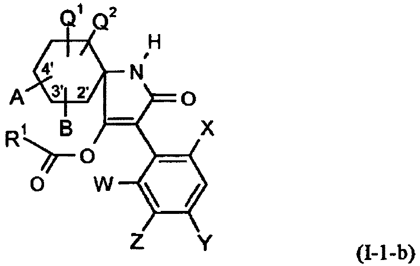 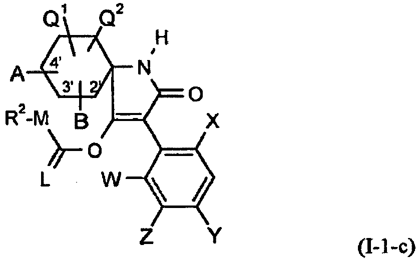 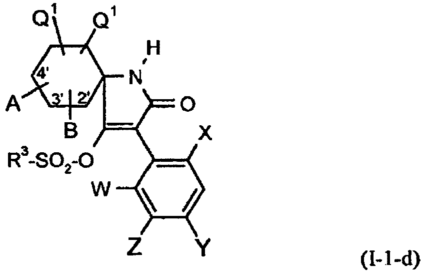 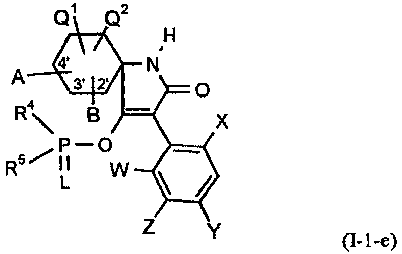 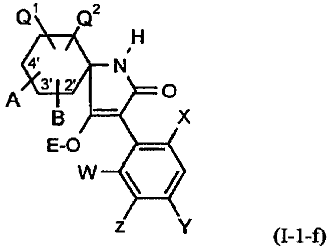 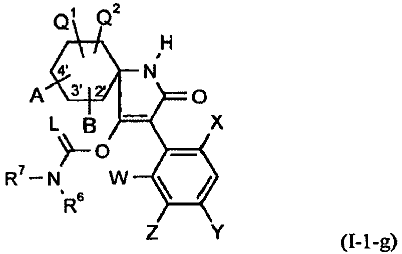 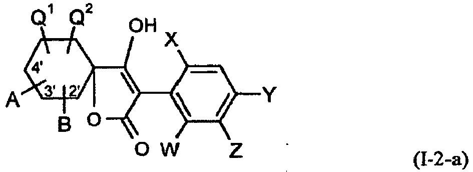 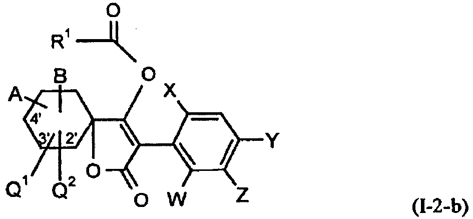 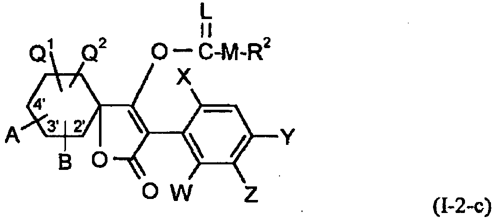 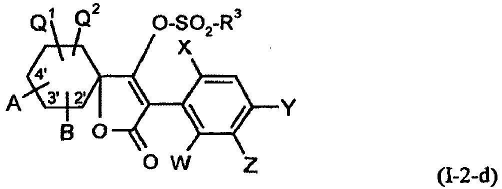 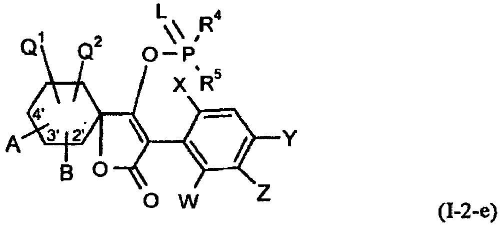 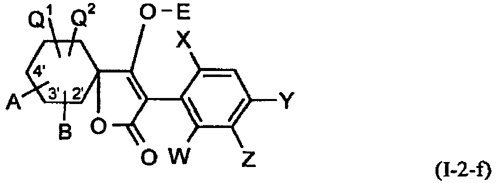 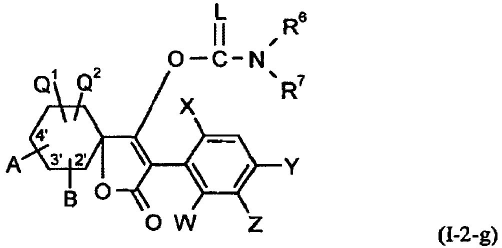 in which A, B, D, G, Q¹, Q², W, X, Y and Z are as defined above, compounds of the formula (XIV) 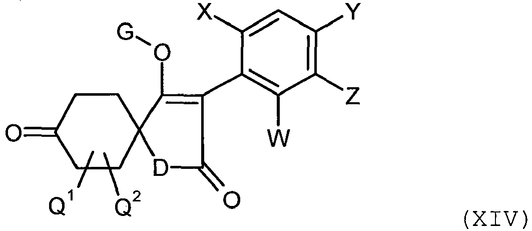 in which
D, G, Q¹, Q², W, X, Y and Z are as defined above
are reacted with diols of the formula (XV)
HO - A - B - OH (XV)
in which
A and B are as defined above,
if appropriate in the presence of a diluent, in the presence of an acidic catalyst and under dehydrating conditions.

7. Composition for controlling pests, unwanted vegetation and/or unwanted microorganisms, **characterized in that** it comprises at least one compound of the formula (I) according to Claim 1.

8. Method for controlling animal pests, unwanted vegetation and/or unwanted microorganisms, **characterized in that** compounds of the formula (I) according to Claim 1 are allowed to act on pests, unwanted vegetation, unwanted microorganisms and/or their habitat, there being excluded methods for the surgical or therapeutic treatment of the human or animal body.

9. Use of compounds of the formula (I) according to Claim 1 for controlling animal pests, unwanted vegetation and/or unwanted microorganisms, except for use in methods for the surgical or therapeutic treatment of the human or animal body.

10. Process for preparing compositions for controlling pests, unwanted vegetation and/or unwanted microorganisms, **characterized in that** compounds of the formula (I) according to Claim 1 are mixed with extenders and/or surfactants.

11. Use of compounds of the formula (I) according to Claim 1 for preparing compositions for controlling pests, unwanted vegetation and/or unwanted microorganisms.

12. Composition comprising an effective amount of an active compound combination comprising, as components,
(a') at least one compound of the formula (I) according to Claim 1 in which A, B, D, G, Q¹, Q², W, X, Y and Z are as defined above
and
(b') at least one crop plant compatibility-improving compound from the following group of compounds:
4-dichloroacetyl-1-oxa-4-azaspiro[4.5]decane (AD-67, MON-4660), 1-dichloroacetylhexahydro-3,3,8a-trimethylpyrrolo[1,2-a]pyrimidin-6(2H)-one (dicyclonon, BAS-145138), 4-dichloroacetyl-3,4-dihydro-3-methyl-2H-1,4-benzoxazine (benoxacor), 1-methylhexyl 5-chloroquinoline-8-oxyacetate (cloquintocet-mexyl -, 3-(2-chlorobenzyl)-1-(1-methyl-1-phenylethyl)urea (cumyluron), α-(cyanomethoximino)phenylacetonitrile (cyometrinil), 2,4-dichlorophenoxyacetic acid (2,4-D), 4-(2,4-dichlorophenoxy)butyric acid (2,4-DB), 1-(1-methyl-1-phenylethyl)-3-(4-methyl-phenyl)urea (daimuron, dymron), 3,6-dichloro-2-methoxybenzoic acid (dicamba), S-1-methyl-1-phenylethyl piperidine-1-thiocarboxylate (dimepiperate), 2,2-dichloro-N-(2-oxo-2-(2-propenylamino)ethyl)-N-(2-propenyl)acetamide (DKA-24), 2,2-dichloro-N,N-di-2-propenylacetamide (dichlormid), 4,6-dichloro-2-phenylpyrimidine (fenclorim), ethyl 1-(2,4-dichlorophenyl)-5-trichloromethyl-1H-1,2,4-triazole-3-carboxylate (fenchlorazole-ethyl -), phenylmethyl 2-chloro-4-trifluoromethylthiazole-5-carboxylate (flurazole), 4-chloro-N-(1,3-dioxolan-2-ylmethoxy)-α-trifluoroacetophenone oxime (fluxofenim), 3-dichloroacetyl-5-(2-furanyl)-2,2-dimethyloxazolidine (furilazole, MON-13900), ethyl 4,5-dihydro-5,5-diphenyl-3-isoxazolecarboxylate (isoxadifen-ethyl -), 1-(ethoxycarbonyl)ethyl 3,6-dichloro-2-methoxybenzoate (lactidichlor), (4-chloro-o-tolyloxy)acetic acid (MCPA), 2-(4-chloro-o-tolyloxy)propionic acid (mecoprop), diethyl 1-(2,4-dichorophenyl)-4,5-dihydro-5-methyl-1H-pyrazole-3,5-dicarboxylate (mefenpyr-diethyl -), 2-dichloromethyl-2-methyl-1,3-dioxolane (MG-191), 2-propenyl-1-oxa-4-azaspiro[4.5]decane-4-carbodithioate (MG-838), 1,8-naphthalic anhydride, α-(1,3-dioxolan-2-ylmethoximino)phenylacetonitrile (oxabetrinil), 2,2-dichloro-N-(1,3-dioxolan-2-ylmethyl)-N-(2-propenyl)acetamide (PPG-1292), 3-dichloroacetyl-2,2-dimethyloxazolidine (R-28725), 3-dichloroacetyl-2,2,5-trimethyloxazolidine (R-29148), 4-(4-chloro-o-tolyl)butyric acid, 4-(4-chlorophenoxy)butyric acid, diphenylmethoxyacetic acid, methyl diphenylmethoxyacetate, ethyl diphenylmethoxyacetate, methyl 1-(2-chlorophenyl)-5-phenyl-1H-pyrazole-3-carboxylate, ethyl 1-(2,4-dichlorophenyl)-5-methyl-1H-pyrazole-3-carboxylate, ethyl 1-(2,4-dichlorophenyl)-5-isopropyl-1H-pyrazole-3-carboxylate, ethyl 1-(2,4-dichlorophenyl)-5-(1,1-dimethylethyl)-1H-pyrazole-3-carboxylate, ethyl 1-(2,4-dichlorophenyl)-5-phenyl-1H-pyrazole-3-carboxylate, ethyl 5-(2,4-dichlorobenzyl)-2-isoxazoline-3-carboxylate, ethyl 5-phenyl-2-isoxazoline-3-carboxylate, ethyl 5-(4-fluorophenyl)-5-phenyl-2-isoxazoline-3-carboxylate, 1,3-dimethylbut-1-yl 5-chloroquinoline-8-oxyacetate, 4-allyloxybutyl 5-chloroquinoline-8-oxyacetate, 1-allyloxyprop-2-yl 5-chloroquinoline-8-oxyacetate, methyl 5-chloroquinoxaline-8-oxyacetate, ethyl 5-chloroquinoline-8-oxyacetate, allyl 5-chloroquinoxaline-8-oxyacetate, 2-oxoprop-1-yl 5-chloroquinoline-8-oxyacetate, diethyl 5-chloroquinoline-8-oxymalonate, diallyl 5-chloroquinoxaline-8-oxymalonate, diethyl 5-chloroquinoline-8-oxymalonate (), 4-carboxychroman-4-ylacetic acid (AC-304415,), 4-chlorophenoxyacetic acid, 3,3`-dimethyl-4-methoxybenzophenone, 1-bromo-4-chloromethyl-sulphonylbenzene, 1-[4-(N-2-methoxybenzoylsulphamoyl)phenyl]-3-methylurea (also known as N-(2-methoxybenzoyl)-4-[(methylaminocarbonyl)-amino]benzenesulphonamide), 1-[4-(N-2-methoxybenzoylsulphamoyl)phenyl]-3,3-dimethylurea, 1-[4-(N-4,5-dimethylbenzoylsulphamoyl)phenyl]-3-methylurea, 1-[4-(N-naphthylsulphamoyl)phenyl]-3,3-dimethylurea, N-(2-methoxy-5-methylbenzoyl)-4-(cyclopropylaminocarbonyl)benzenesulphonamide,
and/or one of the following compounds, defined by general formulae,
of the general formula (IIa) 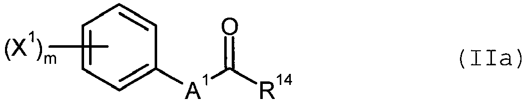 or of the general formula (IIb) 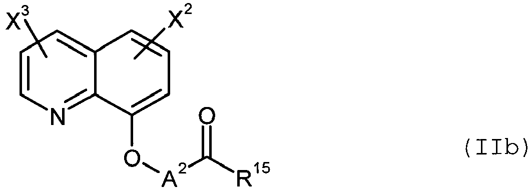 or of the formula (IIc) 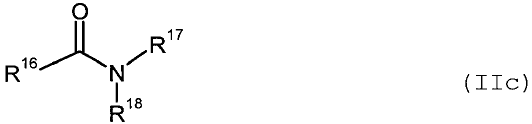 where
m represents a number 0, 1, 2, 3, 4 or 5,
A¹ represents one of the divalent heterocyclic groupings shown below 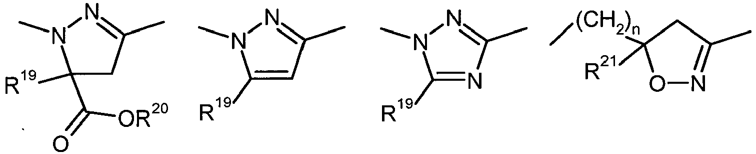
n represents a number 0, 1, 2, 3, 4 or 5,
A² represents optionally C₁-C₄-alkyl- and/or C₁-C₄-alkoxy-carbonyl- and/or C₁-C₄-alkenyloxy-carbonyl-substituted alkanediyl having 1 or 2 carbon atoms,
R¹⁴ represents hydroxyl, mercapto, amino, C₁-C₆-alkoxy, C₁-C₆-alkylthio, C₁-C₆-alkylamino or di (C₁-C₄-alkyl)-amino,
R¹⁵ represents hydroxyl, mercapto, amino, C₁-C₇-alkoxy, C₁-C₆-alkylthio, C₁-C₆-alkenyloxy, C₁-C₆-alkenyloxy-C₁-C₆-alkoxy, C₁-C₆-alkylamino or di (C₁-C₄-alkyl)-amino,
R¹⁶ represents optionally fluorine-, chlorine- and/or bromine-substituted C₁-C₄-alkyl,
R¹⁷ represents hydrogen, in each case optionally fluorine-, chlorine- and/or bromine-substituted C₁-C₆-alkyl, C₂-C₆-alkenyl or C₂-C₆-alkynyl, C₁-C₄-alkoxy-C₁-C₄-alkyl, dioxolanyl-C₁-C₄-alkyl, furyl, furyl-C₁-C₄-alkyl, thienyl, thiazolyl, piperidinyl, or optionally fluorine-, chlorine- and/or bromine- or C₁-C₄-alkyl-substituted phenyl,
R¹⁸ represents hydrogen, in each case optionally fluorine-, chlorine- and/or bromine-substituted C₁-C₆-alkyl, C₂-C₆-alkenyl or C₂-C₆-alkynyl, C₁-C₄-alkoxy-C₁-C₄-alkyl, dioxolanyl-C₁-C₄-alkyl, furyl, furyl-C₁-C₄-alkyl, thienyl, thiazolyl, piperidinyl, or optionally fluorine-, chlorine- and/or bromine- or C₁-C₄-alkyl-substituted phenyl, R¹⁷ and R¹⁸ also together represent C₃-C₆-alkanediyl or C₂-C₅-oxaalkanediyl, each of which is optionally substituted by C₁-C₄-alkyl, phenyl, furyl, a fused benzene ring or by two substituents which, together with the C atom to which they are attached, form a 5- or 6-membered carboxycle,
R¹⁹ represents hydrogen, cyano, halogen, or represents in each case optionally fluorine-, chlorine- and/or bromine-substituted C₁-C₄-alkyl, C₃-C₆-cycloalkyl or phenyl,
R²⁰ represents hydrogen, in each case optionally hydroxyl-, cyano-, halogen- or C₁-C₄-alkoxy-substituted C₁-C₆-alkyl, C₃-C₆-cycloalkyl or tri- (C₁-C₄-alkyl) -silyl,
R²¹ represents hydrogen, cyano, halogen, or represents in each case optionally fluorine-, chlorine- and/or bromine-substituted C₁-C₄-alkyl, C₃-C₆-cycloalkyl or phenyl,
X¹ represents nitro, cyano, halogen, C₁-C₄-alkyl, C₁-C₄-haloalkyl, C₁-C₄-alkoxy or C₁-C₄-haloalkoxy,
X² represents hydrogen, cyano, nitro, halogen, C₁-C₄-alkyl, C₁-C₄-haloalkyl, C₁-C₄-alkoxy or C₁-C₄-haloalkoxy,
X³ represents hydrogen, cyano, nitro, halogen, C₁-C₄-alkyl, C₁-C₄-haloalkyl, C₁-C₄-alkoxy or C₁-C₄-haloalkoxy,
and/or the following compounds, defined by general formulae, of the general formula (IId) 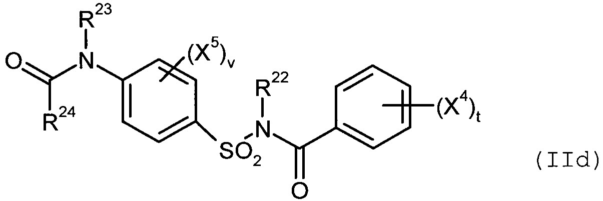 or of the general formula (IIe) 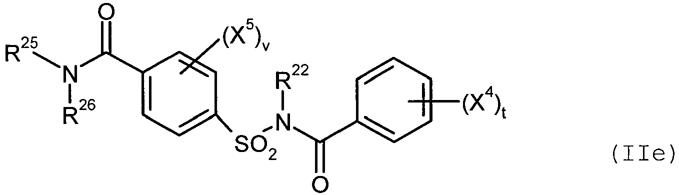 where
t represents a number between 0 and 5,
v represents a number between 0 and 5,
R²² represents hydrogen or C₁-C₄-alkyl,
R²³ represents hydrogen or C₁-C₄-alkyl,
R²⁴ represents hydrogen, in each case optionally cyano-, halogen- or C₁-C₄-alkoxy-substituted C₁-C₆-alkyl, C₁-C₆-alkoxy, C₁-C₆-alkylthio, C₁-C₆-alkylamino or di (C₁-C₄-alkyl) -amino, or in each case optionally cyano-, halogen- or C₁-C₄-alkyl-substituted C₃-C₆-cycloalkyl, C₃-C₆-cycloalkyloxy, C₃-C₆-cycloalkylthio or C₃-C₆-cycloalkylamino,
R²⁵ represents hydrogen, optionally cyano-, hydroxyl-, halogen- or C₁-C₄-alkoxy-substituted C₁-C₆-alkyl, in each case optionally cyano- or halogen-substituted C₃-C₆-alkenyl or C₃-C₆-alkynyl, or optionally cyano-, halogen- or C₁-C₄-alkyl-substituted C₃-C₆-cycloalkyl,
R²⁶ represents hydrogen, optionally cyano-, hydroxyl-, halogen- or C₁-C₄-alkoxy-substituted C₁-C₆-alkyl, in each case optionally cyano- or halogen-substituted C₃-C₆-alkenyl or C₃-C₆-alkynyl, optionally cyano-, halogen- or C₁-C₄-alkyl-substituted C₃-C₆-cycloalkyl, or optionally nitro-, cyano-, halogen-, C₁-C₄-alkyl-, C₁-C₄-haloalkyl-, C₁-C₄-alkoxy- or C₁-C₄-haloalkoxy-substituted phenyl, or together with R²⁵ represents in each case optionally C₁-C₄-alkyl-substituted C₂-C₆-alkanediyl or C₂-C₅-oxaalkanediyl,
X⁴ represents nitro, cyano, carboxyl, carbamoyl, formyl, sulphamoyl, hydroxyl, amino, halogen, C₁-C₄-alkyl, C₁-C₄-haloalkyl, C₁-C₄-alkoxy or C₁-C₄-haloalkoxy, and
X⁵ represents nitro, cyano, carboxyl, carbamoyl, formyl, sulphamoyl, hydroxyl, amino, halogen, C₁-C₄-alkyl, C₁-C₄-haloalkyl, C₁-C₄-alkoxy or C₁-C₄-haloalkoxy.

13. Composition according to Claim 12, in which the crop plant compatibility-improving compound is selected from the following group of compounds: cloquintocet-mexyl, fenchlorazole-ethyl, isoxadifen-ethyl, mefenpyr-diethyl, furilazole, fenclorim, cumyluron, dymron or the compounds 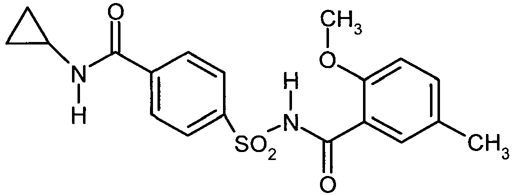 and

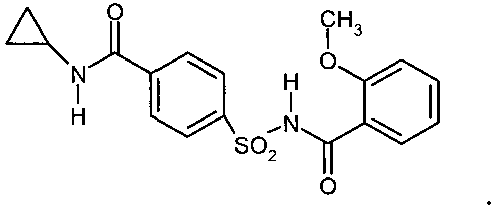

14. Composition according to one of Claims 12 and 13, in which the crop plant compatibility-improving compound is cloquintocet-mexyl.

15. Composition according to one of Claims 12 and 13, in which the crop plant compatibility-improving compound is mefenpyr-diethyl.

16. Method for controlling unwanted vegetation, **characterized in that** a composition according to Claim 12 is allowed to act on the plants or their habitat.

17. Use of a composition according to Claim 12 for controlling unwanted vegetation.

18. Method for controlling unwanted vegetation, **characterized in that** a compound of the formula (I) according to Claim 1 and the crop plant compatibility-improving compound according to Claim 12 are allowed to act, separately in close temporal succession, on the plants or their habitat.

19. Compounds of the formula (II) 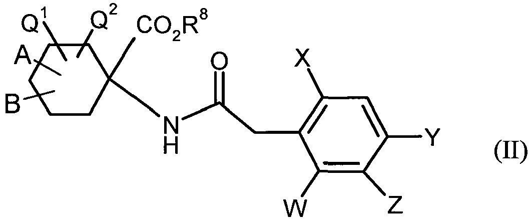 in which A, B, Q¹, Q², R⁸, W, X, Y and Z are as defined in Claims 1 and 6.

20. Compounds of the formula (III) 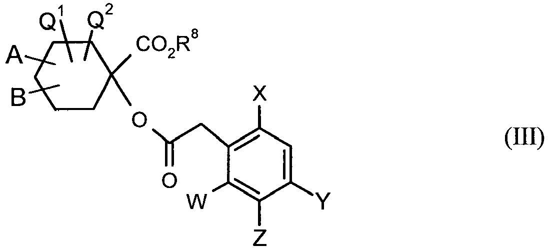 in which
A, B, Q¹, Q², W, X, Y, Z and R⁸ are as defined in Claims 1 and 6.

21. Compounds of the formula (XVIII) 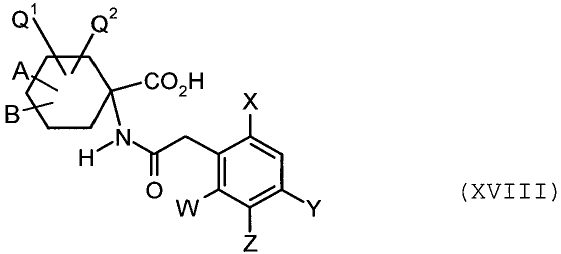 in which
A, B, Q¹, Q², W, X, Y and Z are as defined in Claim 1.

22. Compounds of the formula (XXI) 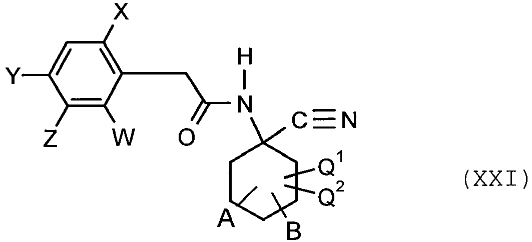 in which
A, B, Q¹, Q², W, X, Y and Z are as defined in Claim 1.

23. Compound of the formula (XXII) 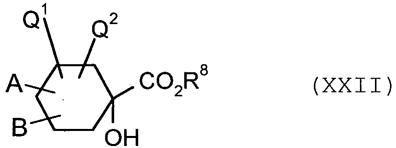 in which
A, B, Q¹, Q² and R⁸ are as defined in Claims 1 and 6, with the exception of

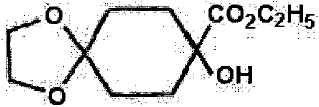

24. Compound of the formula (XVI') and their salts 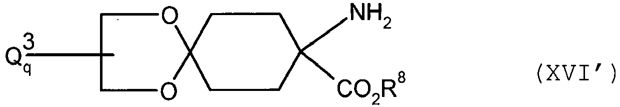 in which
Q³ represents C₁-C₄-alkyl, C₁-C₃-haloalkyl, C₁-C₄-alkoxy or C₁-C₄-alkoxy-C₁-C₂-alkyl,
q represents 1, 2 or 3,
R⁸ is as defined in Claim 6.

25. Compounds of the formula (XVI") and their salts 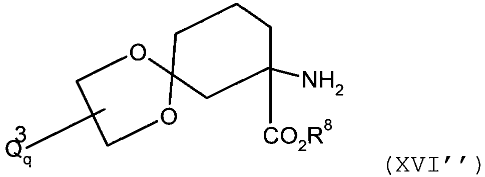 in which
Q³ and R⁸ are as defined in Claims 6 and 24 and
q represents 0, 1, 2 or 3.

26. Compounds of the formula (XVI"') and their salts 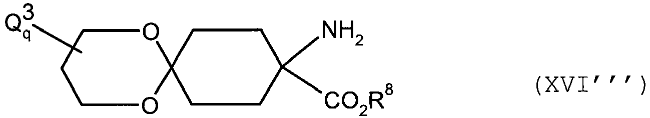 in which
Q³ and R⁸ are as defined in Claims 6 and 24 and
q represents 0, 1, 2 or 3.

27. Compounds of the formula (XIX') 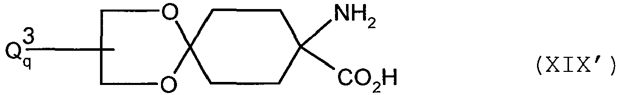 in which
Q³ is as defined in Claims 6 and 24 and
q represents 1, 2 or 3.

28. Compounds of the formula (XIX") 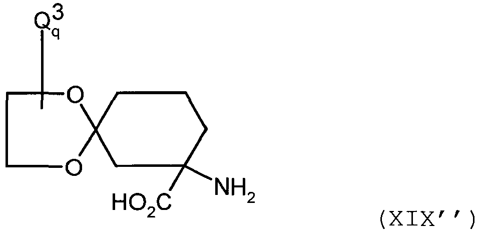 in which
Q³ is as defined in Claim 24 and
q represents 0, 1, 2 or 3.

29. Compounds of the formula (XIX"') 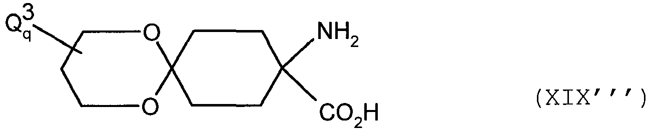 in which
Q³ is as defined in Claim 24 and
q represents 0, 1, 2 or 3.

30. Compounds of the formula (XXIII') 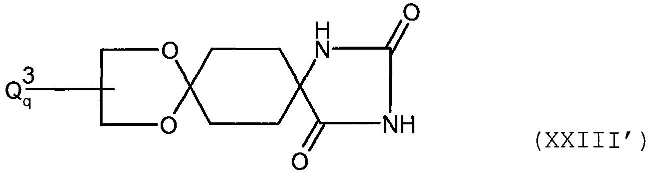 in which
Q³ is as defined in Claim 24 and
q represents 1, 2 or 3.

31. Compounds of the formula (XXIII") 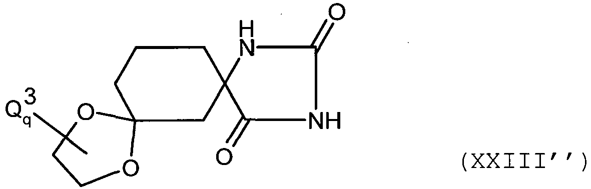 in which
Q³ is as defined in Claim 24 and
q represents 0, 1, 2 or 3.

32. Compounds of the formula (XXIII"') 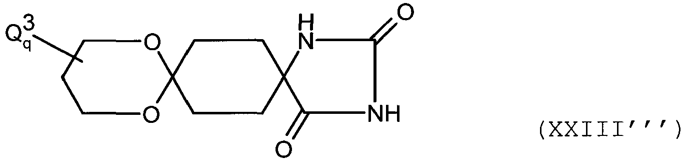 in which
Q³ is as defined in Claim 24 and
q represents 0, 1, 2 or 3.

33. Compound of the formula (I) according to Claim 1 in which the substituents are as defined in the Table:
| W | X | Y | Z | D | Q¹ | Q² | G | A | B |
|---|---|---|---|---|---|---|---|---|---|
| CH₃ | CH₃ | CH₃ | H | NH | H | H | H | 4'-O-(CH₂)₂-O- | |
| CH₃ | CH₃ | Cl | H | NH | H | H | H | 4'-O-(CH₂)₂-O- | |
| CH₃ | CH₃ | Br | H | NH | H | H | H | 4'-O-(CH₂)₂-O- | |
| CH₃ | CH₃ | CH₃ | H | NH | H | H | H | 4'-O-(CH₂)₃-O- | |
| CH₃ | CH₃ | Cl | H | NH | H | H | H | 4'-O-(CH₂)₃-O- | |
| CH₃ | CH₃ | Br | H | NH | H | H | H | 4'-O-(CH₂)₃-O- | |

## Revendications

1. Composés de formule (I) 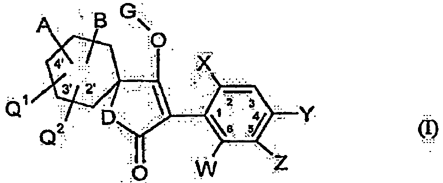 dans laquelle
W représente hydrogène, alkyle, alcényle, alcynyle, alcoxy, halogène, alcényloxy, halogénoalkyle, halogénoalcoxy ou cyano,
X représente halogène, alkyle, alcényle, alcynyle, alcoxy, alcényloxy, alkylthio, alkylsulfinyle, alkylsulfonyle, halogénoalkyle, halogénoalcoxy, halogénoalcényloxy, nitro ou cyano,
Y et Z représentent indépendamment l'un de l'autre hydrogène, alkyle, alcényle, alcynyle, alcoxy, halogène, halogénoalkyle, halogénoalcoxy, cyano, nitro ; ou aryle ou hétaryle, chacun éventuellement substitué,
à condition qu'au moins un des radicaux W ou Z soit différent de l'hydrogène lorsque X et Y représentent méthyle,
A et B et l'atome de carbone auquel ils sont reliés représentent un cétal, thiocétal ou dithiocétal de cinq à sept éléments, chacun éventuellement substitué par alkyle, halogénoalkyle, alcoxy, alcoxyalkyle ou phényle éventuellement substitué, qui peut éventuellement être interrompu par un hétéroatome supplémentaire,
D représente NH et oxygène,
Q¹, Q² représentent indépendamment l'un de l'autre hydrogène, alkyle, halogénoalkyle ou alcoxy,
G représente hydrogène (a) ou un des groupes 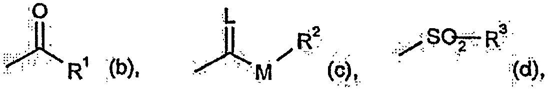 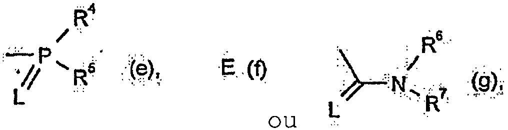
E représentant un ion métallique ou un ion ammonium,
L représentant l'oxygène ou le soufre,
M représentant l'oxygène ou le soufre,
R¹ représentant alkyle, alcényle, alcoxyalkyle, alkylthioalkyle ou polyalcoxyalkyle, chacun éventuellement substitué par halogène ou cyano ; ou cycloalkyle ou hétérocyclyle, chacun éventuellement substitué par halogène, alkyle ou alcoxy ; ou phényle, phénylalkyle, hétaryle, phénoxyalkyle ou hétaryloxyalkyle, chacun éventuellement substitué,
R² représentant alkyle, alcényle, alcoxyalkyle ou polyalcoxyalkyle, chacun éventuellement substitué par halogène ou cyano ; ou cycloalkyle, phényle ou benzyle, chacun éventuellement substitué,
R³, R⁴ et R⁵ représentant indépendamment les uns des autres alkyle, alcoxy, alkylamino, dialkylamino, alkylthio, alcénylthio ou cycloalkylthio, chacun éventuellement substitué par halogène ; ou phényle, benzyle, phénoxy ou phénylthio, chacun éventuellement substitué,
R⁶ et R⁷ représentant indépendamment l'un de l'autre hydrogène ; alkyle, cycloalkyle, alcényle, alcoxy, alcoxyalkyle, chacun éventuellement substitué par halogène ou cyano ; phényle ou benzyle, chacun éventuellement substitué ; ou formant conjointement avec l'atome N auquel ils sont reliés un cycle contenant éventuellement de l'oxygène ou du soufre et éventuellement substitué.

2. Composés de formule (I) selon la revendication 1, dans laquelle
W représente hydrogène, alkyle en C₁-C₆, alcényle en C₂-C₆, alcynyle en C₂-C₆, halogène, alcoxy en C₁-C₆, halogénoalkyle en C₁-C₄, halogénoalcoxy en C₁-C₄ ou cyano,
X représente halogène, alkyle en C₁-C₆, alcényle en C₂-C₆, alcynyle en C₂-C₆, halogénoalkyle en C₁-C₆, alcoxy en C₁-C₆, alcényloxy en C₃-C₆, alkylthio en C₁-C₆, alkylsulfinyle en C₁-C₆, alkylsulfonyle en C₁-C₆, halogénoalcoxy en C₁-C₆, halogénoalcényloxy en C₃-C₆, nitro ou cyano,
Y et Z représentent indépendamment l'un de l'autre hydrogène, halogène, alkyle en C₁-C₆, alcényle en C₂-C₆, alcynyle en C₂-C₆, alcoxy en C₁-C₆, halogénoalkyle en C₁-C₆, halogénoalcoxy en C₁-C₆, cyano, alcényle en C₂-C₆, alcynyle en C₂-C₆ ou un des radicaux (Het)-aryle 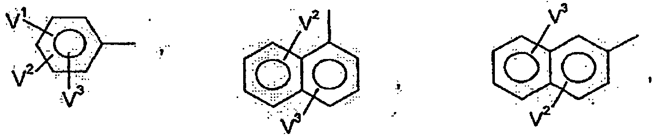 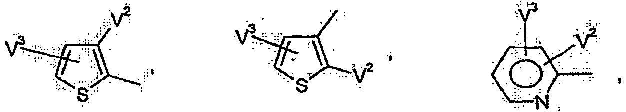 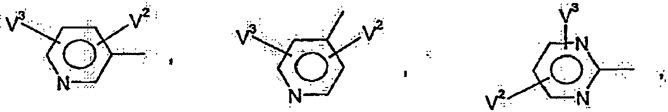 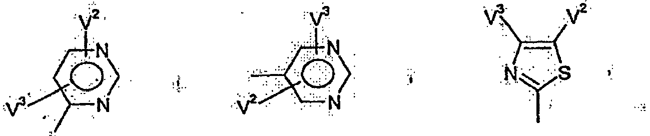 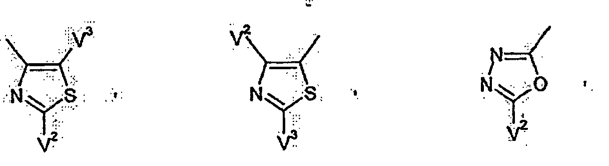 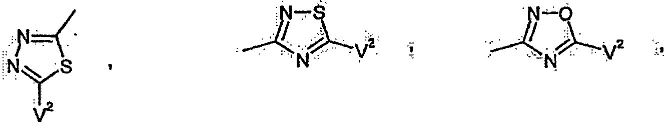 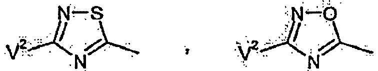 en cas de (Het)-aryle, seul un des radicaux Y ou Z pouvant représenter (Het)-aryle,
V¹ représentant hydrogène, halogène, alkyle en C₁-C₁₂, alcoxy en C₁-C₆, alkylthio en C₁-C₆, alkylsulfinyle en C₁-C₆, alkylsulfonyle en C₁-C₆, halogénoalkyle en C₁-C₄, halogénoalcoxy en C₁-C₄, nitro, cyano ; ou phényle, phénoxy, phénoxy-alkyle en C₁-C₄, phényl-alcoxy en C₁-C₄, phénylthio-alkyle en C₁-C₄ ou phényl-alkylthio en C₁-C₄, chacun éventuellement substitué une ou plusieurs fois par halogène, alkyle en C₁-C₆, alcoxy en C₁-C₆, halogénoalkyle en C₁-C₄, halogénoalcoxy en C₁-C₄, nitro ou cyano,
V² et V³ représentant indépendamment l'un de l'autre hydrogène, halogène, alkyle en C₁-C₆, alcoxy en C₁-C₆, halogénoalkyle en C₁-C₄ ou halogénoalcoxy en C₁-C₄,
à condition qu'au moins un des radicaux W ou Z soit différent de l'hydrogène lorsque X et Y représentent méthyle,
A et B et l'atome de carbone auquel ils sont reliés représentent un cétal, thiocétal ou dithiocétal de cinq à sept éléments, éventuellement substitué une à quatre fois par alkyle en C₁-C₆, halogénoalkyle en C₁-C₄, alcoxy en C₁-C₆ ou alcoxy en C₁-C₄-alkyle en C₁-C₄, qui peut éventuellement être interrompu par un atome d'oxygène, un atome de soufre supplémentaire ou par le groupe 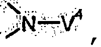
V⁴ représentant hydrogène, alkyle en C₁-C₆, alcényle en C₃-C₆ ou les groupes -CO-V⁵, -CO₂V⁵, CO-NH-V⁵ ou CO-NH-O-V⁵,
V⁵ représentant alkyle en C₁-C₆,
D représente NH (1) ou oxygène (2),
Q¹, Q² représentent indépendamment l'un de l'autre hydrogène, alkyle en C₁-C₆, halogénoalkyle en C₁-C₂ ou alcoxy en C₁-C₄,
G représente hydrogène (a) ou un des groupes 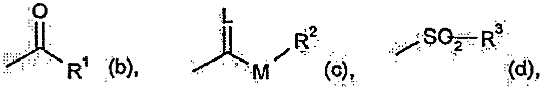 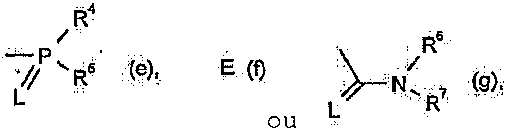
E représentant un ion métallique ou un ion ammonium,
L représentant l'oxygène ou le soufre et
M représentant l'oxygène ou le soufre,
R¹ représentant alkyle en C₁-C₂₀, alcényle en C₂-C₂₀, alcoxy en C₁-C₈-alkyle en C₁-C₈, alkylthio en C₁-C₈-alkyle en C₁-C₈ ou polyalcoxy en C₁-C₈-alkyle en C₁-C₈, chacun éventuellement substitué par halogène ou cyano ; ou cycloalkyle en C₃-C₈ éventuellement substitué par halogène, alkyle en C₁-C₆ ou alcoxy en C₁-C₆, dans lequel un ou deux groupes méthylène non directement voisins sont éventuellement remplacés par oxygène et/ou soufre,
phényle éventuellement substitué par halogène, cyano, nitro, alkyle en C₁-C₆, alcoxy en C₁-C₆, halogénoalkyle en C₁-C₆, halogénoalcoxy en C₁-C₆, alkylthio en C₁-C₆ ou alkylsulfonyle en C₁-C₆, phényl-alkyle en C₁-C₆ éventuellement substitué par halogène, nitro, cyano alkyle en C₁-C₆, alcoxy en C₁-C₆, halogénoalkyle en C₁-C₆ ou halogénoalcoxy en C₁-C₆,
hétaryle de 5 ou 6 éléments éventuellement substitué par halogène ou alkyle en C₁-C₆, contenant un ou deux hétéroatomes de la série oxygène, soufre et azote, phénoxy-alkyle en C₁-C₆ éventuellement substitué par halogène ou alkyle en C₁-C₆,
hétaryloxy-alkyle de 5 ou 6 éléments en C₁-C₆ éventuellement substitué par halogène, amino ou alkyle en C₁-C₆, contenant un ou deux hétéroatomes de la série oxygène, soufre et azote,
R² représentant alkyle en C₁-C₂₀, alcényle en C₂-C₂₀, alcoxy en C₁-C₈-alkyle en C₂-C₈ ou polyalcoxy en C₁-C₈-alkyle en C₂-C₈, chacun éventuellement substitué par halogène ou cyano,
cycloalkyle en C₃-C₈, éventuellement substitué par halogène, alkyle en C₁-C₆ ou alcoxy en C₁-C₆, ou phényle ou benzyle, chacun éventuellement substitué par halogène, cyano, nitro, alkyle en C₁-C₆, alcoxy en C₁-C₆, halogénoalkyle en C₁-C₆ ou halogénoalcoxy en C₁-C₆,
R³ représentant alkyle en C₁-C₈ éventuellement substitué par halogène ; ou phényle ou benzyle, chacun éventuellement substitué par halogène, alkyle en C₁-C₆, alcoxy en C₁-C₆, halogénoalkyle en C₁-C₄, halogénoalcoxy en C₁-C₄, cyano ou nitro,
R⁴ et R⁵ représentant indépendamment l'un de l'autre alkyle en C₁-C₈, alcoxy en C₁-C₈, alkylamino en C₁-C₈, di-(alkyle en C₁-C₈) amino, alkylthio en C₁-C₈ ou alcénylthio en C₃-C₈, chacun éventuellement substitué par halogène ; ou phényle, phénoxy ou phénylthio, chacun éventuellement substitué par halogène, nitro, cyano, alcoxy en C₁-C₄, halogénoalcoxy en C₁-C₄, alkylthio en C₁-C₄, halogénoalkylthio en C₁-C₄, alkyle en C₁-C₄ ou halogénoalkyle en C₁-C₄,
R⁶ et R⁷ représentant indépendamment l'un de l'autre hydrogène ; alkyle en C₁-C₈, cycloalkyle en C₃-C₈, alcoxy en C₁-C₈, alcényle en C₃-C₈ ou alcoxy en C₁-C₈-alkyle en C₂-C₈, chacun éventuellement substitué par halogène ou cyano ; phényle ou benzyle, chacun éventuellement substitué par halogène, alkyle en C₁-C₈, halogénoalkyle en C₁-C₈ ou alcoxy en C₁-C₈, ou représentant ensemble un radical alkylène en C₃-C₆ éventuellement substitué par alkyle en C₁-C₆, dans lequel un groupe méthylène est éventuellement remplacé par oxygène ou soufre.

3. Composés de formule (I) selon la revendication 1, dans laquelle
W représente hydrogène, chlore, brome, alkyle en C₁-C₄, alcényle en C₂-C₄, alcynyle en C₂-C₄, alcoxy en C₁-C₄, halogénoalkyle en C₁-C₂ ou halogénoalcoxy en C₁-C₂,
X représente chlore, brome, alkyle en C₁-C₄, alcényle en C₂-C₄, alcynyle en C₂-C₄, alcoxy en C₁-C₄, halogénoalkyle en C₁-C₄, halogénoalcoxy en C₁-C₄ ou cyano,
Y et Z représentent indépendamment l'un de l'autre hydrogène, fluor, chlore, brome, alkyle en C₁-C₄, alcényle en C₂-C₄, alcynyle en C₂-C₄, alcoxy en C₁-C₆, halogénoalkyle en C₁-C₄, halogénoalcoxy en C₁-C₄, cyano, alcényle en C₂-C₄, alcynyle en C₂-C₄ ou un des radicaux (Het)-aryle 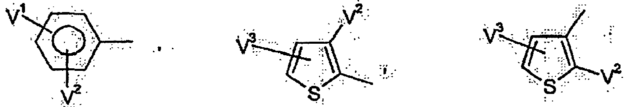 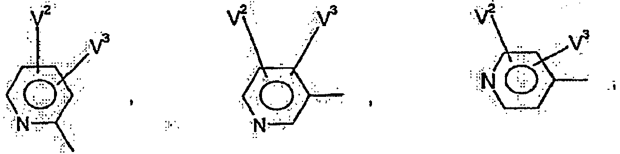 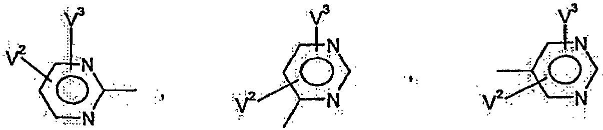 en cas de (Het)-aryle, seul un des radicaux Y ou Z pouvant représenter (Het)-aryle,
V¹ représentant hydrogène, fluor, chlore, brome, alkyle en C₁-C₆, alcoxy en C₁-C₄, halogénoalkyle en C₁-C₂, halogénoalcoxy en C₁-C₂, nitro, cyano ; ou phényle éventuellement substitué une à deux fois par fluor, chlore, brome, alkyle en C₁-C₄, alcoxy en C₁-C₄, halogénoalkyle en C₁-C₂, halogénoalcoxy en C₁-C₂, nitro ou cyano,
V² et V³ représentant indépendamment l'un de l'autre hydrogène, fluor, chlore, brome, alkyle en C₁-C₄, alcoxy en C₁-C₄, halogénoalkyle en C₁-C₂ ou halogénoalcoxy en C₁-C₂,
à condition qu'au moins un des radicaux W ou Z soit différent de l'hydrogène lorsque X et Y représentent méthyle,
A et B et l'atome de carbone auquel ils sont reliés représentent un cétal de cinq, six ou sept éléments, éventuellement substitué une à trois fois par alkyle en C₁-C₄, halogénoalkyle en C₁-C₃, alcoxy en C₁-C₄ ou alcoxy en C₁-C₄-alkyle en C₁-C₂, qui peut éventuellement être interrompu par un atome d'oxygène supplémentaire,
D représente NH (1) ou oxygène (2),
Q¹, Q² représentent indépendamment l'un de l'autre hydrogène, méthyle, éthyle, trifluorométhyle, méthoxy ou éthoxy,
G représente hydrogène (a) ou un des groupes 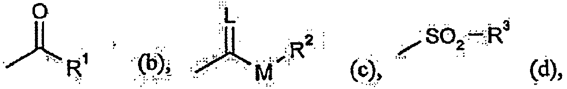 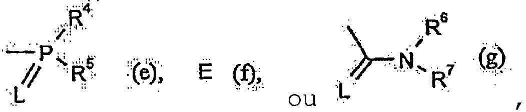
E représentant un ion métallique ou un ion ammonium,
L représentant l'oxygène ou le soufre et
M représentant l'oxygène ou le soufre,
R¹ représentant alkyle en C₁-C₁₆, alcényle en C₂-C₁₆, alcoxy en C₁-C₆-alkyle en C₁-C₄, alkylthio en C₁-C₆-alkyle en C₁-C₄ ou polyalcoxy en C₁-C₆-alkyle en C₁-C₄, chacun éventuellement substitué une à trois fois par fluor ou chlore ; ou cycloalkyle en C₃-C₇ éventuellement substitué une à deux fois par fluor, chlore, alkyle en C₁-C₅ ou alcoxy en C₁-C₅, dans lequel un ou deux groupes méthylène non directement voisins sont éventuellement remplacés par oxygène et/ou soufre,
phényle éventuellement substitué une à trois fois par fluor, chlore, brome, cyano, nitro, alkyle en C₁-C₄, alcoxy en C₁-C₄, halogénoalkyle en C₁-C₃, halogénoalcoxy en C₁-C₃, alkylthio en C₁-C₄ ou alkylsulfonyle en C₁-C₄,
phényl-alkyle en C₁-C₄ éventuellement substitué une à deux fois par fluor, chlore, brome, alkyle en C₁-C₄, alcoxy en C₁-C₄, halogénoalkyle en C₁-C₃ ou halogénoalcoxy en C₁-C₃,
pyrazolyle, thiazolyle, pyridyle, pyrimidyle, furanyle ou thiényle, chacun éventuellement substitué une à deux fois par fluor, chlore, brome ou alkyle en C₁-C₄,
phénoxy-alkyle en C₁-C₅ éventuellement substitué une à deux fois par fluor, chlore, brome ou alkyle en C₁-C₄, ou
pyridyloxy-alkyle en C₁-C₅, pyrimidyloxy-alkyle en C₁-C₅ ou thiazolyloxy-alkyle en C₁-C₅, chacun éventuellement substitué une à deux fois par fluor, chlore, brome, amino ou alkyle en C₁-C₄,
R² représentant alkyle en C₁-C₁₆, alcényle en C₂-C₁₆, alcoxy en C₁-C₆-alkyle en C₂-C₆ ou polyalcoxy en C₁-C₆-alkyle en C₂-C₆, chacun éventuellement substitué une à trois fois par fluor ou chlore, cycloalkyle en C₃-C₇, éventuellement substitué une à deux fois par fluor, chlore, alkyle en C₁-C₄ ou alcoxy en C₁-C₄, ou phényle ou benzyle, chacun éventuellement substitué une à trois fois par fluor, chlore, brome, cyano, nitro, alkyle en C₁-C₄, alcoxy en C₁-C₃, halogénoalkyle en C₁-C₃ ou halogénoalcoxy en C₁-C₃,
R³ représentant alkyle en C₁-C₆ éventuellement substitué une à trois fois par fluor ou chlore ; ou phényle ou benzyle, chacun éventuellement substitué une à deux fois par fluor, chlore, brome, alkyle en C₁-C₄, alcoxy en C₁-C₄, halogénoalcoxy en C₁-C₂, halogénoalkyle en C₁-C₂, cyano ou nitro,
R⁴ et R⁵ représentant indépendamment l'un de l'autre alkyle en C₁-C₆, alcoxy en C₁-C₆, alkylamino en C₁-C₆, di-(alkyle en C₁-C₆) amino, alkylthio en C₁-C₆ ou alcénylthio en C₃-C₄, chacun éventuellement substitué une à trois fois par fluor ou chlore ; ou phényle, phénoxy ou phénylthio, chacun éventuellement substitué une à deux fois par fluor, chlore, brome, nitro, cyano, alcoxy en C₁-C₃, halogénoalcoxy en C₁-C₃, alkylthio en C₁-C₃, halogénoalkylthio en C₁-C₃, alkyle en C₁-C₃ ou halogénoalkyle en C₁-C₃,
R⁶ et R⁷ représentant indépendamment l'un de l'autre hydrogène ; alkyle en C₁-C₆, cycloalkyle en C₃-C₆, alcoxy en C₁-C₆, alcényle en C₃-C₆ ou alcoxy en C₁-C₆-alkyle en C₂-C₆, chacun éventuellement substitué une à trois fois par fluor ou chlore ; phényle ou benzyle, chacun éventuellement substitué une à trois fois par fluor, chlore, brome, halogénoalkyle en C₁-C₅, alkyle en C₁-C₅ ou alcoxy en C₁-C₅; ou représentant ensemble un radical alkylène en C₃-C₆ éventuellement substitué par alkyle en C₁-C₄, dans lequel un groupe méthylène est éventuellement remplacé par oxygène ou soufre.

4. Composés de formule (I) selon la revendication 1, dans laquelle
W représente hydrogène, chlore, brome, méthyle, éthyle, vinyle, éthynyle, propynyle, méthoxy, éthoxy ou trifluorométhyle,
X représente chlore, brome, méthyle, éthyle, propyle, isopropyle, vinyle, éthynyle, propynyle, méthoxy, éthoxy, trifluorométhyle, difluorométhoxy, trifluorométhoxy ou cyano,
Y et Z représentent indépendamment l'un de l'autre hydrogène, fluor, chlore, brome, méthyle, éthyle, vinyle, éthynyle, propynyle, méthoxy, trifluorométhyle, trifluorométhoxy, cyano ou un radical phényle, 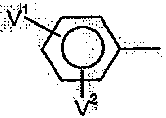 en cas de phényle, seul un des radicaux Y ou Z pouvant représenter phényle,
V¹ représentant hydrogène, fluor, chlore, brome, méthyle, éthyle, n-propyle, isopropyle, tert.-butyle, méthoxy, éthoxy, n-propoxy, isopropoxy, trifluorométhyle ou trifluorométhoxy,
V² représentant hydrogène, fluor, chlore, méthyle, éthyle, n-propyle, isopropyle, méthoxy, éthoxy ou trifluorométhyle,
à condition qu'au moins un des radicaux W ou Z soit différent de l'hydrogène lorsque X et Y représentent méthyle,
A et B et l'atome de carbone auquel ils sont reliés représentent un cétal de cinq, six ou sept éléments, éventuellement substitué une à deux fois par méthyle, éthyle, propyle, trifluorométhyle, monochlorométhyle, méthoxy, éthoxy, méthoxyméthyle ou éthoxyméthyle, qui peut éventuellement être interrompu par un atome d'oxygène supplémentaire,
D représente NH (1) ou oxygène (2),
Q¹, Q² représentent hydrogène,
G représente hydrogène (a) ou un des groupes 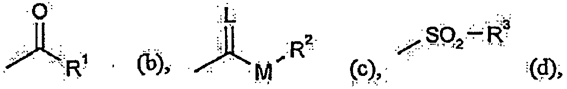 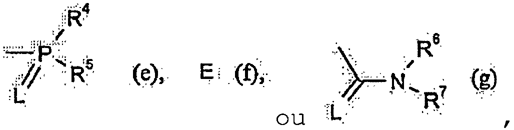
E représentant un ion métallique ou un ion ammonium,
L représentant l'oxygène ou le soufre et
M représentant l'oxygène ou le soufre,
R¹ représentant alkyle en C₁-C₁₀, alcényle en C₂-C₁₀, alcoxy en C₁-C₄-alkyle en C₁-C₂, alkylthio en C₁-C₄-alkyle en C₁-C₂, chacun éventuellement substitué une à trois fois par fluor ou chlore ; ou cycloalkyle en C₃-C₆ éventuellement substitué une fois par fluor, chlore, méthyle, éthyle ou méthoxy, phényle éventuellement substitué une à deux fois par fluor, chlore, brome, cyano, nitro, méthyle, éthyle, n-propyle, i-propyle, méthoxy, éthoxy, trifluorométhyle ou trifluorométhoxy, furanyle, thiényle ou pyridyle, chacun éventuellement substitué une fois par chlore, brome ou méthyle,
R² représentant alkyle en C₁-C₁₀, alcényle en C₂-C₁₀ ou alcoxy en C₁-C₄-alkyle en C₂-C₄, chacun éventuellement substitué une à trois fois par fluor ou chlore, cyclopentyle ou cyclohexyle, ou phényle ou benzyle, chacun éventuellement substitué une à deux fois par fluor, chlore, cyano, nitro, méthyle, éthyle, méthoxy, trifluorométhyle ou trifluorométhoxy,
R³ représentant méthyle, éthyle, propyle ou isopropyle, chacun éventuellement substitué une à trois fois par fluor ou chlore ; ou phényle éventuellement substitué une fois par fluor, chlore, brome, méthyle, éthyle, isopropyle, tert.-butyle, méthoxy, éthoxy, isopropoxy, trifluorométhyle, trifluorométhoxy, cyano ou nitro,
R⁴ et R⁵ représentant indépendamment l'un de l'autre alcoxy en C₁-C₄ ou alkylthio en C₁-C₄ ; ou phényle, phénoxy ou phénylthio, chacun éventuellement substitué une fois par fluor, chlore, brome, nitro, cyano, méthyle, méthoxy, trifluorométhyle ou trifluorométhoxy,
R⁶ et R⁷ représentant indépendamment l'un de l'autre hydrogène, alkyle en C₁-C₄, cycloalkyle en C₃-C₆, alcoxy en C₁-C₄, alcényle en C₃-C₄ ou alcoxy en C₁-C₄-alkyle en C₂-C₄ ; phényle éventuellement substitué une à deux fois par fluor, chlore, brome, méthyle, méthoxy ou trifluorométhyle ; ou représentant ensemble un radical alkylène en C₅-C₆, dans lequel un groupe méthylène est éventuellement remplacé par oxygène ou soufre.

5. Composés de formule (I) selon la revendication 1, dans laquelle
W représente hydrogène, chlore, brome, méthyle, éthyle ou méthoxy,
X représente chlore, brome, méthyle, éthyle ou méthoxy,
Y et Z représentent indépendamment l'un de l'autre hydrogène, chlore, brome, méthyle ou le radical 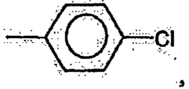 dans ce cas, seul un des radicaux Y ou Z pouvant
représenter 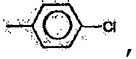
à condition qu'au moins un des radicaux W ou Z soit différent de l'hydrogène lorsque X et Y représentent méthyle,
A et B et l'atome de carbone auquel ils sont reliés représentent un cétal de cinq ou six éléments, éventuellement substitué une à deux fois par méthyle, éthyle, propyle, monochlorométhyle,
D représente NH (1) ou oxygène (2),
Q¹, Q² représentent hydrogène,
G représente hydrogène (a) ou un des groupes 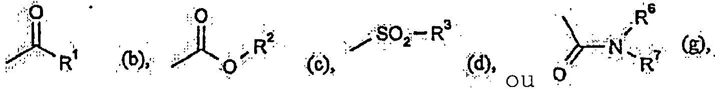
R¹ représentant alkyle en C₁-C₁₀, alcoxy en C₁-C₄-alkyle en C₁-C₂, cycloalkyle en C₃-C₆, phényle éventuellement substitué une fois par chlore ; ou thiényle,
R² représentant alkyle en C₁-C₁₀, alcényle en C₂-C₁₀ ou benzyle,
R³ représentant méthyle,
R⁶ et R⁷ représentant ensemble un radical alkylène en C₅-C₆, dans lequel un groupe méthylène est éventuellement remplacé par oxygène ou soufre.

6. Procédé de fabrication de composés de formule (I) selon la revendication 1, **caractérisé en ce que**, pour obtenir
(A) des composés de formule (I-1a) 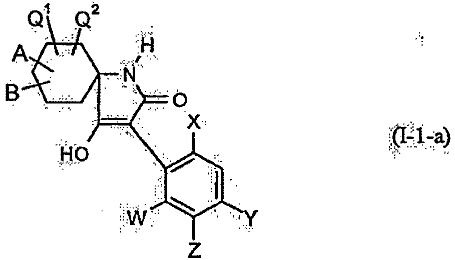 dans laquelle
A, B, Q¹, Q², W, X, Y et Z ont les significations indiquées précédemment,
des composés de formule (II) 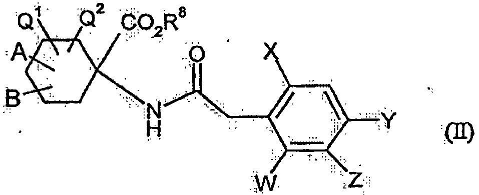 dans laquelle
A, B, Q¹, Q², W, X, Y et Z ont les significations indiquées précédemment,
et
R⁸ représente alkyle,
sont condensés intramoléculairement en présence d'un diluant et en présence d'une base,
(B) des composés de formule (I-2-a) 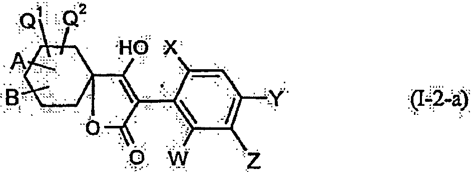 dans laquelle
A, B, Q¹, Q², W, X, Y et Z ont les significations indiquées précédemment,
des composés de formule (III) 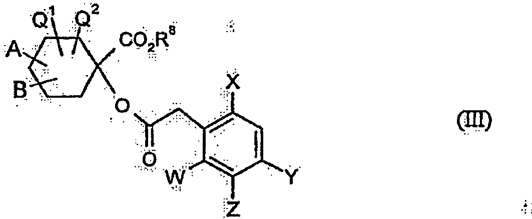 dans laquelle
A, B, Q¹, Q², W, X, Y, Z et R⁸ ont les significations indiquées précédemment,
sont condensés intramoléculairement en présence d'un diluant et en présence d'une base,
(C) des composés des formules (I-1-b) à (I-2-b) 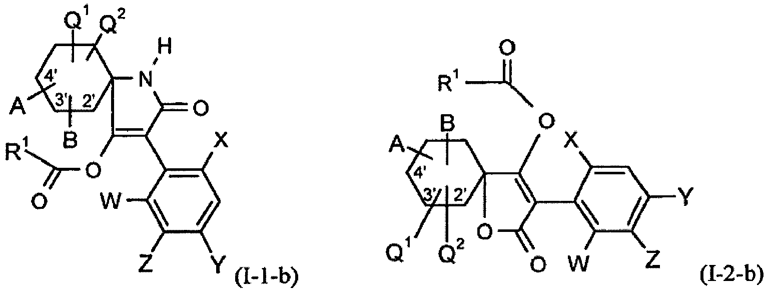 dans lesquelles R¹, A, B, Q¹, Q², W, X, Y et Z ont les significations indiquées précédemment, des composés des formules (I-1-a) à (I-2-a) représentées précédemment, dans lesquelles A, B, Q¹, Q², W, X, Y et Z ont les significations indiquées précédemment, sont à chaque fois mis en réaction
α) avec des composés de formule (IV) 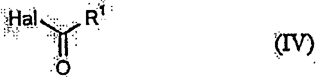 dans laquelle
R¹ a la signification indiquée précédemment et
Hal représente un halogène,
ou
β) avec des anhydrides d'acides carboxyliques de formule (V)
R¹-CO-O-CO-R¹ (V)
dans laquelle
R¹ a la signification indiquée précédemment éventuellement en présence d'un diluant et éventuellement en présence d'un liant d'acide ;
(D) des composés des formules (I-1-c) à (I-2-c) 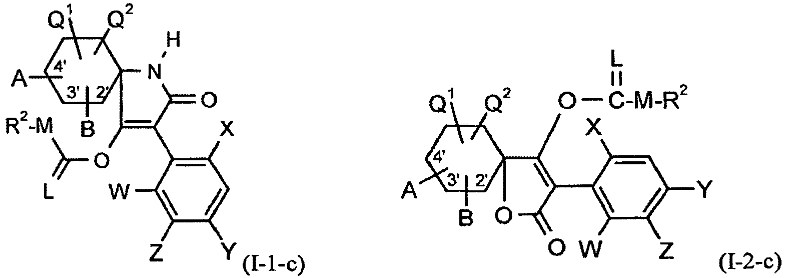 dans lesquelles R², A, B, Q¹, Q², W, M, X, Y et Z ont les significations indiquées précédemment et L représente l'oxygène, des composés des formules (I-1-a) à (I-2-a) représentées précédemment, dans lesquelles A, B, Q¹, Q², W, X, Y et Z ont les significations indiquées précédemment, sont à chaque fois mis en réaction avec des esters de l'acide chloroformique ou des thioesters de l'acide chloroformique de formule (VI)
R²-M-CO-Cl (VI)
dans laquelle
R² et M ont les significations indiquées précédemment, éventuellement en présence d'un diluant et éventuellement en présence d'un liant d'acide ;
(E) des composés des formules (I-1-c) à (I-2-c) 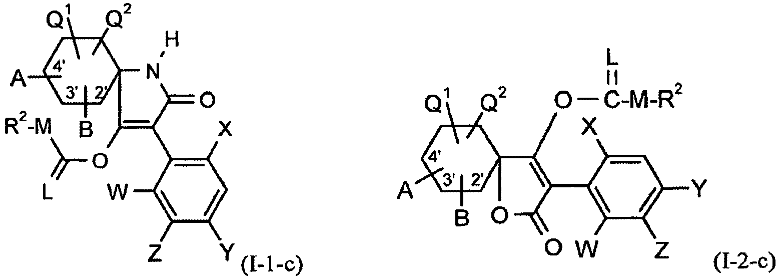 dans lesquelles R², A, B, Q¹, Q², W, M, X, Y et Z ont les significations indiquées précédemment et L représente le soufre, des composés des formules (I-1-a) à (I-2-a) représentées précédemment, dans lesquelles A, B, Q¹, Q², W, X, Y et Z ont les significations indiquées précédemment, sont à chaque fois mis en réaction avec des esters de l'acide chloromonothioformique ou des esters de l'acide chlorodithioformique de formule (VII) 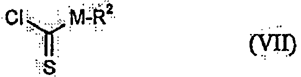 dans laquelle
M et R² ont les significations indiquées précédemment,
éventuellement en présence d'un diluant et éventuellement en présence d'un liant d'acide ;
(F) des composés des formules (I-1-d) à (I-2-d) 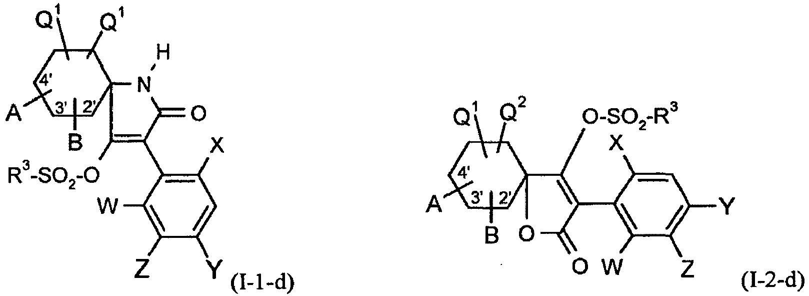 dans lesquelles R³, A, B, W, Q¹, Q², X, Y et Z ont les significations indiquées précédemment, des composés des formules (I-1-a) à (I-2-a) représentées précédemment, dans lesquelles A, B, Q¹, Q², W, X, Y et Z ont les significations indiquées précédemment, sont à chaque fois mis en réaction
avec des chlorures d'acides sulfoniques de formule (VIII)
R³-SO₂-Cl (VIII)
dans laquelle
R³ a la signification indiquée précédemment, éventuellement en présence d'un diluant et éventuellement en présence d'un liant d'acide ;
(G) des composés des formules (I-1-e) à (I-2-e) 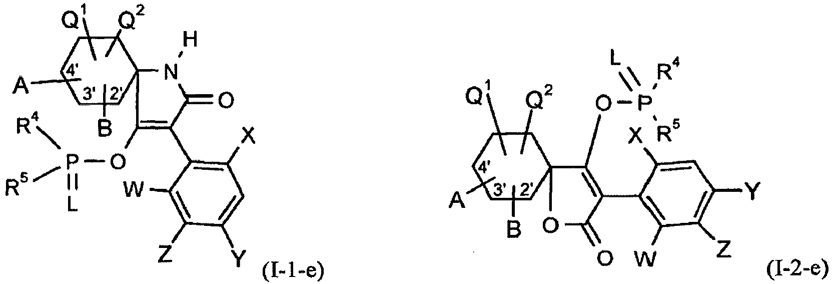 dans lesquelles L, R⁴, R⁵, A, B, Q¹, Q², W, X, Y et Z ont les significations indiquées précédemment, des composés des formules (I-1-a) à (I-2-a) représentées précédemment, dans lesquelles A, B, Q¹, Q², W, X, Y et Z ont les significations indiquées précédemment, sont à chaque fois mis en réaction
avec des composés phosphorés de formule (IX) 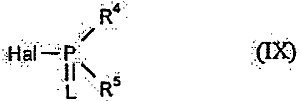 dans laquelle
L, R⁴ et R⁵ ont les significations indiquées précédemment et
Hal représente un halogène,
éventuellement en présence d'un diluant et éventuellement en présence d'un liant d'acide ;
(H) des composés des formules (I-1-f) à (1-2-f) 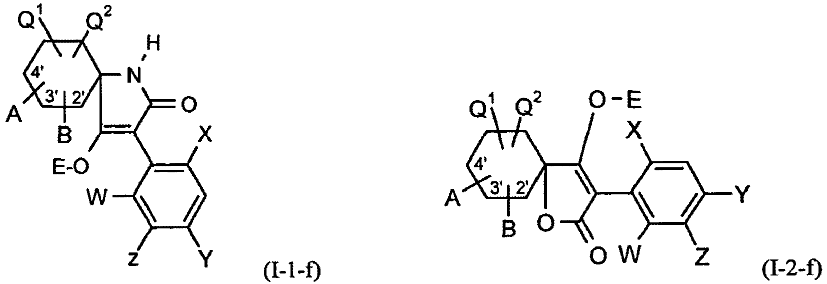 dans lesquelles E, A, B, Q¹, Q², W, X, Y et Z ont les significations indiquées précédemment, des composés des formules (I-1-a) à (I-2-a), dans lesquelles A, B, Q¹, Q², W, X, Y et Z ont les significations indiquées précédemment, sont à chaque fois mis en réaction avec des composés de métaux ou des amines des formules (X) ou (XI) 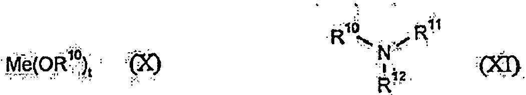 dans lesquelles
Me représente un métal mono- ou bivalent,
t représente le nombre 1 ou 2 et
R¹⁰, R¹¹, R¹² représentent indépendamment les uns des autres l'hydrogène ou un alkyle,
éventuellement en présence d'un diluant ;
(I) des composés des formules (I-1-g) à (1-2-g) 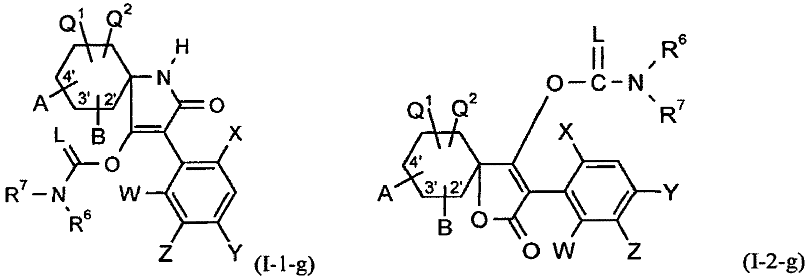 dans lesquelles L, R⁶, R⁷, A, B, Q¹, Q², W, X, Y et Z ont les significations indiquées précédemment, des composés des formules (I-1-a) à (I-2-a) représentées précédemment, dans lesquelles A, B, Q¹, Q², W, X, Y et Z ont les significations indiquées précédemment, sont à chaque fois mis en réaction
α) avec des isocyanates ou des isothiocyanates de formule (XII)
R⁶-N=C=L (XII)
dans laquelle
R⁶ et L ont les significations indiquées précédemment, éventuellement en présence d'un diluant et éventuellement en présence d'un catalyseur, ou
β) avec des chlorures de l'acide carbamique ou des chlorures de l'acide thiocarbamique de formule (XIII) 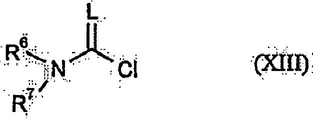 dans laquelle
L, R⁶ et R⁷ ont les significations indiquées précédemment
éventuellement en présence d'un diluant et éventuellement en présence d'un liant d'acide ;
(J) des composés des formules (I-1-a) à (I-1-g) et (I-2-a) à (I-2-g) 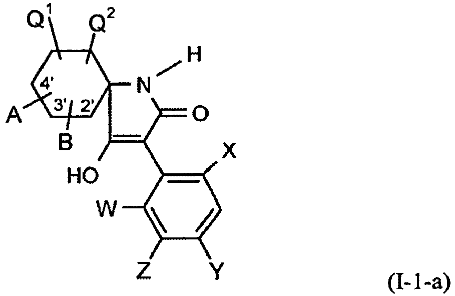 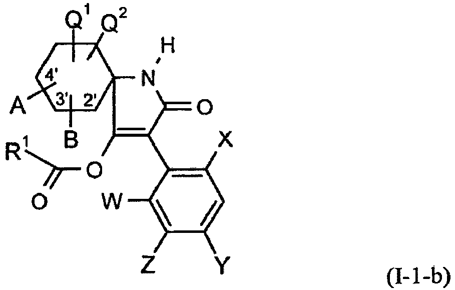 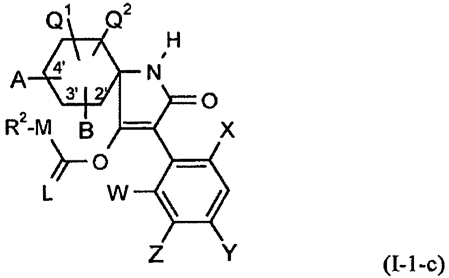 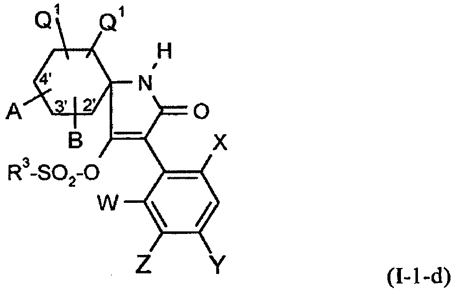 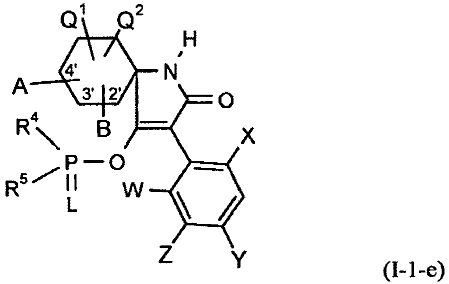 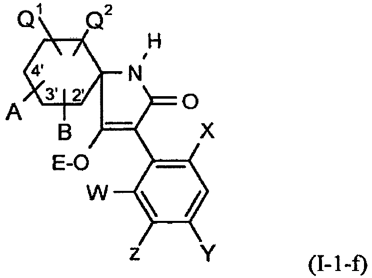 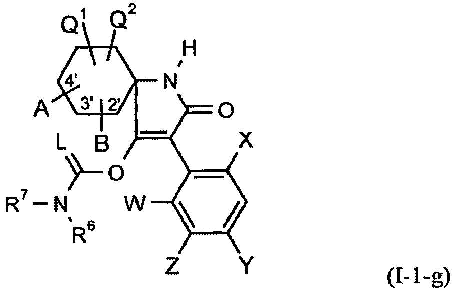 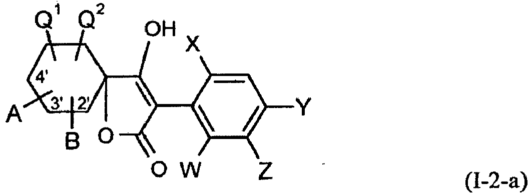 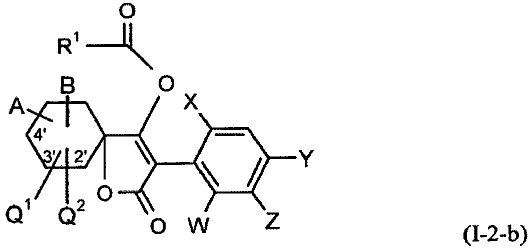 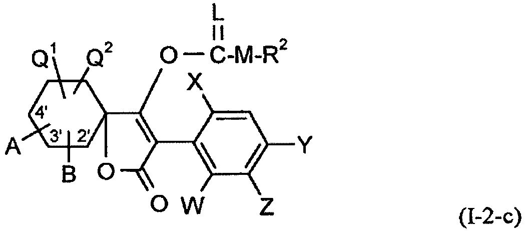 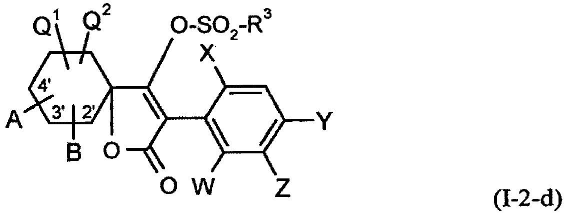 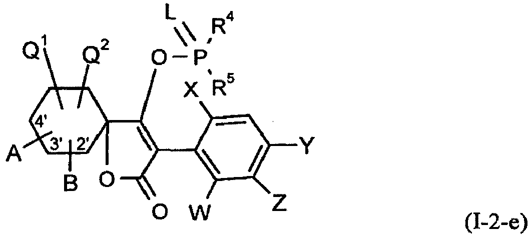 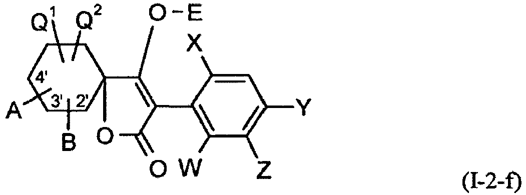 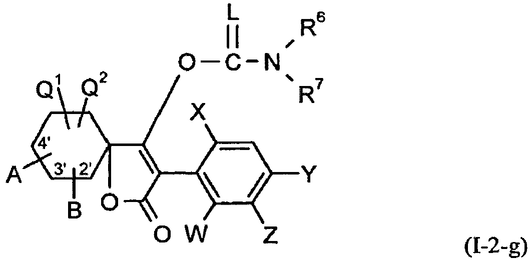 dans lesquelles A, B, D, G, Q¹, Q², W, X, Y et Z ont les significations indiquées précédemment, des composés de formule (XIV) 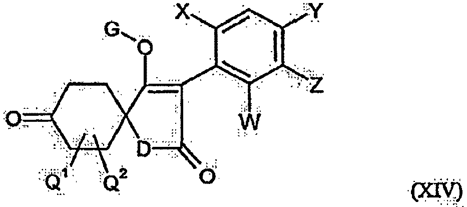 dans laquelle
D, G, Q¹, Q², W, X, Y et Z ont les significations indiquées précédemment,
sont mis en réaction avec des diols de formule (XV)
HO-A-B-OH (XV)
dans laquelle
A et B ont la signification indiquée précédemment, éventuellement en présence d'un diluant, en présence d'un catalyseur acide, en conditions d'exclusion de l'eau.

7. Agent de lutte contre les animaux nuisibles, la végétation indésirable et/ou les microorganismes indésirables, **caractérisé par** une teneur en au moins un composé de formule (I) selon la revendication 1.

8. Procédé de lutte contre les animaux nuisibles, la végétation indésirable et/ou les microorganismes indésirables, **caractérisé en ce que** des composés de formule (I) selon la revendication 1 sont laissés agir sur les animaux nuisibles, la végétation indésirable, les microorganismes indésirables et/ou leur habitat, à l'exception des procédés de traitement chirurgical ou thérapeutique du corps humain ou animal.

9. Utilisation de composés de formule (I) selon la revendication 1 pour lutter contre les animaux nuisibles, la végétation indésirable et/ou les microorganismes indésirables, à l'exception de l'utilisation dans des procédés de traitement chirurgical ou thérapeutique du corps humain ou animal.

10. Procédé de fabrication d'agents de lutte contre les animaux nuisibles, la végétation indésirable et/ou les microorganismes indésirables, **caractérisé en ce que** des composés de formule (I) selon la revendication 1 sont mélangés avec des diluants et/ou des substances tensioactives.

11. Utilisation de composés de formule (I) selon la revendication 1 pour la fabrication d'agents pour lutter contre les animaux nuisibles, la végétation indésirable et/ou les microorganismes indésirables.

12. Agent contenant une teneur active en une combinaison d'agents actifs comprenant en tant que composants
(a') au moins un composé de formule (I) selon la revendication 1, dans laquelle A, B, D, G, Q¹, Q², W, X, Y et Z ont la signification indiquée précédemment
et
(b') au moins un composé améliorant la compatibilité avec les plantes cultivées, du groupe suivant de composés :
4-dichloroacétyl-1-oxa-4-aza-spiro[4.5]-décane (AD-67, MON-4660), 1-dichloroacétyl-hexahydro-3,3,8a-triméthylpyrrolo[1,2-a]-pyrimidin-6(2H)-one (Dicyclonon, BAS-145138), 4-dichloroacétyl-3,4-dihydro-3-méthyl-2H-1,4-benzoxazine (Benoxacor), ester (1-méthyl-hexylique) de l'acide 5-chloro-quinolin-8-oxy-acétique (Cloquintocet-mexyl), 3-(2-chloro-benzyl)-1-(1-méthyl-1-phényl-éthyl)-urée (Cumyluron), α-(cyanométhoximino)-phénylacéto-nitrile (Cyométrinil), acide 2,4-dichloro-phénoxyacétique (2,4-D), acide 4-(2,4-dichloro-phénoxy)-butyrique (2,4-DB), 1(1-méthyl-1-phényl-éthyl)-3-(4-méthyl-phényl)-urée (Daimuron, Dymron), acide 3,6-dichloro-2-méthoxy-benzoïque (Dicamba), ester S-1-méthyl-1-phényl-éthylique de l'acide pipéridine-1-thio-carboxylique (Dimépipérate), 2,2-dichloro-N-(2-oxo-2-(2-propénylamino)-éthyl)-N-(2-propényl)-acétamide (DKA-24), 2,2-dichloro-N,N-di-2-propényl-acétamide (Dichlormid), 4,6-dichloro-2-phényl-pyrimidine (Fenclorim), ester éthylique de l'acide 1-(2,4-dichloro-phényl)-5-trichlorométhyl-1H-1,2,4-triazole-3-carboxylique (Fenchlorazole-éthyle), ester phénylméthylique de l'acide 2-chloro-4-trifluorométhyl-thiazole-5-carboxylique (Flurazole),4-chloro-N-(1,3-dioxolan-2-yl-méthoxy)-α-trifluoro-acétophénonoxime (Fluxofénim), 3-dichloroacétyl-5-(2-furanyl)-2,2-diméthyl-oxazolidine (Furilazole, MON-13900), carboxylate d'éthyl-4,5-dihydro-5,5-diphényl-3-isoxazole (Isoxadifène-éthyle), 3,6-dichloro-2-méthoxybenzoate de 1-(éthoxycarbonyl)-éthyle (Lactidichlor), acide (4-chloro-o-tolyloxy)-acétique (MCPA), acide 2-(4-chloro-o-tolyloxy)-propionique (Mecoprop), 3,5-dicarboxylate de diéthyl-1-(2,4-dichloro-phényl)-4,5-dihydro-5-méthyl-1H-pyrazole (Mefenpyr-diéthyle), 2-dichloro-méthyl-2-méthyl-1,3-dioxolane (MG-191), 4-carbodithioate de 2-propényl-1-oxa-4-azaspiro[4.5]décane (MG-838), anhydride de l'acide 1,8-naphtalique, α-(1,3-dioxolan-2-yl-méthoximino)-phénylacétonitrile (Oxabétrinil), 2,2-dichloro-N-(1,3-dioxolan-2-yl-méthyl)-N-(2-propényl)-acétamide (PPG-1292), 3-dichloroacétyl-2,2-diméthyl-oxazolidine (R-28725), 3-dichloroacétyl-2,2,5-triméthyl-oxazolidine (R-29148), acide 4-(4-chloro-o-tolyl)-butyrique, acide 4-(4-chloro-phénoxy)-butyrique, acide diphénylméthoxyacétique, ester méthylique de l'acide diphénylméthoxyacétique, ester éthylique de l'acide diphénylméthoxyacétique, ester méthylique de l'acide 1-(2-chloro-phényl)-5-phényl-1H-pyrazole-3-carboxylique, ester éthylique de l'acide 1-(2,4-dichloro-phényl)-5-méthyl-1H-pyrazole-3-carboxylique, ester éthylique de l'acide 1-(2,4-dichloro-phényl)-5-isopropyl-1H-pyrazol-3-carboxylique, ester éthylique de l'acide 1-(2,4-dichloro-phényl)-5-(1,1-diméthyl-éthyl)-1H-pyrazole-3-carboxylique, ester éthylique de l'acide 1-(2,4-dichloro-phényl)-5-phényl-1H-pyrazole-3-carboxylique, ester éthylique de l'acide 5-(2,4-dichloro-benzyl)-2-isoxazoline-3-carboxylique, ester éthylique de l'acide 5-phényl-2-isoxazoline-3-carboxylique, ester éthylique de l'acide 5-(4-fluorophényl)-5-phényl-2-isoxazoline-3-carboxylique, ester (1,3-diméthyl-but-1-ylique) de l'acide 5-chloroquinolin-8-oxy-acétique, ester 4-allyloxy-butylique de l'acide 5-chloro-quinolin-8-oxy-acétique, ester 1-allyloxy-prop-2-ylique de l'acide 5-chloro-quinolin-8-oxy-acétique, ester méthylique de l'acide 5-chloroquinoxalin-8-oxy-acétique, ester éthylique de l'acide 5-chloro-quinolin-8-oxy-acétique, ester allylique de l'acide 5-chloro-quinoxalin-8-oxy-acétique, ester 2-oxo-prop-1-ylique de l'acide 5-chloro-quinolin-8-oxy-acétique, ester diéthylique de l'acide 5-chloroquinolin-8-oxy-malonique, ester diallylique de 1"acide 5-chloro-quinoxalin-8-oxy-malonique, ester diéthylique de l'acide 5-chloro-quinolin-8-oxy-malonique, acide 4-carboxy-chroman-4-yl-acétique (AC-304415), acide 4-chloro-phénoxy-acétique, 3,3'-diméthyl-4-méthoxy-benzophénone, 1-bromo-4-chlorométhyl-sulfonyl-benzène, 1-[4-(N-2-méthoxybenzoylsulfamoyl)-phényl]-3-méthylurée (alias N-(2-méthoxy-benzoyl)-4-[(méthylamino-carbonyl)-amino]-benzènesulfonamide), 1-[4-(N-2-méthoxybenzoylsufamoyl)-phényl]-3,3-diméthyl-urée, 1-[4-(N-4,5-diméthylbenzoyl-sulfamoyl)-phényl]-3-méthyl-urée, 1-[4-(N-naphtylsulfamoyl)-phényl]-3,3-diméthyl-urée, N-(2-méthoxy-5-méthyl-benzoyl)-4-(cyclopropylaminocarbonyl)-benzènesulfonamide,
et/ou un des composés suivants définis par les formules générales
de formule générale (IIa) 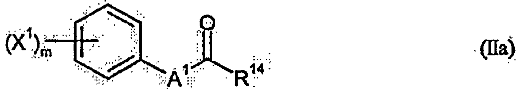 ou de formule générale (IIb) 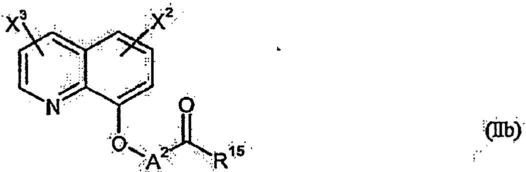 ou de formule (IIc) 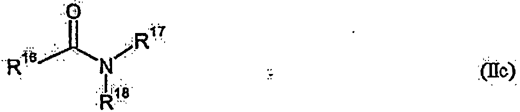 dans lesquelles
m représente un nombre 0, 1, 2, 3, 4 ou 5,
A¹ représente un des groupes hétérocycliques bivalents cités ci-dessous 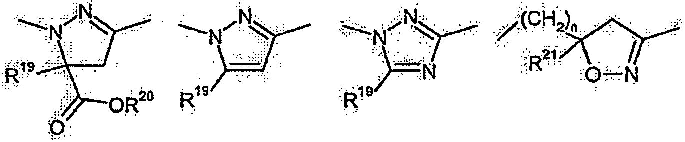
n représente un nombre 0, 1, 2, 3, 4 ou 5,
A² représente alcanediyle éventuellement substitué par alkyle en C₁-C₄ et/ou alcoxy-carbonyle en C₁-C₄ et/ou alcényloxy-carbonyle en C₁-C₄, contenant 1 ou 2 atomes de carbone,
R¹⁴ représente hydroxy, mercapto, amino, alcoxy en C₁-C₆, alkylthio en C₁-C₆. alkylamino en C₁-C₆ ou di (alkyle en C₁-C₄) -amino,
R¹⁵ représente hydroxy, mercapto, amino, alcoxy en C₁-C₇, alkylthio en C₁-C₆, alcényloxy en C₁-C₆, alcényloxy en C₁-C₆-alcoxy en C₁-C₆, alkylamino en C₁-C₆ ou di (alkyle en C₁-C₄)-amino,
R¹⁶ représente alkyle en C₁-C₄ éventuellement substitué par fluor, chlore et/ou brome,
R¹⁷ représente hydrogène ; alkyle en C₁-C₆, alcényle en C₂-C₆ ou alcynyle en C₂-C₆, alcoxy en C₁-C₄-alkyle en C₁-C₄, dioxolanyl-alkyle en C₁-C₄, furyle, furyl-alkyle en C₁-C₄, thiényle, thiazolyle, pipéridinyle, chacun éventuellement substitué par fluor, chlore et/ou brome ; ou phényle éventuellement substitué par fluor, chlore et/ou brome ou alkyle en C₁-C₄,
R¹⁸ représente hydrogène ; alkyle en C₁-C₆, alcényle en C₂-C₆ ou alcynyle en C₂-C₆, alcoxy en C₁-C₄-alkyle en C₁-C₄, dioxolanyl-alkyle en C₁-C₄, furyle, furyl-alkyle en C₁-C₄, thiényle, thiazolyle, pipéridinyle, chacun éventuellement substitué par fluor, chlore et/ou brome ; ou phényle éventuellement substitué par fluor, chlore et/ou brome ou alkyle en C₁-C₄; R¹⁷ et R¹⁸ représentant également ensemble alcanediyle en C₃-C₆ ou oxaalcanediyle en C₂-C₅, chacun éventuellement substitué par alkyle en C₁-C₄, phényle, furyle, un cycle benzène annelé ou par deux substituants, qui forment ensemble avec l'atome C auquel ils sont reliés un carbocycle de 5 ou 6 éléments,
R¹⁹ représente hydrogène, cyano, halogène ; ou alkyle en C₁-C₄, cycloalkyle en C₃-C₆ ou phényle, chacun éventuellement substitué par fluor, chlore et/ou brome,
R²⁰ représente hydrogène ; alkyle en C₁-C₆, cycloalkyle en C₃-C₆ ou tri-(alkyle en C₁-C₄) -silyle, chacun éventuellement substitué par hydroxy, cyano, halogène ou alcoxy en C₁-C₄,
R²¹ représente hydrogène, cyano, halogène ; ou alkyle en C₁-C₄, cycloalkyle en C₃-C₆ ou phényle, chacun éventuellement substitué par fluor, chlore et/ou brome,
X¹ représente nitro, cyano, halogène, alkyle en C₁-C₄, halogénoalkyle en C₁-C₄, alcoxy en C₁-C₄ ou halogénoalcoxy en C₁-C₄,
X² représente hydrogène, cyano, nitro, halogène, alkyle en C₁-C₄, halogénoalkyle en C₁-C₄, alcoxy en C₁-C₄ ou halogénoalcoxy en C₁-C₄,
X³ représente hydrogène, cyano, nitro, halogène, alkyle en C₁-C₄, halogénoalkyle en C₁-C₄, alcoxy en C₁-C₄ ou halogénoalcoxy en C₁-C₄,
et/ou les composés suivants définis par les formules générales
de formule générale (IId) 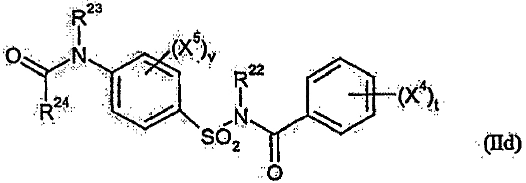 ou de formule générale (IIe) 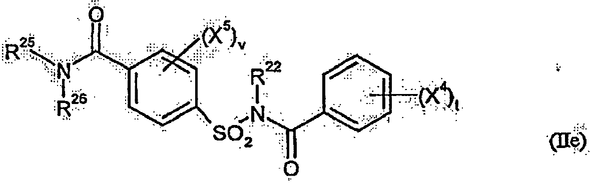 dans lesquelles
t représente un nombre 0, 1, 2, 3, 4 ou 5,
v représente un nombre 0, 1, 2, 3, 4 ou 5,
R²² représente hydrogène ou alkyle en C₁-C₄,
R²³ représente hydrogène ou alkyle en C₁-C₄,
R²⁴ représente hydrogène ; alkyle en C₁-C₆, alcoxy en C₁-C₆, alkylthio en C₁-C₆, alkylamino en C₁-C₆ ou di-(alkyle en C₁-C₄)-amino, chacun éventuellement substitué par cyano, halogène ou alcoxy en C₁-C₄ ; ou cycloalkyle en C₃-C₆, cycloalkyloxy en C₃-C₆, cycloalkylthio en C₃-C₆ ou cycloalkylamino en C₃-C₆, chacun éventuellement substitué par cyano, halogène ou alkyle en C₁-C₄,
R²⁵ représente hydrogène ; alkyle en C₁-C₆ éventuellement substitué par cyano, hydroxy, halogène ou alcoxy en C₁-C₄ ; alcényle en C₃-C₆ ou alcynyle en C₃-C₆, chacun éventuellement substitué par cyano ou halogène ; ou cycloalkyle en C₃-C₆ éventuellement substitué par cyano, halogène ou alkyle en C₁-C₄,
R²⁶ représente hydrogène ; alkyle en C₁-C₆ éventuellement substitué par cyano, hydroxy, halogène ou alcoxy en C₁-C₄ ; alcényle en C₃-C₆ ou alcynyle en C₃-C₆, chacun éventuellement substitué par cyano ou halogène ; cycloalkyle en C₃-C₆ éventuellement substitué par cyano, halogène ou alkyle en C₁-C₄ ; ou phényle éventuellement substitué par nitro, cyano, halogène, alkyle en C₁-C₄, halogénoalkyle en C₁-C₄, alcoxy en C₁-C₄ ou halogénoalcoxy en C₁-C₄ ; ou représente, conjointement avec R²⁵, alcanediyle en C₂-C₆ ou oxaalcanediyle en C₂-C₅, chacun éventuellement substitué par alkyle en C₁-C₄,
X⁴ représente nitro, cyano, carboxy, carbamoyle, formyle, sulfamoyle, hydroxy, amino, halogène, alkyle en C₁-C₄, halogénoalkyle en C₁-C₄, alcoxy en C₁-C₄ ou halogénoalcoxy en C₁-C₄, et
X⁵ représente nitro, cyano, carboxy, carbamoyle, formyle, sulfamoyle, hydroxy, amino, halogène, alkyle en C₁-C₄, halogénoalkyle en C₁-C₄, alcoxy en C₁-C₄ ou halogénoalcoxy en C₁-C₄.

13. Agent selon la revendication 12, dans lequel le composé améliorant la compatibilité avec les plantes cultivées est choisi dans le groupe suivant de composés :
Cloquintocet-mexyle, Fenchlorazole-éthyle, Isoxadifène-éthyle, Méfenpyr-diéthyle, Furilazole, Fenclorim, Cumyluron, Dymron ou les composés 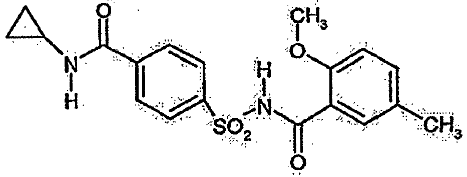 et

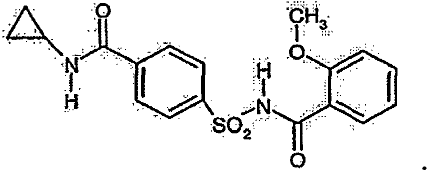

14. Agent selon l'une quelconque des revendications 12 ou 13, dans lequel le composé améliorant la compatibilité avec les plantes cultivées est le Cloquintocet-mexyl.

15. Agent selon l'une quelconque des revendications 12 ou 13, dans lequel le composé améliorant la compatibilité avec les plantes cultivées est le Mefenpyr-diéthyle.

16. Procédé de lutte contre la végétation indésirable, **caractérisé en ce qu'**un agent selon la revendication 12 est laissé agir sur les plantes ou leur environnement.

17. Utilisation d'un agent selon la revendication 12 pour lutter contre la végétation indésirable.

18. Procédé de lutte contre la végétation indésirable, **caractérisé en ce qu'**un composé de formule (I) selon la revendication 1 et le composé améliorant la compatibilité avec les plantes cultivées selon la revendication 12 sont laissés agir séparément, en succession temporelle rapprochée, sur les plantes ou leur environnement.

19. Composés de formule (II) 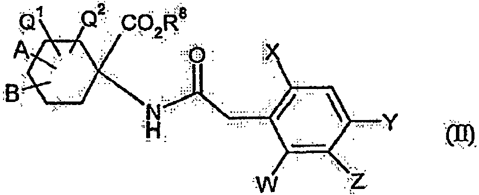 dans laquelle
A, B, Q¹, Q², R⁸, W, X, Y et Z ont les significations indiquées dans les revendications 1 et 6.

20. Composés de formule (III) 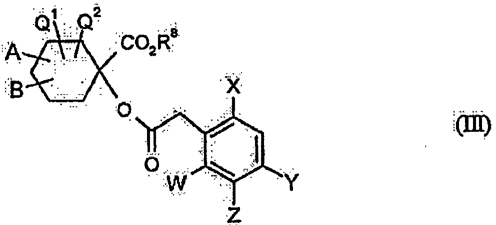 dans laquelle A, B, Q¹, Q², W, X, Y, Z et R⁸ ont les significations indiquées dans les revendications 1 et 6.

21. Composés de formule (XVIII) 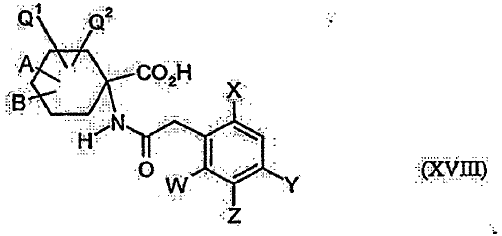 dans laquelle
A, B, Q¹, Q², W, X, Y et Z ont les significations indiquées dans la revendication 1.

22. Composés de formule (XXI) 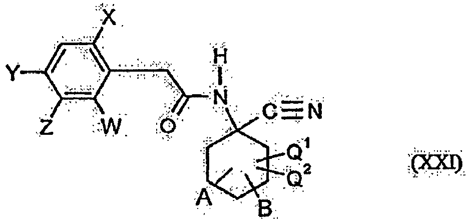 dans laquelle
A, B, Q¹, Q², W, X, Y et Z ont les significations indiquées dans la revendication 1.

23. Composés de formule (XXII) 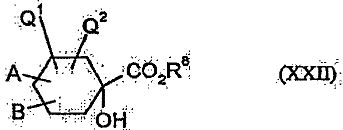 dans laquelle A, B, Q¹, Q² et R⁸ ont les significations indiquées dans les revendications 1 et 6, à l'exception de

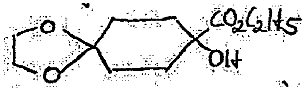

24. Composés de formule (XVI') et leurs sels 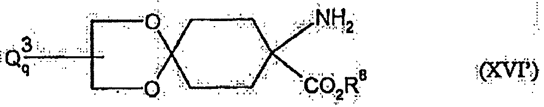 dans laquelle
Q³ représente alkyle en C₁-C₄, halogénoalkyle en C₁-C₃, alcoxy en C₁-C₄ ou alcoxy en C₁-C₄-alkyle en C₁-C₂,
q représente 1, 2 ou 3,
R⁸ a la signification indiquée dans la revendication 6.

25. Composés de formule (XVI'') et leurs sels 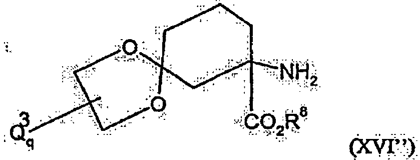 dans laquelle
Q³ et R⁸ ont les significations indiquées dans les revendications 6 et 24, et
q représente 0, 1, 2 ou 3.

26. Composés de formule (XVI''') et leurs sels 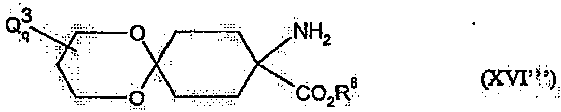 dans laquelle
Q³ et R⁸ ont les significations indiquées dans les revendications 6 et 24, et
q représente 0, 1, 2 ou 3.

27. Composés de formule (XIX') 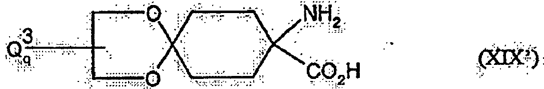 dans laquelle
Q³ a les significations indiquées dans les revendications 6 et 24, et
q représente 1, 2 ou 3.

28. Composés de formule (XIX'') 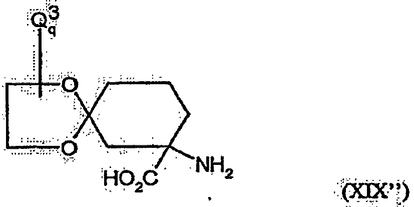 dans laquelle
Q³ a les significations indiquées dans la revendication 24, et
q représente 0, 1, 2 ou 3.

29. Composés de formule (XIX''') 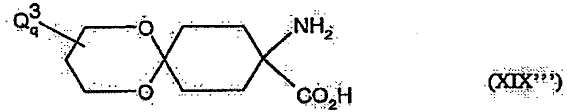 dans laquelle
Q³ a les significations indiquées dans la revendication 24, et
q représente 0, 1, 2 ou 3.

30. Composés de formule (XXIII') 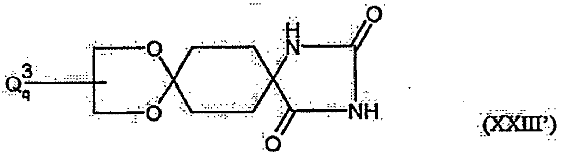 dans laquelle
Q³ a les significations indiquées dans la revendication 24, et
q représente 1, 2 ou 3.

31. Composés de formule (XXIII'') 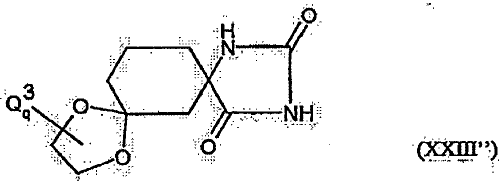 dans laquelle
Q³ a les significations indiquées dans la revendication 24, et
q représente 0, 1, 2 ou 3.

32. Composés de formule (XXIII''') 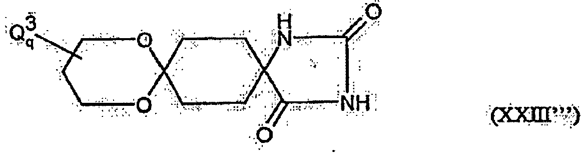 dans laquelle
Q³ a les significations indiquées dans la revendication 24, et
q représente 0, 1, 2 ou 3.

33. Composé de formule (I) selon la revendication 1, dans lequel les substituants ont les significations indiquées dans le tableau :
| W | X | Y | Z | D | Q¹ | Q² | G | A | B |
|---|---|---|---|---|---|---|---|---|---|
| CH₃ | CH₃ | CH₃ | H | NH | H | H | H | 4' -O- (CH₂)₂-O- | |
| CH₃ | CH₃ | Cl | H | NH | H | H | H | 4' -O- (CH₂)₂-O- | |
| CH₃ | CH₃ | Br | H | NH | H | H | H | 4'-O-(CH₂)₂-O- | |
| CH₃ | CH₃ | CH₃ | H | NH | H | H | H | 4'-O-(CH₂)₃-O- | |
| CH₃ | CH₃ | Cl | H | NH | H | H | H | 4'-O-(CH₂)₃-O- | |
| CH₃ | CH₃ | Br | H | NH | H | H | H | 4'-O-(CH₂)₃-O- | |
